(19) Europäisches Patentamt / European Patent Office / Office européen des brevets

(11) **EP 3 935 388 B1**

(12) **EUROPEAN PATENT SPECIFICATION**

(45) Date of publication and mention of the grant of the patent:
**15.01.2025 Bulletin 2025/03**

(21) Application number: **20766865.8**

(22) Date of filing: **05.03.2020**

(51) International Patent Classification (IPC):
**G01N 33/53** *(2006.01)*    **G01N 33/15** *(2006.01)*
**A61K 31/37** *(2006.01)*    **A61P 25/00** *(2006.01)*
**G01N 33/68** *(2006.01)*

(52) Cooperative Patent Classification (CPC):
**G01N 33/6896; A61K 31/37; A61P 25/00;**
**G01N 2800/52**

(86) International application number:
**PCT/SE2020/050245**

(87) International publication number:
**WO 2020/180238 (10.09.2020 Gazette 2020/37)**

(54) **TREATMENT EFFICIENCY EVALUATION**

BEURTEILUNG DER THERAPIEEFFIZIENZ

ÉVALUATION DE L'EFFICACITÉ D'UN TRAITEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2019 SE 1950292**

(43) Date of publication of application:
**12.01.2022 Bulletin 2022/02**

(73) Proprietor: **TX Medic AB**
**263 03 Viken (SE)**

(72) Inventor: **BRUCE, Lars**
**263 61 Viken (SE)**

(74) Representative: **Barker Brettell Sweden AB**
**Östermalmsgatan 87B**
**114 59 Stockholm (SE)**

(56) References cited:
EP-A1- 3 217 988       WO-A1-2008/134430
WO-A1-2017/018922      WO-A1-2018/054959

WO-A1-2018/212708      WO-A1-2019/050460
JP-A- 2016 132 619     US-A1- 2017 151 275

• DI BATTISTA ALEX P. ET AL: "Altered Blood Biomarker Profiles in Athletes with a History of Repetitive Head Impacts", PLOS ONE, vol. 11, no. 7, 1 January 2016 (2016-01-01), US, pages e0159929 - e0159929, XP055975601, ISSN: 1932-6203, DOI: 10.1371/journal.pone.0159929
• ADRIAN HAREL ET AL: "Biomarkers of Traumatic Brain Injury: Temporal Changes in Body Fluids", ENEURO, vol. 3, no. 6, 1 November 2016 (2016-11-01), XP055946870, DOI: 10.1523/ENEURO.0294-16.2016
• CHICOINE, L. M. ET AL.: "Excitotoxic Protection by Polyanionic Polysaccharide: Evidence of a Cell Survival Pathway Involving AMPA Receptor-MAPK Interactions", J NEUROSCI RES, vol. 85, 2007, pages 294 - 302, XP055581942, DOI: 10.1002/jnr.21117

**Description**

TECHNICAL FIELD

The present invention generally relates to treatment efficiency evaluation, and in particular to a method of determining an efficiency of dextran sulfate treatment of a patient.

BACKGROUND

In neurological diseases, such as Alzheimer's disease (AD), Parkinson's disease (PD), Huntington's disease (HD), amyotrophic lateral sclerosis (ALS) and multiple sclerosis (MS), and damages to the central nervous system (CNS) or peripheral nervous system (PNS), such as traumatic brain injury (TBI), stroke and sub-arachnoid hemorrhage (SAH), loss of differentiation of neurons and glial cells, such as oligodendrocytes and Schwann cells, is one of the first disease stages, followed by cell death. The function of the cells is also compromised as seen in impaired metabolic function and mitochondrial energy metabolism and elevated oxygen stress. Damaged neurons furthermore release glutamate having an excitotoxicity effect on nearby neurons, in turn causing further cell damage and cell death.

Accordingly, there are a multitude of deleterious mechanisms taking place in neurological diseases, disorders and conditions. There is therefore a general need for a treatment that is effective in combating such deleterious mechanisms and therefore could be of benefit for patients suffering from such neurological diseases, disorders and conditions. It is furthermore a particular need for treatment efficiency evaluation to determine whether the treatment has the desired effect in patients.

SUMMARY

It is a general objective to provide a treatment efficiency evaluation.

It is a particular objective to provide a method of determining an efficiency of dextran sulfate treatment of a patient.

These and other objectives are met by embodiments as disclosed herein.

The invention is defined in the independent claim. Further embodiments of the invention are defined in dependent claims.

According to the invention, an efficiency of a dextran sulfate treatment of a patient suffering from a neurological disease, disorder or condition is determined by determining an amount of at least one biomarker selected from each group of group nos. 1 to 6 in a first biological sample taken from the patient prior to administration of dextran sulfate, or a pharmaceutically acceptable salt thereof, to the patient. An amount of the at least one biomarker selected from each group of the group nos. 1 to 6 is also determined in a second biological sample taken from the patient following administration of the dextran sulfate, or the pharmaceutically acceptable salt thereof, to the patient. A difference is then determined for each biomarker between the amount of the biomarker in the second biological sample and the amount of the biomarker in the first biological sample. The efficiency of the dextran sulfate treatment is determined based on the differences.

In an embodiment, group no. 1 consists of platelet factor 4 (PFA4) and vav guanine nucleotide exchange factor 3 (VAV3); group no. 2 consists of tumor necrosis factor (TNF) superfamily member 15 (TNFSF15), interleukin 17B (IL-17B), thymic stromal lymphopoietin (TSLP) and corticotropin releasing hormone (CRH); group no. 3 consists of fibroblast growth factor 1 (FGF1) and KIT-ligand (KITLG); group no. 4 consists of brain derived neutrophic factor (BDNF), noggin (NOG) and heparin binding epidermal growth factor (EGF) like growth factor (HBEGF); group no. 5 consists of alpha fetoprotein (AFP), sarcoplasmic/endoplasmic reticulum calcium ATPase 3 (ATP2A3), solute carrier family 29 member 1 (SLC29A1), solute carrier family 40 member 1 (SLC40A1) and transthyretin (TTR); and group no. 6 consists of solute carrier family 1 member 4 (SLC1A4), solute carrier family 7 member 11 (SLC7A11), solute carrier family 16 member 7 (SLC16A7), low density lipoprotein receptor (LDLR) and ATPase phospholipid transporting 8A1 (ATP8A1).

The biomarkers of the present invention are useful in assessing the efficiency of the dextran sulfate treatment. Hence, the biomarkers can be used to verify whether an initial treatment regimen achieves the desired effect in the patient or whether the treatment regimen should be adjusted in order to obtain the desired effect.

BRIEF DESCRIPTION OF THE DRAWINGS

The embodiments, together with further objects and advantages thereof, may best be understood by making reference to the following description taken together with the accompanying drawings, in which:

Fig. 1 is a diagram illustrating changes in brain glutamate levels.

Figs. 2A-2D are diagrams illustrating changed levels of adenine nucleotides (ATP, ADP, AMP) and ATP/ADP ratio as a measurement of mitochondrial phosphorylating capacity.

Figs. 3A-3D are diagrams illustrating changed levels of oxidative and reduced nicotinic coenzymes.

Figs. 4A-4C are diagrams illustrating changed levels of biomarkers representative of oxidative stress.

Fig. 5 is a diagram illustrating changed levels of nitrate as a measurement of NO-mediated nitrosative stress.

Figs. 6A-6C are diagrams illustrating changed levels of N-acetylaspartate (NAA) and its substrates.

Fig. 7 illustrates concentrations of NAA measured in deproteinized brain homogenates of rats sacrificed at 2 days post-TBI without and with a single administration of increasing doses of LWM-DS (1, 5 and 15 mg/kg b.w.), performed 30 minutes after trauma induction. Controls are represented by sham-operated animals. Values are the mean of 12 animals. Standard deviations are represented by vertical bars. *significantly different from controls, p < 0.01. "significantly different from sTBI 2 days, p < 0.01.

Fig. 8 illustrates concentrations of ATP measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean of 12 animals. Standard deviations are represented by vertical bars. *significantly different from controls, p < 0.01. "significantly different from sTBI 2 days, p < 0.01.

Fig. 9 illustrates concentrations of ascorbic acid measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean of 12 animals. Standard deviations are represented by vertical bars. *significantly different from controls, p < 0.01. "significantly different from sTBI 2 days, p < 0.01.

Fig. 10 illustrates concentrations of glutathione (GSH) measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean of 12 animals. Standard deviations are represented by vertical bars. *significantly different from controls, p < 0.01. "significantly different from sTBI 2 days, p < 0.01.

Fig. 11 illustrates concentrations of NAA measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean of 12 animals. Standard deviations are represented by vertical bars. *significantly different from controls, p < 0.01. "significantly different from sTBI 2 days, p < 0.01.

Fig. 12 is a flow chart illustrating an embodiment of a method of determining an efficiency of dextran sulfate treatment of a patient.

DETAILED DESCRIPTION

The present invention generally relates to treatment efficiency evaluation, and in particular to a method of determining an efficiency of dextran sulfate treatment of a patient.
A neurological disorder is any disorder of the body nervous system, i.e., the brain, spine and the nerves that connect them. Structural, biochemical or electrical abnormalities in the brain, spinal cord or other nerves can result in a range of symptoms. Although the brain and spinal cord are surrounded by tough membranes, enclosed in the bones of the skull and spinal vertebrae, and chemically isolated by the blood-brain barrier, they are very susceptible if compromised. Nerves tend to lie deep under the skin but can still become exposed to damage. Individual neurons, and the neural networks and nerves into which they form, are susceptible to electrochemical and structural disruption. Neuroregeneration may occur in the peripheral nervous system and, thus, overcome or work around injuries to some extent, but it is thought to be rare in the brain and spinal cord.
The specific causes of neurological problems vary, but can include genetic disorders, congenital abnormalities or disorders, infections, lifestyle or environmental health problems including malnutrition, and brain injury, spinal cord injury or nerve injury. The problem may start in another body system that interacts with the nervous system. For example, cerebrovascular disorders involve brain injury due to problems with the blood vessels, i.e., the cardiovascular system, supplying the brain; autoimmune disorders involve damage caused by the body's own immune system; lysosomal storage diseases, such as Niemann-Pick disease, can lead to neurological deterioration.

A neurodegenerative disease, disorder or condition is a disease, disorder or condition causing progressive loss of structure and/or function of neurons, including death of neurons. Non-limiting examples of such neurodegenerative diseases, disorders or conditions include Alzheimer's disease (AD), Parkinson's disease (PD), Huntington's disease (HD) and amyotrophic lateral sclerosis (ALS).

A neurological disease, disorder or condition may be a demyelinating disease, disorder or condition. A demyelinating disease, disorder or condition is a disease of the nervous system in which the myelin sheath of neurons is damaged. Such damage impairs the conduction of signals in the affected nerves and thereby causing deficiency in sensation, movement, cognition and other functions depending on the nerves involved in the damage. Non-limiting examples of such demyelinating diseases, disorders or conditions include multiple sclerosis (MS), acute disseminated encephalomyelitis (ADEM), central nervous system (CNS) neuropathies, central pontine myelinolysis (CPM), myelopathies, leukoencephalopathies and leukodystrophies (all affecting the CNS), and Guillain-Barre syndrome (GBS), peripheral neuropathies and Charcot-Marie-Tooth (CMT) disease (all affecting the peripheral nervous system (PNS)).

Dextran sulfate, or a pharmaceutically acceptable salt thereof, affects a large number of molecules with downstream effects that lead to complex biological changes useful in treating, for instance, neurological diseases, disorders or conditions in patients.

In neurological disorders loss of differentiation of neurons and glial cells, such as oligodendrocytes and Schwann cells, is one of the first stages in the disease progress. Generally, the disorders subsequently progress with cell death of such neurons and glial cells. Dextran sulfate, or a pharmaceutically acceptable salt thereof, is capable of promoting differentiation of neuronal and glial cells. This effect of dextran sulfate is seen both for cortical neurons and motor neurons and for neurons from both mouse and human origin. In more detail, dextran sulfate is capable of inducing an increase in beta-tubulin, in particular βIII-tubulin, expression in the neurons. During differentiation, tubulin is increased in the cell and builds up microtubule, which allows the differentiating neurons to extend or retract growing axons in response to guidance cues in order to maintain directional growth towards post-synaptic targets.

Dextran sulfate does not only induce differentiation of cells of the CNS and PNS, which is beneficial in neurological diseases, disorders and conditions, dextran sulfate also has positive effect in combating metabolic modifications that are seen in neurological diseases, disorders and conditions, such as traumatic brain injury (TBI). Thus, many neurological diseases, disorders and conditions are characterized by modifications of various metabolites connected to the cell energy state and mitochondrial functions. Furthermore, modifications in amino acid metabolisms are seen in many neurological diseases, disorders and conditions. These metabolic modifications are early cellular signals that influence changes in enzymatic activities and gene and protein expressions indicative of a pathological tissue response. Dextran sulfate acts to positively regulate cellular metabolism in the compromised tissues, thereby inhibiting or at least suppressing any subsequent modifications in enzyme activity and gene and protein expression that contribute to adverse outcomes.

In more detail, dextran sulfate is capable of reducing levels of glutamate excitotoxicity and ameliorated adverse changes in metabolic hemostastis, thereby efficiently protecting mitochondrial function and providing a neuroprotective effect. Dextran sulfate positively affects various compounds related to energy metabolism and mitochondrial functions. Particularly interesting are the concentrations of adenine nucleotides and ATP/ADP ratio as measurement of mitochondrial phosphorylating capacity.

Dextran sulfate also leads to a significant reduction in oxidative stress. In particular, the levels of ascorbic acid, as the main water-soluble brain antioxidant, and glutathione (GSH), as the major intracellular sulfhydryl group (SH) donor, are significantly improved. In addition, malondialdehyde (MDA) levels, as end product of polyunsaturated fatty acids of membrane phospholipids and therefore taken as a marker of reactive oxygen species (ROS) mediated lipid peroxidation, shows a significant reduction after dextran sulfate administration. The oxidative stress markers described above all indicate an improvement in the recovery of antioxidant status after dextran sulfate treatment.

Dextran sulfate administration also significantly decreases the nitrate concentrations in both acute and chronic phases of neurological diseases, disorders and conditions. Accordingly, dextran sulfate has a positive effect on NO-mediated nitrosative stress.

N-acetylaspartate (NAA) is a brain specific metabolite and a valuable biochemical marker for monitoring deterioration or recovery after neurological diseases, disorders and conditions, such as TBI. NAA is synthesized in neurons from aspartate and acetyl-CoA by aspartate N-acetyltransferase. Dextran sulfate shows significant improvements in NAA levels.

Dextran sulfate treatment can thereby protect against the cell loss that occurs due to oxidative stress and/or glutamate excitotoxicity in the diseased and damaged nervous system. By protecting cell metabolism, dextran sulfate may be a useful protective treatment in many degenerative conditions where cells are progressively lost due to ischemic, oxidative and/or traumatic damage, such as stroke, ALS, MND, MS, dementia, TBI, SCI, retinal damage, etc. These neurological diseases, disorders and conditions have a common link in terms of death and compromise of neuronal function of neurons that occurs in all conditions. There are commonalities in the causes of this of neuronal death. Of particular relevance is the toxicity caused by the high levels of the neurotransmitter glutamate that is released from dying neurons. Dextran sulfate induces scavenging of released glutamate in glial cells and thereby prevents accumulation of toxic amounts of glutamate in the neuronal clefts. This will be useful in all neurodegenerative diseases, disorders and conditions, both acute and chronic, where neurons are dying.

Excitotoxicity is the pathological process by which nerve cells are damaged or killed by excessive stimulation by neurotransmitters, in particular glutamate. This occurs when receptors for the excitatory neurotransmitter glutamate, such as the N-methyl-D-aspartate (NMDA) receptor and the $\alpha$-amino-3-hydroxy-5-methyl-4-isoxazolepropionic acid (AMPA) receptor, are overactivated by glutamatergic storm or when neurons are damaged or dies, releasing their content of glutamate.

Excitotoxicity may be involved in SCI, stroke, TBI, hearing loss (through noise overexposure or ototoxicity), and in neurodegenerative diseases of the CNS, such as MS, AD, ALS, PD, alcoholism or alcohol withdrawal and especially over-rapid benzodiazepine withdrawal, and also HS. Other common conditions that cause excessive glutamate concentrations around neurons are hypoglycemia.

During normal conditions, glutamate concentration can be increased up to 1 mM in the synaptic cleft, which is rapidly decreased in the lapse of milliseconds. When the glutamate concentration around the synaptic cleft cannot be decreased or reaches higher levels, the neuron kills itself by a process called apoptosis. This pathologic phenomenon can also occur after brain injury, such as in TBI, and SCI. Within minutes after the injury, damaged neural cells within the lesion site spill glutamate into the extracellular space where glutamate can stimulate presynaptic glutamate receptors to enhance the release of additional glutamate. Brain trauma or stroke can cause ischemia, in which blood flow is reduced to inadequate levels. Ischemia is followed by accumulation of glutamate in the extracellular fluid, causing cell death, which is aggravated by lack of oxygen and glucose. The biochemical cascade resulting from ischemia and involving excitotoxicity is called the ischemic cascade. Because of the events resulting from ischemia and glutamate receptor activation, a deep chemical coma may be induced in patients with brain injury to reduce the metabolic rate of the brain, its need for oxygen and glucose, and save energy to be used to remove glutamate actively.

Furthermore, increased extracellular glutamate levels leads to the activation of $Ca^{2+}$ permeable $N$-methyl-D-aspartate (NMDA) receptors on myelin sheaths and oligodendrocytes, leaving oligodendrocytes susceptible to $Ca^{2+}$ influxes and subsequent excitotoxicity. One of the damaging results of excess calcium in the cytosol is initiating apoptosis through cleaved caspase processing. Another damaging result of excess calcium in the cytosol is the opening of the mitochondrial permeability transition pore, a pore in the membranes of mitochondria that opens when the organelles absorb too much calcium. Opening of the pore may cause mitochondria to swell and release reactive oxygen species and various proteins that can lead to apoptosis. The pore can also cause mitochondria to release more calcium. In addition, production of adenosine triphosphate (ATP) may be stopped, and ATP synthase may in fact begin hydrolyzing ATP instead of producing it.

Inadequate ATP production resulting from brain trauma can eliminate electrochemical gradients of certain ions. Glutamate transporters require the maintenance of these ion gradients to remove glutamate from the extracellular space. The loss of ion gradients results in not only halting of glutamate uptake, but also the reversal of the transporters. The Na+-glutamate transporters on neurons and astrocytes can reverse their glutamate transport and start secreting glutamate at a concentration capable of inducing excitotoxicity. This results in a buildup of glutamate and further damaging activation of glutamate receptors.

On the molecular level, calcium influx is not the only factor responsible for apoptosis induced by excitotoxicity. Recently, it has been noted that extrasynaptic NMDA receptor activation, triggered by both glutamate exposure or hypoxic/ischemic conditions, activate a cAMP response element binding (CREB) protein shut-off, which in turn caused loss of mitochondrial membrane potential and apoptosis.

Thus, the activation of glutamate transporter in glial cells by dextran sulfate to prevent or at least inhibit accumulation of toxic levels of glutamate will effectively protect surrounding neurons from glutamate excitotoxicity. As a result, dextran sulfate protects neurons from damages and cell death that is otherwise the result of this glutamate excitotoxicity.

Also, when any tissue, including the CNS and PNS, and the brain, which is particularly sensitive to changes in oxygen/energy supply, is damaged or diseased, the energy supply to cells is compromised. As a result, the cells in the tissue, such as CNS, PNS or brain, cannot function efficiently. Accordingly, the reduction in oxidative stress by dextran sulfate, i.e., the protection of the mitochondrial energy supply, allows surviving cells to function more efficiently and will also protect compromised neurons from dying by apoptosis.

Thus, dextran sulfate is effective in restoring mitochondrial related energy metabolism, profoundly imbalanced in subject suffering from brain damages, such as severe TBI (sTBI), with positive effects on the concentration of triphosphates purine and pyrimidine nucleotides. Particularly, ATP levels were only 16% lower than the value of healthy control subjects, whilst in untreated sTBI subjects a 35% decrease was found. Remarkably, NAA concentration in sTBI subjects treated with dextran sulfate was only 16% lower than the value of healthy control subjects, whilst sTBI subjects showed 48% lower values of this compound. This finding once again strongly confirms the strict connection between the homeostasis of NAA and correct mitochondrial energy metabolism, and underlines the importance of pharmacological interventions capable to act positively on mitochondrial functioning.

The general amelioration of brain metabolism produced by dextran sulfate treatment also involves nicotinic coenzymes and metabolism of free CoA-SH and CoA-SH derivatives. This implies that dextran sulfate treated subjects, notwithstanding submitted to sTBI, have quasi-normal coenzymes to ensure correct oxido-reductive reactions and to allow a good functioning of the TCA cycle.

The aforementioned improvement of brain metabolism further contributes to the other remarkable dextran sulfate effects, i.e., the abolishment of glutamate excitotoxicity. Additionally, dextran sulfate affects sulfur-containing amino acids. Possibly, this effect might be related to the dextran sulfate molecule that contains S atoms. Increasing the bioavailability of this atom might produce a net increase in the biosynthesis of these amino acids, one of them (MET) is crucial in the methylation reaction and in the so called methyl cycle.

Further positive effects recorded are the increase in antioxidants and the decrease of biochemical signatures of oxidative/nitrosative stress in sTBI subjects receiving administration of dextran sulfate. Of relevance is that the effects of dextran sulfate are more evident at 7 days post sTBI than at 2 days post sTBI. This strongly suggests that the general amelioration of brain metabolism caused by the dextran sulfate administration is not a transitory phenomenon.

The large number of molecules affected by dextran sulfate treatment as described above and further disclosed herein may have genetic variations among the human population that will affect the activity of these molecules. For instance, due to the knock on effect of a loss of function mutation in one molecule, there might be quite a large patient-to-patient variation in the response to dextran sulfate treatment. The rule of thumb is that the more molecules are involved in achieving the therapeutic response the more likely to have variations in this therapeutic response. Additionally, the involvement of the large number of molecules in the therapeutic response will result in a shaded drug response (continuum) rather than a simple effect/no effect. This will also be complicated by the different severity (stages) of the disease, disorder or condition in patients. In the case of nervous system diseases, disorders or conditions, the expected response to dextran sulfate treatment may also be affected by the 'functional reserve' of the individual patient.

While the prediction of disease severity (as opposed to clinical manifestation) and functional reserve would require disease biomarkers, the variations in the response to dextran sulfate treatment due to genetic variations, absorption problems, etc. can be detected by biomarkers directly related to the effect of dextran sulfate treatment.

Experimental data as presented herein have indicated a large number of molecules that are upregulated or downregulated by dextran sulfate treatment and may therefore be useful as biomarkers for assessing treatment efficiency.

As a starting point, a list and expression levels of molecules differentially regulated by dextran sulfate relative to a TBI model for the three different doses (1 mg/kg, 5 mg/kg and 15 mg/kg) of dextran sulfate at day 7 following TBI were established. As the expression experiments were done in rats, not all proteins have a known or established human counterpart. As a consequence genes and proteins having no known human counterpart were removed from the list in a first filtering step. Most likely biomarker candidates are proteins that are secreted. In a second filtering step only growth factors, cytokines and transporters were therefore retained in the list. Of these growth factors, cytokines and transporters, the onse that are not known in the literature to be present either in whole blood or in the blood plasma or blood serum were filtered out. This second filtering step thereby retained genes that encode proteins that are highly likely to appear in blood, blood plasma and/or blood serum.

Dextran sulfates are available in a wide range of molecular weights from low molecular weight dextran sulfate (LMW-DS), generally having an average molecular weight of equal to or below 10 kDa, to high molecular weight dextran sulfates having several tens of kDa or several hundred of kDa as average molecular weight. The dextran sulfates having higher molecular weights are marred by severe side effects when administered to human patients. In a third filtering step, the genes regulated by high molecular weight dextran sulfates were removed from the list.

In a preferred embodiment, a biomarker is useful for clinical applications when the biomarker meets the following criteria:

1. the biomarker is upregulated or downregulated by dextran sulfate irrespective of the dose, i.e., has a consistent dose-independent effect;
2. the biomarker is upregulated or downregulated in a dose dependent manner, i.e., the effect appears with increasing doses of dextran sulfate;
3. the effect is easily measurable with existing technologies, such as enzyme-linked immunosorbent assay (ELISA), other protein assays or gene arrays.

In an embodiment, the effect induced by dextran sulfate treatment is a minimum of 20 %, i.e., a fold change (FC) of 1.2 or more if upregulated by dextran sulfate and a FC of -1.2 or below if downregulated by dextran sulfate.

In a fourth filtering step, the biomarkers that did not show the expression patterns (criteria 1-3 and a FC≥1.2 or a FC≤ -1.2) in response to dextran sulfate treatment were eliminated from the biomarker list.

The filtering strategies mentioned above left 24 molecules in the potential biomarker list, see Tables 1 and 2. Of these eight are known to be detectable from blood plasma or blood serum and not just whole blood, see Table 2. The biomarkers were grouped in 7 groups based on the type of molecule, upregulation or downregulation expected in response to dextran sulfate treatment and consistency of dextran sulfate effect.

Group no. 1 consists of platelet factor 4 (PFA4), also referred to as chemokine (C-X-C motif) ligand 4 (CXCL4); and vav guanine nucleotide exchange factor 3 (VAV3).

Group no. 2 consists of tumor necrosis factor (TNF) superfamily member 15 (TNFSF15), also referred to as vascular endothelial growth inhibitor (VEGI) or TNF-like ligand 1A (TL1A); interleukin 17B (IL-17B); thymic stromal lymphopoietin (TSLP); and corticotropin releasing hormone (CRH), also referred to as corticotropin-releasing factor (CRF) or corticoliberin.

Group no. 3 consists of fibroblast growth factor 1 (FGF1), also referred to as acidic fibroblast growth factor (aFGF); and KIT-ligand (KITLG), also referred to as stem cell factor (SCF) or steel factor.

Group no. 4 consists of brain derived neutrophic factor (BDNF); noggin (NOG); and heparin binding epidermal growth factor (EGF) like growth factor (HBEGF).

Group no. 5 consists of alpha fetoprotein (AFP), also referred to as alpha-1-fetoprotein, alpha-fetoglobulin, or alpha fetal protein; sarcoplasmic/endoplasmic reticulum calcium ATPase 3 (ATP2A3); solute carrier family 29 member 1 (SLC29A1), also referred to as equilibrative nucleoside transporter 1 (ENT1); solute carrier family 40 member 1 (SLC40A1), also referred to as ferroportin-1 or iron-regulated transporter 1 (IREG1); and transthyretin (TTR).

Group no. 6 consists of solute carrier family 1 member 4 (SLC1A4), also referred to as neutral amino acid transporter A; solute carrier family 7 member 11 (SLC7A11), also referred to as cystine/glutamate transporter; solute carrier family 16 member 7 (SLC16A7), also referred to as monocarboxylate transporter 2 (MCT2); low density lipoprotein receptor (LDLR); and ATPase phospholipid transporting 8A1 (ATP8A1).

Group no. 7 consists of interleukin 36 receptor antagonist (IL36RN); golgi soluble N-ethylmaleimide-sensitive factor (NSF) attachment protein (SNAP) receptor complex 1 (GOSR1); and solute carrier family 4 member 1 (SLC4A1), also referred to as band 3 anion transport protein, anion exchanger 1 (AE1) or band 3.

Table 1 and 2 below provide more information of the biomarkers in the seven groups.

Table 1 - biomarkers

| Group | Symbol | Entrez gene name | Family | Effect for efficient DS* treatment |
|---|---|---|---|---|
| 1 | PF4 | platelet factor 4 | cytokine | downregulated (FC≤-1.2) |
| 1 | VAV3 | vav guanine nucleotide exchange factor 3 | cytokine | downregulated (FC≤-1.2) |
| 2 | TNFSF15 | TNF superfamily member 15 | cytokine | upregulated (FC≥1.2) |
| 2 | IL-17B | interleukin 17B | cytokine | upregulated (FC≥1.2) |
| 2 | TSLP | thymic stromal lymphopoietin | cytokine | upregulated (FC≥1.2) |
| 2 | CRH | corticotropin releasing hormone | cytokine | upregulated (FC≥1.2) |
| 3 | FGF1 | fibroblast growth factor 1 | growth factor | downregulated (FC≤-1.2) |
| 3 | KITLG | KIT-ligand | growth factor | downregulated (FC≤-1.2) |
| 4 | BDNF | brain derived neutrophic factor | growth factor | upregulated (FC≥1.2) |
| 4 | NOG | noggin | growth factor | upregulated (FC≥1.2) |
| 4 | HBEGF | heparin binding EGF like growth factor | growth factor | upregulated (FC≥1.2) |
| 5 | AFP | alpha fetoprotein | transporter | downregulated (FC≤-1.2) |
| 5 | ATP2A3 | sarcoplasmic/endoplasmic reticulum calcium ATPase 3 | transporter | downregulated (FC≤-1.2) |
| 5 | SLC29A1 | solute carrier family 29 member 1 | transporter | downregulated (FC≤-1.2) |
| 5 | SLC40A1 | solute carrier family 40 member 1 | transporter | downregulated (FC≤-1.2) |
| 5 | TTR | transthyretin | transporter | downregulated (FC≤-1.2) |
| 6 | SLC1A4 | solute carrier family 1 member 4 | transporter | upregulated (FC≥1.2) |
| 6 | SLC7A11 | solute carrier family 7 member 11 | transporter | upregulated (FC≥1.2) |

(continued)

| Group | Symbol | Entrez gene name | Family | Effect for efficient DS* treatment |
|---|---|---|---|---|
| 6 | SLC16A7 | solute carrier family 16 member 7 | transporter | upregulated (FC≥1.2) |
| 6 | LDLR | low density lipoprotein receptor | transporter | upregulated (FC≥1.2) |
| 6 | ATP8A1 | ATPase phospholipid transporting 8A1 | transporter | upregulated (FC≥1.2) |
| 7 | IL36RN | interleukin 36 receptor antagonist | cytokine | upregulated (FC≥1.2) |
| 7 | GOSR1 | golgi SNAP receptor complex 1 | transporter | upregulated (FC≥1.2) |
| 7 | SLC4A1 | solute carrier family 4 member 1 | transporter | downregulated (FC≤-1.2) |
| * DS = dextran sulfate | | | | |

Table 2 - biomarkers

| Group | Symbol | FC(DS* 1 mg/kg) | FC(DS* 5 mg/kg) | FC(DS* 15 mg/kg) | Blood | Plasma Serum |
|---|---|---|---|---|---|---|
| 1 | PF4 | -1.453 | -1.331 | -1.244 | x | x |
| 1 | VAV3 | -1.505 | -1.418 | -1.318 | x | |
| 2 | TNFSF15 | 3.878 | 4.302 | 3.939 | x | |
| 2 | IL-17B | 1.863 | 1.765 | 1.729 | x | |
| 2 | TSLP | 1.389 | 1.446 | 1.322 | x | |
| 2 | CRH | 1.253 | 1.316 | 1.293 | x | x |
| 3 | FGF1 | -1.366 | -1.382 | -1.213 | x | |
| 3 | KITLG | -1.251 | -1.274 | -1.261 | x | x |
| 4 | BDNF | 1.635 | 1.708 | 1.557 | x | x |
| 4 | NOG | 1.304 | 1.305 | 1.281 | x | |
| 4 | HBEGF | 1.235 | 1.274 | 1.250 | x | |
| 5 | AFP | -1.439 | -1.280 | -1.208 | x | x |
| 5 | ATP2A3 | -1.284 | -1.205 | -1.216 | x | x |
| 5 | SLC29A1 | -1.370 | -1.377 | -1.316 | x | |
| 5 | SLC40A1 | -1.345 | -1.338 | -1.348 | x | |
| 5 | TTR | -2.531 | -1.915 | -2.179 | x | x |
| 6 | SLC1A4 | 1.498 | 1.531 | 1.518 | x | |
| 6 | SLC7A11 | 1.490 | 1.469 | 1.477 | x | x |
| 6 | SLC16A7 | 1.493 | 1.441 | 1.408 | x | |
| 6 | LDLR | 1.364 | 1.291 | 1.212 | x | |
| 6 | ATP8A1 | 1.280 | 1.223 | 1.212 | x | |
| 7 | IL36RN | | | 1.284 | x | |
| 7 | GOSR1 | | | 1.233 | x | |
| 7 | SLC4A1 | | -1.442 | -1.213 | x | |
| * DS = dextran sulfate | | | | | | |

In an embodiment, the effects of dextran sulfate treatment are as expected in the patient if, within one week of treatment, there is at least 20 % downregulation of at least one of the molecules in groups 1, 3 and 5 combined with at least 20 % upregulation of at least one of the molecules in groups 2, 4 and 6 relative to the baseline level of these molecules in the patient, i.e., before the treatment started. A lack of change or lesser change than 20 % indicates low efficacy of the dextran sulfate treatment.

In an embodiment, unexpected effects of dextran sulfate treatment can be expected in a patient where there is at least 20 % upregulation of at least one of the molecules in groups 1, 3 and 5 and/or at least 20 % downregulation of at least one of the molecules in groups 2, 4 and 6 relative to the baseline level of these molecules in the patient, i.e., before the treatment started. This would indicate an effect that is opposite to expectation and may lead to side effects in the patient due to the dextran sulfate treatment.

In an embodiment, the efficacy of the dextran sulfate treatment is indicated by the level of downregulation of the molecules in groups 1, 3 and 5 combined with the level of upregulation of the molecules in groups 2, 4 and 6 relative to the baseline level of these molecules in the patient, i.e., before the treatment started. A lack of change or lesser change than 20 % indicates low efficacy of the dextran sulfate treatment in the patient.

In cases of low or no efficacy of the current dextran sulfate treatment as indicated by the biomarkers, the dextran sulfate treatment may be changed, such as by increasing the dextran sulfate dose.

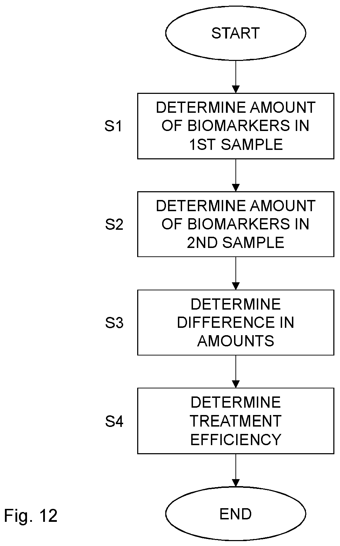

An aspect of the invention relates to a method of determining an efficiency of dextran sulfate treatment of a patient suffering from a neurological disease, disorder or condition, see Fig. 12. The method comprises determining, in step S1, an amount of at least one biomarker selected from each group of group nos. 1 to 6 in a first biological sample taken from the patient prior to administration of dextran sulfate, or a pharmaceutically acceptable salt thereof, to the patient. The method also comprises determining, in step S2, an amount of the at least one biomarker selected from each group of the group nos. 1 to 6 in a second biological sample taken from the patient following administration of the dextran sulfate, or the pharmaceutically acceptable salt thereof, to the patient. The method further comprises determining, in step S3 and for each biomarker, a difference between the amount of the biomarker in the second biological sample and the amount of the biomarker in the first biological sample. The method additionally comprises determining, in step S4, the efficiency of the dextran sulfate treatment based on the differences.

Hence, in an embodiment, steps S1 and S2 involve determining the amount of at least one biomarker from group 1, at least one biomarker from group 2, at least one biomarker from group 3, at least one biomarker from group 4, at least one biomarker from group 5 and at least one biomarker from group 6 in the first and second biological samples.

In an embodiment, the first biological sample and the second biological sample are a first body fluid sample and a second body fluid sample. In an embodiment, the body fluid is selected from the group consisting of blood, blood serum and blood plasma, preferably the body fluid is blood, such as whole blood.

In an embodiment, step S2 comprises determining the amount of the at least one biomarker selected from each group of the group nos. 1 to 6 in the second biological sample taken from the patient within a time period of from one day up to fourteen days following administration of the dextran sulfate, or the pharmaceutically acceptable salt thereof, to the patient. In a particular embodiment, step S2 comprises determining the amount of the at least one biomarker selected from each group of the group nos. 1 to 6 in the second biological sample taken from the patient within a time period of from four days up to ten days following administration of the dextran sulfate, or the pharmaceutically acceptable salt thereof, to the patient. More preferably, step S2 comprises determining the amount of the at least one biomarker selected from each group of the group nos. 1 to 6 in the second biological sample taken from the patient seven days following administration of the dextran sulfate, or the pharmaceutically acceptable salt thereof, to the patient.

In an embodiment, step S1 comprises determining the amount of multiple, i.e., at least two, biomarkers selected from each group of the group nos. 1 to 6 in the first biological sample taken from the patient prior to administration of dextran sulfate, or the pharmaceutically acceptable salt thereof, to the patient. In this embodiment, step S2 comprises determining the amount of the multiple biomarkers selected from each group of the group nos. 1 to 6 in the second biological sample taken from the patient following administration of the dextran sulfate, or the pharmaceutically acceptable salt thereof, to the patient.

In a particular embodiment, step S1 comprises determining to the patient, the amount of all biomarkers from each group of the group nos. 1 to 6 in the first biological sample taken from the patient prior to administration of dextran sulfate, or the pharmaceutically acceptable salt thereof. In this particular embodiment, step S2 comprises determining the amount of the all biomarkers from each group of the group nos. 1 to 6 in the second biological sample taken from the patient following administration of the dextran sulfate, or the pharmaceutically acceptable salt thereof, to the patient.

In an embodiment, step S4 comprises determining the dextran sulfate treatment to be efficient if the amounts of the biomarkers selected from group nos. 1, 3 and 5 are reduced in the second biological sample relative to the first biological sample and if the amounts of the biomarkers selected from group nos. 2, 4 and 6 are increased in the second biological sample relative to the first biological sample.

In an embodiment, step S3 comprises determining, for each biomarker $i$, a change $c_i$ in the amount of the biomarker between the first biological sample and the second biological sample relative to the amount of the biomarker in

$$c_i = 100 \times \frac{A2_i - A1_i}{A1_i}$$

the first biological sample. In this embodiment, and $A1_i$ represents the amount of the biomarker $i$ in the first biological sample and $A2_i$ represents the amount of the biomarker $i$ in the second biological sample.

In an embodiment, step S4 comprises determining the dextran sulfate treatment to be efficient if the change $c_i$ is

equal to or larger than X for the biomarkers selected from group nos. 1, 3 and 5 and the change $c_i$ is equal to or smaller than -X for the biomarkers selected from group nos. 2, 4 and 6, wherein X is a threshold value. In an embodiment, step S4 comprises determining the dextran sulfate treatment to be inefficient if the change $c_i$ is below X for at least one of the biomarkers selected from group nos. 1, 3 and 5 and/or the change $c_i$ is above -X for at least one of the biomarkers selected from group nos. 2, 4 and 6, wherein X is a threshold value. In a particular embodiment, X is 20.

In an embodiment, the method comprises determining an amount of at least one of IL36RN, GOSR1 and SLC4A1 in the first biological sample taken from the patient prior to administration of the dextran sulfate, or the pharmaceutically acceptable salt thereof, to the patient. In this embodiment, the method also comprises determining an amount of the at least one of IL36RN, GOSR1 and SLC4A1 in the second biological sample taken from the patient following administration of the dextran sulfate, or the pharmaceutically acceptable salt thereof, to the patient. The method further comprises, in this embodiment, determining a difference between the amount of the at least one of IL36RN, GOSR1 and SLC4A1 in the second biological sample and the amount of the at least one of IL36RN, GOSR1 and SLC4A1 in the first biological sample. In this embodiment, step S4 comprises determining the efficiency of the dextran sulfate treatment based on the differences for biomarkers in group nos. 1 to 6 and the difference between the amount of the at least one of IL36RN, GOSR1 and SLC4A1.

In an embodiment, the method also comprises adjusting the dextran sulfate treatment based on the determined efficiency.

In an embodiment, adjusting the dextran sulfate treatment comprises selecting, based on the determined efficiency, a dose of the dextran sulfate, or the pharmaceutically acceptable salt thereof, to be administered to the patient. Alternatively, or in addition, adjusting the dextran sulfate treatment comprises selecting, based on the determined efficiency, a frequency of administration of the dextran sulfate, or the pharmaceutically acceptable salt thereof, to the patient. Alternatively, or in addition, adjusting the dextran sulfate treatment comprises selecting, based on the determined efficiency, a duration of administration of the dextran sulfate, or the pharmaceutically acceptable salt thereof, to the patient. Alternatively, or in addition, adjusting the dextran sulfate treatment comprises selecting, based on the determined efficiency, a dosage regimen of the dextran sulfate, or the pharmaceutically acceptable salt thereof, for the patient

In an embodiment, the patient is suffering from a neurological disease, disorder or condition. In a particular embodiment, the neurological disease, disorder or condition is selected from the group consisting of traumatic brain injury (TBI), amyotrophic lateral sclerosis (ALS), Alzheimer's disease (AD), sub-arachnoid hemorrhage (SAH), Parkinson's disease (PD), Huntington's disease (HD), multiple sclerosis (MS), acute disseminated encephalomyelitis (ADEM), central nervous system (CNS) neuropathies, central pontine myelinolysis (CPM), myelopathies, leukoencephalopathies, leukodystrophies, Guillain-Barre syndrome (GBS), peripheral neuropathies, Charcot-Marie-Tooth (CMT) disease, hereditary spastic paraplegia (HSP), primary lateral sclerosis (PLS), progressive muscular atrophy (PMA), progressive bulbar palsy (PBP) pseudobulbar palsy, spinal muscular atrophy (SMA) and post-polio syndrome (PPS), preferably selected from the group consisting of TBI, ALS, AD and SAH and more preferably being TBI or ALS.

In the following, reference to (average) molecular weight and sulfur content of dextran sulfate applies also to any pharmaceutically acceptable salt of dextran sulfate. Hence, the pharmaceutically acceptable salt of dextran sulfate preferably has the average molecular weight and sulfur content as discussed in the following embodiments.

Dextran sulfate outside of the preferred ranges of the embodiments are believed to have inferior effect and/or causing negative side effects to the cells or subject.

For instance, dextran sulfate of a molecular weight exceeding 10,000 Da (10 kDa) generally has a lower effect vs. side effect profile as compared to dextran sulfate having a lower average molecular weight. This means that the maximum dose of dextran sulfate that can be safely administered to a subject is lower for larger dextran sulfate molecules (>10,000 Da) as compared to dextran sulfate molecules having an average molecular weight within the preferred ranges. As a consequence, such larger dextran sulfate molecules are less appropriate in clinical uses when the dextran sulfate is to be administered to subjects *in vivo.*

Dextran sulfate is a sulfated polysaccharide and in particular a sulfated glucan, i.e., polysaccharide made of many glucose molecules. Average molecular weight as defined herein indicates that individual sulfated polysaccharides may have a molecular weight different from this average molecular weight but that the average molecular weight represents the mean molecular weight of the sulfated polysaccharides. This further implies that there will be a natural distribution of molecular weights around this average molecular weight for a dextran sulfate sample.

Average molecular weight, or more correctly weight average molecular weight ($M_w$), of dextran sulfate is typically determined using indirect methods such as gel exclusion/penetration chromatography, light scattering or viscosity. Determination of average molecular weight using such indirect methods will depend on a number of factors, including choice of column and eluent, flow rate, calibration procedures, etc.

$$(M_w): \frac{\sum M_i^2 N_i}{\sum M_i N_i}$$

Weight average molecular weight , typical for methods sensitive to molecular size rather than numerical value, e.g., light scattering and size exclusion chromatography (SEC) methods. If a normal distribution is

assumed, then a same weight on each side of $M_w$, i.e., the total weight of dextran sulfate molecules in the sample having a molecular weight below $M_w$ is equal to the total weight of dextran sulfate molecules in the sample having a molecular weight above $M_w$. The parameter $N_i$ indicates the number of dextran sulfate molecules having a molecular weight of $M_i$ in a sample or batch.

In an embodiment, the dextran sulfate, or the pharmaceutically acceptable salt thereof, has a $M_w$ equal to or below 10,000 Da. In a particular embodiment, the dextran sulfate, or the pharmaceutically acceptable salt thereof, has a $M_w$ within an interval of from 2,000 Da to 10,000 Da.

In another embodiment, the dextran sulfate, or the pharmaceutically acceptable salt thereof, has a $M_w$ within an interval of from 2,500 Da to 10,000 Da, preferably within an interval of from 3,000 Da to 10,000 Da. In a particular embodiment, the dextran sulfate, or the pharmaceutically acceptable salt thereof, has a $M_w$ within an interval of from 3,500 Da to 9,500 Da, such as within an interval of from 3,500 Da to 8,000 Da.

In another particular embodiment, the dextran sulfate, or the pharmaceutically acceptable salt thereof, has a $M_w$ within an interval of from 4,500 Da to 7,500 Da, such as within an interval of from 4,500 Da and 6,500 Da or within an interval of from 4,500 Da and 5,500 Da.

Thus, in some embodiments, the dextran sulfate, or the pharmaceutically acceptable salt thereof, has a $M_w$ equal to or below 10,000 Da, equal to or below 9,500 Da, equal to or below 9,000 Da, equal to or below 8,500 Da, equal to or below 8,000 Da, equal to or below 7,500 Da, equal to or below 7,000 Da, equal to or below 6,500 Da, equal to or below 6,000 Da, or equal to or below 5,500 Da.

In some embodiments, the dextran sulfate, or the pharmaceutically acceptable salt thereof, has a $M_w$ equal to or above 1,000 Da, equal to or above 1,500 Da, equal to or above 2,000 Da, equal to or above 2,500 Da, equal to or above 3,000 Da, equal to or above 3,500 Da, equal to or above 4,000 Da. or equal to or above 4,500 Da. Any of these embodiments may be combined with any of the above presented embodiments defining upper limits of the $M_w$, such combined with the upper limit of equal to or below 10,000 Da.

In a particular embodiment, the $M_w$ of dextran sulfate, or the pharmaceutically acceptable salt thereof, as presented above is average $M_w$, and preferably determined by gel exclusion/penetration chromatography, size exclusion chromatography, light scattering or viscosity-based methods.

Number average molecular weight $(M_n)$: $\dfrac{\sum M_i N_i}{\sum N_i}$ , typically derived by end group assays, e.g., nuclear magnetic resonance (NMR) spectroscopy or chromatography. If a normal distribution is assumed, then a same number of dextran sulfate molecules can be found on each side of $M_n$, i.e., the number of dextran sulfate molecules in the sample having a molecular weight below $M_n$ is equal to the number of dextran sulfate molecules in the sample having a molecular weight above $M_n$.

In an embodiment, the dextran sulfate, or the pharmaceutically acceptable salt thereof, has a $M_n$ as measured by NMR spectroscopy within an interval of from 1,850 to 3,500 Da.

In a particular embodiment, the dextran sulfate, or the pharmaceutically acceptable salt thereof, has a $M_n$ as measured by NMR spectroscopy within an interval of from 1,850 Da to 2,500 Da, preferably within an interval of from 1,850 Da to 2,300 Da, such as within an interval of from 1,850 Da to 2,000 Da.

Thus, in some embodiments, the dextran sulfate, or the pharmaceutically acceptable salt thereof, has a $M_n$ equal to or below 3,500 Da, equal to or below 3,250 Da, equal to or below 3,000 Da, equal to or below 2,750 Da, equal to or below 2,500 Da, equal to or below 2,250 Da, or equal to or below 2,000 Da. In addition, the dextran sulfate, or the pharmaceutically acceptable salt thereof, has a $M_n$ equal to or above 1,850 Da.

In an embodiment, the dextran sulfate, or the pharmaceutically acceptable salt thereof, has an average sulfate number per glucose unit within an interval of from 2.5 to 3.0.

In a particular embodiment, the dextran sulfate, or the pharmaceutically acceptable salt thereof, has an average sulfate number per glucose unit within an interval of from 2.5 to 2.8, preferably within an interval of from 2.6 to 2.7.

In an embodiment, the dextran sulfate, or the pharmaceutically acceptable salt thereof, has an average number of glucose units within an interval of from 4.0 to 6.0.

In a particular embodiment, the dextran sulfate, or the pharmaceutically acceptable salt thereof, has an average number of glucose units within an interval of from 4.5 to 5.5, preferably within an interval of from 5.0 to 5.2.

In an embodiment, the dextran sulfate, or the pharmaceutically acceptable salt thereof, has a $M_n$ as measured by NMR spectroscopy within an interval of from 1,850 to 3,500 Da, an average sulfate number per glucose unit within an interval of from 2.5 to 3.0, and an average sulfation of C2 position in the glucose units of the dextran sulfate is at least 90 %.

In an embodiment, the dextran sulfate has an average number of glucose units of about 5.1, an average sulfate number per glucose unit within an interval of from 2.6 to 2.7 and a $M_n$ within an interval of from 1,850 Da and 2,000 Da.

In an embodiment, the pharmaceutically acceptable salt of dextran sulfate is a sodium salt of dextran sulfate. In a particular embodiment, the sodium salt of dextran sulfate has an average number of glucose units of about 5.1, an average sulfate number per glucose unit within an interval of from 2.6 to 2.7 and a $M_n$ including the Na+ counter ion within an interval of from 2,100 Data 2,300 Da.

In an embodiment, the dextran sulfate has an average number of glucose units of 5.1, an average sulfate number per glucose unit of 2.7, an average $M_n$ without Na+ as measured by NMR spectroscopy of about 1,900-1,950 Da and an average $M_n$ with Na+ as measured by NMR spectroscopy of about 2,200-2,250 Da.

The dextran sulfate according to the embodiments can be provided as a pharmaceutically acceptable salt of dextran sulfate, such as a sodium or potassium salt.

The subject is preferably a mammalian subject, more preferably a primate and in particular a human subject. The dextran sulfate, or the pharmaceutically acceptable salt thereof, can, however, be used also in veterinary applications. Non-limiting example of animal subjects include primate, cat, dog, pig, horse, mouse, rat.

The dextran sulfate, or the pharmaceutically acceptable salt thereof, is preferably administered by injection to the subject and in particular by intravenous (*i.v.*) injection, subcutaneous (s.c.) injection or (i.p.) intraperitoneal injection, preferably *i.v.* or *s.c.* injection. Other parenteral administration routes that can be used include intramuscular and intraarticular injection. Injection of the dextran sulfate, or the pharmaceutically acceptable derivative thereof, could alternatively, or in addition, take place directly in, for instance, a tissue or organ or other site in the subject body, at which the target effects are to take place.

The dextran sulfate, or the pharmaceutically acceptable salt thereof, may alternatively, or in addition, be administered intrathecally. For instance, the dextran sulfate, or the pharmaceutically acceptable salt thereof, can be injected together with a suitable aqueous carrier or solution into the spinal canal, or into the subarachnoid space so that it reaches the cerebrospinal fluid (CSF). A further administration route is intraocular administration.

The dextran sulfate, or the pharmaceutically acceptable salt thereof, of the embodiments is preferably formulated as an aqueous injection solution with a selected solvent or excipient. The solvent is advantageously an aqueous solvent and in particular a buffer solution. A non-limiting example of such a buffer solution is a citric acid buffer, such as citric acid monohydrate (CAM) buffer, or a phosphate buffer. For instance, dextran sulfate of the embodiments can be dissolved in saline, such as 0.9 % NaCl saline, and then optionally buffered with 75 mM CAM and adjusting the pH to about 5.9 using sodium hydroxide. Also non-buffered solutions are possible, including aqueous injection solutions, such as saline, i.e., NaCl (aq). Furthermore, other buffer systems than CAM could be used if a buffered solution are desired.

The embodiments are not limited to injections and other administration routes can alternatively be used including orally, nasally, bucally, rectally, dermally, tracheally, bronchially, or topically. The active compound, dextran sulfate, is then formulated with a suitable excipient or carrier that is selected based on the particular administration route.

Suitable dose ranges for the dextran sulfate, or the pharmaceutically acceptable salt thereof, may vary according to the application, such as *in vitro* versus *in vivo*, the size and weight of the subject, the condition for which the subject is treated, and other considerations. In particular for human subjects, a possible dosage range could be from 1 $\mu$g/kg to 100 mg/kg of body weight, preferably from 10 $\mu$g/kg to 50 mg/kg of body weight.

In preferred embodiments, the dextran sulfate, or the pharmaceutically acceptable salt thereof, is formulated to be administered at a dosage in a range from 0.05 to 50 mg/kg of body weight of the subject, preferably from 0.05 or 0.1 to 40 mg/kg of body weight of the subject, and more preferably from 0.05 or 0.1 to 30 mg/kg, or 0.1 to 25 mg/kg or from 0.1 to 15 mg/kg or 0.1 to 10 mg/kg body weight of the subject.

The dextran sulfate, or the pharmaceutically acceptable derivative thereof, can be administered at a single administration occasion, such as in the form of a single bolus injection. This bolus dose can be injected quite quickly to the subject but is advantageously infused over time so that the dextran sulfate solution is infused over a few minutes of time to the patient, such as during 5 to 10 minutes.

Alternatively, the dextran sulfate, or the pharmaceutically acceptable salt thereof, can be administered at multiple, i.e., at least two, occasions during a treatment period.

The dextran sulfate, or the pharmaceutically acceptable salt thereof, can be administered together with other active agents, either sequentially, simultaneously or in the form of a composition comprising the dextran sulfate, or the pharmaceutically acceptable salt thereof, and at least one other active agent. The at least one active agent can be selected among any agent useful in any of the above mentioned diseases, disorders or conditions. The at least one active agent could also be in the form of cells in cell therapy, such as stem cells including, but not limited to, embryonic stem cells (ESCs) and mesenchymal stromal cells (MSCs).

As previously described herein, the dextran sulfate treatment can be adjusted based on the efficiency as determined in step S4 in Fig. 12. For instance, such an adjustment may include at least one of selecting, based on the determined efficiency, a dose of dextran sulfate, or the pharmaceutically acceptable salt thereof, to be administered to the patient; selecting, based on the determined efficiency, a frequency of administration of dextran sulfate, or the pharmaceutically acceptable salt thereof, to the patient; selecting, based on the determined efficiency, a duration of administration of dextran sulfate, or the pharmaceutically acceptable salt thereof, to the patient; and selecting, based on the determined efficiency, a dosage regimen of dextran sulfate, or pharmaceutically acceptable salt thereof, for the patient

Comparative EXAMPLES

In the following examples, a sodium salt of dextran sulfate, denoted low molecular weight dextran sulfate (LMW-DS) herein, was used (Tikomed AB, Sweden, WO 2016/076780).

EXAMPLE 1

The effects of daily sub-cutaneous injections of LMW-DS on glutamate excitotoxicity and mitochondrial function after severe traumatic brain injury (sTBI) in rats were evaluated by high-performance liquid chromatography (HPLC) analysis of frozen brain samples. The results suggest that LMW-DS interferes with mitochondrial function to improve energy metabolism and also decreases glutamate excitotoxicity.

MATERIALS AND METHODS

*Induction of sTBI and drug administration protocol*

The experimental protocol used in this study was approved by the Ethical Committee of the Catholic University of Rome, according to international standards and guidelines for animal care. Male Wistar rats of 300-350 g body weight (b.w.) were fed with standard laboratory diet and water ad libitum in a controlled environment.

They were divided into three groups:

1) n = 6 animals subjected to sTBI, with drug administration after 30 minutes and sacrifice at 2 days post-TBI (Acute phase 1)
2) n= 6 animals subjected to sTBI, with drug administration after 30 minutes and sacrifice at 7 days post-TBI (Acute phase 2).
3) n= 6 animals subjected to sTBI, with drug administration after 3 days and sacrifice at 7 days post-TBI (Chronic phase).

As the anesthetic mixture, animals received 35 mg/kg b.w. ketamine and 0.25 mg/kg b.w. midazolam by *i.p.* injection. sTBI was induced by dropping a 450 g weight from 2 m height on to the rat head that had been protected by a metal disk previously fixed on the skull, according to the "weight drop" impact acceleration model (Marmarou et al., J Neurosurg. 1994; 80: 291-300). Rats that suffered from skull fracture, seizures, nasal bleeding, or did not survive the impacts, were excluded from the study. At the end of each period of treatment, rats were anesthetized again and then immediately sacrificed.

The drug treatment was a subcutaneous injection of 0.5 ml of LMW-DS (15 mg/kg) and administered according to the aforementioned schematic protocol.

*Cerebral tissue processing*

An *in vivo* craniectomy was performed in all animals during anesthesia, after carefully removing the rat's skull, the brain was exposed and removed with a surgical spatula and quickly dropped in liquid nitrogen. After the wet weight (w.w.) determination, tissue preparation was affected as previously disclosed (Tavazzi et al., Neurosurgery. 2005; 56: 582-589; Vagnozzi et al., Neurosurgery. 2007; 61: 379-388; Tavazzi et al., Neurosurgery. 2007; 61: 390-395; Amorini et al., J Cell Mol Med. 2017; 21: 530-542.). Briefly, whole brain homogenization was performed with 7 ml of ice-cold, nitrogen-saturated, precipitating solution composed by $CH_3CN$ + 10 mM $KH_2PO_4$, pH 7.40, (3:1; v:v), and using an Ultra-Turrax set at 24,000 rpm/min (Janke & Kunkel, Staufen, Germany). After centrifugation at 20,690 $\times$ g, for 10 min at 4°C, the clear supernatants were saved, pellets were supplemented with 3 ml of the precipitating solution and homogenized again as described above. A second centrifugation was performed (20,690 $\times$ g, for 10 min at 4°C), pellets were saved, supernatants combined with those previously obtained, extracted by vigorous agitation with a double volume of HPLC-grade $CHCl_3$ and centrifuged as above. The upper aqueous phases containing water-soluble low-molecular weight compounds were collected, subjected to chloroform washings for two more times (this procedure allowed the removal of all the organic solvent and of any lipid soluble compound from the buffered tissue extracts), adjusted in volumes with 10 mM $KH_2PO_4$, pH 7.40, to have ultimately aqueous 10 % tissue homogenates and saved at -80°C until assayed.

*HPLC analyses of purine-pyrimidine metabolites*

Aliquots of each deproteinized tissue samples were filtered through a 0.45 $\mu$m HV Millipore filter and loaded (200 $\mu$l) onto a Hypersil C-18, 250 $\times$ 4.6 mm, 5 $\mu$m particle size column, provided with its own guard column (Thermo Fisher Scientific, Rodano, Milan, Italy) and connected to an HPLC apparatus consisting of a Surveyor System (Thermo Fisher Scientific, Rodano, Milan, Italy) with a highly sensitive diode array detector (equipped with a 5 cm light path flow cell) and set up between 200 and 300 nm wavelength. Data acquisition and analysis were performed by a PC using the ChromQuest® software package provided by the HPLC manufacturer.

Metabolites belonging to the purine-pyrimidine profiles (listed below) and related to tissue energy state, mitochondrial function and relative to oxidative-nitrosative stresses were separated, in a single chromatographic run, according to slight modifications of existing ion-pairing HPLC methods (Lazzarino et al., Anal Biochem. 2003; 322: 51-59; Tavazzi et al., Clin Biochem. 2005; 38: 997-1008). Assignment and calculation of the compounds of interest in chromatographic runs of tissue extracts were carried out at the proper wavelengths (206, 234 and 260 nm) by comparing retention times, absorption spectra and areas of peaks with those of peaks of chromatographic runs of freshly-prepared ultra-pure standard mixtures with known concentrations.

List of compounds: Cytosine, Creatinine, Uracil, Beta-Pseudouridine, Cytidine, Hypoxanthine, Guanine, Xanthine, Cytidine diphosphate-Choline (CDP-Choline), Ascorbic Acid, Uridine, Adenine, Nitrite ($-NO_2^-$), reduced glutathione (GSH), Inosine, Uric Acid, Guanosine, Cytidine monophosphate (CMP), malondialdehyde (MDA), Thyimidine, Orotic Acid, Nitrate ($-NO_3^-$), Uridine monophosphate (UMP), Nicotinamide adenine dinucleotide, oxidized (NAD+), Adenosine (ADO), Inosine monophosphate (IMP), Guanosine monophosphate (GMP), Uridine diphosphate-glucose (UDP-Glc), UDP-galactose (UDP-Gal), oxidized glutathione (GSSG), UDP-N-acetyl-glucosamine (UDP-GlcNac), UDP-N-acetylgalactosamine (UDP-GalNac), Adenosine monophosphate (AMP), Guanosine diphosphate-glucose (GDP-glucose), Cytidine diphosphate (CDP), UDP, GDP, Nicotinamide adenine dinucleotide phosphate, oxidized (NADP+), Adenosine diphosphate-Ribose (ADP-Ribose), Cytidine triphosphate (CTP), ADP, Uridine triphosphate (UTP), Guanosine triphosphate (GTP), Nicotinamide adenine dinucleotide, reduced (NADH), Adenosine triphosphate (ATP), Nicotinamide adenine dinucleotide phosphate, reduced (NADPH), Malonyl-CoA, Coenzyme A (CoA-SH), Acetyl-CoA, N-acetylaspartate (NAA).

*HPLC analyses of free amino acids and amino group containing compounds*

The simultaneous determination of primary free amino acids (FAA) and amino group containing compounds (AGCC) (listed below) was performed using the precolumn derivatization of the sample with a mixture of Ortho-phthalaldehyde (OPA) and 3-Mercaptopropionic acid (MPA), as described in detail elsewhere (Amorini et al., J Cell Mol Med. 2017; 21: 530-542; Amorini et al., Mol Cell Biochem. 2012; 359: 205-216). Briefly, the derivatization mixture composed by 25 mmol/l OPA, 1 % MPA, 237.5 mmol/l sodium borate, pH 9.8 was prepared daily and placed in the autosampler. The automated precolumn derivatization of the samples (15 $\mu$l) with OPA-MPA was carried out at 24°C and 25 $\mu$l of the derivatized mixture were loaded onto the HPLC column (Hypersil C-18, 250 $\times$ 4.6 mm, 5 $\mu$m particle size, thermostated at 21°C) for the subsequent chromatographic separation. In the case of glutamate, deproteinized brain extracts were diluted 20 times with HPLC-grade $H_2O$ prior to the derivatization procedure and subsequent injection. Separation of OPA-AA and OPA-AGCC was carried out at a flow rate of 1.2 ml/min using two mobile phases (mobile phase A = 24 mmol/l $CH_3COONa$ + 24 mmol/l $Na_2HPO_4$ + 1 % tetrahydrofurane + 0.1 % trifluoroacetic acid, pH 6.5; mobile phase B = 40 % $CH_3OH$ + 30 % $CH_3CN$ + 30 % $H_2O$), using an appropriate step gradient (Amorini et al., J Cell Mol Med. 2017; 21: 530-542; Amorini et al., Mol Cell Biochem. 2012; 359: 205-216).

Assignment and calculation of the OPA-AA and OPA-AGCC in chromatographic runs of whole brain extracts were carried out at 338 nm wavelengths by comparing retention times and areas of peaks with those of peaks of chromatographic runs of freshly-prepared ultra-pure standard mixtures with known concentrations.

List of FAA and ACGC compounds: aspartate (ASP), glutamate (GLU), asparagine (ASN), serine (SER), glutamine (GLN), histidine (HIS), glycine (GLY), threonine (THR), citrulline (CITR), arginine (ARG), alanine (ALA), taurine (TAU), gamma-aminobutyric acid (GABA), tyrosine (TYR), S-adenosylhomocysteine (SAH), L-cystathionine (L-Cystat), valine (VAL), methionine (MET), tryptophan (TRP), phenylalanine (PHE), isoleucine (ILE), leucine (LEU), ornithine (ORN), lysine (LYS).

*Statistical analysis*

Normal data distribution was tested using the Kolmogorov-Smirnov test. Differences across groups were estimated by the two-way ANOVA for repeated measures. Fisher's protected least square was used as the post hoc test. Only two-tailed p-values of less than 0.05 were considered statistically significant

RESULTS

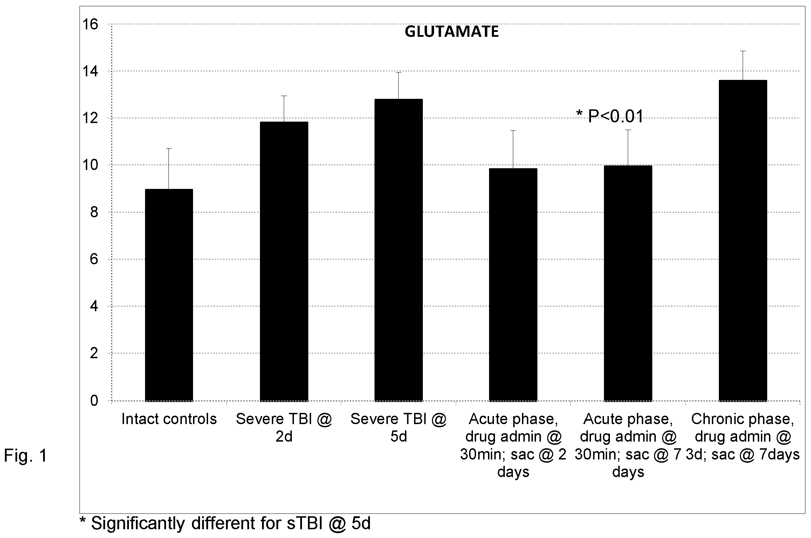

The most evident result among the cerebral values of the 24 standard and non-standard amino acids and primary amino-group containing compounds was that LMW-DS treatment had a remarkable inhibition of the increase in glutamate (GLU) induced by sTBI (Fig. 1), thus certainly causing a decrease of excitotocity consequent to excess of this compound.

This effect was, however, visible only if the drug was administered early post-injury (30 min following sTBI), with no efficacy on this excitotoxicity marker when LMW-DS was injected at 3 days after sTBI. It is also worth underlining that LMW-DS had significant beneficial effects on compounds involved in the so-called methyl cycle (Met, L-Cystat, SAH), see Table 3.

Table 3 - concentrations of cerebral compounds

| | ASP | GLU | ASN | SER | GLN | HIS |
|---|---|---|---|---|---|---|
| Control | 2.67±0.45 | 8.95±1.76 | 0.11±0.02 | 0.56±0.14 | 3.70±0.72 | 0.045±0.01 |
| TBI 2 days | 3.86±0.80 | 11.8±1.15 | 0.12±0.02 | 0.85±0.17 | 4.81±0.78 | 0.060±0.01 |
| TBI 5 days | 3.85±0.91 | 12.77±1.17 | 0.09±0.03 | 0.69±0.19 | 3.57±0.62 | 0.046±0.008 |
| Acute phase 1 | 2.40±0.56[d,i] | 9.81±1.66[i] | 0.12±0.02[i] | 0.88±0.25[a] | 4.78±1.09[a] | 0.068±0.015[b] |
| Acute phase 2 | 2.94±0.98[f,j] | 9.93±1.56[e,i] | 0.13±0.03[i] | 0.71±0.28[b] | 3.66±0.41 | 0.055±0.019 |
| Chronic phase | 4.46±0.70[a,f] | 13.58±1.28[a] | 0.18±0.02[a] | 0.93±0.27[a,e] | 3.98±0.34 | 0.047±0.021 |
| | GLY | THR | CITR | ARG | ALA | TAU |
| Control | 0.65±0.10 | 0.58±0.15 | 0.018±0.002 | 0.16±0.034 | 0.30±0.067 | 3.60±0.89 |
| TBI 2 days | 1.54±0.16 | 0.78±0.17 | 0.017±0.006 | 0.098±0.029 | 0.66±0.17 | 4.93±0.79 |
| TBI 5 days | 0.84±0.13 | 0.60±0.12 | 0.017±0.007 | 0.13±0.52 | 0.35±0.047 | 4.00±0.97 |
| Acute phase 1 | 0.83±0.25[a,c] | 0.92±0.29[a] | 0.018±0.004 | 0.13±0.02[b,d] | 0.50±0.12[a] | 4.86±0.85[b] |
| Acute phase 2 | 0.71±0.16[f,i] | 0.66±0.23 | 0.018±0.008 | 0.16±0.03 | 0.52±0.24[a,e] | 3.80±1.19 |
| Chronic phase | 1.05±0.13[a,f] | 0.75±0.24[a,e] | 0.020±0.006 | 0.14±0.02 | 0.57±0.28[a,e] | 4.49±0.43[a] |
| | GABA | TYR | SAH | L-Cystat | VAL | MET |
| Control | 1.15±0.40 | 0.120±0.022 | 0.26±0.010 | 0.147±0.080 | 0.049±0.005 | 0.015±0.002 |
| TBI 2 days | 1.74±0.35 | 0.160±0.023 | 0.077±0.009 | 0.337±0.011 | 0.057±0.005 | 0.011±0.001 |
| TBI 5 days | 1.50±0.30 | 0.123±0.013 | 0.043±0.013 | 0.202±0.061 | 0.042±0.014 | 0.010±0.001 |
| Acute phase 1 | 1.43±0.25[a] | 0.15±0.03 | 0.033±0.008[b,c,j] | 0.185±0.031[b,c,i] | 0.042±0.011 | 0.016±0.005[d,j] |
| Acute phase 2 | 1.60±0.24[a] | 0.172±0.046[b,f] | 0.026±0.010[f,i] | 0.173±0.038[b,f,i] | 0.057±0.017 | 0.022±0.006[b,e,i] |
| Chronic phase | 1.85±0.65[a] | 0.21±0.05[f] | 0.050±0.013[a] | 0.26±0.05[a,f] | 0.040±0.016[b] | 0.009±0.004[b] |
| | TRP | PHE | ILE | LEU | ORN | LYS |
| Control | 0.013±0.002 | 0.023±0.001 | 0.030±0.010 | 0.015±0.002 | 0.012±0.003 | 0.206±0.042 |
| TBI 2 days | 0.023±0.004 | 0.046±0.011 | 0.043±0.005 | 0.014±0.007 | 0.013±0.015 | 0.202±0.023 |
| TBI 5 days | 0.012±0.003 | 0.033±0.006 | 0.038±0.010 | 0.014±0.005 | 0.009±0.002 | 0.19±0.092 |
| Acute phase 1 | 0.030±0.007[b,dg,i] | 0.031±0.011[b,d] | 0.038±0.007 | 0.021±0.005[a,c] | 0.014±0.007 | 0.236±0.057[b,d,h] |

| | TRP | PHE | ILE | LEU | ORN | LYS |
|---|---|---|---|---|---|---|
| Acute phase 2 | 0.015±0.006 | 0.028±0.010 | 0.048±0.017[a] | 0.018±0.004 | 0.011±0.005 | 0.32±0.04[a,e,i] |
| Chronic phase | 0.012±0.007 | 0.033±0.011[b] | 0.041±0.016[b] | 0.024±0.032[b,f] | 0.017±0.009[a,e] | 0.179±0.036 |

[a] $p<0.01$ (comparison with control), [b] $p<0.05$ (comparison with control), [c] $p<0.01$ (comparison with TBI 2 days), [d] $p<0.05$ (comparison with TBI 2 days), [e] $p<0.01$ (comparison with TBI 5 days), [f] $p<0.05$ (comparison with TBI 5 days), a $p<0.01$ (comparison with Acute phase 2), [h] $p<0.05$ (comparison with Acute phase 2), [i] $p<0.01$ (comparison with Chronic phase), [j] $p<0.05$ (comparison with Chronic phase) Table 3 lists the compounds in μmol/g (w.w.)

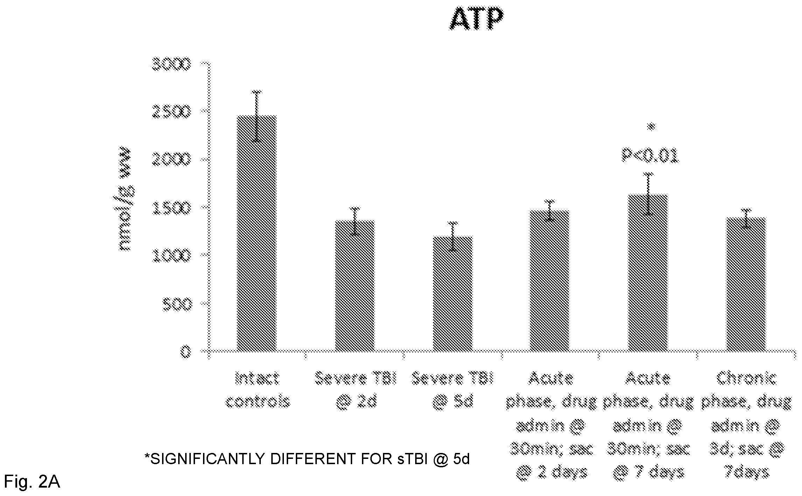

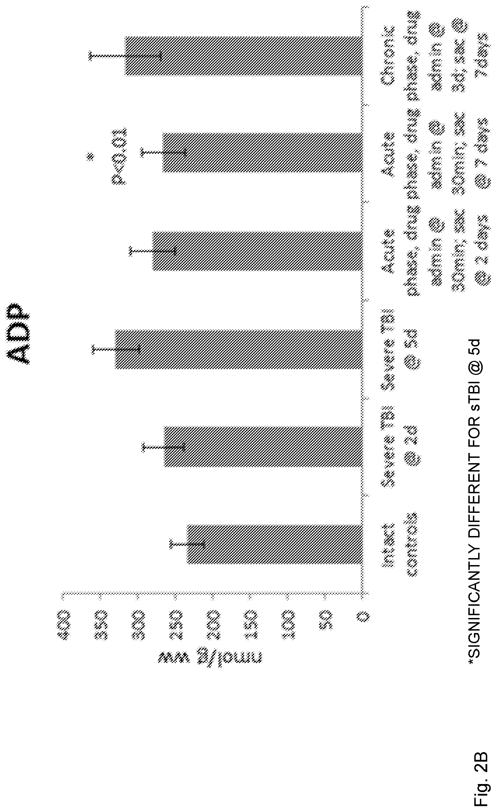

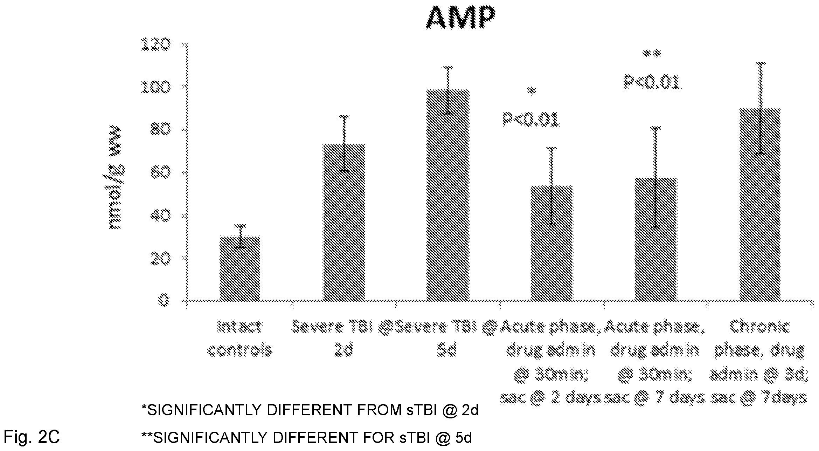

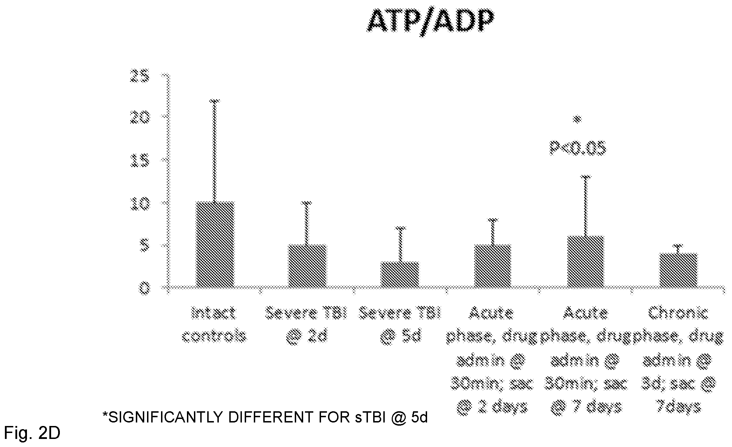

As is seen in Table 4, LMW-DS positively affected various compounds related to energy metabolism and mitochondrial functions. Particularly interesting are the concentrations of adenine nucleotides and ATP/ADP ratio as measurement of mitochondrial phosphorylating capacity (Figs. 2A-2D).

Table 4 - concentrations of energy metabolites

| | | cytosine | creatinine | uracil | β-pseudouridine | cytidine |
|---|---|---|---|---|---|---|
| | Control | 12.89±1.77 | 18.77±2.09 | 10.65±1.11 | 6.32±1.11 | 12.54±1.84 |
| | TBI 2 days | 23.58±5.62 | 28.61±3.33 | 17.32±1.54 | 8.45±0.98 | 11.33±1.23 |
| | TBI 5 days | 21.56±2.88 | 76.03±8.19 | 24.31±2.60 | 18.66±1.29 | 26.12±2.37 |
| | Acute phase 1 | 17.69±2.50[b,d] | 24.55±3.20[b,g,i] | 14.56±5.44 | 6.65±1.30[g,i] | 15.40±3.04 |
| | Acute phase 2 | 15.70±4.10[f] | 37.27±5.82[a,e,j] | 19.40±7.52[a,e] | 13.26±3.16[a,e,j] | 16.18±4.21[e] |
| | Chronic phase | 15.58±2.50[b,f] | 51.25±10.17[a,f] | 16.57±2.99[a,f] | 18.62±2.80[a] | 14.71±2.83[e] |
| | | hypoxanthine | guanine | xanthine | CDP choline | ascorbic acid |
| | Control | 7.21±1.22 | 3.12±0.78 | 8.09±1.48 | 7.50±1.01 | 4954.36±212.43 |
| | TBI 2 days | 11.36±1.52 | 5.42±0.87 | 13.15±2.88 | 9.83±1.71 | 3186.09±287.87 |
| | TBI 5 days | 16.83±2.13 | 4.56±1.29 | 14.14±2.11 | 8.12±1.55 | 2234.51±198.62 |
| | Acute phase 1 | 14.47±2.87[a] | 4.80±1.24[b] | 9.46±2.34[d] | 10.93±3.22[b,h] | 3733.10±277.88[a,d] |
| | Acute phase 2 | 12.90±2.58[a,j] | 4.73±1.07 | 10.41±2.11[f] | 6.91±1.86 | 3512.58±224.62[a,e] |
| | Chronic phase | 17.97±4.49[a] | 5.31±1.04[b] | 9.35±0.83[f] | 8.37±2.19 | 3375.03±856.41[a,e] |
| | | uridine | adenine | NO$_2$ | GSH | inosine |
| | Control | 56.17±3.88 | 23.14±2.16 | 151.21±16.79 | 3810.29±200.65 | 94.33±17.48 |
| | TBI 2 days | 112.09±15.65 | 54.85±8.88 | 233.14±25.48 | 2109.89±156.71 | 126.36±14.06 |
| | TBI 5 days | 94.8±10.75 | 76.55±6.33 | 256.28±28.07 | 1902.56±183.42 | 137.73±24.82 |
| | Acute phase 1 | 76.35±12.85[a,c] | 44.82±6.31[a,d,g] | 216.03±41.74[a] | 2649.50±397.31[a,d] | 92.55±31.20[c] |
| | Acute phase 2 | 63.02±9.66[b,e] | 58.16±6.36[a,f] | 226.40±30.95[b] | 2821.50±242.82[a,e] | 85.52±20.36[e] |
| | Chronic phase | 63.28±3.37[f] | 52.94±8.59[a,f] | 217.67±55.04[a] | 2608.67±358.07[a,e] | 105.81±25.57[f] |
| | | uric acid | guanosine | CMP | MDA | thymidine |
| | Control | 2.75±0.35 | 18.96±2.90 | 12.16±1.61 | 1.13±0.25 | 0.54±0.16 |
| | TBI 2 days | 30.84±5.13 | 17.52±2.44 | 30.83±4.81 | 28.37±3.37 | 0.67±0.19 |
| | TBI 5 days | 23.63±3.40 | 21.32±3.04 | 27.20±3.76 | 7.69±2.18 | 0.97±0.32 |
| | Acute phase 1 | 23.62±3.77[a,d,h] | 20.71±5.66 | 30.12±9.97[a,h] | 12.47±2.09[a,c,g] | 0.69±0.11 |
| | Acute phase 2 | 19.17±2.15[a,h,i] | 17.90±3.24[j] | 15.68±2.12[f,j] | 4.82±1.73[a,e,i] | 0.49±0.20[f] |
| | Chronic phase | 27.77±3.60[a] | 28.87±7.60[a,f] | 20.51±3.73[a,f] | 11.62±3.90[a,e] | 0.71±0.11 |
| | | orotic acid | NO$_3$ | UMP | NAD+ | ADO |
| | Control | 5.67±0.85 | 178.66±37.75 | 96.21±10.51 | 506.88±59.15 | 50.73±8.29 |
| | TBI 2 days | 10.09±1.54 | 265.31±47.68 | 116.06±13.55 | 322.37±30.87 | 66.19±11.06 |
| | TBI 5 days | 14.27±1.67 | 325.19±60.08 | 128.70±28.28 | 261.67±49.97 | 78.91±20.42 |
| | Acute phase 1 | 8.80±2.45[b,h,j] | 210.64±91.95[d] | 107.80±21.62 | 404.63±51.10[a,c,i] | 71.67±15.87 |
| | Acute phase 2 | 13.34±3.65[a] | 198.56±25.93[e,i] | 138.73±32.01[b] | 401.18±34.53[a,e,i] | 82.11±16.51[a] |
| | Chronic phase | 12.05±1.50[a] | 241.27±18.84[e] | 103.11±29.79 | 301.13±29.90[a] | 89.97±12.98[a] |
| | | IMP | GMP | UDP-Glc | UDP-Gal | GSSG |
| | Control | 54.09±12.15 | 98.93±10.42 | 47.23±3.14 | 120.18±10.99 | 189.21±20.19 |

(continued)

|  | IMP | GMP | UDP-Glc | UDP-Gal | GSSG |
|---|---|---|---|---|---|
| TBI 2 days | 50.82±10.45 | 181.94±27.20 | 45.17±6.67 | 131.19±18.49 | 179.51±29.17 |
| TBI 5 days | 124.46±18.97 | 158.35±40.43 | 41.43±5.14 | 112.26±17.36 | 196.65±33.48 |
| Acute phase 1 | 67.71±10.63[g,i] | 177.00±32.39[a,g] | 32.14±4.59[g] | 119.45±12.50 | 185.21±48.10 |
| Acute phase 2 | 102.63±22.09[a] | 91.47±12.35[e,i] | 44.44±7.59[j] | 145.14±27.76 | 219.54±53.36 |
| Chronic phase | 99.29±13.82[a] | 148.56±31.21[a] | 35.79±3.45[b] | 122.29±12.15 | 231.08±44.34[b,f] |
|  | UDP-GlcNac | UDP-GalNac | AMP | GDP glucose | CDP |
| Control | 93.71±14.16 | 35.09±3.07 | 30.31±5.12 | 34.89±8.18 | 14.08±1.14 |
| TBI 2 days | 93.71±14.16 | 20.17±3.33 | 73.32±12.88 | 39.16±6.87 | 18.31±2.15 |
| TBI 5 days | 129.54121.21 | 10.5612.89 | 98.32±10.99 | 59.88±12.54 | 19.03±6.45 |
| Acute phase 1 | 95.85±19.73[h,i] | 19.17±4.01[a] | 53.61±17.91[a,c,j] | 38.71±6.86 | 25.53±6.83[a,c] |
| Acute phase 2 | 130.65±28.41[a] | 19.90±3.12[a,e] | 57.70±23.01[a,e,j] | 49.25±10.33[a] | 24.29±6.76[a] |
| Chronic phase | 129.42±15.88[b] | 21.84±2.80[a,e] | 90.01±21.24[a] | 43.85±5.06[b] | 23.55±6.45[a] |
|  | UDP | GDP | NADP+ | ADP-ribose | CTP |
| Control | 26.06±7.32 | 61.78±17.09 | 27.52±2.58 | 48.88±5.61 | 38.90±4.64 |
| TBI 2 days | 55.47±6.70 | 149.02±19.09 | 16.36±4.41 | 133.31±30.02 | 21.57±3.19 |
| TBI 5 days | 43.71±8.81 | 113.11±28.34 | 12.50±2.97 | 221.80±36.72 | 18.79±3.69 |
| Acute phase 1 | 61.83±10.23[a,g] | 158.72±24.57[a] | 17.95±3.28[a] | 137.87±43.18[a] | 18.98±6.58[a,g] |
| Acute phase 2 | 40.38±8.50[a,i] | 126.70±31.35[a,j] | 21.27±4.19[b,e,j] | 141.96±23.56[a,e,j] | 32.63±3.99[e,i] |
| Chronic phase | 57.40±5.88[a,f] | 173.05±28.68[a,e] | 16.44±2.66[a,f] | 173.94±8.45[a] | 25.23±2.93[a,f] |
|  | ADP | UTP | GTP | NADH | ATP |
| Control | 233.19±21.33 | 138.95±28.89 | 567.33±54.79 | 14.50±2.75 | 2441.66±257.71 |
| TBI 2 days | 264.71±26.31 | 107.77±12.83 | 208.13±28.36 | 8.54±1.73 | 1350.25±140.87 |
| TBI 5 days | 328.26±31.30 | 90.50±18.69 | 191.81±37.56 | 6.77±1.58 | 1195.81±137.82 |
| Acute phase 1 | 279.34±29.59[b] | 123.46±15.42[d] | 255.29±45.21[a,g] | 15.49±2.05[c,j] | 1464.25±99.09[a,h] |
| Acute phase 2 | 264.07±28.29[b,e,j] | 146.71±32.68[e] | 336.65±35.18[a,e,j] | 13.12±4.19[e] | 1632.23±90.07[a,e,j] |
| Chronic phase | 315.53±46.53[a] | 136.80±33.25[f] | 290.92±34.68[a,f] | 11.78±3.32[e] | 1381.03±212.64[a] |
|  | NADPH | malonyl-CoA | CoA-SH | acetyl-CoA | NAA |
| Control | 7.95±1.38 | 15.83±1.31 | 28.91±3.19 | 38.97±5.79 | 9141.22±366.64 |
| TBI 2 days | 8.14±1.69 | 10.46±2.56 | 19.64±2.37 | 21.76±4.49 | 5570.00±912.08 |
| TBI 5 days | 9.24±2.07 | 11.89±1.96 | 21.77±1.44 | 18.94±3.75 | 4300.00±480.84 |
| Acute phase 1 | 6.22±1.73 | 12.33±1.82[b] | 21.61±3.42[a,h] | 21.56±6.22[a,g,i] | 6147.91±989.12[a] |
| Acute phase 2 | 7.05±2.21 | 11.29±2.27[b] | 30.57±6.02[f] | 36.86±4.11[e] | 7262.84±749.73[a,e] |
| Chronic phase | 7.34±2.65[f] | 10.00±1.95[b] | 27.58±6.24[f] | 35.68±6.55[e] | 6375.36±974.12[a,e] |

[a] $p<0.01$ (comparison with control), [b] $p<0.05$ (comparison with control), [c] $p<0.01$ (comparison with TBI 2 days), [d] $p<0.05$ (comparison with TBI 2 days), [e] $p<0.01$ (comparison with TBI 5 days), [f] $p<0.05$ (comparison with TBI 5 days), a $p<0.01$ (comparison with Acute phase 2), [h] $p<0.05$ (comparison with Acute phase 2), [i] $p<0.01$ (comparison with Chronic phase), [j] $p<0.05$ (comparison with Chronic phase) Table 4 lists the compounds in nmol/g (w.w.)

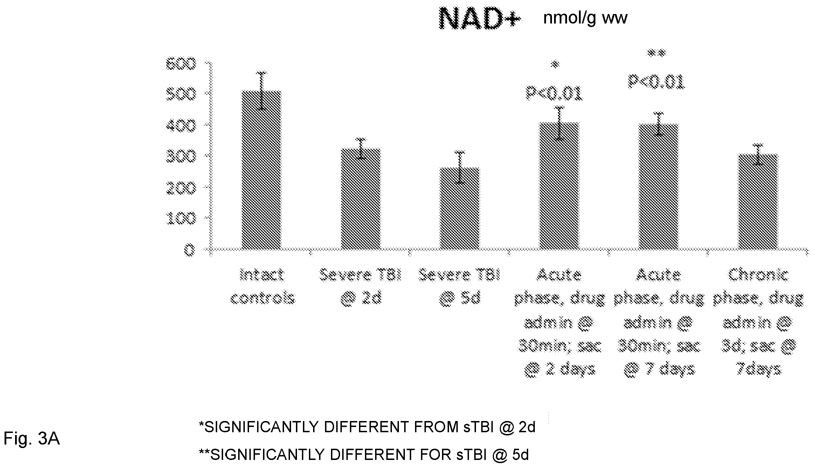

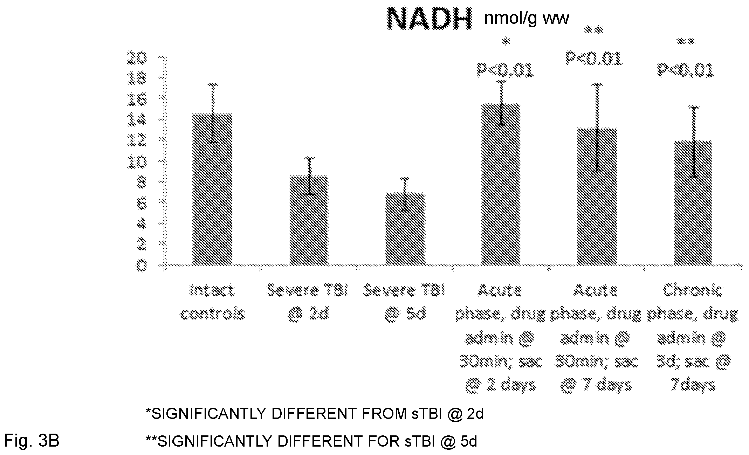

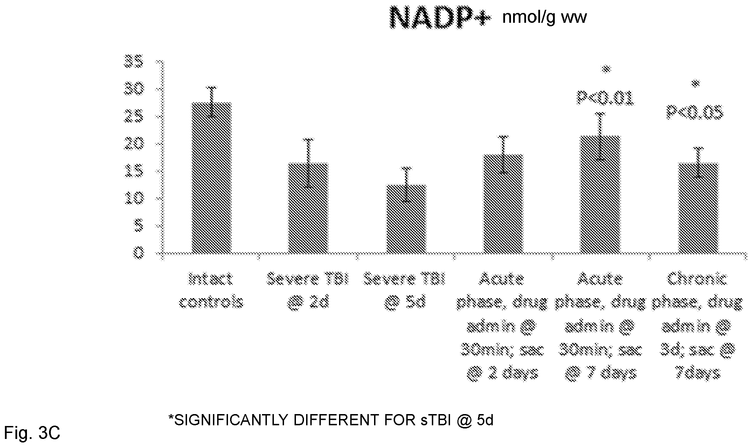

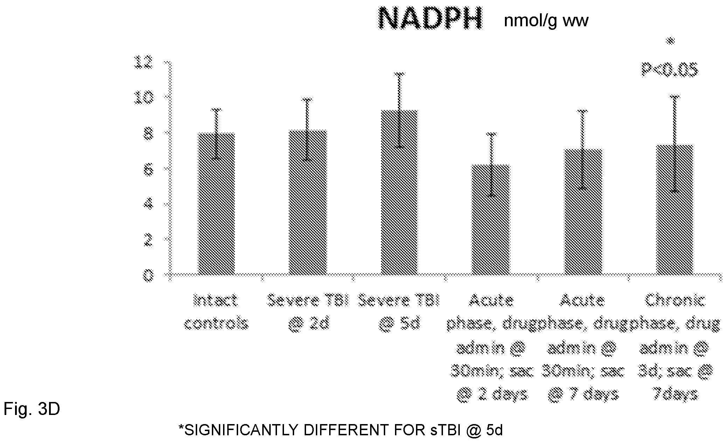

Remarkable changes of oxidative and reduced nicotinic coenzymes were also observed (Figs. 3A-3D).

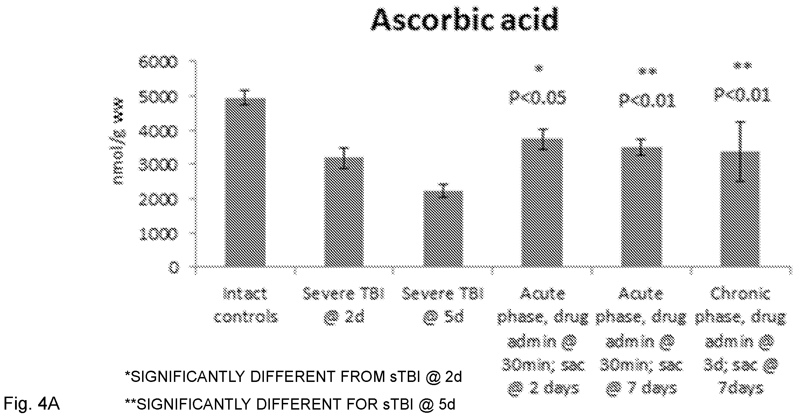

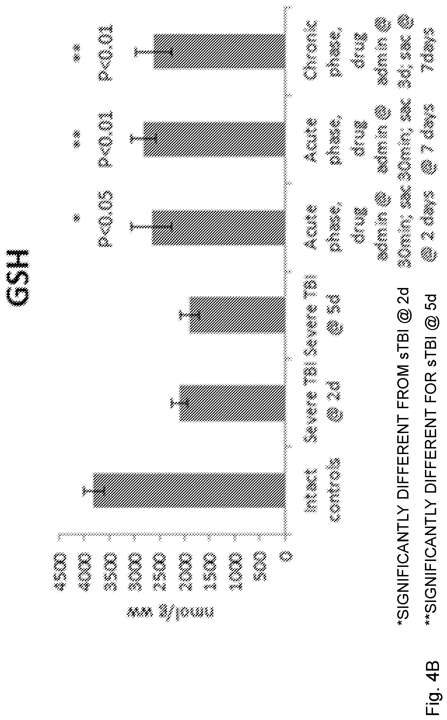

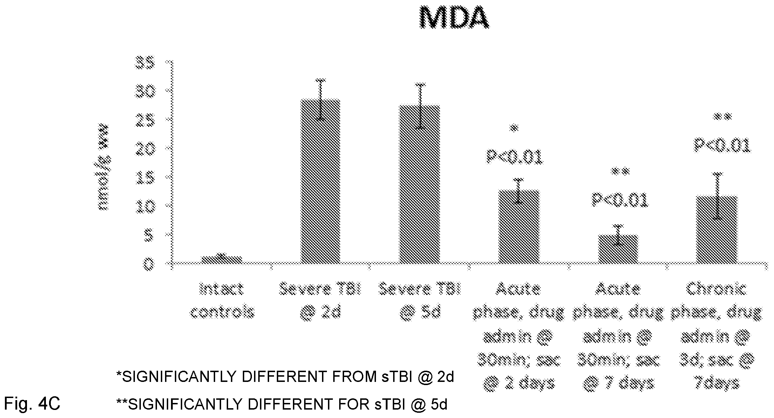

Parameters related to oxidative stress were also measured and a significant reduction of oxidative stress was detected after administration of LMW-DS. In particular, ascorbic acid, as the main water-soluble brain antioxidant, and GSH, as the major intracellular-SH donor, were measured. Results showed a significant improvement in their levels after administration of LMW-DS as shown in Table 4 and Figs. 4A-4C.

In addition, MDA, as end product of polyunsaturated fatty acids of membrane phospholipids and therefore taken as a marker of ROS-mediated lipid peroxidation, was also measured. MDA levels showed a significant reduction after administration of LMW-DS. The oxidative stress markers described above all indicated an improvement in the recovery of antioxidant status after treatment with LMW-DS (Figs. 4A-4C).

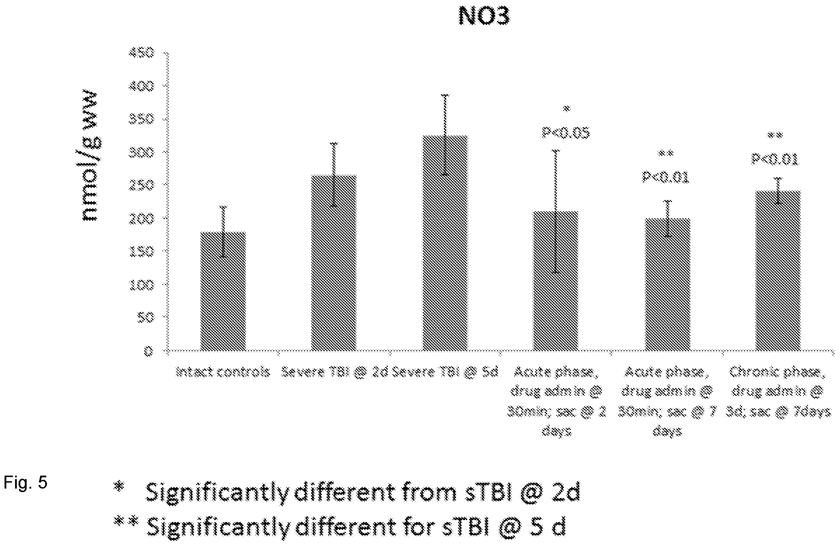

Indices of representative of NO-mediated nitrosative stress (nitrite and nitrate) were also analyzed. LMW-DS administration significantly decreased the nitrate concentrations in both the acute and chronic phases of sTBI (Fig. 5).

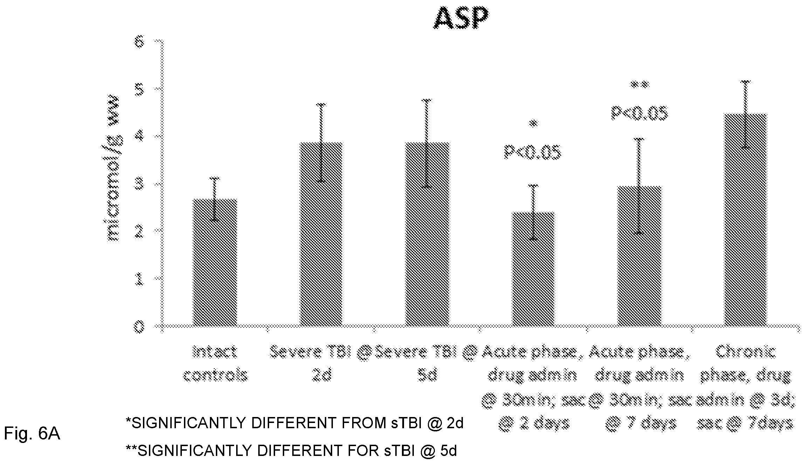

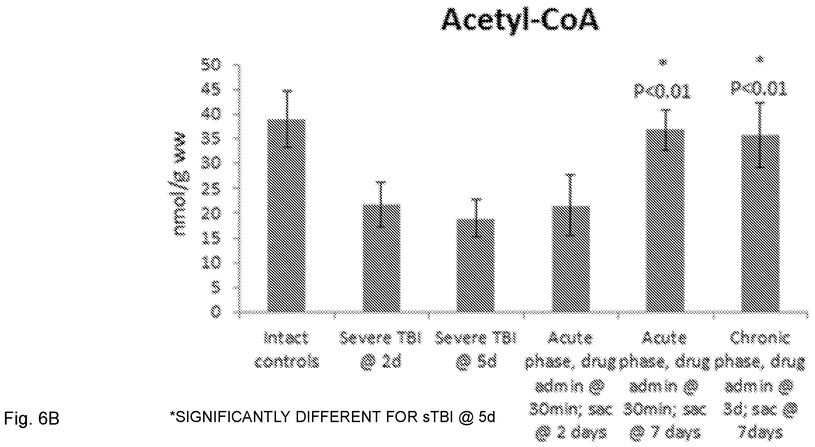

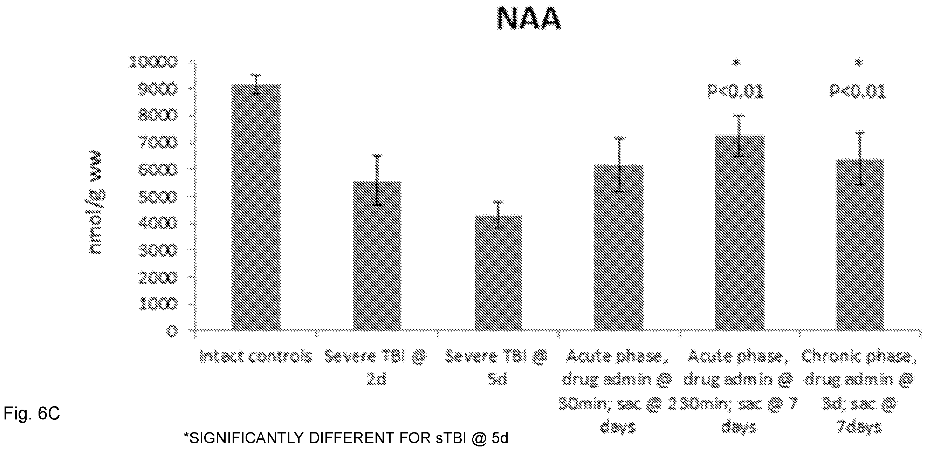

NAA is a brain specific metabolite and a valuable biochemical marker for monitoring deterioration or recovery after TBI. NAA is synthesized in neurons from aspartate and acetyl-CoA by aspartate N-acetyltransferase. To ensure NAA turnover, the molecule must move between cellular compartments to reach oligodendrocytes where it is degraded into acetate and aspartate by aspartoacylase (ASPA). An upregulation of the catabolic enzyme ASPA and an NAA decrease in order to supply the availability of the substrates aspartate and acetyl-CoA are an indication of the status of metabolic impairment. In this study NAA and its substrates were measured after sTBI and showed significant improvements in levels after LMW-DS administration (Figs. 6A-6C).

These effects on energy metabolites were particularly evident when animals received the LMW-DS administration early post-injury (30 mins). It is important to note that the overall beneficial effects of LMW-DS were observed either when the animals were sacrificed 2 days after sTBI or when sacrifice occurred 7 days post sTBI. In these groups of animals, the general amelioration of metabolism connected to AGCC and energy metabolites was more evident, suggesting a long-lasting positive effect of the LMW-DS administration on brain metabolism.

DISCUSSION

TBI is the leading cause of death and disability in the first four decades of life. The cost to the UK economy alone is estimated to be £8 billion per year, for comparison this is a greater cost to the economy than stroke. In the USA, the combined healthcare and socioeconomic costs of TBI are estimated to exceed $60 billion per year, not including military expenditure. In addition, the last few years have seen a massive surge of interest in sport concussion on both sides of the Atlantic.

Despite the obvious clinical need, there are currently no approved pharmacological treatments for TBI. Whilst the primary insult (contusion) associated with TBI may be amenable to surgical treatment, reduction in the subsequent secondary non-mechanical damage of surrounding brain tissue (penumbra) offers greater potential therapeutic opportunities.

Using a well-established rodent model of severe traumatic brain injury (sTBI), characterized by diffuse axonal damage of TBI, it has previously been shown that severely injured animals have long-lasting modifications of various metabolites connected to the cell energy state and mitochondrial functions (Vagnozzi et al., J Neurotrauma. 1999; 16: 903-913; Signoretti et al., J Neurotrauma. 2001; 18: 977-993; Tavazzi et al., Neurosurgery. 2005; 56: 582-589; Vagnozzi et al., Neurosurgery. 2007; 61: 379-388; Tavazzi et al., Neurosurgery. 2007; 61: 390-395), as well as to amino acidic metabolism (Amorini et al., J Cell Mol Med. 2017; 21: 530-542). In the complex molecular mechanisms causing TBI-induced cerebral damages, it appears that metabolic modifications are early cellular signals that influence the changes in enzymatic activities and gene and protein expression indicative of the pathological tissue response (Di Pietro et al., Mol Cell Biochem. 2013; 375: 185-198; Di Pietro et al., Mol Med. 2014; 20: 147-157; Di Pietro et al., Free Radic Biol Med. 2014; 69: 258-264; Amorini et al., Biochim Biophys Acta Mol Basis of Dis. 2016; 1862: 679-687). This implies that agents that act to positively regulate cellular metabolism in the compromised tissues might decrease the subsequent TBI-associated modifications in enzyme activity and gene and protein expression that contribute to adverse outcomes.

The data presented herein suggests that early administration of LMW-DS reduced levels of glutamate excitotoxicity and ameliorated adverse changes in metabolic homeostasis by protecting mitochondrial function, indicating a neuroprotective effect of the compound after severe TBI. Accordingly, LMW-DS has a potential to be used in the treatment or inhibition of TBI, including STBI.

EXAMPLE 2

An analysis of changes in gene-expression induced by LMW-DS was investigated in cell lines.

MATERIALS AND METHODS

*Experimental design*

For each cell line, n=8 × 25 cm$^2$ culture flasks were set up. Two flasks were harvested for each cell type on the day of treatment (24 hours after seeding). This represents the Day0 time point. From the remaining flasks, three flasks were treated with Control Medium and three were treated with Culture Medium (CM) containing LMW-DS to give a final concentration of 0.01 mg/ml. Cells from the treated flasks were collected after 48 hours. Therefore the collected data represent (a) untreated cells (Day0 Controls and Day2 Controls) and (b) cells treated with LMW-DS for 48 hours (Day2 LMW-DS treated).

*Coating of tissue culture plates for all cells*

25 cm$^2$ flasks were coated by adding 2 ml per flask of a solution of 50 $\mu$g/ml poly-d-lysine in Hank's balanced salt solution (HBSS) and incubating overnight at 37°C in the dark. Flasks were washed with cell culture water and air-dried for 30 min in the dark. Flasks were coated by adding 1 ml per flask of a solution of 25 $\mu$g/ml laminin in phosphate-buffered saline (PBS) and incubating for 2 hour at 37°C in the dark. The laminin flasks were washed with PBS three times before plating cells.

*Human umbilical vein endothelial cells (HUVECs)*

Medium 200 + Large Vessel Endothelial Supplement (M200+LVES) additive (1:50) was prepared and pre-warmed to 37°C. Cells were thawed in a 37°C water bath for no longer than 2 min and gently transferred into a 50 ml tube containing 20 ml Dulbecco's Modified Eagle Medium, Nutrient Mixture F-12 (DMEM-F12). The cell suspension was mixed by inverting the tube carefully twice. Cells were spun at 400 $\times$ g for 10 minutes. Supernatant removed and cells were re-suspended in 10 ml of culture media (M200+LVES additive).
Cells were counted with the Cellometer. 1,000,000 cells/flask were seeded in 25 cm$^2$ flasks (n=8) and medium was topped up to a total of 5 ml per flask. Cells were incubated at 37°C with 5 % $CO_2$. Cells were allowed to settle for 24 hours before LMW-DS treatment.

*Human Schwann cells*

Schwann cells growth medium was prepared by adding 10 % of fetal bovine serum (FBS) to high-glucose DMEM and pre-warmed to 37°C. Cells were thawed in a 37°C water bath for no longer than 2 min.
Cells from 12 vials were each gently transferred to a tube containing 10 ml of high-glucose DMEM medium and centrifuged at 400 relative centrifugal field (RCF) for 10 min. Pellet was re-suspended in culture medium. The cells from the 12 vials were mixed and distributed equally into the previously coated 25 cm$^2$ flasks (n=8). Cells were incubated at 37°C with 5 % $CO_2$. Cells were allowed to settle for 24 hours before LMW-DS treatment.

*Mouse cortical neurons (Lonza)*

Medium was prepared by adding 10 ml B-27 Serum-Free Supplement and 2.5 ml GlutaMAX™-I Supplement to 500 ml of Neurobasal medium. The medium was pre-warmed to 37°C. Cells from 12 vials were thawed sequentially in a 37°C water bath for no longer than 2 min and gently transferred into a 15 ml tube. 9 ml of medium was gently added drop-wise to each. The cell suspension was mixed by inverting the tubes carefully twice.
The cells were centrifuged for 5 minutes at 200 $\times$ g. Supernatant was removed (to the last 0.5 ml) and cells were gently re-suspended by trituration. The cells from the 12 vials were mixed and distributed equally into the previously coated 25 cm$^2$ flasks (n=8). Cells were incubated at 37°C with 5 % $CO_2$ for 24 hours.

*Mouse Motor neurons (Aruna)*

The culture medium was prepared according to Table 5.

Table 5 - Preparation of culture medium

| Component | Stock concentration | Final concentration | For 50 ml |
|---|---|---|---|
| Advanced DMEM/F12 | | | 25 ml |
| AB2™ Basal Neural Medium | | | 25 ml |
| Knockout Serum Replacement | | | 5 ml |
| L-Glutamate | 100 X | 1 X | 0.5 ml |
| Penicillin/Streptomycin | 100 X | 1 X | 0.5 ml |

(continued)

| Component | Stock concentration | Final concentration | For 50 ml |
|---|---|---|---|
| B-mercaptoethanol | 1 M (diluted in PBS) | 0.1 mM | 5 μl |
| Glial cell-derived neurotrophic factor (GDNF) | 100 μg/ml in $H_2O$ | 10 ng/ml | 5 μl |
| Ciliary neurotrophic factor (CNTF) | 100 μg/ml in PBS with 0.1 % BSA | 10 ng/ml | 5 μl |

Medium (see Table 5) was pre-warmed to 37°C. Cells were thawed in a 37°C water bath for no longer than 2 min. 9 ml of media was gently added drop-wise. The cell suspension was mixed by inverting the tube carefully twice. The cells were counted with a Cellometer. The cells were centrifuged for 5 minutes at $200 \times g$. Supernatant was removed (to the last 0.5 ml) and cells were gently re-suspended by trituration. The cells from the 8 vials were mixed and distributed equally into the previously coated 25 cm² flasks (n=8). Cells were incubated at 37°C with 5 % $CO_2$ for 24 hours before treatment.

*Drug treatment*

LMW-DS was provided at a stock concentration of 20 mg/ml and was kept in a temperature monitored refrigerator at 4°C. A fresh 100X LMW-DS stock (1.0 mg/ml) was prepared in sterile DMEM-F12. The concentrated drug stock was sterile filtered and added to the respective culture media (19.6 ml CM and 0.4 ml LMW-DS stock solution). The Control was made using 19.6 ml CM and 0.4 ml of DMEM-F12. LMW-DS and CM were added to the respective flasks (5 ml each) to reach the 0.01 mg/ml concentration of LMW-DS in each dish with a total of 10 ml CM each.

*Culture collection and cell lysis*

CM was aspirated into a clean and labelled 15 ml Falcon tube. The flasks (without culture medium) were placed into the -80°C freezer for 30 minutes. The CM in the Falcon tubes was spun at $3000 \times g$ for 5 minutes. Supernatant was removed and the small pellet was re-suspended in 2.5 ml Trizol:Water (4:1) solution at room temperature (RT, ~22°C).
The frozen flasks were removed one-by one from the freezer and the Trizol-Water from the appropriate tubes was moved to the flask. Flasks were left at RT for 5 minutes before the content was aspirated back into the 15 ml Falcon tube (after washing the bottom of the flask with the solution thoroughly). The flasks were inspected under the microscope to ensure full removal of cells. The collected lysates in the 15 ml Falcon tubes were placed into the -80°C freezer.

*RNA extraction*

Falcon tubes containing the homogenates were removed from the freezer and stored for 5 minutes at RT to permit the complete dissociation of nucleoprotein complexes.
Two aliquots of 1 ml lysate were removed from each sample and 200 μl of chloroform was added to each (0.2 ml of chloroform per 1 ml of TRIzol Reagent used during the cell lysis step) and the tube was shaken vigorously. Samples were stored at RT for 2-3 minutes and subsequently centrifuged at $12,000 \times g$ for 15 minutes at 4°C.
The mixture separated into three layers: a lower red phenol-chloroform phase, an interphase and a colorless upper aqueous phase. The RNA remained in the top aqueous phase, DNA in the white middle (interphase) phase and protein in the pink bottom (organic) phase. The top ¾ of the aqueous phase was transferred to a new clean Eppendorf tube.
The RNA was precipitated from the aqueous phase by adding an equal amount of 100 % ethanol. The precipitated RNA was fixed onto a Spin Cartridge, washed twice and dried. The RNA was eluted in 50 μl warm RNase-Free Water. The amount and quality of the purified RNA was measured by Nanodrop. The RNA was stored at -80°C before transfer to Source Bioscience for Array analysis.

*Analysis plan for expression data*

The expression data were downloaded into separate files for each cell line. The 'Background corrected' expression is the data from the "gProcessedSignal" of the arrays that is the result of the background signal extracted from the actual signal of the relevant probe. This is the most often used variable in array analysis. The background corrected signal was log2 transformed for all samples for statistical analysis. To reduce the false discovery rate in the samples, the signals that were below 'expression level' were removed. The 'below expression' level was set at 5 for the log2 transformed expression values.

*Statistical analysis*

Based on the expression pattern of the Control probes on each array it was decided to carry out Median Centering for all arrays before analysis to reduce the variability of the results. Data were grouped by cell type and each cell type was analyzed using the following algorithms:

- Comparison of D0 control to D2 control samples - expression changes seen in the cells in normal cultures
- Comparison of D0 control to D2 LMW-DS treated samples - expression changes seen in the cells in the LMD-DS treated cultures
- Comparison of D2 control to D2 LMD-DS treated samples - differential expression induced by LMW-DS in the culture.

A preliminary analysis was carried out to screen out genes that were not differentially expressed between any combination of the three datasets. Simple, non-stringent ANOVA (p<0.05) was carried out to look for patterns of expression. Probes with no changes across the three datasets were eliminated. The remaining probe sets were analyzed for fold change and significance using Volcano plots. More than 20 % change in the expression of a probe (fold change (FC) $\geq 1.2$ or FC $\leq 0.84$) was regarded as significant in the first instance to allow the detection of expression patterns.

*Quality parameters*

Seeding densities were calculated from the cell counts retrieved from the cell stocks for the Schwann cells. The HUVECS were seeded at their optimum density.

The additional quality control from the Array service provider indicated that the RNA was high quality (no degradation) and the amounts were within the parameters of the Low input RNA microarray from Agilent.

The analysis of the raw data indicated that, as expected, there were significant differences between arrays. These differences (reflected by differences in the same control samples included on all arrays), were, however, easily eliminated by normalization techniques. The chosen median centering of the data that eliminates the array-to-array variation did not affect the overall differences expected to be seen between the controls representing different concentrations of RNA.

*Expression analysis of Schwann cells*

As described in the foregoing, genes not expressed in the Schwann cells were removed prior to data analysis. The 'below expression' level was set at 5 for the log2 transformed expression values. This left 15,842 unique probes to analyze in the Schwann cell cultures. In the next step of the analysis, three sets of data (comparison of D0 control to D2 control samples; comparison of D0 control to D2 LMW-DS treated samples; comparison of D2 control to D2 LMD-DS treated samples) were analyzed to establish the effect of the CM on the cells and the relative changes induced by LMW-DS.

585 genes were differentially expressed in Schwann cell cultures when comparing the D0 control to the D2 control samples. The molecular functions influenced by these genes relate to cellular movement (1.14E-07-2.49E-03); cell morphology (5.56E-07-2.36E-03); cellular development (7.3E-06-2.48E-03); cellular growth and proliferation (7.3E-06-2.48E-03); cellular assembly and organization (1.23E-05-2.36E-03); cellular function and maintenance (1.23E-05-2.47E-03); cell death and survival (1.53E-05-2.51E-03); lipid metabolism (8.14E-05-1.6E-03); small molecule biochemistry (8.14E-05-1.6E-03); molecular transport (1.18E-04-2.29E-03); protein trafficking (1.62E-04-1.6E-03); carbohydrate metabolism (3.22E-04-1.78E-03); gene expression (3.98E-04-2.2E-03); cell signaling (4.39E-04-2.25E-03); cell-to-cell signaling and interaction (5.05E-04-2.48E-03); cellular compromise (7.69E-04-1.58E-03); cell Cycle (1.12E-03-1.8E-03); amino acid metabolism (1.6E-03-1.6E-03); and nucleic acid metabolism (1.6E-03-1.6E-03).

The values presented above are p-values representing the statistical significance of the association of these genes with the different pathways. The two p values represent the lower and upper limits of the statistical significance observed (p<0.05 is significant).

LMW-DS induced differential expression in Schwann cell culture of 1244 genes as assessed when comparing the D0 control to the D2 LMW-DS treated samples. The molecular functions influenced by these genes relate to cell morphology (1.43E-08-8.39E-04); cellular movement (1.4E-07-9.6E-04); post-translational modification (3.93E-07-6.71E-05); protein synthesis (3.93E-07-1.08E-04); protein trafficking (3.93E-07-1.26E-06); cell death and survival (2.13E-06-8.65E-04); cellular assembly and organization (7.46E-06-8.24E-04); DNA replication, recombination, and repair (7.46E-06-7.46E-06); cellular function and maintenance (9.53E-06-6.46E-04); gene expression (1.27E-05-4.92E-04); cellular development (1.29E-05-9.06E-04); cellular growth and proliferation (1.29E-05-9.06E-04); cell-to-cell signaling and interaction (1.97E-05-8.81E-04); amino acid metabolism (4.22E-05-8.24E-04); small molecule biochemistry (4.22E-05-8.24E-04); lipid metabolism (4.81E-05-3.64E-04); molecular transport (3.64E-04-3.64E-04); and cell cycle (4.53E-04-4.86E-04).

LMW-DS induced differential expression in Schwann cell culture of 700 genes as assessed when comparing the D2 control to the D2 LMW-DS treated samples. The molecular functions influenced by these genes relate to cell morphology (1.49E-07-5.62E-03); cellular assembly and organization (1.49E-07-5.95E-03); cellular movement (7.24E-07-6.06E-03); cell death and survival (9.41E-06-5.95E-03); amino acid metabolism (2.56E-05-3.7E-03); post-translational modification (2.56E-05-1.05E-03); small molecule biochemistry (2.56E-05-3.7E-03); cell-to-cell signaling and interaction (5.05E-05-5.76E-03); gene expression (7.18E-05-4.94E-03); cell cycle (1.06E-04-5.95E-03); cellular development (1.06E-04-5.95E-03); cellular function and maintenance (1.96E-04-5.95E-03); cellular growth and proliferation (2.35E-04-5.95E-03); DNA replication, recombination and repair (2.75E-04-5.95E-03); cell signaling (5.92E-04-2.54E-03); cellular comprise (6.26E-04-6.26E-04); lipid metabolism (6.26E-04-1.85E-03); molecular transport (6.26E-04-5.95E-03); protein synthesis (1.05E-03-1.93E-03); cellular response to therapeutics (1.85E-03-1.85E-03); protein trafficking (2.66E-03-5.95E-03); and RNA post-transcriptional modification (4.32E-03-4.32E-03).

The mechanistic molecular network model simulates the effect of the differentially regulated molecules by LMW-DS enabling the functional consequences of these changes to be evaluated. The *in silico* model indicated that LMW-DS inhibits neuronal cell death; apoptosis; and synthesis of protein and activates angiogenesis; migration of cells; cell viability; cell survival; cell movement; proliferation of cells; differentiation of cells; cellular homeostasis; cell cycle progression; cell transformation; and expression of RNA.

Table 6 summarizes the results of the gene expression changes in the cultured Schwann cells.

Table 6 - Overall pattern of gene expression changes in Schwann cells

| | abolished nutrient effect | enhanced response to nutrients | new effect induced by LMW-DS | not different from control | total |
|---|---|---|---|---|---|
| no effect | 21 | | | | 21 |
| significant downregulation | 1 | 122 | 352 | 42 | 517 |
| significant upregulation | 13 | 441 | 74 | 373 | 901 |
| total | 35 | 563 | 426 | 415 | 1439 |

21 genes that have altered expression in the Control cultures in the two days did not show any changes at all in the LMW-DS treated cultures during the same two days. 1 gene that had increased expression in the control cultures was downregulated in the LMW-DS treated cultures during the same two days. 13 genes that were downregulated in the control cultures were upregulated in the LMW-DS treated cultures during the two days. 122 genes were significantly downregulated by growth factors in the culture medium and this downregulation was even stronger in the LMW-DS treated cultures. 441 genes were upregulated in the Control cultures and the addition of LMW-DS made this upregulation significantly stronger.

*Expression analysis of HUVECs*

As described in the foregoing, genes that are not expressed in the HUVECs have been removed before attempting any analysis. The 'below expression' level was set at 5 for the log2 transformed expression values. This left 15,239 unique probes to analyze in HUVEC cultures. In the next step, the three sets of data were analyzed to establish the effect of the CM on gene expression in the cells and the differences induced by LMW-DS. A preliminary analysis was carried out to screen out genes that were not differentially expressed between any combination of the three datasets. Simple, non-stringent ANOVA ($p < 0.05$) was carried out to look for patterns of expression. Genes with no changes across the three datasets were eliminated, leaving a total of 12,313 probes (10,368 genes) to analyze.

1551 genes were differentially expressed in HUVEC cultures when comparing the D0 control to the D2 control samples. The molecular functions influenced by these genes relate to cellular assembly and organization (2.55E-15-1.29E-03); cellular function and maintenance (2.55E-15-1.29E-03); cell cycle (1.98E-11-1.32E-03); cell morphology (3.18E-10-1.29E-03); gene expression (1.05E-08-2.01E-04); cellular development (1.66E-07-1.37E-03); cellular growth and proliferation (1.66E-07-1.37E-03); DNA replication, recombination, and repair (2.04E-07-9.84E-04); cell death and survival (2.09E-07-1.3E-03); RNA post-transcriptional modification (4.86E-06-6.53E-04); cellular movement (9.9E-06-1.18E-03); post-translational modification (1.92E-05-1.34E-03); cell-to-cell signaling and interaction (2.19E-05-9.1E-04); protein synthesis (5.49E-05-1.14E-03); cellular compromise (8.16E-05-8.16E-05); molecular transport (6.27E-04-6.27E-04); protein trafficking (6.27E-04-6.27E-04); cell signaling (8.86E-04-8.86E-04); cellular response to therapeutics (9.84E-04-9.84E-04); and protein degradation (1.14E-03-1.14E-03).

LMW-DS induced differential expression in HUVEC culture of 1779 genes as assessed when comparing the D0 control to the D2 LMW-DS treated samples. The molecular functions influenced by these genes relate to cellular assembly and organization (4.14E-17-9.7E-04); cellular function and maintenance (4.14E-17-8.05E-04); cell cycle (5.83E-14-9.85E-04); cell morphology (1.69E-10-7.48E-04); gene expression (7.99E-09-8.62E-04); cell death and survival (2E-08-8.4E-04); cellular development (1.28E-07-8.88E-04); cellular growth and proliferation (1.28E-07-8.88E-04); DNA replication, recombination, and repair (3.07E-07-9.7E-04); RNA post-transcriptional modification (1.13E-06-6.31E-04); cellular movement (1.42E-06-8.34E-04); post-translational modification (3.4E-05-9.17E-04); cell-to-cell signaling and interaction (6.97E-05-9.56E-04); molecular transport (7.43E-05-9.7E-04); protein trafficking (7.43E-05-7.43E-05); RNA trafficking (1.57E-04-5.72E-04); protein synthesis (1.92E-04-9.02E-04); cellular compromise (2.47E-04-6.28E-04); and cell signaling (4.64E-04-9.02E-04).

LMW-DS induced differential expression in HUVEC culture of 76 genes as assessed when comparing the D2 control to the D2 LMW-DS treated samples. The molecular functions influenced by these genes relate to DNA replication, recombination, and repair (9.62E-05-2.57E-02); cell cycle (1.22E-04-2.4E-02); cellular development (1.59E-04-2.67E-02); cell morphology (4.64E-04-2.42E-02); cellular function and maintenance (4.64E-04-2.57E-02); lipid metabolism (9.49E-04-1.07E-02); molecular transport (9.49E-04-1.61E-02); small molecule biochemistry (9.49E-04-1.87E-02); cellular compromise (1.6E-03-2.62E-02); cell death and survival (2.06E-03-2.67E-02); amino acid metabolism (2.7E-03-2.7E-03); carbohydrate metabolism (2.7E-03-1.07E-02); cell-to-cell signaling and interaction (2.7E-03-2.4E-02); cellular assembly and organization (2.7E-03-2.57E-02); cellular growth and proliferation (2.7E-03-2.4E-02); cellular movement (2.7E-03-2.4E-02); energy production (2.7E-03-2.7E-03); nucleic acid metabolism (2.7E-03-1.07E-02); post-translational modification (2.7E-03-1.61E-02); gene expression (5.39E-03-2.36E-02); RNA post-transcriptional modification (5.39E-03-2.4E-02); drug metabolism (8.07E-03-1.61E-02); vitamin and mineral metabolism (8.07E-03-8.07E-03); protein synthesis (1.07E-02-1.07E-02); RNA trafficking (1.07E-02-1.07E-02); cellular response to therapeutics (1.24E-02-1.24E-02); and free radical scavenging (1.43E-02-1.43E-02).

Although the overall difference between Control and LMW-DS-treated cultures after 2 days of treatment at first hand does not appear to be large, the effects of LMW-DS on gene expression changes were significant, in particular when considering the modulation of growth factor induced gene expression by LMW-DS.

Using the mechanistic molecular network model it is possible to simulate the effect of the genes differentially regulated by LMW-DS to look for the functional consequences of these changes. The *in silico* model indicated that LMW-DS inhibits neuronal cell death; apoptosis; and synthesis of protein and activates angiogenesis; migration of cells; cell viability; cell survival; cell movement; proliferation of cells; differentiation of cells; cellular homeostasis; cell cycle progression; cell transformation; and expression of RNA.

The HUVEC control cultures comprise growth factors. In the treated cultures, LMW-DS was added to the culture medium that already contained growth factors.

Table 7 summarizes the results of the gene expression changes in the cultured HUVECs. 67 genes that have altered expression in the Control cultures in the two days (under the effect of the growth factors) did not show any changes at all in the LMW-DS treated cultures during the same two days. 4 genes that had increased expression in the control cultures with the growth factors were downregulated in the LMW-DS treated cultures during the same two days. 11 genes that were downregulated by the growth factors in the control cultures were upregulated in the LMW-DS treated cultures during the two days. 120 genes were significantly downregulated by growth factors and this downregulation was even stronger in the LMW-DS treated cultures. 229 genes were upregulated in the Control cultures and the addition of LMW-DS made this upregulation significantly stronger.

Table 7 - Overall pattern of gene expression changes in HUVECs

|  | abolished nutrient effect | enhanced response to nutrients | not different from control | total |
|---|---|---|---|---|
| no effect | 67 |  |  | 67 |
| significant downregulation | 4 | 120 | 167 | 291 |
| significant upregulation | 11 | 229 | 1326 | 1566 |
| total | 82 | 349 | 1493 | 1924 |

The effect of LMW-DS on several molecular pathways that are important for different disease conditions and therapeutic applications were analyzed. For the analysis, the effects of adding LMW-DS on gene expression was compared to that seen in cells in CM and the functional effects were predicted based on the observed changes in the expression patterns.

*Expression analysis of motor neurons*

As described in the foregoing, genes that are not expressed in the motor neurons have been removed before attempting any analysis. The 'below expression' level was set at 5 for the log2 transformed expression values. This left 12,240 unique probes where the expression threshold was met by at least three samples in the series. In the next step, the three sets of data were analyzed to establish the effect of the CM on the cells and the differences induced by the LMW-DS.

The changes in gene expression under normal culture conditions mimic the normal developmental processes of the motor neurons, when from a dissociated set of cells they develop a motor neuron phenotype. The growth factors in the normal culture medium are those necessary for these cells to differentiate. The stress factor present in these cultures is the oxidative stress (normal for tissue culture conditions).

485 genes were differentially expressed in motor neuron cultures when comparing the D0 control to the D2 control samples. The molecular functions influenced by these genes relate to cell death and survival (1.99E-17-1.98E-04); cellular movement (1.14E-16-1.91E-04); cellular assembly and organization (1.22E-16-1.93E-04); cellular function and maintenance (1.22E-16-1.95E-04); cell morphology (6.46E-16-1.74E-04); cell-to-cell signaling and interaction (3.16E-12-1.95E-04); cellular development (1.59E-10-1.93E-04); cellular growth and proliferation (1.59E-10-1.9E-04); molecular transport (4.27E-10-1.89E-04); protein synthesis (9.85E-09-5.03E-05); lipid metabolism (1.08E-08-1.61E-04); small molecule biochemistry (1.08E-08-1.89E-04); gene expression (8.45E-08-3.8E-05); cell cycle (4.55E-07-1.09E-04); free radical scavenging (7.12E-07-1.65E-04); cell signaling (1.23E-05-1.89E-04); vitamin and mineral metabolism (1.23E-05-1.89E-04); protein degradation (3.07E-05-1.31E-04); carbohydrate metabolism (3.32E-05-1.61E-04); drug metabolism (4.16E-05-4.16E-05); post-translational modification (7.1E-05-1.31E-04); and protein folding (7.1E-05-7.1E-05).

LMW-DS induced differential expression in motor neurons of 315 genes as assessed when comparing the D0 control to the D2 LMW-DS treated samples. The molecular functions influenced by these genes relate to cell death and survival (6.54E-08-9.06E-03), cellular movement (8.21E-08-5.42E-03); cellular assembly and organization (8.36E-08-9.01E-03); cellular function and maintenance (8.36E-08-9.01E-03); cell morphology (2.9E-06-8.75E-03); cellular development (1.04E-05-9.01E-03); cellular growth and proliferation (1.04E-05-7.83E-03); DNA replication, recombination, and repair (2.79E-05-8.01E-03); cell-to-cell signaling and interaction (8.18E-05-7.11E-03); post-translational modification (1.32E-04-7.56E-03); protein degradation (1.32E-04-4.35E-03); protein synthesis (1.32E-04-5.09E-03); gene expression (1.9E-04-9.01E-03); cellular compromise (3.58E-04-9.01E-03); cell cycle (6.08E-04-9.01E-03); free radical scavenging (7.41E-04-7.31E-03); amino acid metabolism (7.67E-04-6.61E-03); small molecule biochemistry (7.67E-04-9.01E-03); vitamin and mineral metabolism (7.67E-04-1.13E-03); lipid metabolism (1.05E-03-9.01E-03); molecular transport (1.05E-03-9.01E-03); cell signaling (1.13E-03-5.09E-03); and carbohydrate metabolism (4.71E-03-4.71E-03).

LMW-DS induced differential expression in motor neurons of 425 genes as assessed when comparing the D0 control to the D2 LMW-DS treated samples. The molecular functions influenced by these genes relate to cell death and survival (2.87E-08-6.27E-03); cellular movement (4.73E-07-6.47E-03); cell morphology (4.95E-07-7.47E-03); cellular development (1.02E-06-7.13E-03); cellular growth and proliferation (1.02E-06-7.48E-03); cellular assembly and organization (7.03E-06-7.47E-03); cellular function and maintenance (7.03E-06-7.47E-03); gene expression (1.95E-05-6.18E-03); cell cycle (2.88E-05-7.48E-03); DNA replication, recombination, and repair (3.39E-05-5.16E-03); amino acid metabolism (7.75E-05-4.68E-03); small molecule biochemistry (7.75E-05-4.68E-03); cellular compromise (8.23E-05-4.61E-03); cell-to-cell signaling and interaction (3.27E-04-7.48E-03); vitamin and mineral metabolism (3.27E-04-3.27E-04); protein synthesis (8.94E-04-5.29E-03); post-translational modification (9.67E-04-9.67E-04); molecular transport (9.7E-04-4.68E-03); protein trafficking (9.7E-04-9.7E-04); carbohydrate metabolism (1.44E-03-1.92E-03); cellular response to therapeutics (1.92E-03-1.92E-03); and lipid metabolism (4.68E-03-4.68E-03).

Table 8 - Overall pattern of gene expression changes in motor neurons

|  | abolished nutrient effect | enhanced response to nutrients | new effect induced by LMW-DS | not different from control | total |
|---|---|---|---|---|---|
| no effect | 177 |  | 108 |  | 285 |
| significant downregulation | 47 | 36 | 375 | 104 | 562 |
| significant upregulation | 40 | 103 | 71 | 75 | 289 |
| total | 264 | 139 | 554 | 179 | 1136 |

*Expression analysis of cortical neurons*

As described in the foregoing, genes that are not expressed in the motor neurons have been removed before attempting any analysis. The 'below expression' level was set at 5 for the log2 transformed expression values. This left 10,653 unique probes where the expression threshold was met by at least three samples in the series. In the next step, the three sets of data were analyzed to establish the effect of the CM on the cells and the differences induced by the LMW-DS.

The changes in gene expression under normal culture conditions mimic the normal developmental processes of the cortical neurons, when from a dissociated set of cells they develop a cortical neuron phenotype. The growth factors in the normal culture medium are those necessary for these cells to differentiate. The stress factor present in these cultures is the oxidative stress (normal for tissue culture conditions).

1101 genes were differentially expressed in motor neuron cultures when comparing the D0 control to the D2 control samples. The molecular functions influenced by these genes relate to cellular assembly and organization (3.57E-25-6.65E-04); cellular function and maintenance (3.57E-25-6.65E-04); cell morphology (4.28E-22-6.36E-04); cellular development (4.28E-22-6.53E-04); cellular growth and proliferation (4.28E-22-6.6E-04); cell-to-cell signaling and interaction (2.16E-13-6.65E-04); molecular transport (5.18E-12-4.95E-04); cellular movement (1.86E-11-6.65E-04); cell death and survival (3.37E-11-6.41E-04); gene expression (1.27E-08-8.96E-05); protein synthesis (3.84E-07-8.69E-05); small molecule biochemistry (6.65E-07-5.18E-04); cellular compromise (7.12E-06-4.54E-04); protein degradation (1.62E-05-1.62E-05); amino acid metabolism (2.11E-05-4.25E-04); protein trafficking (3.4E-05-3.4E-05); cell signaling (8.69E-05-3E-04); post-translational modification (8.69E-05-2.15E-04); protein folding (2.15E-04-2.15E-04); cell cycle (2.69E-04-3.07E-04); DNA replication, recombination, and repair (2.69E-04-4.77E-04); nucleic acid metabolism (2.69E-04-2.69E-04); lipid metabolism (3.12E-04-5.18E-04); and carbohydrate metabolism (5.18E-04-5.18E-04).

LMW-DS induced differential expression in motor neurons of 609 genes as assessed when comparing the D0 control to the D2 LMW-DS treated samples. The molecular functions influenced by these genes relate to cellular assembly and organization (3.91E-15-1.83E-03); cellular function and maintenance (3.91E-15-1.83E-03); cell morphology (2.53E-13-1.43E-03); cellular development (2.53E-13-1.81E-03); cellular growth and proliferation (2.53E-13-1.83E-03); cellular movement (4.95E-09-1.2E-03); cell-to-cell signaling and interaction (5.96E-09-1.47E-03); cell death and survival (2.25E-08-1.77E-03); molecular transport (7.08E-08-1.79E-03); DNA replication, recombination, and repair (3.03E-06-1.71E-03); cellular compromise (9.23E-06-7.65E-04); amino acid metabolism (1.75E-05-1.64E-03); cell cycle (1.75E-05-1.77E-03); small molecule biochemistry (1.75E-05-1.79E-03); protein synthesis (2.77E-05-1.5E-03); protein trafficking (2.77E-05-1.9E-04); cell signaling (7.65E-05-1.73E-03); post-translational modification (3.01E-04-1.4E-03); gene expression (3.65E-04-1.15E-03); drug metabolism (6.49E-04-6.49E-04); carbohydrate metabolism (6.95E-04-7.69E-04); vitamin and mineral metabolism (1.09E-03-1.09E-03); and nucleic acid metabolism (1.44E-03-1.73E-03).

LMW-DS induced differential expression in motor neurons of 247 genes as assessed when comparing the D0 control to the D2 LMW-DS treated samples. The molecular functions influenced by these genes relate to cell morphology (6.01E-08-1.01E-02); cellular development (7.46E-08-1.01E-02); cellular growth and proliferation (7.46E-08-1.01E-02); cell death and survival (4.23E-07-1.01E-02); cellular movement (2.69E-06-9.91E-03); cellular assembly and organization (1.57E-05-1.01E-02); cellular function and maintenance (1.57E-05-1.01E-02); cell cycle (1.01E-04-1.01E-02); cell-to-cell signaling and interaction (1.01E-04-1.01E-02); lipid metabolism (1.56E-04-1.01E-02); small molecule biochemistry (1.56E-04-1.01E-02); gene expression (2.28E-04-3.38E-03); RNA damage and repair (2.28E-04-2.28E-04); RNA post-transcriptional modification (2.28E-04-2.28E-04); molecular transport (4.18E-04-8.32E-03); cellular compromise (4.47E-04-2.2E-03); protein synthesis (2.66E-03-7.29E-03); protein trafficking (4.11E-03-8.32E-03); protein degradation (5.64E-03-7.29E-03); and DNA replication, recombination, and repair (7.31E-03-1.01E-02).

Table 9 - Overall pattern of gene expression changes in cortical neurons

|  | abolished nutrient effect | enhanced response to nutrients | new effect induced by LMW-DS | not different from control | total |
|---|---|---|---|---|---|
| no effect | 572 |  | 19 |  | 591 |
| significant downregulation | 7 | 158 | 22 | 95 | 282 |
| significant upregulation | 33 | 43 | 7 | 221 | 304 |
| total | 612 | 612 | 48 | 316 | 1177 |

*The effect of LMW-DS on oxidative stress pathways in mitochondria*

The oxidative stress pathways occurring in mitochondria are important not just for cancer but also for ageing and age-related degenerative diseases. Normal growth conditions trigger a certain amount of oxidative stress in cells, which contributes to both the *in vivo* and the *in vitro* ageing process.

In Schwann cells cultured in normal conditions, Complex I (NADH dehydrogenase) was inhibited while Complex IV (cytochrome c oxidase) was activated. When LMW-DS was added to the cultures Complex III (cytochrome bc1) was inhibited. The inhibition of Complex III inhibits the oxidative stress phenomena that are involved in the pathogenesis of cancer and neurological diseases.

Complex III, sometimes referred to as coenzyme Q : cytochrome c - oxidoreductase or the cytochrome bc1 complex, is the third complex in the electron transport chain (EC 1.10.2.2), playing a critical role in biochemical generation of ATP (oxidative phosphorylation). Complex III is a multi-subunit transmembrane protein encoded by both the mitochondrial (cytochrome b) and the nuclear genomes (all other subunits). Complex III is present in the mitochondria of all animals and all aerobic eukaryotes and the inner membranes of most eubacteria. Mutations in Complex III cause exercise intolerance as well as multisystem disorders. The bc1 complex contains 11 subunits, 3 respiratory subunits (cytochrome B, cytochrome C1, Rieske protein), 2 core proteins and 6 low-molecular weight proteins.

In HUVECs no significant modulation of the effects of oxidative stress on mitochondria was detected following treatment with LMW-DS.

In normal culture conditions the motor neurons appear to suffer from significant oxidative stress. This leads to the activation of some apoptotic mechanisms and involving activation of cytochrome C, AIF, Caspase 3, 8 and 9. In addition, the motor neurons are characterized by production of amyloid-$\beta$ in the cells further exacerbating oxidative stress and mitochondrial fragmentation, via FIAS1, as well as the oxidation of fatty acids. Furthermore, Complex V was activated.

The addition of LMW-DS to the cultures ameliorated these negative effects by preventing and inhibiting apoptosis by preventing amyloid-$\beta$ production and its negative effects on mitochondrial fragmentation and dysfunction and subsequent damage and by inhibiting fatty acid oxidation. LMD-DS also inhibited the reaction path involving TRAK1 and PINK1, thereby contributing to improved mitochondrial function. LMW-DS further reduced the level of $H_2O_2$. A further effect was the inhibition of HtrA2 contributing to inhibition of apoptosis.

In normal culture conditions the cortical neurons are exposed to significant oxidative stress leading to the production of amyloid-$\beta$ and Lewy body formation and involving activation of Synuclein $\alpha$ and increased levels of ROS; apoptosis; mitochondrial fragmentation; and reduction of mitochondrial function and involving C161. The addition of LMW-DS to the cultures was able to prevent and reverse most of these deleterious effects, such as the accumulation of the amyloid-$\beta$ and Lewy body pathology, mitochondrial dysfunction. Some apoptosis inducing mechanisms remain active probably due to strong activation in the cultures.

*The effect of LMW-DS on glutamate excitotoxicity*

Glutamate is an essential excitatory amino acid involved in long-term potentiation (LTP), i.e., learning and memory functions. However, too much glutamate is also associated with excitotoxicity, leading to neuronal death. This later phenomenon is hypothesized to be involved in the neuronal death triggered in chronic neurodegenerative conditions but also in TBI. The genes involved in glutamate signaling are not expressed in HUVECs but are present in the Schwann and neuron cell lines used in this study.

Glutamate production was inhibited by the baseline conditions in the motor neuron cultures. The inhibition was not affected by LMW-DS. Glutamate production was elevated in the cortical neurons at baseline. The addition of LMW-DS did not alter the glutamate production in these cells.

The addition of LMW-DS to the CM of the Schwan cells induced the expression of a protein complex (CALM, G$\beta\gamma$, GRM7, PICK1). More importantly, LMW-DS increased activity and/or levels of glutamate transporters in the Schwann cells, and in particular of SLC1A2/3, thereby leading to a scavenging of glutamate produced by and released from the presynaptic neuron. Accordingly, LMW-DS induced the Schwann cells to remove the toxic glutamate from the synaptic cleft, thereby preventing it from exerting its excitotoxicity.

SLC1A3, solute carrier family 1 (glial high-affinity glutamate transporter), member 3, is a protein that, in humans, is encoded by the SLC1A3 gene. SLC1A3 is also often called the GLutamate ASpartate Transporter (GLAST) or Excitatory Amino Acid Transporter 1 (EAAT1). SLC1A3 is predominantly expressed in the plasma membrane, allowing it to remove glutamate from the extracellular space. It has also been localized in the inner mitochondrial membrane as part of the malate-aspartate shuttle. SLC1A3 functions *in vivo* as a homotrimer. SLC1A3 mediates the transport of glutamic and aspartic acid with the cotransport of three Na+ and one H+ cations and counter transport of one K+ cation. This co-transport coupling (or symport) allows the transport of glutamate into cells against a concentration gradient. SLC1A3 is expressed throughout the CNS, and is highly expressed in astrocytes and Bergmann glia in the cerebellum. In the retina, SLC1A3 is expressed in Muller cells. SLC1A3 is also expressed in a number of other tissues including cardiac myocytes.

SLC1A2, solute carrier family 1 member 2, also known as excitatory amino acid transporter 2 (EAAT2) and glutamate transporter 1 (GLT-1), is a protein that in humans is encoded by the SLC1A2 gene. SLC1A2 is a member of a family of the solute carrier family of proteins. The membrane-bound protein is the principal transporter that clears the excitatory neurotransmitter glutamate from the extracellular space at synapses in the CNS. Glutamate clearance is necessary for proper synaptic activation and to prevent neuronal damage from excessive activation of glutamate receptors. SLC1A2 is responsible for over 90 % of glutamate reuptake within the brain.

These findings indicate that LMW-DS may be useful for the prevention of glutamate excitotoxicity in conditions where its high extracellular levels are harmful, like after TBI.

*The effect of LMW-DS on cell adhesion*

One of the strong noticeable phenotypic effects of LMW-DS was the effect on cell adhesion, which was cell type specific. Cell adhesion was affected in neurons most strongly, then in Schwann cells, while HUVECs were not affected.

The analysis of gene expression indicated that this is due to the effect of LMW-DS on the expression of enzymes that regulate cell attachment including metallopeptidases, also referred to as matrix metalloproteinases (MMPs), see Table 10.

The aggregate effect of these molecules on the pathways regulating cell movement and attachment in Schwann cells (17 molecules, see Table 10) was such that cell adhesion would be inhibited while cell movement would be activated, while in HUVECs (1 molecule, ADAM11) adhesion would not be affected but angiogenesis would be activated.

Table 10 - Molecules of the pathway regulating cell movement and attachment in Schwann cells

| Symbol | Entrez gene name | Location | Type(s) |
|---|---|---|---|
| A2M | alpha-2-macroglobulin | Extracellular Space | transporter |
| ADAM10 | ADAM metallopeptidase domain 10 | Plasma Membrane | peptidase |
| ADAM23 | ADAM metallopeptidase domain 23 | Plasma Membrane | peptidase |
| ADAMTS9 | ADAM metallopeptidase with thrombospondin type 1 motif 9 | Extracellular Space | peptidase |
| CDH11 | cadherin 11 | Plasma Membrane | other |
| CSF3 | colony stimulating factor 3 | Extracellular Space | cytokine |
| FAS | Fas cell surface death receptor | Plasma Membrane | transmembrane receptor |
| HIF1A | hypoxia inducible factor 1 alpha subunit | Nucleus | transcription regulator |
| IL6 | interleukin 6 | Extracellular Space | cytokine |
| IL15 | interleukin 15 | Extracellular Space | cytokine |
| LUM | lumican | Extracellular Space | other |
| MMP3 | matrix metallopeptidase 3 | Extracellular Space | peptidase |
| POSTN | periostin | Extracellular Space | other |
| RECK | reversion inducing cysteine rich protein with kazal motifs | Plasma Membrane | other |
| SERPINA3 | serpin family A member 3 | Extracellular Space | other |
| TNC | tenascin C | Extracellular Space | other |
| VCAM1 | vascular cell adhesion molecule 1 | Plasma Membrane | transmembrane receptor |

The effect of differential gene expression induced by LMW-DS in neurons was analyzed. In the motor neurons the same metallopeptidase-dependent pathways could be responsible for the cell detachment seen in the Schwann cells, see Table 11.

Table 11 - Molecules of the pathway regulating cell movement and attachment in motor neurons

| Symbol | Entrez Gene Name | Location | Type(s) |
|---|---|---|---|
| ADAM11 | ADAM metallopeptidase domain 11 | Plasma Membrane | peptidase |

(continued)

| Symbol | Entrez Gene Name | Location | Type(s) |
|---|---|---|---|
| ADAM19 | ADAM metallopeptidase domain 19 | Plasma Membrane | peptidase |
| ADAMTS7 | ADAM metallopeptidase with thrombospondin type 1 motif 7 | Extracellular Space | peptidase |
| ADORA1 | adenosine A1 receptor | Plasma Membrane | G-protein coupled receptor |
| AGT | angiotensinogen | Extracellular Space | growth factor |
| APP | amyloid beta precursor protein | Plasma Membrane | other |
| CD44 | CD44 molecule (Indian blood group) | Plasma Membrane | other |
| F2R | coagulation factor II thrombin receptor | Plasma Membrane | G-protein coupled receptor |
| FAS | Fas cell surface death receptor | Plasma Membrane | transmembrane receptor |
| FGF2 | fibroblast growth factor 2 | Extracellular Space | growth factor |
| FN1 | fibronectin 1 | Extracellular Space | enzyme |
| HBEGF | heparin binding EGF like growth factor | Extracellular Space | growth factor |
| ITGAM | integrin subunit alpha M | Plasma Membrane | transmembrane receptor |
| JUN | Jun proto-oncogene, AP-1 transcription factor subunit | Nucleus | transcription regulator |
| KDR | kinase insert domain receptor | Plasma Membrane | kinase |
| MMP15 | matrix metallopeptidase 15 | Extracellular Space | peptidase |
| MMP17 | matrix metallopeptidase 17 | Extracellular Space | peptidase |
| NREP | neuronal regeneration related protein | Cytoplasm | other |
| PLAT | plasminogen activator, tissue type | Extracellular Space | peptidase |
| PPIA | peptidylprolyl isomerase A | Cytoplasm | enzyme |
| PSEN1 | presenilin 1 | Plasma Membrane | peptidase |
| SDC1 | syndecan 1 | Plasma Membrane | enzyme |
| SERPINE2 | serpin family E member 2 | Extracellular Space | other |
| SNAP23 | synaptosome associated protein 23 | Plasma Membrane | transporter |
| STX12 | syntaxin 12 | Cytoplasm | other |
| TIMP3 | TIMP metallopeptidase inhibitor 3 | Extracellular Space | other |
| TIMP4 | TIMP metallopeptidase inhibitor 4 | Extracellular Space | other |
| TPSAB1/ TPSB2 | tryptase alpha/beta 1 | Extracellular Space | peptidase |

However, none of the MMP-related genes were found to be differentially expressed in the cortical neurons.

This finding led to the re-assessment of all molecular interactions that affect cell attachment and adhesion related molecules and their effect on cellular attachment in the four different cultures. The full list of the 217 attachment-related molecules (197 genes and 20 drugs) is presented below:

ACE2, ACP1, ADAM15, ADGRB1, ADGRE2, ADIPOQ, AG490, AMBN, ANGPT1, ANTXR1, ARAP3, ARMS2, batimastat, BCAM, BCAP31, BCAR1, benzyloxycarbonyl-Leu-Leu-Leu-aldehyde, BMP2, BMP4, BTC, C1QBP, $Ca^{2+}$, CA9, CADM1, CALR, calyculin A, caspase, CBL, CD209, CD36, CD44, CD46, CDH13, cerivastatin, chloramphenicol, chondroitin sulfate, CLEC4M, colchicine, Collagen type I, Collagen(s), COMP, CRK, CRP, CSF1, CSF2RB, CTGF, curcumin, CXCL12, cyclic AMP, DAB2, DAG1, DCN, DDR1, desferriexochelin 772SM, DOCK2, DSG2, DSG4, durapatite, Efna, EFNA1, EFNB, EFNB1, EGF, EGFR, EGR1, ELN, ENG, EP300, Eph Receptor, EPHA8, EPHB1, eptifibatide, ethylenediaminetetraacetic acid, ETS1, F11R, F3, FBLN5, FBN1, Fc receptor, FCN2, FERMT2, FES, FGF2, FGFR1, Fibrin, FN1, Focal adhesion kinase, FSH, FUT3, FUT6, FUT7, FYN, HACD1, heparin, Histone h3, Histone h4, HRAS, HSPG2, HTN1, hyaluronic acid, hydrocortisone, hydrogen peroxide, ICAM1, ICAM2, IGF1R, IgG, Igg3, IL1, IL1B, IL6, ILK, Integrin, Integrin alpha4 beta 1, Integrina, IPO9, ITGA1, ITGA2, ITGA3, ITGA5, ITGA6, ITGB1, ITGB2, ITGB3, ITGB5, JAK2, Jnk, KP-SD-1, LAMC1, Laminin, Laminin1, levothyroxine, LGALS3, LIF, lipopolysaccharide, LOX, LRP1, LRPAP1, MAD1L1, mannose, MAPK7, MBL2, MERTK, metronidazole, MGAT5, MMP2, $Mn^{2+}$, NCK, NEDD9, NRG1, okadaic acid, OLR1, P38 MAPK, PDGF BB, phosphatidylinositol, PKM, platelet activating factor, PLD1, PLG, PMP22, PODXL, POSTN, PRKCD, PTAFR, PTEN, PTGER2, PTK2, PTK2B, PTN, PTPN11, PTPRZ1, pyrrolidine dithiocarbamate, Rac, RALB, RANBP9, RHOA, RHOB, RPSA, SDC3, SELE, Selectin, SELL, SEMA3A, simvastatin, SIRPA, SPARC, sphingosine-1-phosphate, SPI1, SPP1, SPRY2, SRC, STARD13, SWAP70, TEK, TFPI, TFPI2, TGFA, TGFB1, TGFBI, TGM2, THBS2, THY1, thyroid hormone, TIMP2, tirofiban, TLN1, TLN2, TNF, TP63, tretinoin, VAV1, VCAM1, VCAN, Vegf, VHL, VTN, VWF, and WRR-086.

Of the 197 genes regulating cell attachment none are differentially regulated by LMW-DS in HUVECs. In the Schwann cell cultures, the 17 molecules differentially expressed lead to an overall slightly increased attachment. However, in the neurons the expression patterns lead to significant inhibition of cellular attachment in these cells.

*Upstream regulator pathways affected by LMW-DS*

In Schwann cells, the upstream regulator analysis revealed that LMW-DS modulated the effect of several growth factors by either increasing their activation or reducing their inhibition in the system as shown in Table 12.

Table 12 - Upstream regulator comparison in Schwann cells

| Analysis | Upstream regulator | Predicted activation state relative D2 control | Activation z-score | p-value of overlap |
|---|---|---|---|---|
| D2 control | ANGPT2 | | 1.062 | 0.003 |
| D2 LMW-DS treatment | | Activated | 1.283 | 0.00373 |
| D2 control | BMP2 | | 0.674 | 0.0126 |
| D2 LMW-DS treatment | | Activated | 1.395 | 0.00326 |
| D2 control | BMP4 | | -0.272 | 0.00253 |
| D2 LMW-DS treatment | | Activated | 0.927 | 0.000663 |
| D2 control | BMP7 | | 1.45 | 0.0346 |
| D2 LMW-DS treatment | | Activated | 1.86 | 0.0225 |
| D2 control | EGF | | -0.015 | 0.0000927 |
| D2 LMW-DS treatment | | Activated | 2.059 | 0.00735 |
| D2 control | FGF2 | | 1.366 | 0.0000142 |
| D2 LMW-DS treatment | | Activated | 2.37 | 0.000395 |
| D2 control | GDF2 | | 1.556 | 0.000299 |
| D2 LMW-DS treatment | | Activated | 2.561 | 0.000106 |
| D2 control | HGF | | -0.823 | 0.0114 |
| D2 LMW-DS treatment | | Activated | 1.432 | 0.0161 |
| D2 control | IGF1 | | 0.365 | 0.00883 |
| D2 LMW-DS treatment | | Activated | 1.332 | 0.0132 |
| D2 control | NRG1 | | 1.073 | 0.0473 |
| D2 LMW-DS treatment | | Activated | 1.768 | 0.143 |
| D2 control | NRTN | | | 0.0118 |
| D2 LMW-DS treatment | | Activated | 0.958 | 0.0149 |
| D2 control | PGF | | 0 | 0.00185 |
| D2 LMW-DS treatment | | Activated | 0.254 | 0.00871 |
| D2 control | TGFβ1 | | -1.239 | 0.0000354 |
| D2 LMW-DS treatment | | Less inhibited | 1.05 | 0.0000691 |

(continued)

| Analysis | Upstream regulator | Predicted activation state relative D2 control | Activation z-score | p-value of overlap |
|---|---|---|---|---|
| D2 control | VEGFA | | 1.909 | 0.00981 |
| D2 LMW-DS treatment | | Activated | 3.4 | 0.00186 |
| D2 control | WISP2 | | -1.067 | 0.0323 |
| D2 LMW-DS treatment | | Less inhibited | -0.896 | 0.0349 |

In HUVECs, the number of growth factors whose effect was enhanced by LMW-DS was relatively smaller but still highly significant, see Table 13.

Table 13 - Upstream regulator comparison in HUVECs

| Analysis | Upstream regulator | Predicted activation state relative D2 control | Activation z-score | p-value of overlap |
|---|---|---|---|---|
| D2 control | HGF | | 2.602 | 0.0000181 |
| D2 LMW-DS treatment | | Activated relative to control | 3.194 | 0.00000793 |
| D2 control | TGFβ1 | | 0.682 | 0.00328 |
| D2 LMW-DS treatment | | Activated relative to control | 1.429 | 0.0338 |
| D2 control | VEGF | | 3.113 | 2.78E-08 |
| D2 LMW-DS treatment | | Activated relative to control | 3.432 | 6.33E-09 |

In the motor neurons, the upstream regulator analysis revealed that LMW-DS affected the effect of several growth factors either increasing their activation or reducing the inhibitions present in the system as shown in Table 14.

Table 14 - Upstream regulator comparison in motor neurons

| Analysis | Upstream regulator | Predicted activation state relative D2 control | Activation z-score |
|---|---|---|---|
| D0 to D2 control | AGT | Activated | 2.292 |
| D0 to LMW-DS treatment | | Activated | 2.631 |
| D0 to D2 control | BMP4 | | 0.798 |
| D0 to LMW-DS treatment | | More activated relative to control | 0.972 |
| D0 to D2 control | BMP6 | | -0.269 |
| D0 to LMW-DS treatment | | More activated relative to control | 0.13 |
| D0 to D2 control | BMP7 | | -0.862 |
| D0 to LMW-DS treatment | | More activated relative to control | 1.092 |
| D0 to D2 control | INHA | | 2.292 |
| D0 to LMW-DS treatment | | More activated relative to control | 0.588 |

In cortical neurons, in normal culture conditions, most growth factor dependent pathways were significantly activated by the normal culture medium. In most instances this activation was not altered by LMW-DS. However, LMW-DS activated molecules that are the downstream effector of GDF7 indicating that the effect of this growth factor was enhanced by LMW-DS. As GDF7 is a powerful differentiation factor for neurons, and the additional activation of these growth factors, to the activation of BDNF and NT3, provide a good explanation for the enhanced differentiation of these cells in culture.

DISCUSSION

The normal culture conditions for HUVECs mimics the environment following tissue hypoxia and reperfusion, containing a high nutrient content and growth factors also supplemented with heparin. The LMW-DS-treated cultures mimicked the effect of LMW-DS added after 24 hours of hypoxia and reperfusion. The real life scenario this relates to is that of angiogenesis following ischemic conditions, such as stroke.

In Schwann cells, the control cultures, with high nutrient content and glucose, recapitulate the activation of Schwann cells. The LMW-DS-treated cultures mimicked the effect of LMW-DS added after 24 hours of glial activation. The real life scenario that this recapitulates is glial activation following damage to the nervous system, such as following TBI.

The normal culture conditions for the neurons, both motor neurons and cortical neurons, with high nutrient content and growth factors mimic the environment during normal neuronal differentiation. The only negative effect in these cultures is the oxidative stress the cells suffer. The real life scenario this relates to is the degenerative conditions driven by oxidative stress in the presence of ample growth and differentiation factors. This corresponds to an early stage of a neurodegenerative disease or condition where oxidative stress plays a pivotal role.

It is clear from the cell types that the molecular effects seen in Schwann cells and in HUVECs support a role for LMW-DS in protection against apoptosis; induction of angiogenesis; increased migration and movement of cells; increased cell viability and survival; and induction of cellular differentiation. The analysis of pivotal molecular pathways indicated that in neurons LMW-DS will reduce the effect of oxidative stress on mitochondria and will reduce neurodegeneration-related molecules, such as amyloid-$\beta$ and Lewy bodies.

Accordingly, the results from the HUVEC cell model indicates that LMW-DS can protect against cell damage and promotes the development of new blood vessels in injured or diseased tissue, such as following stroke. The results from the Schwann cells indicate that LMW-DS can protect against cell loss in a diseased or damaged nervous system, such as due to TBI or a neurodegenerative disease.

The analysis of pivotal molecular pathways indicated that in Schwann cells LMW-DS reduced the effect of oxidative stress on mitochondria and increased the uptake of glutamate. The results in Schwann cells indicate that LMW-DS can protect against cell loss that occurs due to oxidative stress and glutamate excitotoxicity in the diseased or damaged nervous system, which is of relevance in, for instance, neurodegenerative diseases and TBI.

Of particular importance, LMW-DS increased the glutamate uptake in glia cells, as presented by Schwann cells. However, LMW-DS did not alter the production of glutamate by neurons. This is important since glutamate is needed for LTP, learning and memory. Thus, it is beneficial that LMW-DS did not alter production of glutamate by neurons since this glutamate is needed for the normal neurotransmission in the above mentioned processed. However, the increased levels of glutamate released from damaged or dying cells will be effectively taken up by surrounding glial cells due to the effects of LMW-DS. Thus, the activation of glutamate transporters in the glial cells caused by LMW-DS effectively removed the glutamate released by the damaged or dying neurons from the neural cleft. This in turn prevented the glutamate from exerting its excitotoxicity and thereby damaging further neurons. Accordingly, LMW-DS induced the uptake of the potentially harmful neurotoxic amounts of glutamate by the glial cells.

The results in the neurons therefore confirm the potential therapeutic usefulness of LMW-DS in neurodegenerative diseases, disorders and conditions by reducing secondary tissue damage due to oxidative stress, promoting repair, and reducing degeneration-related protein accumulation.

Taken together the results support the role of LMW-DS in protection against apoptosis in general and protection against neuronal cell death in particular, induction of angiogenesis, increased migration and movement of cells, increased cell viability and survival, induction of cellular differentiation, reduction of the effects of oxidative stress, reduction of glutamate excitotoxicity and reduction of the production of degeneration-related protein products, such as amyloid-$\beta$ and Lewy bodies.

Cell adhesion was affected mainly in neurons and Schwann cells, where LMW-DS promoted cell detachment and movement. In HUVECs, cell adhesion was not affected. The effect on cell adhesion was mainly due to the expression of metalloproteinase-type enzymes, but the modulation of other adhesion molecules contributed to this effect as well.

Scarring as a pathological reaction is driven by TGF$\beta$. TGF$\beta$ induces a large interconnected network of 171 molecules causing adhesion of immune cells, activation of cells, cell movement, aggregation of cells, fibrosis and induction of TGF$\beta$. Administration of LMW-DS totally abolished the TGF$\beta$-induced effect in adhesion of immune cells, activation of cells, aggregation of cells, fibrosis and self-activation of TGF$\beta$. These inactivating effects of LMW-DS on the molecular networks driven by TGF$\beta$ in Schwann cells are also seen even when TGF$\beta$ is activated, i.e., even in the presence of excessive TGF$\beta$.

These studies therefore confirm the potential therapeutic usefulness of LMW-DS in treating neurodegenerative diseases, disorders and conditions, where it could promote neuronal survival, differentiation and ultimately repair.

The analysis of the upstream regulators of the genes regulated by LMW-DS indicated that LMW-DS enhanced the effect of existing growth factors on cells, similar to the effect of heparin. A hypothesis is that LMW-DS binds to the growth factor molecules and facilitates binding to their receptors.

This hypothesis is also supported by the observation that the LMW-DS-induced differential gene expression in HUVECs, where the normal CM already contains heparin, was relatively smaller than in the Schwann cells where the normal CM did not contain heparin.

This mechanism of action also explains why LMW-DS is effective in the acute stage of TBI as seen in Example 1, when growth factors are present, but less effective at later stage when the initial repair attempt has already diminished.

Thus, it could be possible that at least some of the therapeutic effects of LMW-DS depend on existing repair mechanisms, which are amplified by it. In such a case, it is generally recommended that in any neurodegenerative condition LMW-DS is given in the early stage of the disease or condition when there is enough repair potential in the tissue.

By protecting cell metabolism, LMW-DS may be a useful protective treatment in many degenerative conditions where cells are progressively lost due to ischemic, oxidative or traumatic damage. Nonlimiting, but illustrative, examples of such degenerative conditions include stroke, ALS, MS, dementia, TBI, SCI, retinal damage, AD, etc. LMW-DS may help those damaged tissues to recover some lost function as it enhances the residual intrinsic repair mechanisms.

EXAMPLE 3

The aim of this study was to evaluate the potential neuroprotective effects of LMW-DS on biochemical, molecular and histo-anatomical damages produced by the experimental model of closed-head diffuse severe TBI (sTBI) in rat. In the present study, results were obtained through HPLC analyses of low molecular weight metabolites representative of energy metabolism, oxidative/nitrosative stress, antioxidants and free amino acids in cerebral tissue extracts of treated animals.

MATERIALS AND METHODS

*Induction of sTBI and drug administration protocol*

Male Wistar rats (n=160) of 300-350 g body weight were used in this study. They were fed with standard laboratory diet and water *ad libitum* in a controlled environment.

As the accepted anesthetic mixture, animals received 35 mg/kg b.w. ketamine and 0.25 mg/kg body weight midazolam by intramuscular injection. Diffuse sTBI was induced according to the "weight drop" impact acceleration model set up by Marmarou et al. J. Neurosurg. 1994, 80: 291-300. This model causes diffuse axonal injury and it is able to reproduce the physical and mechanical characteristics of the diffuse TBI in humans.

Severe TBI was induced by dropping a 450 g weight from 2 meters height onto the rat head protected by a helmet (metal disk previously fixed on the skull using dental cement) in order to uniformly distribute the mechanical force to the brain. Rats were placed prone on a bed of specific polyurethane foam inserted in a special container. This foam dissipates the major part of the potential energy (deriving from the mechanical forces) and prevents any rebound of the animal after the impact that could produce spinal damages.

Rats suffering from skull fracture, seizures, nasal bleeding, or did not survive the impact, were excluded from the study. After 2 or 7 days from TBI induction, rats were anesthetized again and then immediately sacrificed. These time points are coincident with the worst biochemical derangement (2 days) or, in the case of a mildly injured brain, with a full metabolic recovery (7 days).

The drug treatment consisted in a subcutaneous injection of 0.5 ml of LMW-DS (Tikomed) and administered at 3 different concentrations (1, 5 and 15 mg/kg body weight), according to the schematic protocol described below.

Sham-operated animals underwent the same procedure of anesthesia but TBI and were used as the control group.

*Experimental design*

Rats used in this study were divided into 4 groups in order to carry out a study on the efficacy of three different concentrations of LMW-DS at two different times post TBI. As subsequently specified, in each group there were animals subjected to a specific treatment for metabolic analyses and other animals intended to histo-morphological studies, according to the procedures described below.

*Group-1*

Controls (n = 12) dedicated to the biochemical evaluation. Four additional animals were used for the histo-morphological studies. Total rats in this group: n = 16

*Group-2*

Rats subjected to sTBI with no pharmacological treatment were divided into the following subgroups:

1. 12 animals subjected to sTBI and sacrificed after 2 days post-TBI
2. 12 animals subjected to sTBI and sacrificed after 7 days post-TBI

Four additional rats to each subgroup were used for the histo-morphological studies. Total rats in this group: n = 32.

*Group-3*

Rats subjected to sTBI and receiving a single administration of LMW-DS after 30 minutes post-TBI, with sacrifice at 2 days post-TBI. Animals were divided in the following subgroups:

1. 12 animals subjected to sTBI and treated with 1 mg/kg b.w. LMW-DS
2. 12 animals subjected to sTBI and treated with 5 mg/kg b.w. LMW-DS
3. 12 animals subjected to sTBI and treated with 15 mg/kg b.w. LMW-DS

Four additional rats to each subgroup were used for the histo-morphological studies. Total rats in this group: n = 48.

*Group-4*

Rats subjected to sTBI and receiving a single administration of LMW-DS after 30 minutes post-TBI, with sacrifice at 7 days post-TBI. Animals were divided in the following subgroups:

1. 12 animals subjected to sTBI and treated with 1 mg/kg b.w. LMW-DS
2. 12 animals subjected to sTBI and treated with 5 mg/kg b.w. LMW-DS
3. 12 animals subjected to sTBI and treated with 15 mg/kg b.w. LMW-DS

Four additional rats to each subgroup were used for the histo-morphological studies. Total rats in this group: n = 48.

*Group-5*

Rats (n = 12) subjected to sTBI and receiving repeated administrations of the maximal dose of LMW-DS (15 mg/kg b.w.) after 30 minutes, 3 days and 5 days post-TBI, with sacrifice at 7 days post-TBI. Four additional rats were used for the histo-morphological studies. Total rats in this group: n=16

*Cerebral tissue processing for biochemical and gene expression analyses*

To minimize metabolite loss, an *in vivo* craniectomy was performed in all animals during anesthesia. The rat skull was carefully removed, the brain was exposed, sharply cut along the sagittal fissure and the two hemispheres were separated. The hemispheres dedicated to biochemical analyses were freeze-clamped by aluminum tongues pre-cooled in liquid nitrogen and then immersed in liquid nitrogen. The freeze-clamping procedure was introduced to accelerate freezing of the tissue, thus minimizing potential metabolite loss.

The remaining hemispheres, dedicated to molecular biology analyses, were placed in 5-10 volumes of RNAlater® Solution (Invitrogen Life Technologies), a RNA stabilization solution that stabilize and protect RNA from degradation. Brain samples were stored at 4°C overnight to allow the solution to completely penetrate tissue.

Tissue homogenization for metabolite analyses was effected as described below. After the wet weight (w.w.) determination, the frozen hemispheres were placed into 7 ml of ice-cold, nitrogen-saturated, precipitating solution (1:10 w/v) composed by $CH_3CN$ + 10 mM $KH_2PO_4$, pH 7.40, (3:1; v:v), and the homogenization was performed using an Ultra-Turrax homogenizer set at 24,000 rpm/min (Janke & Kunkel, Staufen, Germany). After centrifugation at 20,690 × g, for 10 min at 4°C, the clear supernatants were saved, pellets were supplemented with an aliquot of 10 mM $KH_2PO_4$ and homogenized again as described above and saved overnight at -20°C in order to obtain a complete recovery of aqueous phase from tissue. A second centrifugation was performed (20,690 × g, for 10 min at 4°C) and supernatants combined with those previously obtained were extracted by vigorous agitation with a double volume of HPLC-grade $CHCl_3$ and centrifuged as above. The upper aqueous phases (containing water-soluble low-molecular weight compounds) were collected, subjected to chloroform washings for two more times (this procedure allowed the removal of all the organic solvent and of any lipid soluble compound from the buffered tissue extracts), adjusted in volumes with 10 mM $KH_2PO_4$, pH 7.40, to have ultimately aqueous 10% tissue homogenates and saved at -80°C until assayed.

*HPLC analysis of energy metabolites, antioxidants and oxidative/nitrosative stress biomarkers*

Aliquots of each deproteinized tissue sample were filtered through a 0.45 $\mu$m HV Millipore filter and loaded (200 $\mu$l) onto a Hypersil C-18, 250 × 4.6 mm, 5 $\mu$m particle size column, provided with its own guard column (Thermo Fisher Scientific, Rodano, Milan, Italy) and connected to an HPLC apparatus consisting of a Surveyor System (Thermo Fisher Scientific, Rodano, Milan, Italy) with a highly sensitive diode array detector (equipped with a 5 cm light path flow cell) and set up between 200 and 300 nm wavelength. Data acquisition and analysis were performed by a PC using the ChromQuest® software package provided by the HPLC manufacturer.

Metabolites (listed below) related to tissue energy state, mitochondrial function antioxidants and representative of oxidative/nitrosative stress were separated, in a single chromatographic run, according to slight modifications of existing ion-pairing HPLC methods formerly (Lazzarino et al., Anal Biochem. 2003, 322: 51-59; Tavazzi et al., Clin Biochem. 2005, 38: 997-1008). Assignment and calculations of the compounds of interest in chromatographic runs of tissue extracts were carried out at the proper wavelengths (206, 234 and 260 nm) by comparing retention times, absorption spectra and areas of peaks with those of peaks of chromatographic runs of freshly-prepared ultra-pure standard mixtures with known concentrations.

**List of compounds:** Cytosine, Creatinine, Uracil, Beta-Pseudouridine, Cytidine, Hypoxanthine, Guanine, Xanthine, CDP-Choline, Ascorbic Acid, Uridine, Nitrite ($NO_2$), reduced glutathione (GSH), Inosine, Uric Acid, Guanosine, CMP, Malondialdehyde (MDA), Nitrate (NOs), UMP, $NAD^+$, ADO, IMP, GMP, UDP-glucose (UDP-Glc), UDP-galactose (UDP-Gal), UDP-N-acetyl-glucosamine (UDP-GlcNac), UDP-N-acetyl-galactosamine (UDP-GalNac), AMP, GDP-glucose, UDP, GDP, $NADP^+$, ADP-Ribose, CTP, ADP, UTP, GTP, NADH, ATP, NADPH, Malonyl-CoA, Coenzyme A (CoA-SH), Acetyl-CoA, N-acetylaspartate (NAA).

*HPLC analysis of free amino acids and amino group containing compounds*

The simultaneous determination of primary free amino acids (FAA) and amino group containing compounds (AGCC) (listed below) was performed using the precolumn derivatization of the sample with a mixture of OPA and MPA, as described in (Amorini et al., J Cell Mol Med. 2017, 21(3): 530-542; Amorino et al., Mol Cell Biochem. 2012, 359: 205-216). Briefly, the derivatization mixture composed by 25 mmol/l OPA, 1% MPA, 237.5 mmol/l sodium borate, pH 9.8 was prepared daily and placed in the autosampler. The automated precolumn derivatization of the samples (15 $\mu$l) with OPA-MPA was carried out at 24°C and 25 $\mu$l of the derivatized mixture were loaded onto the HPLC column (Hypersil C-18, 250 × 4.6 mm, 5 $\mu$m particle size, thermostated at 21°C) for the subsequent chromatographic separation. In order to quantify correctly Glutamate, deproteinized brain extracts were diluted 20 times with HPLC-grade $H_2O$ prior to the derivatization procedure and subsequent injection. Separation of OPA-AA and OPA-AGCC was carried out at a flow rate of 1.2 ml/min using two mobile phases (mobile phase A = 24 mmol/l $CH_3COONa$ + 24 mmol/l $Na_2HPO_4$ + 1% tetrahydrofurane + 0.1% trifluoroacetic acid, pH 6.5; mobile phase B = 40% $CH_3OH$ + 30 $CH_3CN$ + 30% $H_2O$), using an appropriate step gradient.

Assignment and calculation of the OPA-AA and OPA-AGCC in chromatographic runs of whole brain extracts were carried out at 338 nm wavelengths by comparing retention times and areas of peaks with those of peaks of chromatographic runs of freshly-prepared ultra-pure standard mixtures with known concentrations.

**List of FAA and AGCC compounds:** aspartate (ASP), glutamate (GLU), asparagine (ASN), serine (SER), glutamine (GLN), histidine (HIS), glycine (GLY), threonine (THR), citrulline (CITR), arginine (ARG), alanine (ALA), taurine (TAU), $\gamma$-aminobutyric acid (GABA), tyrosine (TYR), S-adenosylhomocysteine (SAH), L-cystathionine (L-Cystat), valine (VAL), methionine (MET), tryptophan (TRP), phenylalanine (PHE), isoleucine (ILE), leucine (LEU), ornithine (ORN), lysine (LYS).

*Brain tissue processing for histo-morphological analyses*

After adequate anesthesia rats were transcardially perfused as described in (Di Pietro et al., Sci Rep. 2017, 7(1): 9189). Briefly, a thoracotomy was performed and a heparin solution was administered into the portal vein to avoid blood coagulation during all the operation. Afterwards, a right atrial incision was carried out and the perfusion needle was advanced into the ascending aorta. Perfusion was performed with 100 ml of Phosphate Buffer Solution (PBS) at pH 7.4 in order to wash out blood before further perfusion with 100 ml 4% paraformaldehyde (PFA) in PBS solution at pH 7.4. After rapid removal from the skull, each brain was post fixed by immersion in 4% PFA in PBS solution for 2 hours at 4°C.

Cryoprotection was obtained by immersing the whole brain in PBS enriched with increasing sucrose solutions (10%, 20%, and 30%) for 24 hours at 4°C, then implanted in optimal cutting temperature embedding medium (OCT) (Thermo Shandon, Runcorn, UK) in peel-away mould containers (Agar Scientific, Essex, UK). Brain immersed in OCT was rapidly frozen in crushed dry ice before storage at -80°C.

*Statistical analysis*

Differences across groups were estimated by the Student's t-test. Only two-tailed p-values of less than 0.05 were considered statistically significant.

RESULTS

SUMMARY OF BIOCHEMICAL DATA RECORDED AT 2 DAYS POST sTBI

*Effects of increasing doses of LMW-DS on brain energy metabolism measured*

Table 15 summarizes values referring to phosphorylated high-energy purine and pyrimidine compounds. It is particularly evident the depletion of triphosphate nucleotides (ATP, GTP, UTP and CTP) caused by sTBI, that was accompanied by an increase in ADP and in the N-acetylated derivatives of UDP-glucose (UDP-GlcNac) and UDP-galactose (UDP- GalNac).

At this time post injury, treatment with LMW-DS was only partly effective in improving cell energy metabolism. Significantly higher values of high energy phosphates (ATP, GTP, and CTP) were recorded with all the three dosages of the drug tested. No effects were seen on the concentrations of UTP and ADP. It is worth recalling that 48 hours post TBI in rats represents a critical time point for brain metabolism, coincident with maximal alterations of mitochondrial functions including changes in the mitochondrial quality control. In this experimental model of TBI, this time point could be considered a sort of "turning point" at which recovery or no recovery of cerebral metabolism is defined.

Table 15 - Concentrations of energy metabolites (phosphorylated purines and pyrimidines) measured in deproteinized brain homogenates of rats sacrificed at 2 days post-sTBI, without and with a single administration of increasing doses of LWM-DS (1, 5 and 15 mg/kg b.w.), performed 30 minutes after brain trauma induction. Controls are represented by sham-operated animals. Values are the mean $\pm$ S.D. of 12 animals in each group and are expressed as nmol/g w.w.

| Compound | Controls | TBI only) | LMW-DS 1 | LMW-DS 5 | LMW-DS 15 |
|---|---|---|---|---|---|
| CMP | 13.52 $\pm$ 3.44 | **34.85 $\pm$ 7.11** | **29.39 $\pm$ 6.00** | ***28.94 $\pm$ 5.91*** | **25.61 $\pm$ 5.23** |
| UMP | 82.30 $\pm$ 9.82 | **151.45 $\pm$ 20.92** | **148.04 $\pm$ 20.45** | ***97.98 $\pm$ 13.53*** | **147.53 $\pm$ 20.38** |
| IMP | 51.57 $\pm$ 4.610 | 55.06 $\pm$ 10.36 | **45.97 $\pm$ 8.65** (underlined) | ***33.19 $\pm$ 6.25*** | **68.95 $\pm$ 12.98** |
| GMP | 82.81 $\pm$ 7.821 | **186.08 $\pm$ 23.36** | **205.06 $\pm$ 25.74** | ***167.44 $\pm$ 8.47*** | **178.88 $\pm$ 22.46** |
| UDP-Glc | 51.00 $\pm$ 10.89 | 48.87 $\pm$ 7.24 | 45.14 $\pm$ 6.68 | ***28.60 $\pm$ 4.23*** | 43.41 $\pm$ 6.43 |
| UDP-Gal | 131.00 $\pm$ 13.26 | 127.11 $\pm$ 10.61 | **118.50 $\pm$ 9.89** | ***116.42 $\pm$ 9.72*** | ***116.34 $\pm$ 9.71*** |
| UDP-GlcNac | 88.77 $\pm$ 19.55 | **102.34 $\pm$ 9.32** | 96.62 $\pm$ 8.80 | ***140.58 $\pm$ 12.80*** | **108.74 $\pm$ 9.90** |
| UDP- GalNac | 38.82 $\pm$ 9.83 | **22.10 $\pm$ 3.26** | **21.24 $\pm$ 3.13** | **20.75 $\pm$ 3.06** | **22.37 $\pm$ 3.30** |
| GDP Glucose | 85.35 $\pm$ 12.76 | 89.05 $\pm$ 39.68 | 65.66 $\pm$ 41.61 | 83.81 $\pm$ 37.35 | 84.24$\pm$ 37.54 |
| AMP | 43.59 $\pm$ 9.90 | 65.13 $\pm$ 41.27 | **62.04 $\pm$ 7.46** | **67.03 $\pm$ 11.85** | **66.26 $\pm$ 10.74** |
| UDP | 23.94 $\pm$ 6.75 | **64.40 $\pm$ 6.60** | ***83.06 $\pm$ 8.52*** | ***70.93 $\pm$ 7.27*** | ***80.00 $\pm$ 8.20*** |
| GDP | 57.40 $\pm$ 14.06 | **167.28 $\pm$ 23.11** | **189.85 $\pm$ 26.23** | **183.27 $\pm$ 25.32** | **194.61 $\pm$ 26.88** |
| ADP-Ribose | 12.69 $\pm$ 1.43 | 13.85 $\pm$ 2.78 | ***25.69 $\pm$ 5.16*** | ***21.65 $\pm$ 4.35*** | **23.06 $\pm$ 4.63** |
| CTP | 41.85 $\pm$ 10.32 | **28.32 $\pm$ 5.73** | **33.01 $\pm$ 7.63** | **37.72 $\pm$ 7.63** | **37.53 $\pm$ 7.59** (underlined) |
| ADP | 222.67 $\pm$ 30.99 | **297.53 $\pm$ 25.59** | ***333.90 $\pm$ 28.72*** | ***364.92 $\pm$ 31.39*** | ***346.37 $\pm$ 29.79*** |
| UTP | 152.64 $\pm$ 17.39 | **100.79 $\pm$ 15.83** | **104.07 $\pm$ 16.34** | **142.82 $\pm$ 22.43** (underlined) | **108.21 $\pm$ 16.99** |
| GTP | 569.00 $\pm$ 45.32 | **202.19 $\pm$ 21.33** | ***169.98 $\pm$ 17.93*** | ***180.01 $\pm$ 18.99*** | ***179.07 $\pm$ 18.89*** |
| ATP | 2390.14 $\pm$ 213.98 | **1330.60 $\pm$ 77.96** | ***1696.96 $\pm$ 99.43*** | ***1683.87 $\pm$ 98.66*** | ***1556.54 $\pm$ 91.20*** |

In Tables 15 - 34, **bold** indicates significantly different from controls (p < 0.05); **bold underlined** indicates significantly different from TBI (p < 0.05); and ***bold italic*** indicates significantly different from both controls and TBI (p < 0.05).

*Effects of increasing doses of LMW-DS on nicotinic coenzymes*

Values of oxidized (NAD+ and NADP+) and reduced (NADH and NADPH) nicotinic coenzymes are summarized in Table 16. This Table 16 also reports the calculated, adimensional values of the NAD+/NADH ratio which is suitable to evaluate how much metabolism is dependent on glycolysis or on mitochondrial oxidative phosphorylation.

As previously observed herein, sTBI caused decrease of $NAD^+$, $NADP^+$ and of the NAD+/NADH ratio. At this time point, treatment with LMW-DS was effective only at the maximal dose tested (15 mg/kg b.w.) that produced significant protection of the nicotinic coenzyme pool and avoid the metabolic switch towards glycolysis, thereby indirectly suggesting overall better mitochondrial functions.

Table 16 - Concentrations of nicotinic coenzymes measured in deproteinized brain homogenates of rats sacrificed at 2 days post-sTBI, without and with a single administration of increasing doses of LWM-DS (1, 5 and 15 mg/kg b.w.), performed 30 minutes after brain trauma induction. Controls are represented by sham-operated animals. Values are the mean $\pm$ S.D. of 12 animals in each group and are expressed as nmol/g w.w. The NAD+/NADH ratio is adimensional.

| Compound | Controls | TBI only | LMW-DS 1 | LMW-DS 5 | LMW-DS 15 |
|---|---|---|---|---|---|
| NAD+ | 485.74 $\pm$ 37.06 | **379.70 $\pm$ 64.64** | *325.87 $\pm$ 55.47* | 376.85 $\pm$ 64.15 | <u>475.32 $\pm$ 80.91</u> |
| NADH | 13.57 $\pm$ 1.94 | 12.45 $\pm$ 1.82 | *9.42 $\pm$ 1.38* | *10.37 $\pm$ 1.19* | *10.83 $\pm$ 1.58* |
| NADP+ | 23.17 $\pm$ 4.58 | **17.68 $\pm$ 4.04** | *11.79 $\pm$ 2.70* | *11.86 $\pm$ 2.71* | <u>17.75 $\pm$ 4.06</u> |
| NADPH | 8.51 $\pm$ 0.71 | 7.94 $\pm$ 0.66 | *13.07 $\pm$ 1.09* | *37.48 $\pm$ 3.11* | <u>8.93 $\pm$ 0.74</u> |
| NAD+/NADH | 36.47 $\pm$ 5.46 | **34.99 $\pm$ 6.05** | 33.91 $\pm$ 9.32 | **36.61 $\pm$ 6.09** | <u>44.40 $\pm$ 7.67</u> |

*Effects of increasing doses of LMW-DS on CoA-SH derivatives*

Table 17 reports data referring to free CoA-SH and CoA-SH derivatives. Particularly Acetyl-CoA is a crucial compound for mitochondrial metabolism allowing correct functioning of the tricarboxylic acid cycle (TCA cycle), thus ensuring continuous electron supply for the electron transfer chain (ETC). TCA is the major cell cycle for the generation of reduced coenzymes (NADH and $FADH_2$) which, by transferring their electrons to mitochondrial complexes I and II, respectively, are the fuel for ETC and oxidative metabolism. All compounds, particularly Acetyl-CoA, were significantly affected by sTBI. A partial rescue of this compound was observed when 5 or 15 mg/kg b.w. LWM-DS was administered to animals 30 minutes post injury.

Table 17 - Concentrations of free CoA-SH and CoA-SH derivatives (Acetyl-CoA and Malonyl-CoA) measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 |
|---|---|---|---|---|---|
| Malonyl-CoA | 15.02 $\pm$ 2.38 | **11.82 $\pm$ 2.50** | **19.06 $\pm$ *4.04*** | *35.58 $\pm$ 7.54* | *28.73 $\pm$ 6.09* |
| CoA-SH | 26.31 $\pm$ 3.86 | **21.00 $\pm$ 2.32** | *9.42 $\pm$ 1.04* | *7.46 $\pm$ 0.82* | *9.35 $\pm$ 1.03* |
| Acetyl-CoA | 36.97 $\pm$ 5.43 | **28.32 $\pm$ 3.29** | 27.74 $\pm$ 3.23 | <u>34.85 $\pm$ 4.05</u> | 32.38 $\pm$ 3.77 |

*Effects of increasing doses of LMW-DS on antioxidants and oxidative/nitrosative stress biomarkers* Table 18 shows the concentrations of the main water-soluble brain antioxidants (ascorbic acid and GSH) and of biomarkers of oxidative (MDA) and nitrosative stress ($-NO_2^-$ and $-NO_3^-$). Malondialdehyde (MDA) originates from decomposition of unsaturated fatty acids of membrane phospholipids as a consequence of ROS-mediated lipid peroxidation. Nitrites ($-NO_2^-$) and nitrates ($-NO_3^-$) are stable end products of nitric oxide (NO) metabolism which, under pathological conditions, is generated in excess by an inducible form of nitric oxide synthase (iNOS) and gives raise to reactive nitrogen species (RNS) through the reaction with ROS:

At two days post impact, 25 to 45% decrease in both water-soluble antioxidants occurred in rats experiencing sTBI. Consequent increase in signatures of oxidative/nitrosative stress was also recorded. Administration of LWM-DS significantly ameliorated the concentrations of both ascorbic acid and reduced glutathione (GSH) with evident decrease of cerebral tissue nitrites and nitrates. These effects were more remarkable when 15 mg kg/b.w. where used.

Table 18 - Concentrations of antioxidants and oxidative/nitrosative stress biomarkers measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 |
|---|---|---|---|---|---|
| ASCORBIC ACID | 3315.38 $\pm$ 351.59 | **2577.87 $\pm$ 148.36** | **2567.35 $\pm$ 147.76** | **2626.68 $\pm$ 151.17** | *2783.04 $\pm$ 160.17* |
| GSH | 3521.63 $\pm$ 275.04 | **1972.14 $\pm$ 287.59** | *2337.06 $\pm$ 340.81* | **2067.79 $\pm$ 301.54** | *2418.94 $\pm$ 352.75* |
| MDA | 0.85 $\pm$ 0.26 | **27.30 $\pm$ 4.45** | *44.00 $\pm$ 7.17* | *32.73 $\pm$ 5.33* | *18.28 $\pm$ 2.98* |
| NO$_2$ | 142.93 $\pm$ 28.19 | **232.31 $\pm$ 27.99** | **158.36 $\pm$ 19.08** | **218.12 $\pm$ 26.28** | **72.29 $\pm$ 8.71** |
| NOs | 169.51 $\pm$ 20.79 | **266.82 $\pm$ 58.06** | *99.16 $\pm$ 21.58* | **148.41 $\pm$ 32.30** | *56.50 $\pm$ 12.30* |

*Effects of increasing doses of LMW-DS on de-phosphorylated purines and pyrimidines*

The majority of the compounds reported in Table 19 originates from the degradation pathways of purine and pyrimidine nucleotides and are indirectly connected to cell energy metabolism. Rats receiving sTBI had higher cerebral concentrations of all these compounds, but CDP-choline, most of which were positively affected by the drug administration.

Table 19 - Concentrations of de-phosphorylated purines and pyrimidines measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 |
|---|---|---|---|---|---|
| CYTOSINE | 14.14 $\pm$ 3.38 | **20.19 $\pm$ 2.47** | **13.47 $\pm$ 1.65** | **13.65 $\pm$ 1.67** | **13.57 $\pm$ 1.66** |
| CREATININE | 17.12 $\pm$ 2.49 | **31.08 $\pm$ 5.79** | **17.66 $\pm$ 3.29** | *10.22 $\pm$ 1.90* | *11.77 $\pm$ 2.19* |
| URACIL | 10.91 $\pm$ 2.27 | **15.64 $\pm$ 3.06** | 17.18 $\pm$ 3.36 | 17.83 $\pm$ 3.48 | 15.55 $\pm$ 3.04 |
| β-PSEUDOURIDINE | 6.89 $\pm$ 1.27 | **8.51 $\pm$ 1.71** | *11.64 $\pm$ 2.3* | *10.41 $\pm$ 2.09* | 7.84 $\pm$ 1.57 |
| CYTIDINE | 12.76 $\pm$ 2.59 | **10.07 $\pm$ 1.82** | **13.79 $\pm$ 2.49** | *7.47 $\pm$ 1.35* | 11.46 $\pm$ 2.07 |
| HYPOXANTHINE | 7.57 $\pm$ 0.93 | **15.22 $\pm$ 2.49** | *4.02 $\pm$ 0.66* | *4.18 $\pm$ 0.68* | **6.82 $\pm$ 1.12** |
| GUANINE | 3.34 $\pm$ 0.88 | **5.11 $\pm$ 1.28** | *1.62 $\pm$ 0.41* | *1.68 $\pm$ 0.42* | *1.61 $\pm$ 0.40* |
| XANTHINE | 7.61 $\pm$ 1.39 | **15.82 $\pm$ 1.64** | *13.79 $\pm$ 1.43* | *6.71 $\pm$ 0.70* | *13.87 $\pm$ 1.44* |
| CDP choline | 7.97 $\pm$ 1.370 | 8.25 $\pm$ 1.23 | 8.23 $\pm$ 1.22 | *5.16 $\pm$ 0.77* | **7.07 $\pm$ 1.05** |
| URIDINE | 64.08 $\pm$ 14.14 | **131.59 $\pm$ 23.17** | *79.93 $\pm$ 14.07* | *117.21 $\pm$ 20.64* | *87.55 $\pm$ 15.41* |
| INOSINE | 89.43 $\pm$ 15.04 | **134.31 $\pm$ 17.51** | *113.85 $\pm$ 14.84* | *114.89 $\pm$ 14.98* | *142.91 $\pm$ 18.63* |
| URIC ACID | 3.36 $\pm$ 0.64 | **37.73 $\pm$ 7.74** | *52.42 $\pm$ 10.75* | *11.22 $\pm$ 2.30* | *26.00 $\pm$ 5.33* |
| GUANOSINE | 21.10 $\pm$ 5.56 | 19.69 $\pm$ 3.27 | **16.46 $\pm$ 2.73** | *30.97 $\pm$ 5.15* | **24.35 $\pm$ 4.05** |
| ADENOSINE | 46.71 $\pm$ 7.39 | **68.07 $\pm$ 16.30** | 68.91 $\pm$ 16.50 | 92.58 $\pm$ 22.16 | **53.25 $\pm$ 12.75** |

*Effects of increasing doses of LMW-DS on N-acetylaspartate (NAA)*

NAA is the most abundant N-acetylated amino acid of the mammalian brain, with concentrations almost equaling those of the neurotransmitter glutamate in humans. Notwithstanding the biological role of NAA has not yet been fully elucidated, it has been shown, in both preclinical and clinical studies, that TBI decreases NAA concentrations and that its time course changes following head injury mirrors those of ATP. Particularly, sTBI causes an irreversible modification in NAA homeostasis, therefore NAA is a good surrogate marker of brain energy metabolism and decrease and recovery of NAA levels are much slower than symptom clearance in post-concussed athletes. Hence, NAA has a particular relevance in studies on TBI.

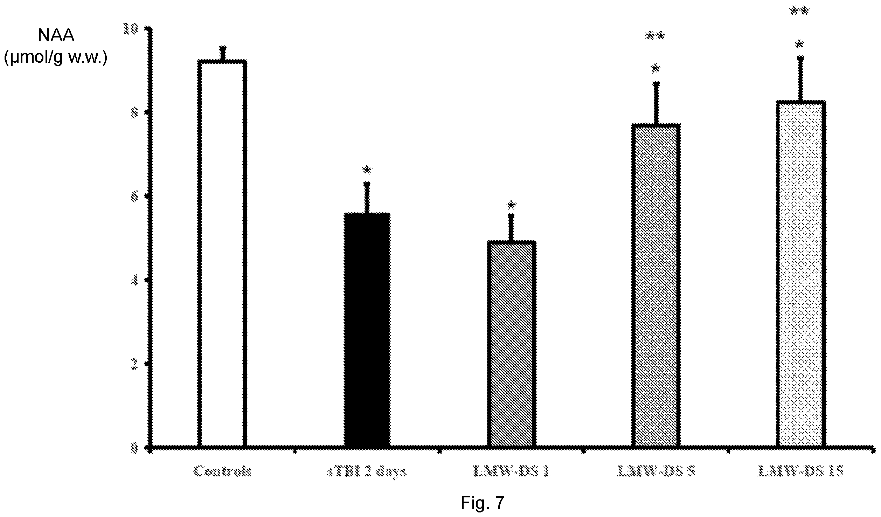

Decrease by 40% in whole brain NAA was observed in sTBI rats (Fig. 7) at two days post impact. LMW-DS produced beneficial effects on NAA concentrations when administered at 5 or 15 mg/kg b.w. Although significantly lower than controls, NAA in rats administered with either one of the two drug dosages was significantly higher than values found in sTBI rats, with highest NAA levels found in rats receiving the highest dose of LMW-DS.

*Effects of increasing doses of LMW-DS on free amino acids involved in neurotransmission*

Compounds listed in Table 20 are amino acids directly (GLU, GABA) of indirectly (GLN, ASP, ASN, GLY, SER, THR, ALA) involved in neurotransmission. Particularly, GLU is the main excitatory amino acid, counteracted in its action by GABA. Excitotoxicity of GLU is modulated by SER, GLY, THR and ALA and it is linked to the function of the GLU-GLN cycle involving neurons and astrocytes. As shown in a previous study (Amorini et al., J Cell Mol Med. 2017; 21(3): 530-542), most of these amino acids increased in sTBI rats at two days post injury. Treating animals with a single administration of LMW-DS was partly effective when the drug was subcutaneously infused at 5 or 15 mg/kg b.w. In most cases, values of the different compounds were significantly better than those found in the group of untreated sTBI animals but not than those of controls.

Table 20 - Concentrations of free amino acids with neurotransmitter functions measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 |
|---|---|---|---|---|---|
| ASP | 2.88 ± 0.88 | **4.55 ± 0.63** | **4.17 ± 0.99** | **4.15 ± 0.95** | <u>**3.05 ± 0.42**</u> |
| GLU | 9.92 ± 0.83 | **12.88 ± 0.60** | **12.52 ± 0.91** | **11.93 ± 0.55** | *11.79 ± 0.55* |
| ASN | 0.10 ± 0.03 | **0.14 ± 0.02** | **0.13 ± 0.02** | *0.17± 0.03* | *0.17± 0.03* |
| SER | 0.64 ± 0.17 | **0.82 ± 0.07** | *0.91 ± 0.07* | *0.91 ± 0.07* | *0.76 ± 0.06* |
| GLN | 3.89 ± 0.87 | 4.34 ± 0.42 | 4.37 ± 0.59 | **4.55 ± 0.44** | **4.21** ± 0.51 |
| GLY | 0.78 ± 0.13 | **1.38 ± 0.27** | **1.35 ± 0.26** | **1.43 ± 0.28** | **1.18 ± 0.23** |
| THR | 0.69 ± 0.18 | 0.76 ± 0.16 | 0.70 ± 0.15 | 0.77 ± 0.17 | <u>**0.61 ± 0.13**</u> |
| ALA | 0.41 ± 0.11 | **0.58 ± 0.06** | *0.76 ± 0.08* | *0.79 ± 0.08* | *0.68 ± 0.07* |
| GABA | 1.36 ± 0.22 | **1.93** ± 0.17 | **1.87 ± 0.17** | **1.99 ± 0.18** | *1.58 ± 0.14* |

*Effects of increasing doses of LMW-DS on free amino acids involved in the methyl cycle*

Free amino acids reported in Table 21 are involved either in the so called methyl cycle, regulating the homeostasis of compounds acting as methyl donors in cell metabolism, or in the formation of cysteine, the sole amino acid having a free -SH group. Severe head trauma caused significant changes in the main actors of this important metabolic pathway. Restoration of methionine was accomplished by LWM-DS at any dose tested. Drug treatment was partly effective in normalizing the other amino acids. Comments to changes in L-Cystathionine (L-Cystat) will be given in the corresponding Table at 7 days post impact.

Table 21 - Concentrations of free amino acids involved in the methyl cycle and homeostasis of -SH groups measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean $\pm$ S.D. of 12 animals in each group and are expressed as nmol/g w.w.

| Compound | Controls | TBI only | LMW-DS 1 | LMW-DS 5 ( | LMW-DS 15 |
|---|---|---|---|---|---|
| SAH | 0.03 ± 0.01 | **0.07 ± 0.01** | **0.07 ± 0.02** | **0.07 ± 0.02** | **0.06 ± 0.02** |
| L-Cystat | 0.15 ± 0.03 | **0.31 ± 0.06** | *0.25 ± 0.05* | **0.31 ± 0.06** | *0.41 ± 0.08* |
| MET | 0.03 ± 0.01 | **0.02 ± 0.01** | <u>**0.04 ± 0.01**</u> | *0.05 ± 0.01* | <u>**0.03 ± 0.01**</u> |

*Effects of increasing doses of LMW-DS on free amino acids involved in the generation of nitric oxide (NO)*

Table 22 illustrates concentrations of the free amino acids directly involved in the generation of NO, in the reaction catalyzed by nitric oxide synthases (NOS), a family of enzymes existing in three isoforms: endothelial NOS (eNOS), neuronal NOS (nNOS), inducible NOS (iNOS). The last isoform (iNOS) is the one involved in nitrosative stress. Nitric oxide is generated through a complex reaction in which arginine (ARG) donates a nitrogen atom undergoing a partial oxidation and forming citrulline (CITR) and NO. Animals at 2 days post sTBI showed concomitant decrease in ARG and increase in CITR, in line with data showing increase in the stable NO end products nitrites and nitrates (Table 18). Administration of LMW-DS was particularly effective when the 15 mg/kg b.w. dose was used.

Table 22 - Concentrations of free amino acids involved in nitric oxide formation measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 |
|---|---|---|---|---|---|
| CITR | 0.03 ± 0.01 | **0.03 ±0.01** | *0.01 ± 0.01* | *0.02 ± 0.01* | *0.01 ± 0.01* |
| ARG | 0.17 ± 0.03 | **0.11 ± 0.03** | **0.13 ± 0.03** | **0.13 ± 0.03** | <u>**0.16 ± 0.04**</u> |
| ORN | 0.02 ± 0.01 | 0.02 ± 0.01 | 0.02 ± 0.01 | 0.01 ± 0.01 | 0.02 ± 0.01 |

*Effects of increasing doses of LMW-DS on long-chain free amino acids*

The free amino acids reported in Table 23 represents a source of carbon skeleton useful to generate $\alpha$-ketoacids that cells use to replenish the TCA cycle. Among these compounds, only isoleucine (ILE) was significantly affected by sTBI and restored in rats receiving drug treatment.

Table 23 - Concentrations of long chain free amino acids measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 |
|---|---|---|---|---|---|
| VAL | 0.07 ± 0.02 | 0.06 ± 0.03 | 0.07 ± 0.03 | 0.08 ± 0.03 | 0.06 ± 0.03 |
| ILE | 0.03 ± 0.01 | **0.05 ± 0.01** | *0.10 ± 0.02* | *0.10 ± 0.02* | *0.06 ± 0.01* |
| LEU | 0.04 ± 0.01 | 0.04 ± 0.01 | *0.09 ± 0.02* | *0.10 ± 0.02* | 0.04 ± 0.01 |
| LYS | 0.23 ± 0.03 | 0.28 ± 0.10 | 0.29 ± 0.11 | **0.37 ± 0.14** | **0.32 ± 0.12** |

*Effects of increasing doses of LMW-DS on free amino acids acting as osmolytes and aromatic free amino acids*

Results summarized in Table 24 clearly show that sTBI caused the increase in the concentrations of all these free amino acids. Particularly, the increase in taurine (TAU) may suggest the attempt to counteract cell edema by increasing the levels of one of the most important brain osmolyte. Differently, increase in aromatic free amino acids may suggest reduced biosynthesis of the neurotransmitters serotonin (formed from tryptophan) and dopamine (generated from the biotransformation of phenylalanine first and tyrosine then). No remarkable effects of LMW-DS administration were observed at this time point after impact.

Table 24 - Concentrations of free amino acids acting as osmolytes and aromatic free amino acids measured in deproteinized brain homogenates of rats sacrificed at 2 days post-TBI without and with a single administration of increasing doses of LWM-DS (1, 5 and 15 mg/kg b.w.), performed 30 minutes after brain trauma induction. Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 |
|---|---|---|---|---|---|
| TAU | 3.82 ± 0.61 | **4.84 ± 0.46** | **4.98 ± 0.47** | **5.15 ± 0.49** | **4.59 ± 0.43** |
| HYS | 0.05 ± 0.01 | **0.06 ± 0.01** | *0.08 ± 0.01* | *0.11 ± 0.02* | *0.10 ± 0.01* |

(continued)

| Compound | Controls | TBI only | LMW-DS 1 | LMW-DS 5 | LMW-DS 15 |
|---|---|---|---|---|---|
| TYR | 0.13 ± 0.03 | **0.17 ± 0.03** | **0.18 ± 0.03** | **0.20 ± 0.03** | **0.17 ± 0.03** |
| TRP | 0.01 ± 0.01 | **0.02 ± 0.01** | 0.02 ± 0.01 | 0.03 ± 0.01 | *0.04 ± 0.01* |
| PHE | 0.03 ± 0.01 | **0.05 ± 0.01** | *0.07 ± 0.03* | *0.07 ± 0.03* | *0.06 ± 0.01* |

SUMMARY OF BIOCHEMICAL DATA RECORDED AT 7 DAYS POST sTBI

*Effects of increasing doses of LMW-DS on brain energy metabolism measured*

Table 25 summarizes values referring to phosphorylated high-energy purine and pyrimidine compounds. It is particularly evident the no amelioration of the depletion of triphosphate nucleotides (ATP, GTP, UTP and CTP) was observed at 7 days post sTBI. Concomitant increase in AMP and ADP was accompanied by significant changes in the concentrations of UDP derivatives (UDP-Glc, UDP-Gal, UDP-GlcNac and UDP- GalNac). In general, it should be underlined that longer times post injury were often characterized by worsening of the biochemical, metabolic, molecular alterations induced by sTBI.

At this time post injury, treatment with LMW-DS produced a general improvement of cerebral energy metabolism, more evident when drug administration dose was higher than 1 mg/kg b.w. Although differences with controls were recorded even in rats receiving repeat administration of 15 mg/kg b.w. LWM-DS, significantly higher values of nucleotide triphosphates were found in drug treated animals. Of particular relevance is the progressive recovery of the calculated, adimensional value of the ATP/ADP ratio (which is considered as a good indicator of the mitochondrial phosphorylating capacity) that progressively increased by increasing the dose of drug administered to sTBI animals.

Table 25 - Concentrations of energy metabolites (phosphorylated purines and pyrimidines) measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| CMP | 13.52 ± 3.44 | **30.98 ± 3.18** | **25.41 ± 10.81** | **55.67 ± 22.97** | *47.10 ± 20.04* | **31.21 ± 13.28** |
| UMP | 82.30 ± 9.82 | **139.70 ± 27.06** | *103.06 ± 19.96* | 167.18 ± 32.39 | *181.82 ± 35.22* | *107.66 ± 20.86* |
| IMP | 51.57 ± 4.61 | **110.07 ± 28.19** | *80.16 ± 20.53* | *68.72 ± 17.60* | *74.70 ± 19.13* | *141.84 ± 36.32* |
| GMP | 82.81 ± 7.82 | **164.41 ± 77.81** | 113.06 ± 53.51 | <u>101.42 ± 48.00</u> | *41.55 ± 19.66* | *61.86 ± 29.28* |
| UDP-Glc | 51.00 ± 10.89 | **39.28 ± 7.98** | *63.19 ± 12.84* | <u>58.10 ± 11.81</u> | 62.97 ± 12.80 | 61.37 ± 12.47 |
| UDP-Gal | 131.00 ± 13.26 | **112.58 ± 7.74** | <u>130.20 ± 8.95</u> | <u>132.66 ± 9.12</u> | <u>137.57 ± 9.46</u> | <u>135.15 ± 9.29</u> |
| UDP-GlcNac | 88.77 ± 19.55 | **134.24 ± 46.44** | <u>85.36 ± 29.53</u> | <u>85.14 ± 29.45</u> | *67.47 ± 23.34* | <u>86.42 ± 29.89</u> |
| UDP-GalNac | 38.82 ± 9.83 | **13.08 ± 3.75** | **15.85 ± 4.54** | *17.37 ± 4.98* | *17.91 ± 5.13* | 16.50 ± 4.73 |
| GDP Glucose | 85.35 ± 12.76 | 90.43 ± 10.58 | *112.22 ± 13.13* | *104.76 ± 12.25* | *101.65 ± 11.89* | *106.42 ± 12.45* |
| AMP | 43.59 ± 9.90 | **55.86 ± 4.39** | <u>43.13 ± 3.39</u> | 59.50 ± 4.68 | *50.50 ± 3.97* | <u>43.12 ± 3.39</u> |
| UDP | 23.94 ± 6.75 | **45.30 ± 6.37** | *38.59 ± 5.43* | 44.19 ± 6.22 | *37.91 ± 5.33* | *37.12 ± 5.22* |

(continued)

| Compound | Controls | TBI only | LMW-DS 1 | LMW-DS 5 | LMW-DS 15 | LMW-DS 15-R |
|---|---|---|---|---|---|---|
| GDP | 57.40 ± 14.06 | **112.05 ± 12.80** | 121.72 ± 13.91 | *126.82 ± 14.49* | 122.07 ± 13.95 | <u>109.06 ± 12.46</u> |
| ADP-Ribose | 12.69 ± 1.43 | **22.64 ± 5.68** | *7.95 ± 1.99* | *6.76 ± 1.70* | 19.21 ± 4.82 | <u>**13.23 ± 3.32**</u> |
| CTP | 41.85 ± 10.32 | 34.12 ± 9.03 | *81.75 ± 31.55* | *79.08 ± 14.54* | *96.44 ± 25.54* | *92.67 ± 16.27* |
| ADP | 222.67 ± 30.99 | **302.60 ± 40.30** | 286.78 ± 38.19 | 289.27 ± 38.52 | 276.83 ± 36.87 | 260.32 ± 34.67 |
| UTP | 152.64 ± 17.39 | **108.55 ± 19.01** | *179.75 ± 31.48* | *175.02 ± 30.65* | 127.42 ± 22.32 | 133.72 ± 23.42 |
| GTP | 569.00 ± 45.32 | **375.24 ± 34.12** | *438.65 ± 39.88* | *453.86 ± 41.27* | *479.98 ± 43.64* | *466.06 ± 42.38* |
| ATP | 2390.14 ± 213.98 | **1561.36 ± 125.60** | *1792.01 ± 144.16* | 1730.92 ± 139.24 | 1846.63 ± 148.55 | *1971.17 ± 158.57* |
| ATP/ADP | 10.99 ± 2.21 | **5.23 ± 0.66** | *6.12 ± 0.78* | *6.28 ± 0.80* | *6.76 ± 0.86* | *7.67 ± 0.97* |

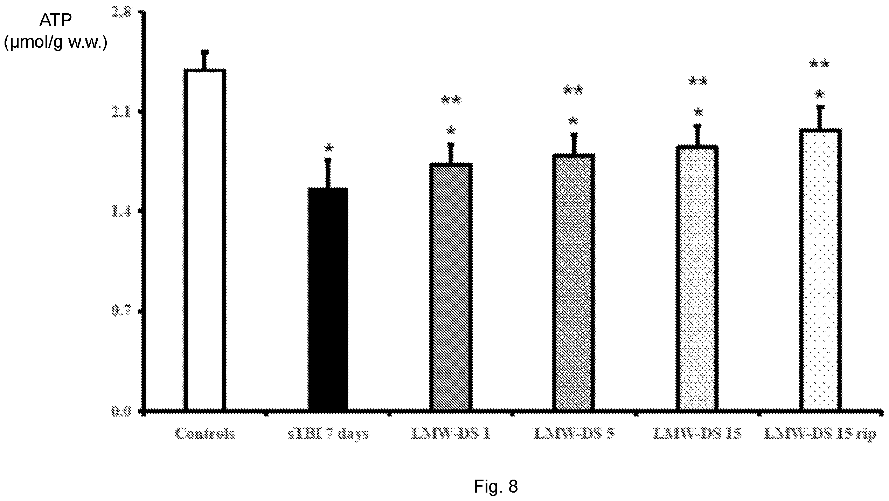

To better show that drug effects were related to the drug dosage, we graphically reported in Fig. 8 results concerning ATP. It is possible to observe that ATP increase was related to the dosage administered and that drug administration produced significant increases of the most important high energy phosphate at any dose tested.

*Effects of increasing doses of LMW-DS on nicotinic coenzymes*

Values of oxidized (NAD+ and NADP+) and reduced (NADH and NADPH) nicotinic coenzymes are summarized in Table 26. Table 26 also reports the calculated, adimensional value of the NADVNADH ratio which is suitable to evaluate how much metabolism is dependent on glycolysis or on mitochondrial oxidative phosphorylation.

As formerly observed, profound decrease of nicotinic coenzymes and of the NAD+/NADH ratio was recorded in sTBI rats at 7 days post injury. With the exclusion of the lowest dose, treatment with LMW-DS produced significant improvement of the concentrations of nicotinic coenzymes. Particularly, single and repeat administration of 15 mg/kg b.w. LMW-DS were able to normalize NAD+ level and to restore the correct NAD+/NADH ratio determined in control animals.

Table 26 - Concentrations of nicotinic coenzymes measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| NAD+ | 485.74 ± 37.06 | **249.37 ± 35.32** | 268.14 ± 37.97 | *293.36 ± 41.55* | <u>491.52 ± 69.61</u> | 401.73 ± 56.89 |
| NADH | 13.57 ± 1.94 | **8.98 ± 1.55** | 8.20 ± 1.41 | 8.83 ± 1.26 | *11.65 ± 1.63* | *11.05 ± 1.52* |
| NADP+ | 23.17 ± 4.58 | **11.69 ± 4.29** | *39.94 ± 14.65* | <u>24.45 ± 8.97</u> | <u>23.75 ± 8.72</u> | *16.56 ± 6.08* |
| NADPH | 8.51 ± 0.71 | **10.66 ± 2.48** | *18.91 ± 4.39* | 12.30 ± 2.86 | *6.66 ± 1.55* | 11.21 ± 2.60 |
| NAD+/NADH | 36.47 ± 5.46 | **27.51 ± 5.83** | 33.91 ± 9.32 | <u>33.90 ± 7.19</u> | *42.51 ± 5.26* | <u>**37.47 ± 9.46**</u> |

*Effects of increasing doses of LMW-DS on CoA-SH derivatives*

Table 27 reports data referring to free CoA-SH and CoA-SH derivatives. Remarkable positive effects of the administration of 5 or 15 mg/kg b.w. (this dose both as a single and repeat administration) were detected both for CoA-SH and Acetyl-CoA, suggesting much more favorable metabolic conditions for the functioning of the TCA cycle.

Table 27 - Concentrations of free CoA-SH and CoA-SH derivatives (Acetyl-CoA and Malonyl-CoA) measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| Malonyl-CoA | 15.02 ± 2.38 | **13.01 ± 2.35** | *5.43 ± 0.98* | *6.02 ± 1.09* | *7.98 ± 1.44* | **12.56 ± 2.27** |
| CoA-SH | 26.31 ± 3.86 | 26.44 ± 3.39 | *38.50 ± 4.94* | *51.86 ± 6.66* | *64.93 ± 8.33* | **45.76 ± 5.87** |
| Acetyl-CoA | 36.97 ± 5.43 | **18.28 ± 3.11** | *27.05 ± 4.61* | *22.87 ± 3.89* | *38.60 ± 6.57* | *37.91 ± 6.46* |

*Effects of increasing doses of LMW-DS on antioxidants and oxidative/nitrosative stress biomarkers* Table 28 shows the concentrations of the main water-soluble brain antioxidants (ascorbic acid and GSH) and of biomarkers of oxidative (MDA) and nitrosative stress (-$NO_2^-$ and -$NO_3^-$). At 7 days post impact, no recovery in the concentrations of both water-soluble antioxidants occurred in rats experiencing sTBI. Remarkably high levels of signatures of oxidative/nitrosative stress were also recorded. The effects of the administration of the highest single and repeat dose of LWM-DS were particularly beneficial to rescue the concentrations of both ascorbic acid and reduced glutathione (GSH) with evident decrease of cerebral tissue nitrites and nitrates. These effects were also significant when 5 mg kg/b.w. where used.

Table 28 - Concentrations of antioxidants and oxidative/nitrosative stress biomarkers measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| ASCORBIC ACID | 3315.38 ± 351.59 | **2251.89 ± 271.20** | **2177.22 ± 262.21** | **2195.87 ± 264.45** | *2853.35 ± 343.64* | *2617.09 ± 315.18* |
| GSH | 3521.63 ± 275.04 | **1752.50 ± 231.01** | **1627.30 ± 214.51** | *2412.17 ± 317.97* | *2390.89 ± 315.16* | *2342.03 ± 308.72* |
| MDA | 0.85 ± 0.26 | **10.70 ± 1.77** | *32.98 ± 5.44* | *17.78 ± 2.94* | *6.23 ± 1.03* | *4.09 ± 0.67* |
| NO$_2$ | 142.93 ± 28.19 | **241.72 ± 52.37** | *93.04 ± 20.16* | *59.61 ± 12.91* | *110.72 ± 23.99* | *130.69 ± 28.31* |
| NO$_3$ | 169.51 ± 20.79 | **315.71 ± 53.92** | *153.62 ± 26.24* | *234.45 ± 40.05* | *161.99 ± 27.67* | *271.69 ± 46.41* |

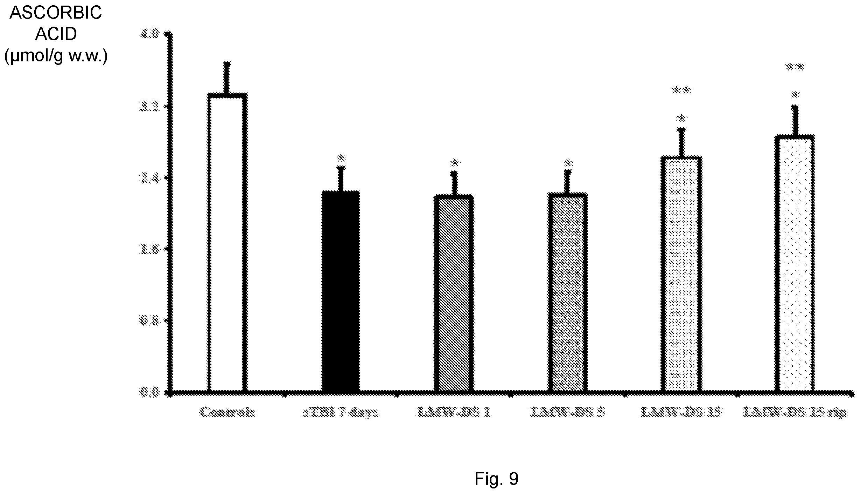

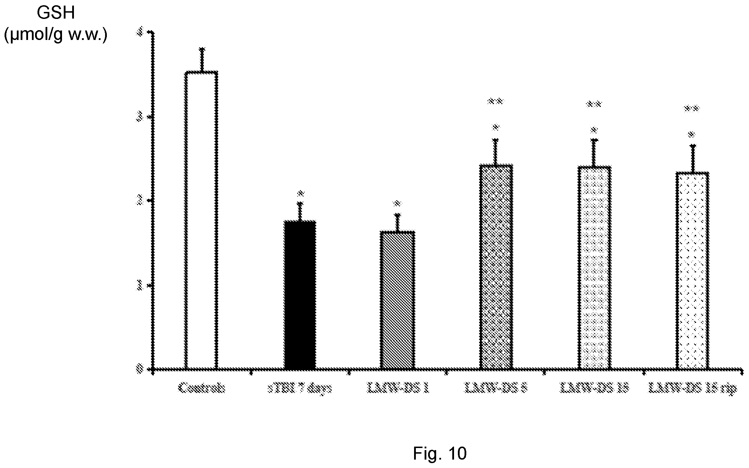

To better appreciate that drug effects were related to the drug dosage, results concerning Ascorbic acid and GSH are graphically reported in Figs. 9 and 10.

*Effects of increasing doses of LMW-DS on de-phosphorylated purines and pyrimidines*

A further worsening in the majority of the compounds reported in Table 29, originating from the degradation pathways of purine and pyrimidine nucleotides and indirectly connected to cell energy metabolism, were observed in rats receiving sTBI at 7 days post injury. Most of these compounds were positively affected by the drug administration.

Table 29 - Concentrations of de-phosphorylated purines and pyrimidines measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean ± S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| CYTOSINE | 14.14 ± 3.38 | **21.43 ± 4.60** | **16.03 ± 3.44** | **12.67 ± 2.72** | **13.87 ± 2.98** | ***8.76 ± 1.88*** |
| CREATININE | 17.12 ± 2.49 | **7.68 ± 1.36** | ***6.57 ± 1.16*** | ***5.48 ± 0.97*** | ***5.23 ± 0.92*** | ***9.07 ± 1.60*** |
| URACIL | 10.91 ± 2.27 | **22.71 ± 4.67** | ***14.78 ± 3.04*** | ***18.34 ± 3.77*** | ***15.92 ± 3.27*** | **24.13 ± 4.96** |
| β-PSEUDOURIDINE | 6.89 ± 1.27 | **23.36 ± 4.33** | **14.00 ± 2.60** | **17.51 ± 3.25** | **63.77 ± 11.83** | ***16.72 ± 3.10*** |
| CYTIDINE | 12.76 ± 2.59 | **29.68 ± 10.44** | **29.67 ± 10.44** | **26.51 ± 9.33** | **33.06 ± 11.63** | ***72.85 ± 25.63*** |
| HYPOXANTHINE | 7.57 ± 0.93 | **24.66 ± 7.18** | ***16.97 ± 4.94*** | **13.45 ± 3.91** | ***10.21 ± 2.97*** | **4.10 ± 1.19** |
| GUANINE | 3.34 ± 0.87 | **5.21 ± 2.22** | **6.86 ± 2.92** | **7.92 ± 3.37** | **5.27 ± 2.24** | **3.32 ± 1.41** |
| XANTHINE | 7.61 ± 1.39 | **13.58 ± 3.84** | **12.53 ± 3.54** | **14.33 ± 4.05** | **12.71 ± 3.60** | **11.24 ± 3.18** |
| CDP choline | 7.97 ± 1.37 | 7.90 ± 2.54 | **6.26 ± 2.01** | **10.37 ± 3.33** | 10.06 ± 3.23 | ***11.72 ± 3.76*** |
| URIDINE | 64.08 ± 14.14 | **84.44 ± 20.01** | ***110.17 ± 26.11*** | **134.60 ± 31.89** | **134.04 ± 31.76** | **97.21 ± 23.03** |
| INOSINE | 89.43 ± 15.04 | **139.98 ± 15.70** | ***124.27 ± 13.94*** | **196.61 ± 22.06** | ***104.41 ± 11.72*** | **139.26 ± 15.62** |
| URIC ACID | 3.36 ± 0.64 | **25.06 ± 5.96** | ***7.13 ± 1.70*** | ***17.26 ± 4.11*** | ***8.60 ± 2.05*** | ***7.80 ± 1.86*** |
| GUANOSINE | 21.10 ± 5.56 | **31.85 ± 6.64** | **19.11 ± 3.98** | **33.42 ± 6.96** | **20.91 ± 4.36** | **19.66 ± 4.10** |
| ADENOSINE | 46.71 ± 7.39 | 69.37 ± 51.38 | ***26.08 ± 19.31*** | ***22.24 ± 16.47*** | 55.95 ± 41.44 | 40.84 ± 30.25 |

*Effects of increasing doses of LMW-DS on N-acetylaspartate (NAA)*

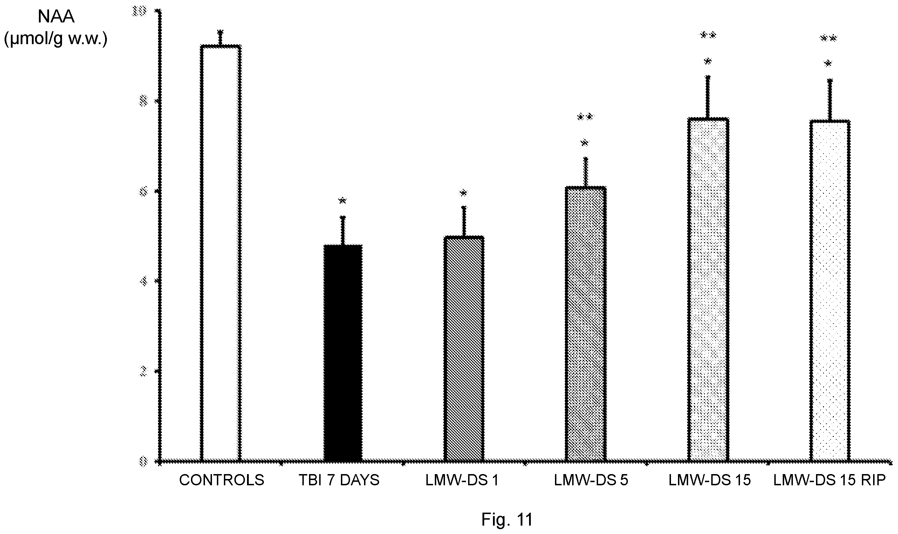

As previously mentioned, sTBI causes an irreversible modification in NAA homeostasis. Even in this study, at 7 days post sTBI whole brain NAA was about 50% lower than that measured in control rats, see Fig. 11 Interestingly, a dose dependent increase in NAA was detected in rats receiving increasing doses of single LMW-DS or repeat administrations of the maximal dose tested.

*Effects of increasing doses of LMW-DS on free amino acids involved in neurotransmission*

Compounds listed in Table 30 are amino acids directly (GLU, GABA) of indirectly (GLN, ASP, AASN, GLY, SER, THR, ALA) involved in neurotransmission. Most of these amino acids had still higher in sTBI rats at 7 days post injury when compared with controls. It is evident from this Table 30 that administration of LMW-DS was effective particularly when the drug was subcutaneously infused at 15 mg/kg b.w., either in a single or in repeat administrations. Particularly relevant is the normalization of GLU, thus indicating that LMW-DS is capable to abolish excitotoxicity cause by excess GLU release after sTBI.

Table 30 - Concentrations of free amino acids with neurotransmitter functions measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| ASP | 2.88 $\pm$ 0.88 | **4.14 $\pm$ 0.75** | **4.17 $\pm$ 0.67** | **3.63 $\pm$ 0.59** | ***2.29 $\pm$ 0.37*** | ***2.42 $\pm$ 0.39*** |
| GLU | 9.92 $\pm$ 0.83 | **12.26 $\pm$ 1.03** | **12.14 $\pm$ 1.02** | **11.82 $\pm$ 0.99** | **10.25 $\pm$ 0.86** | ***10.78 $\pm$ 0.91*** |
| ASN | 0.10 $\pm$ 0.03 | 0.10 $\pm$ 0.02 | 0.10 $\pm$ 0.02 | 0.10 $\pm$ 0.02 | 0.10 $\pm$ 0.02 | 0.10 $\pm$ 0.02 |
| SER | 0.64 $\pm$ 0.17 | **1.04 $\pm$ 0.18** | **0.92 $\pm$ 0.16** | ***0.83 $\pm$ 0.14*** | **0.76 $\pm$ 0.12** | ***0.79 $\pm$ 0.13*** |
| GLN | 3.89 $\pm$ 0.87 | 3.97 $\pm$ 0.41 | 4.10 $\pm$ 0.42 | 3.86 $\pm$ 0.40 | 3.73 $\pm$ 0.38 | 3.88 $\pm$ 0.40 |
| GLY | 0.78 $\pm$ 0.13 | **0.91 $\pm$ 0.17** | **0.98 $\pm$ 0.20** | 0.88 $\pm$ 0.15 | **0.78 $\pm$ 0.12** | **0.78 $\pm$ 0.10** |
| THR | 0.69 $\pm$ 0.18 | 0.76 $\pm$ 0.10 | 0.71 $\pm$ 0.12 | 0.71 $\pm$ 0.15 | 0.72 $\pm$ 0.14 | 0.77 $\pm$ 0.14 |
| ALA | 0.41 $\pm$ 0.11 | **0.51 $\pm$ 0.05** | ***0.57 $\pm$ 0.06*** | **0.44 $\pm$ 0.05** | **0.38 $\pm$ 0.04** | 0.47 $\pm$ 0.05 |
| GABA | 1.36 $\pm$ 0.22 | **1.78 $\pm$ 0.18** | **1.73 $\pm$ 0.18** | ***1.63 $\pm$ 0.17*** | **1.43 $\pm$ 0.15** | **1.38 $\pm$ 0.14** |

*Effects of increasing doses of LMW-DS on free amino acids involved in the methyl cycle*

As shown in Table 31, levels of free amino acids involved either in the so called methyl cycle or in the formation of cysteine, were still different in sTBI rats at 7 days post impact, when compared to corresponding values of controls. Increase in MET was observed in animals receiving the highest dose of LWM-DS (both as single administration or as repeat administrations). As already observed at 2 days post injury, these drug levels produced a significant increase in L-Cystathionine (L-Cystat). Since this compound is an intermediate in the generation of cysteine (CYS), it is conceivable to hypothesize that increase in L-Cystat may produce a consequent increase in CYS. It is worth recalling that determination of CYS requires a specific additional HPLC assay with additional derivatization with F-MOC, a fluorescent compound that reacts with secondary amine and with CYS.

Table 31 - Concentrations of free amino acids involved in the methyl cycle and homeostasis of -SH groups measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| SAH | 0.03 $\pm$ 0.01 | **0.05 $\pm$ 0.01** | **0.04 $\pm$ 0.01** | ***0.04 $\pm$ 0.01*** | **0.04 $\pm$ 0.01** | 0.04 $\pm$ 0.04 |
| L-Cystat | 0.15 $\pm$ 0.03 | **0.23 $\pm$ 0.04** | **0.24 $\pm$ 0.04** | **0.26 $\pm$ 0.04** | **0.25 $\pm$ 0.04** | ***0.44 $\pm$ 0.07*** |
| MET | 0.03 $\pm$ 0.01 | 0.03 $\pm$ 0.01 | 0.03 $\pm$ 0.01 | 0.04 $\pm$ 0.01 | 0.04 $\pm$ 0.01 | ***0.05 $\pm$ 0.01*** |

*Effects of increasing doses of LMW-DS on free amino acids involved in the generation of nitric oxide (NO)*

Table 32 illustrates concentrations of the free amino acids directly involved in the generation of NO. Animals at 7 days post sTBI showed still concomitant decrease in ARG and increase in CITR, in line with data showing increase in the stable NO end products nitrites and nitrates (Table 18). Administration of LMW-DS was particularly effective when 5 or 15 mg/kg b.w. (single and repeat) was used.

Table 32 - Concentrations of free amino acids involved in nitric oxide formation measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| CITR | 0.03 ± 0.01 | **0.04 ± 0.02** | 0.03 ± 0.01 | 0.03 ± 0.01 | 0.03 ± 0.01 | 0.03 ± 0.01 |
| ARG | 0.17 ± 0.03 | **0.13 ± 0.02** | **0.13 ± 0.02** | <u>**0.15 ± 0.02**</u> | <u>**0.14 ± 0.02**</u> | *0.19 ± 0.02* |
| ORN | 0.02 ± 0.01 | **0.01 ± 0.01** | **0.01 ± 0.01** | *0.01 ± 0.01* | *0.01 ± 0.01* | <u>**0.02 ± 0.01**</u> |

*Effects of increasing doses of LMW-DS on long-chain free amino acids*

The free amino acids reported in Table 33, representing a source of carbon skeleton useful to generate α-ketoacids that cells use to replenish the TCA cycle, were practically normal at 7 days post sTBI and any other group of animals treated with the drug of interest.

Table 33 - Concentrations of long chain free amino acids measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| VAL | 0.07 ± 0.02 | 0.07 ± 0.01 | <u>**0.08 ± 0.01**</u> | <u>**0.08 ± 0.01**</u> | *0.10 ± 0.01* | 0.07 ± 0.01 |
| ILE | 0.03 ± 0.01 | 0.03 ± 0.01 | <u>*0.04 ± 0.01*</u> | <u>**0.05 ± 0.01**</u> | *0.07 ± 0.01* | 0.03 ± 0.01 |
| LEU | 0.04 ± 0.01 | 0.04 ± 0.01 | *0.05 ± 0.01* | *0.05 ± 0.01* | *0.07 ± 0.01* | 0.04 ± 0.01 |
| LYS | 0.23 ± 0.03 | **0.19 ± 0.03** | 0.19 ± 0.06 | 0.21 ± 0.04 | 0.21 ± 0.05 | 0.23 ± 0.07 |

*Effects of increasing doses of LMW-DS on free amino acids acting as osmolytes and aromatic free amino acids*

Results summarized in Table 34 clearly show that sTBI caused the increase in the concentrations of taurine (TAU) at 7 days after injury. LMW-DS administration normalized TAU concentrations and caused the increase in aromatic amino acids.

Table 34 - Concentrations of free amino acids acting as osmolytes and aromatic free amino acids measured in deproteinized brain homogenates of rats sacrificed at 7 days post-sTBI, without and with administration of increasing doses of LWM-DS (single administration of 1, 5 and 15 mg/kg b.w. and repeated administration of 15 mg/kg b.w.). Controls are represented by sham-operated animals. Values are the mean $\pm$ S.D. of 12 animals in each group and are expressed as nmol/g w.w.

| Compound | Controls | TBI only | LMW-DS 1 | LMW-DS 5 | LMW-DS 15 | LMW-DS 15-R |
|---|---|---|---|---|---|---|
| HYS | 0.05 ± 0.01 | 0.06 ± 0.01 | 0.06 ± 0.01 | 0.06 ± 0.01 | 0.06 ± 0.01 | 0.06 ± 0.01 |
| TAU | 3.82 ± 0.61 | **4.36 ± 0.56** | 4.02 ± 0.51 | <u>**3.51 ± 0.44**</u> | <u>**3.38 ± 0.44**</u> | <u>**3.47 ± 0.44**</u> |
| TYR | 0.13 ± 0.03 | 0.14 ± 0.02 | 0.13 ± 0.02 | 0.13 ± 0.02 | 0.14 ± 0.02 | 0.14 ± 0.02 |
| TRP | 0.02 ± 0.01 | 0.02 ± 0.01 | *0.01 ± 0.01* | 0.02 ± 0.01 | *0.03 ± 0.01* | *0.03 ± 0.01* |
| PHE | 0.03 ± 0.01 | 0.04 ± 0.01 | *0.05 ± 0.01* | *0.06 ± 0.01* | *0.07 ± 0.01* | *0.05 ± 0.01* |

DISCUSSION

TBI is one of the most common neurodegenerative diseases and represents the leading cause of death under 45 years of age in Western countries. Its incidence is on the rise and by 2020 the World Health Organization estimates that TBI will be the largest cause of disability worldwide. Depending on the severity of the symptoms related to TBI (evaluated by the Glasgow Coma Scale), it is possible to identify three different types of TBI: mild TBI (mTBI), moderate TBI and severe TBI (sTBI). It has been calculated that the ratio in the occurrence of mTBI to sTBI is approximately 22 to 1. Unfortunately, the consequences of a TBI are often invalidating and possibly leading to permanent or temporary impairment of cognitive, physical and psychosocial functions, with an associated diminished or altered state of consciousness. Thus, patients are affected in some important aspects, primarily the ability to be independent, to correctly work and to maintain social relationships.

TBI is considered a complicated pathological process consisting of a primary insult (the impact force acting on the brain tissue) directly inducing a scarcely predictable secondary insult characterized by a cascade of biochemical, metabolic and molecular changes causing profound mitochondrial malfunctioning in cerebral cells. The severity of the damage depends on the impact force acting on the cerebral tissue. In fact, this event induces a stretching of axonal and neuronal fibers, triggering the biochemical and molecular events, which are not simultaneous with the insurgence of clinical symptoms.

To date, there are no satisfying pharmacological treatments capable to decrease mortality and improve recovery of TBI patients. Putative pharmacological treatments are generally tested in their ability to interfere with the neurometabolic cascade triggered by the primary insult, such as the biochemical and molecular alterations occurring to the cerebral tissue metabolism, as well as the vascular and hematic flow changes strictly correlated with tissue damages.

Previous studies have demonstrated a significant correlation between the severity of TBI and energy deficit associated with the increase rate of the anaerobic metabolism, mitochondrial dysfunction, increase in production of reactive oxygen (ROS) and nitrogen species (RNS) and enhance in excitatory amino acid release. Moreover, N-acetylated amino acid N-acetylaspartate (NAA) is a reliable surrogate biomarker useful to monitor *in vivo* the state of the energetic metabolism. Indeed, since mitochondrial NAA biosynthesis has a high indirect energy expenditure, changes in NAA intracerebral concentration are closely related to changes in homeostasis of some parameters related to energy metabolism (ATP, GTP, ADP, AMP, Acetyl-CoA, CoA-SH and NAD+) and to mitochondrial phosphorylating capacity (ATP/ADP).

The study conducted to evaluate the effects of increasing doses of LMW-DS on a large panel of brain metabolites in rats experiencing sTBI at different times post injury evidenced that the administration of this compound produces a general amelioration of cerebral metabolism.

LMW-DS was effective in restoring mitochondrial related energy metabolism, profoundly imbalanced in sTBI animals with no treatment, with positive effects on the concentration of triphosphates purine and pyrimidine nucleotides. Particularly, ATP levels, at 7 days post impact, were only 16% lower than the value of controls, whilst in sTBI rats a 35% decrease was found (Table 25 and Fig. 8). Remarkably, NAA concentration in animals treated with LMW-DS at the same time point was only 16% lower than the value of controls, whilst sTBI animals showed 48% lower values of this compound. This finding once again strongly confirms the strict connection between the homeostasis of NAA and correct mitochondrial energy metabolism, and underlines the importance of pharmacological interventions capable to act positively on mitochondrial functioning.

The general amelioration of brain metabolism produced by LMW-DS administration also involved nicotinic coenzymes and metabolism of free CoA-SH and CoA-SH derivatives. This implies that drug treated animals, notwithstanding submitted to sTBI, had quasi-normal coenzymes to ensure correct oxido-reductive reactions and to allow a good functioning of the TCA cycle.

The aforementioned improvement of brain metabolism certainly contributed to the other remarkable drug effect, i.e., the abolishment of GLU excitotoxicity. Additionally, the drug affected sulphur-containing amino acids. Possibly, this effect might be related to the drug molecule that contains S atoms. Increasing the bioavailability of this atom might have produced a net increase in the biosynthesis of these amino acids, one of them (MET) is crucial in the methylation reaction and in the so called methyl cycle.

Further positive effects recorded in this study were the increase in antioxidants and the decrease of biochemical signatures of oxidative/nitrosative stress in sTBI rats receiving administration of LMW-DS. Even this phenomenon might well be connected with the normalization of mitochondrial functions, since dysfunctional mitochondria are the main intracellular source of both ROS and RNS. Of relevance is that the effects of LMW-DS were more evident at 7 than at 2 days post sTBI. This strongly suggests that the general amelioration of brain metabolism caused by the drug administration is not a transitory phenomenon. Also, it is worth underlining that, under the present experimental conditions, drug effects are often related to the dose administered, even though the repeat administration of 15 mg/kg b.w. was often similar to the single administration of the same dosage. That is, it was not always advantageous to repeat the administration of the drug.

This contradictory result might have the following explanation: 1) it is well known that sTBI induces breakdown of the blood brain barrier (BBB); 2) it is possible that uptake by the brain tissue of LMW-DS is highly favored during period of BBB alterations/breakdown; 3) if the hypothesis in point 2) is correct, then the administration performed at 30 minutes post injury might had occurred when BBB was still open/altered; 4) if the hypotheses of points 2) and 3) are correct, then the administration early post injury, when BBB is still open/altered, might have facilitated the passage of the compound within the cerebral compartment, allowing the drug to elicit its beneficial effects on brain metabolism and functions, including normalization of the BBB; 5) if what reported in point 4) is correct, it means that the administration of 15 mg/kg b.w. of LMW-DS at 30 minutes post sTBlin addition to start brain metabolism normalization, also caused the closure of the BBB so that the second (at 3 days) and the third (at 5 days) drug administrations occurred under unfavorable condition for a further significant passage within the brain compartment, thus limiting the possibility to obtain additional effects with a repeat drug administration protocol.

EXAMPLE 4

The aim of this Example was to determine the neuroprotective effects of different doses of LMW-DS (1, 5 and 15 mg/kg) in sTBI using gene expression studies followed by functional analysis of the differentially regulated genes.

MATERIALS AND METHODS

*Induction of sTBI and drug administration protocol*

The experimental protocol used in this study was approved by the Ethical Committee of the Catholic University of Rome, according to international standards and guidelines for animal care. Male Wistar rats of 300-350 g body weight were fed with standard laboratory diet and water *ad libitum* in a controlled environment. As the anesthetic mixture, the animals received 35 mg/kg b.w. ketamine and 0.25 mg/kg body weight midazolam by *i.p.* injection. Severe traumatic brain injury (sTBI) was induced by dropping a 450 g weight from 2 m height on to the rat head that had been protected by a metal disk previously fixed on the skull, according to the "weight drop" impact acceleration model (Marmarou et al., J Neurosurg. 1994; 80: 291-300). Rats that suffered from skull fracture, seizures, nasal bleeding, or did not survive the impacts, were excluded from the study. At the end of each period of treatment, rats were anesthetized again and then immediately sacrificed.

*Test compound*

LMW-DS (Tikomed AB) was provided at a stock concentration of 20 mg/ml and was kept in a temperature-monitored refrigerator at 4°C. LMW-DS aliquots were diluted to the appropriate dosing concentration in sterile saline prior to delivery of a single subcutaneous injection.

Acute phase -1

Three doses of LMW-DS were administered subcutaneously 30 minutes post-TBI. The animals were sacrificed at 2 days post-TBI. The animals were divided into the following subgroups:

1. n = 4 animals subjected to sTBI and receiving a subcutaneous injection of 0.5 ml of LMW-DS at a concentration of 15 mg/kg
2. n = 4 animals subjected to sTBI and receiving a subcutaneous injection of 0.5 ml of LMW-DS at a concentration of 5 mg/kg
3. n = 4 animals subjected to sTBI and receiving a subcutaneous injection of 0.5 ml of LMW-DS at a concentration of 1 mg/kg

Acute phase - 2

Three doses of LMW-DS were administered subcutaneously 30 minutes post-TBI. The animals were sacrificed at 7 days post-TBI. The animals were divided into the following subgroups:

4. n = 4 animals subjected to sTBI and receiving a subcutaneous injection of 0.5 ml of LMW-DS at a concentration of 15 mg/kg
5. n = 4 animals subjected to sTBI and receiving a subcutaneous injection of 0.5 ml of LMW-DS at a concentration of 5 mg/kg
6. n = 4 animals subjected to sTBI and receiving a subcutaneous injection of 0.5 ml of LMW-DS at a concentration of 1 mg/kg
7. n = 4 animals subjected to sTBI and receiving three repeated subcutaneous injections of 0.5 ml of LMW-DS at a concentration of 15 mg/kg

sTBI - no treatment

8. n = 4 animals subjected to sTBI only and sacrificed at 2 days post-TBI
9. n = 4 animals subjected to sTBI only and sacrificed at 7 days post-TBI

Sham operated (Healthy Control)

10. n = 4 animals receiving anesthesia only.

*Cerebral tissue processing*

An *in vivo* craniectomy was performed on all animals during anesthesia. After carefully removing the rat's skull, the brain was exposed and removed with a surgical spatula and quickly dropped in RNALater and preserved at 4°C for further processing.

*RNA extraction and array analysis*

RNA extraction and array processing was carried out by SourceBioscience. The arrays used were the Agilent Rat expression arrays.

*Statistical analysis*

Statistical analysis was performed to quantitate the effect of sTBI on the brain in this model. The follow-on analyses looked at the effects of LMW-DS in this model using different iterations and algorithms. Statistical analysis was carried out using the Metaboanalyst software package. Gene expression changes of 10% with a $p < 0.05$ were regarded as significant.

RESULTS

*Differential gene expression seen 2 days after sTBI*

Within 2 days of sTBI the brain gene expression changes significantly with a relatively small number of genes (221) up and downregulated.
The administration of 1 mg/kg LMW-DS within 30 minutes after injury altered the TBI-specific gene expression in 372 genes, the administration of 5 mg/kg LMW-DS within 30 minutes after TBI altered the TBI-specific gene expression in 702 genes and the administration of 15 mg/kg within 30 minutes after TBI alters the TBI-specific gene expression in 247 genes within 2 days of sTBI.
The LMW-DS treated animals differed from the healthy controls in the expression of 209 genes (1 mg/kg LMW-DS), 258 genes (5 mg/kg LMW-DS) and 47 genes (15 mg/kg LMW-DS).

*Differential gene expression seen 7 days after sTBI*

Within 7 days of sTBI the brain gene expression changes significantly with a large number of genes (2739) up and downregulated.
The administration of 1 mg/kg LMW-DS within 30 minutes after injury altered the TBI-specific gene expression in 3602 genes, the administration of 5 mg/kg LMW-DS within 30 minutes after TBI altered the TBI-specific gene expression in 3852 genes and the administration of 15 mg/kg within 30 minutes after TBI alters the TBI-specific gene expression in 3901 genes within 7 days of sTBI.
The LMW-DS treated animals differed from the healthy controls in the expression of 282 genes (1 mg/kg LMW-DS), 398 genes (5 mg/kg LMW-DS) and 158 genes (15 mg/kg LMW-DS). The LMW-DS treated animals (3 repeated doses of 15 mg/kg LMW-DS) differed from the healthy controls in the expression of 234 genes.

*Comparison analysis* of *expression changes seen with LMW-DS*

The comparison of the significantly affected genes in different statistical iterations provided information on how LMW-DS changed the TBI induced gene expression.

The comparison for 2 days post-TBI indicated that from the 221 genes deregulated by TBI (2 days) only 22 (10 %), 51 (23 %) and 19 (8.5 %) remained deregulated relative to healthy control animals when 1 mg/kg, 5 mg/kg and 15 mg/kg LMW-DS was given, respectively.

The comparison for 7 days post-TBI indicated that from the 2741 genes deregulated by TBI (7 days) only 124 (4.5 %), 169 (6.1 %) and 85 (3.1 %) remained deregulated relative to healthy control animals when 1 mg/kg, 5 mg/kg and 15 mg/kg LMW-DS was given, respectively. The remaining number of deregulated genes relative healthy animals for the 3 repeated doses of 15 mg/kg LMW-DS relative to healthy control animals were 116 (4.25 %).

*Pathway analysis and mechanistic studies*

Pathway analysis of the differentially regulated genes was carried out using the Ingenuity pathway analysis package. The analysis was performed with special reference to pathways and molecular processes and diseases associated with neurodegenerative disease, including dementia, Alzheimer's disease, ALS, TBI and stroke, and with scarring and fibrosis, including glaucoma and normal pressure hydrocephalus (NPH) after subarachnoid haemorrhage.

Although the effects induced by TBI within 2 days were relatively small, the alterations in many neurodegeneration and scaring-related canonical pathways were significant. Most of these pathway alterations were counteracted by LMW-DS given within 30 minutes of the TBI (Table 35 and 36). Similar to the pathways, the number of significantly affected molecular processes and diseases within 2 days of TBI was modest. However, the effect of TBI was mostly abolished by LMW-DS given 30 minutes after the injury (Table 37 and 38).

Table 35 - Canonical pathways affected by TBI after 2 days and the effects of LMW-DS relative to control (p values and z scores)

| Ingenuity Canonical Pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS |
|---|---|---|---|---|---|---|
| Dendritic Cell Maturation | 10.5 | 33.6 | -1 | * | | |
| Role of NFAT in Regulation of the Immune Response | 5.53 | 15.1 | -0.447 | 0.378 | | |
| Osteoarthritis Pathway | 17.6 | 43.2 | 0.447 | -1.342 | -2.646 | |
| Role of NFAT in Cardiac Hypertrophy | 18.1 | 16.1 | 0.447 | | -1.633 | |
| NF-$_K$B Signaling | 8.97 | 36.4 | 0.447 | | -2 | |
| Ephrin B Signaling | | 4 | 1 | | | |
| RhoA Signaling | | 2.58 | 1 | | | |
| Endothelin-1 Signaling | 12.2 | 14.1 | 1.633 | * | | |
| IL-1 Signaling | 3.22 | 7.14 | 2 | -1 | | |
| Axonal Guidance Signaling | 11 | 17.3 | * | | | |
| CREB Signaling in Neurons | 17.8 | 3.94 | * | | | |
| Phospholipase C Signaling | 4.22 | 11.6 | * | | | |
| Role of Osteoblasts, Osteoclasts and Chondrocytes in Rheumatoid Arthritis | 8.77 | 47.7 | * | | | |
| Thrombin Signaling | 3.11 | 10.2 | * | | | |

(continued)

| Ingenuity Canonical Pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS |
|---|---|---|---|---|---|---|
| Hepatic Fibrosis / Hepatic Stellate Cell Activation | 15.1 | 68.7 | * | | | |
| Fcy Receptor-mediated Phagocytosis in Macrophages and Monocytes | 7.62 | 6.87 | * | | | |
| VDR/RXR Activation | 4.65 | 10.2 | * | | | |
| Role of Wnt/GSK-3$\beta$ Signaling in the Pathogenesis of Influenza | | | * | | | |
| Calcium-induced T Lymphocyte Apoptosis | 3.2 | 4.29 | * | | | |
| Antioxidant Action of Vitamin C | 6.6 | 8.13 | * | | | |
| Phospholipases | | 1.76 | * | | | |
| Cdc42 Signaling | | 1.97 | * | | | |
| Role of Pattern Recognition Receptors in Recognition of Bacteria and Viruses | 11.6 | 28.6 | * | | | |
| Hepatic Cholestasis | 12.5 | 24.6 | * | | | |
| Neuroprotective Role of THOP1 in Alzheimer's Disease | 7.23 | 1.73 | * | | | |
| Type I Diabetes Mellitus Signaling | 6.73 | 24.6 | * | | | |
| Nur77 Signaling in T Lymphocytes | 1.41 | 3.45 | * | | | |
| Cytotoxic T Lymphocyte-mediated Apoptosis of Target Cells | 2.73 | 2.21 | * | | | |
| Th2 Pathway | 5.34 | 28.9 | * | | | |
| Toll-like Receptor Signaling | 4.77 | 16.8 | * | | | |
| Choline Biosynthesis III | | 1.33 | * | | | |
| DNA Methylation and Transcriptional Repression Signaling | | | * | | | |
| T Helper Cell Differentiation | 4.27 | 28.4 | * | | | |
| Role of Cytokines in Mediating Communication between Immune Cells | 3.44 | 17.2 | * | | | |

(continued)

| Ingenuity Canonical Pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS |
|---|---|---|---|---|---|---|
| iCOS-iCOSL Signaling in T Helper Cells | 3.52 | 17.3 | * | | | |
| Allograft Rejection Signaling | | 5.54 | * | | | |
| Autoimmune Thyroid Disease Signaling | | 8.75 | * | | | |
| Graft-versus-Host Disease Signaling | 1.8 | 6.77 | * | | | |
| Communication between Innate and Adaptive Immune Cells | 4.99 | 14.2 | * | | | |
| Crosstalk between Dendritic Cells and Natural Killer Cells | 5.34 | 14.8 | * | | | |
| Systemic Lupus Erythematosus Signaling | 9.46 | 13.3 | * | | | |
| Altered T Cell and B Cell Signaling in Rheumatoid Arthritis | 4.04 | 22.5 | * | | | |
| Role of Hypercytokinemia/hyper chemokinemia in the Pathogenesis of Influenza | 5.07 | 10.7 | * | | | |
| OX40 Signaling Pathway | 1.86 | 3.25 | * | | | |
| Hematopoiesis from Pluripotent Stem Cells | 3.84 | 12.4 | * | | | |
| Antigen Presentation Pathway | 1.69 | 1.29 | * | | | |
| Adrenomedullin Signaling pathway | 10.4 | | * | * | -2.236 | |
| * ambiguous effect | | | | | | |

Table 36 - Canonical pathways affected by TBI after 2 days and the effects of LMW-DS

| Ingenuity Canonical Pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS |
|---|---|---|---|---|---|---|
| Dendritic Cell Maturation | sign affected | sign affected | Inhibited | * | | |
| Role of NFAT in Regulation of the Immune Response | sign affected | sign affected | Inhibited | Activated | | |

(continued)

| Ingenuity Canonical Pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS |
|---|---|---|---|---|---|---|
| Osteoarthritis Pathway | sign affected | sign affected | Activated | Inhibited | Inhibited | |
| Role of NFAT in Cardiac Hypertrophy | sign affected | sign affected | Activated | | Inhibited | |
| NF-$_\kappa$B Signaling | sign affected | sign affected | Activated | | Inhibited | |
| Ephrin B Signaling | | sign affected | Activated | | | |
| RhoA Signaling | | sign affected | Activated | | | |
| Endothelin-1 Signaling | sign affected | sign affected | Activated | * | | |
| IL-1 Signaling | sign affected | sign affected | Activated | Inhibited | | |
| Axonal Guidance Signaling | sign affected | sign affected | * | | | |
| CREB Signaling in Neurons | sign affected | sign affected | * | | | |
| Phospholipase C Signaling | sign affected | sign affected | * | | | |
| Role of Osteoblasts, Osteoclasts and Chondrocytes in Rheumatoid Arthritis | sign affected | sign affected | * | | | |
| Thrombin Signaling | sign affected | sign affected | * | | | |
| Hepatic Fibrosis / Hepatic Stellate Cell Activation | sign affected | sign affected | * | | | |
| Fc$\gamma$ Receptor-mediated Phagocytosis in Macrophages and Monocytes | sign affected | sign affected | * | | | |
| VDR/RXR Activation | sign affected | sign affected | * | | | |
| Role of Wnt/GSK-3$\beta$ Signaling in the Pathogenesis of Influenza | | | * | | | |
| Calcium-induced T Lymphocyte Apoptosis | sign affected | sign affected | * | | | |
| Antioxidant Action of Vitamin C | sign affected | sign affected | * | | | |
| Phospholipases | | sign affected | * | | | |
| Cdc42 Signaling | | sign affected | * | | | |
| Role of Pattern Recognition Receptors in Recognition of Bacteria and Viruses | sign affected | sign affected | * | | | |
| Hepatic Cholestasis | sign affected | sign affected | * | | | |

(continued)

| Ingenuity Canonical Pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS |
|---|---|---|---|---|---|---|
| Neuroprotective Role of THOP1 in Alzheimer's Disease | sign affected | sign affected | * | | | |
| Type I Diabetes Mellitus Signaling | sign affected | sign affected | * | | | |
| Nur77 Signaling in T Lymphocytes | sign affected | sign affected | * | | | |
| Cytotoxic T Lymphocyte-mediated Apoptosis of Target Cells | sign affected | sign affected | * | | | |
| Th2 Pathway | sign affected | sign affected | * | | | |
| Toll-like Receptor Signaling Choline Biosynthesis III | sign affected | sign affected sign affected | * | | | |
| DNA Methylation and Transcriptional Repression Signaling | | | * | | | |
| T Helper Cell Differentiation | sign affected | sign affected | * | | | |
| Role of Cytokines in Mediating Communication between Immune Cells | sign affected | sign affected | * | | | |
| iCOS-iCOSL Signaling in T Helper Cells | sign affected | sign affected | * | | | |
| Allograft Rejection Signaling | | sign affected | * | | | |
| Autoimmune Thyroid Disease Signaling | | sign affected | * | | | |
| Graft-versus-Host Disease Signaling | sign affected | sign affected | * | | | |
| Communication between Innate and Adaptive Immune Cells | sign affected | sign affected | * | | | |
| Crosstalk between Dendritic Cells and Natural Killer Cells | sign affected | sign affected | * | | | |
| Systemic Lupus Erythematosus Signaling | sign affected | sign affected | * | | | |
| Altered T Cell and B Cell Signaling in Rheumatoid Arthritis | sign affected | sign affected | * | | | |

(continued)

| Ingenuity Canonical Pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS |
|---|---|---|---|---|---|---|
| Role of Hypercytokinemia/hyper chemokinemia in the Pathogenesis of Influenza | sign affected | sign affected | * | | | |
| OX40 Signaling Pathway | sign affected | sign affected | * | | | |
| Hematopoiesis from Pluripotent Stem Cells | sign affected | sign affected | * | | | |
| Antigen Presentation Pathway | sign affected | sign affected | * | | | |
| Adrenomedullin Signaling pathway | sign affected | | * | * | Inhibited | |
| * ambiguous effect | | | | | | |

Table 37 - Diseases and molecular functions affected by TBI after 2 days and the effects of LMW-DS (p values and z scores)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| MAPKKK cascade | | | -2.236 | | | |
| Apoptosis of tumor cell lines | 4.41 E-93 | 5.28E-155 | -2.077 | | | 0.09 |
| Abdominal carcinoma | | | -1.98 | -1.715 | -2.631 | |
| Carcinoma | | | -1.941 | -0.127 | -2.071 | |
| Synthesis of cyclic AMP | | | -1.794 | | | |
| Cell death of tumor cell lines | 3.79E-88 | 5.76E-159 | -1.705 | | -1.947 | |
| Survival of organism | 1.39E-73 | 3.6E-208 | -1.599 | -0.095 | | |
| Paired-pulse facilitation | | | -1.4 | | | |
| Resorption of bone | | | -1.353 | | -0.478 | |
| Proliferation of hematopoietic progenitor cells | | | -1.331 | | -2.951 | |
| Epithelial neoplasm | | | -1.223 | | -1.393 | |
| Cytostasis of tumor cell lines | | | -1.2 | | | |
| Self-renewal of cells | | | -1.199 | | | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Digestive system cancer | | | -1.131 | | -2.221 | |
| Cell proliferation of leukocyte cell lines | | | -1.083 | | -2.754 | |
| Paired-pulse facilitation of synapse | | | -1 | | | |
| Osteoclastogenesis of bone cells | | | -1 | | | |
| Development of connective tissue cells | | 1.1E-76 | -0.973 | -0.332 | | |
| Binding of tumor cell lines | | 2.44E-75 | -0.957 | 2.397 | | |
| T cell development | | 4.12E-88 | -0.928 | | | |
| Tumorigenesis of tissue | | | -0.885 | | | |
| Growth of lymphoid organ | | | -0.881 | | | |
| Lymphopoiesis | | 5.45E-106 | -0.874 | 0.583 | -3.105 | |
| Lymphocyte homeostasis | | 6.36E-90 | -0.855 | | -2.94 | |
| Hypersensitive reaction | | 1.77E-82 | -0.832 | | | |
| Behavior | 7.65E-146 | | -0.793 | 1.334 | -2.009 | -0.139 |
| Proliferation of bone marrow cell lines | | | -0.762 | | | |
| Necrosis | 3.13E-153 | 1.37E-251 | -0.719 | -0.361 | -1.503 | -0.477 |
| Proliferation of blood cells | 4.3E-57 | 4.19E-154 | -0.687 | -1.083 | | |
| Feeding | | | -0.668 | | -0.895 | |
| Digestive organ tumor | | | -0.666 | -0.604 | -1.149 | |
| Non-hematologic malignant neoplasm | | | -0.63 | -0.243 | | |
| Analgesia | | | -0.587 | | | |
| Abdominal cancer | | | -0.57 | -1.538 | -2.553 | |
| Differentiation of T lymphocytes | | | -0.568 | | | |
| Proliferation of lymphatic system cells | 4.71E-58 | 2.05E-141 | -0.559 | -1.112 | | |
| Proliferation of thy-mocytes | | | -0.555 | | | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Cell movement of tumor cells | | | -0.555 | | | |
| Protein kinase cascade | | | -0.412 | | | |
| Hepatic injury | 2.69E-66 | | -0.339 | | | |
| Leukopoiesis | | 4.76E-137 | -0.296 | 1.185 | -3.549 | |
| Development of hematopoietic progenitor cells | | | -0.295 | | | |
| Regeneration of neurons | | | -0.277 | | | |
| Quantity of neuroglia | | | -0.277 | | -1.446 | |
| Experimentally-induced arthritis | | | -0.262 | -0.816 | | |
| Proliferation of lymphocytes | 2.25E-52 | 1.05E-119 | -0.244 | -0.852 | | |
| Differentiation of hematopoietic progenitor cells | | | -0.223 | 0.487 | | |
| Cell proliferation of T lymphocytes | | 6.09E-108 | -0.211 | -1.097 | | |
| Place preference | | | -0.192 | | | |
| Non-hematological solid tumor | | | -0.167 | | | |
| Adhesion of tumor cell lines | | | -0.093 | 2.074 | | |
| Inflammation of joint | 3.04E-121 | 4.99E-137 | -0.079 | -0.053 | | |
| Rheumatic Disease | 1.08E-145 | 7.12E-183 | -0.079 | -0.053 | | |
| Hematopoiesis of bone marrow cells | | | -0.07 | | | |
| Hematologic cancer | 1.05E-92 | 2.16E-115 | -0.063 | | -1.067 | |
| Thrombus | | | -0.042 | 1 | | |
| Apoptosis | 7.51E-135 | 1.07E-244 | -0.011 | -0.337 | 0.601 | -0.502 |
| Non-melanoma solid tumor | | | -0.001 | | -1.249 | |
| Formation of osteoclasts | | | Ambiguous effect | | | |
| Atelectasis | | | * | | | |
| Quantity of osteoblasts | | | * | | | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Development of hematopoietic system | | 8.45E-77 | 0.026 | | | |
| Quantity of lymphocytes | | 7.81E-128 | 0.042 | | | -0.943 |
| Cell death of blood cells | 5.88E-70 | 3.48E-151 | 0.045 | 1.082 | | |
| Development of cytoplasm | | | 0.066 | | | |
| Hematopoiesis of hematopoietic progenitor cells | | | 0.083 | | | |
| Cell death of leukemia cell lines | | | 0.084 | | | |
| Concentration of prostaglandin | | | 0.119 | | -0.911 | |
| Polyarthritis | | | 0.133 | | | |
| Cell death | 6.48E-155 | 3.74E-254 | 0.142 | -0.793 | 0.051 | -0.141 |
| Memory deficits | | | 0.152 | | | |
| Differentiation of adipocytes | | | 0.168 | | | |
| Interaction of lymphocytes | | | 0.186 | | | |
| Binding of lymphocytes | | | 0.186 | | | |
| Cellular homeostasis | 1.04E-117 | 1.56E-154 | 0.202 | 0.19 | -3.19 | |
| Incidence of tumor | | | 0.21 | -1.131 | -0.731 | |
| Quantity of lymphatic system cells | | 1.35E-136 | 0.219 | -0.701 | | |
| Cell death of immune cells | 4.29E-72 | 1.75E-147 | 0.225 | 1.001 | -1 | |
| Locomotion | 1.34E-66 | | 0.239 | | -0.039 | |
| Hematopoiesis of bone marrow | | | 0.265 | | | |
| Differentiation of connective tissue cells | 1.6E-52 | 3.39E-143 | 0.278 | 0.73 | | |
| Cell death of antigen presenting cells | | | 0.306 | | -0.62 | |
| Differentiation of osteoclasts | | | 0.339 | -0.223 | | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Lymphatic system tumor | 4.79E-88 | | 0.339 | | | |
| Neoplasia of leukocytes | 5.5E-88 | 1.29E-149 | 0.339 | | -0.48 | |
| Lymphoid cancer | 1.85E-77 | 1.81E-114 | 0.339 | | | |
| Lymphocytic neoplasm | 2.2E-82 | 4.25E-139 | 0.339 | | -0.48 | |
| Lymphocytic cancer | 3.97E-73 | | 0.339 | | -0.48 | |
| Lymphoproliferative disorder | 2.49E-83 | 1.95E-104 | 0.339 | | -0.48 | |
| Release of Ca$^{2+}$ | | | 0.342 | | | |
| Interaction of mononuclear leukocytes | | | 0.343 | 1.626 | | |
| Binding of mononuclear leukocytes | | | 0.343 | | | |
| Concentration of fatty acid | | | 0.395 | | | |
| Edema | 2.05E-71 | 6.78E-82 | 0.447 | | 3.386 | |
| Quantity of osteoclasts | | | 0.447 | | | |
| Quantity of epithelial tissue | | | 0.447 | | -0.028 | |
| Differentiation of bone cells | | 1.39E-102 | 0.463 | -0.341 | | |
| Malignant solid tumor | | | 0.475 | -0.562 | -1.492 | |
| Chemotaxis of tumor cell lines | | | 0.495 | | | |
| Quantity of amino acids | | | 0.516 | | | |
| Quantity of bone cells | | | 0.537 | | | |
| Quantity of mononuclear leukocytes | | 1.1E-133 | 0.539 | | | |
| Formation of reactive oxygen species | | | 0.555 | | | |
| Quantity of blood cells | 8.73E-61 | 1.92E-184 | 0.62 | -1.479 | | -0.34 |
| Quantity of connective tissue cells | | 3.02E-74 | 0.622 | 0.637 | | |
| Abdominal neoplasm | | | 0.628 | -0.154 | -0.927 | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Release of metal | | | 0.647 | | | |
| Angiogenesis of extraembryonic tissue | | | 0.689 | | | |
| Development of extraembryonic tissue | | | 0.689 | | | |
| Hematopoietic neoplasm | 2.37E-95 | | 0.692 | | | |
| Quantity of connective tissue | | 4.84E-113 | 0.702 | | | |
| Concentration of eicosanoid | | | 0.734 | | | |
| Binding of breast cancer cell lines | | | 0.747 | | | |
| Damage of liver | 7.95E-76 | 4.11E-168 | 0.784 | | | |
| Quantity of leukocytes | 7.27E-55 | 1.75E-172 | 0.803 | -1.163 | | |
| Size of body | | | 0.813 | | -4.771 | |
| Cell movement of breast cancer cell lines | | 1.15E-73 | 0.836 | | | |
| Formation of muscle cells | | | 0.842 | | | |
| Migration of breast cell lines | | | 0.849 | | | |
| Vascularization | | 1.92E-105 | 0.881 | | | |
| Vasculogenesis | 3.63E-68 | 6.72E-185 | 0.894 | | -2.274 | |
| Release of prostaglandin E2 | | | 0.911 | | | |
| Cell proliferation of lymphoma cell lines | | | 0.97 | | | |
| Aggregation of blood cells | | | 0.976 | | | |
| Activation of endothelial cells | | | 1 | | | |
| Cell movement of cervical cancer cell lines | | | 1.009 | | | |
| Cell survival | 1.22E-94 | 4.03E-184 | 1.01 | | | |
| Attachment of cells | | | 1.041 | | | |
| Inflammation of organ | 1.21E-228 | * | 1.041 | -1.295 | | |
| Transcription of DNA | | | 1.044 | | | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Metastasis of carcinoma cell lines | | | 1.067 | | | |
| Fusion of muscle cells | | | 1.091 | | | |
| Aggregation of cells | | 1.14E-83 | 1.104 | | | |
| Formation of muscle | | | 1.107 | | | |
| Vascularization of eye | | | 1.109 | | | |
| Differentiation of muscle cell lines | | | 1.117 | | | |
| Quantity of cells | 2.72E-102 | 2.87E-233 | 1.121 | -0.765 | -3.092 | -0.797 |
| Quantity of bone | | | 1.159 | -1.985 | | |
| Cell movement of breast cell lines | | | 1.172 | | | |
| Activation of T lymphocytes | | | 1.193 | | | |
| Activation of lymphocytes | | | 1.221 | -1.158 | | |
| Activation of blood cells | 1.69E-56 | 3.43E-146 | 1.258 | 0.086 | | |
| Quantity of phagocytes | | 4.3E-140 | 1.289 | -2.061 | | |
| Aggregation of blood platelets | | | 1.299 | | | |
| Development of vasculature | 1.8E-77 | 1.84E-221 | 1.299 | | -1.534 | |
| Solid tumor | | | 1.31 | -0.186 | | |
| Extracranial solid tumor | | | 1.311 | 0.056 | -0.992 | |
| Cancer | | | 1.318 | | | |
| Activation of leukocytes | 2.75E-57 | 5.84E-135 | 1.325 | 0.086 | | |
| G1 phase of tumor cell lines | | | 1.342 | | | |
| Myelopoiesis of bone marrow | | | 1.342 | | | |
| Cell-mediated response | | | 1.387 | | | |
| Interaction of protein | | | 1.4 | | | |
| Chemotaxis | | 4.9E-120 | 1.425 | | -3.642 | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Cell movement of epithelial cell lines | | | 1.446 | | | |
| Fusion of cells | | | 1.446 | | | |
| G1/S phase transition | | | 1.455 | | | |
| Apoptosis of muscle cells | | 2.49E-119 | 1.467 | 0.041 | | |
| Pelvic tumor | 1.81E-59 | | 1.491 | -0.651 | | |
| Transcription of RNA | | 2.71E-75 | 1.519 | | -2.488 | |
| Transcription | | 3.3E-92 | 1.537 | | | |
| G1 phase | | 6.31E-76 | 1.609 | | | |
| Migration of brain cells | | | 1.616 | | | |
| Activation of cells | 3.66E-78 | 6.43E-190 | 1.629 | 0.836 | | |
| Proliferation of leukemia cell lines | | 5.94E-78 | 1.662 | | | |
| Migration of neurons | | | 1.676 | | | |
| Neovascularization of eye | | | 1.677 | | | |
| Apoptosis of stem cells | | | 1.686 | | | |
| Leukocyte migration | 1.46E-79 | 3.36E-205 | 1.694 | 1.296 | -2.163 | |
| Expression of RNA | | 5.44E-90 | 1.78 | | | |
| Necrosis of muscle | 3.34E-54 | 1.37E-133 | 1.792 | | | |
| Cell movement of tumor cell lines | 1.17E-69 | 1.12E-156 | 1.812 | | -2.078 | |
| Interphase | | 1.99E-94 | 1.823 | | | |
| Growth of tumor | 2.27E-68 | 2.81E-193 | 1.937 | | -1.233 | |
| Genital tumor | 1.07E-52 | | 1.981 | 0.13 | | |
| Attachment of tumor cell lines | | | 1.982 | | | |
| Adipogenesis of connective tissue | | | 1.982 | | | |
| Quantity of IL-6 in blood | | | 1.982 | | | |
| Quantity of TNF in blood | | | 2 | | | |
| Inflammation of body cavity | 6.8E-184 | * | 2.004 | -1.757 | | |

(continued)

| Diseases of functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS |
|---|---|---|---|---|---|---|
| Inflammation of absolute anatomical region | 1.33E-208 | * | 2.016 | -1.359 | | |
| Cell movement | 1.08E-108 | 5.26E-246 | 2.142 | 1.948 | -3.723 | |
| Metabolism of hormone | | | 2.185 | | -1.632 | |
| Synthesis of hormone | | | 2.185 | 0.977 | -1.632 | |
| Migration of cells | 6.76E-103 | 4.26E-241 | 2.188 | 2.093 | -3.087 | |
| Cell movement of vascular smooth muscle cells | | | 2.213 | -0.588 | | |
| Inflammatory response | 2.02E-74 | 9.77E-181 | 2.246 | 1.159 | | |
| Secretion of molecule | 1.66E-75 | | 2.281 | | 1.634 | |
| Cell movement of muscle cells | | 6.73E-75 | 2.393 | -0.26 | | |
| Transport of molecule | 1.58E-117 | | 2.597 | 2.421 | 0.248 | |
| * ambiguous effect | | | | | | |

Table 38 - Diseases and molecular functions affected by TBI after 2 days and the effects of LMW-DS

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| MAPKKK cascade | | | Inhibited | | | |
| Apoptosis of tumor cell lines | 4.41E-93 | 5.28E-155 | Inhibited | | | Activated |
| Abdominal carcinoma | | | Inhibited | Inhibited | Inhibited | |
| Carcinoma | | | Inhibited | Inhibited | Inhibited | |
| Synthesis of cyclic AMP | | | Inhibited | | | |
| Cell death of tumor cell lines | 3.79E-88 | 5.76E-159 | Inhibited | | Inhibited | |
| Survival of organism | 1.39E-73 | 3.6E-208 | Inhibited | Inhibited | | |
| Paired-pulse facilitation | | | Inhibited | | | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Resorption of bone | | | Inhibited | | Inhibited | |
| Proliferation of hematopoietic progenitor cells | | | Inhibited | | Inhibited | |
| Epithelial neoplasm | | | Inhibited | | Inhibited | |
| Cytostasis of tumor cell lines | | | Inhibited | | | |
| Self-renewal of cells | | | Inhibited | | | |
| Digestive system cancer | | | Inhibited | | Inhibited | |
| Cell proliferation of leukocyte cell lines | | | Inhibited | | Inhibited | |
| Paired-pulse facilitation of synapse | | | Inhibited | | | |
| Osteoclastogenesis of bone cells | | | Inhibited | | | |
| Development of connective tissue cells | | 1.1E-76 | Inhibited | Inhibited | | |
| Binding of tumor cell lines | | 2.44E-75 | Inhibited | Activated | | |
| T cell development | | 4.12E-88 | Inhibited | | | |
| Tumorigenesis of tissue | | | Inhibited | | | |
| Growth of lymphoid organ | | | Inhibited | | | |
| Lymphopoiesis | | 5.45E-106 | Inhibited | Activated | Inhibited | |
| Lymphocyte homeostasis | | 6.36E-90 | Inhibited | | Inhibited | |
| Hypersensitive reaction | | 1.77E-82 | Inhibited | | | |
| Behavior | 7.65E-146 | | Inhibited | Activated | Inhibited | Inhibited |
| Proliferation of bone marrow cell lines | | | Inhibited | | | |
| Necrosis | 3.13E-153 | 1.37E-251 | Inhibited | Inhibited | Inhibited | Inhibited |
| Proliferation of blood cells | 4.3E-57 | 4.19E-154 | Inhibited | Inhibited | | |
| Feeding | | | Inhibited | | Inhibited | |
| Digestive organ tumor | | | Inhibited | Inhibited | Inhibited | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Non-hematologic malignant neoplasm | | | Inhibited | Inhibited | | |
| Analgesia | | | Inhibited | | | |
| Abdominal cancer | | | Inhibited | Inhibited | Inhibited | |
| Differentiation of T lymphocytes | | | Inhibited | | | |
| Proliferation of lymphatic system cells | 4.71E-58 | 2.05E-141 | Inhibited | Inhibited | | |
| Proliferation of thymocytes | | | Inhibited | | | |
| Cell movement of tumor cells | | | Inhibited | | | |
| Protein kinase cascade | | | Inhibited | | | |
| Hepatic injury | 2.69E-66 | | Inhibited | | | |
| Leukopoiesis | | 4.76E-137 | Inhibited | Activated | Inhibited | |
| Development of hematopoietic progenitor cells | | | Inhibited | | | |
| Regeneration of neurons | | | Inhibited | | | |
| Quantity of neuroglia | | | Inhibited | | Inhibited | |
| Experimentally-induced arthritis | | | Inhibited | Inhibited | | |
| Proliferation of lymphocytes | 2.25E-52 | 1.05E-119 | Inhibited | Inhibited | | |
| Differentiation of hematopoietic progenitor cells | | | Inhibited | Activated | | |
| Cell proliferation of T lymphocytes | | 6.09E-108 | Inhibited | Inhibited | | |
| Place preference | | | Inhibited | | | |
| Non-hematological solid tumor | | | Inhibited | | | |
| Adhesion of tumor cell lines | | | Inhibited | Activated | | |
| Inflammation of joint | 3.04E-121 | 4.99E-137 | Inhibited | Inhibited | | |
| Rheumatic Disease | 1.08E-145 | 7.12E-183 | Inhibited | Inhibited | | |
| Hematopoiesis of bone marrow cells | | | Inhibited | | | |
| Hematologic cancer | 1.05E-92 | 2.16E-115 | Inhibited | | Inhibited | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Thrombus | | | Inhibited | Activated | | |
| Apoptosis | 7.51E-135 | 1.07E-244 | Inhibited | Inhibited | Activated | Inhibited |
| Non-melanoma solid tumor | | | Inhibited | | Inhibited | |
| Formation of osteoclasts | | | | | | |
| Atelectasis | | | | | | |
| Quantity of osteoblasts | | | | | | |
| Development of hematopoietic system | | 8.45E-77 | Activated | | | |
| Quantity of lymphocytes | | 7.81E-128 | Activated | | | Inhibited |
| Cell death of blood cells | 5.88E-70 | 3.48E-151 | Activated | Activated | | |
| Development of cytoplasm | | | Activated | | | |
| Hematopoiesis of hematopoietic progenitor cells | | | Activated | | | |
| Cell death of leukemia cell lines | | | Activated | | | |
| Concentration of prostaglandin | | | Activated | | Inhibited | |
| Polyarthritis | | | Activated | | | |
| Cell death | 6.48E-155 | 3.74E-254 | Activated | Inhibited | Activated | Inhibited |
| Memory deficits | | | Activated | | | |
| Differentiation of adipocytes | | | Activated | | | |
| Interaction of lymphocytes | | | Activated | | | |
| Binding of lymphocytes | | | Activated | | | |
| Cellular homeostasis | 1.04E-117 | 1.56E-154 | Activated | Activated | Inhibited | |
| Incidence of tumor | | | Activated | Inhibited | Inhibited | |
| Quantity of lymphatic system cells | | 1.35E-136 | Activated | Inhibited | | |
| Cell death of immune cells | 4.29E-72 | 1.75E-147 | Activated | Activated | Inhibited | |
| Locomotion | 1.34E-66 | | Activated | | Inhibited | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Hematopoiesis of bone marrow | | | Activated | | | |
| Differentiation of connective tissue cells | 1.6E-52 | 3.39E-143 | Activated | Activated | | |
| Cell death of antigen presenting cells | | | Activated | | Inhibited | |
| Differentiation of osteoclasts | | | Activated | Inhibited | | |
| Lymphatic system tumor | 4.79E-88 | | Activated | | | |
| Neoplasia of leukocytes | 5.5E-88 | 1.29E-149 | Activated | | Inhibited | |
| Lymphoid cancer | 1.85E-77 | 1.81E-114 | Activated | | | |
| Lymphocytic neoplasm | 2.2E-82 | 4.25E-139 | Activated | | Inhibited | |
| Lymphocytic cancer | 3.97E-73 | | Activated | | Inhibited | |
| Lymphoproliferative disorder | 2.49E-83 | 1.95E-104 | Activated | | Inhibited | |
| Release of Ca$^2$+ | | | Activated | | | |
| Interaction of mono-nuclear leukocytes | | | Activated | Activated | | |
| Binding of mononuclear leukocytes | | | Activated | | | |
| Concentration of fatty acid | | | Activated | | | |
| Edema | 2.05E-71 | 6.78E-82 | Activated | | Activated | |
| Quantity of osteoclasts | | | Activated | | | |
| Quantity of epithelial tissue | | | Activated | | Inhibited | |
| Differentiation of bone cells | | 1.39E-102 | Activated | Inhibited | | |
| Malignant solid tumor | | | Activated | Inhibited | Inhibited | |
| Chemotaxis of tumor cell lines | | | Activated | | | |
| Quantity of amino acids | | | Activated | | | |
| Quantity of bone cells | | | Activated | | | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Quantity of mono-nuclear leukocytes | | 1.1E-133 | Activated | | | |
| Formation of reactive oxygen species | | | Activated | | | |
| Quantity of blood cells | 8.73E-61 | 1.92E-184 | Activated | Inhibited | | Inhibited |
| Quantity of connective tissue cells | | 3.02E-74 | Activated | Activated | | |
| Abdominal neoplasm | | | Activated | Inhibited | Inhibited | |
| Release of metal | | | Activated | | | |
| Angiogenesis of extraembryonic tissue | | | Activated | | | |
| Development of ex-traembryonic tissue | | | Activated | | | |
| Hematopoietic neo-plasm | 2.37E-95 | | Activated | | | |
| Quantity of connective tissue | | 4.84E-113 | Activated | | | |
| Concentration of eicosanoid | | | Activated | | | |
| Binding of breast cancer cell lines | | | Activated | | | |
| Damage of liver | 7.95E-76 | 4.11E-168 | Activated | | | |
| Quantity of leukocytes | 7.27E-55 | 1.75E-172 | Activated | Inhibited | | |
| Size of body | | | Activated | | Inhibited | |
| Cell movement of breast cancer cell lines | | 1.15E-73 | Activated | | | |
| Formation of muscle cells | | | Activated | | | |
| Migration of breast cell lines | | | Activated | | | |
| Vascularization | | 1.92E-105 | Activated | | | |
| Vasculogenesis | 3.63E-68 | 6.72E-185 | Activated | | Inhibited | |
| Release of prostaglandin E2 | | | Activated | | | |
| Cell proliferation of lymphoma cell lines | | | Activated | | | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Aggregation of blood cells | | | Activated | | | |
| Activation of endothelial cells | | | Activated | | | |
| Cell movement of cervical cancer cell lines | | | Activated | | | |
| Cell survival | 1.22E-94 | 4.03E-184 | Activated | | | |
| Attachment of cells | | | Activated | | | |
| Inflammation of organ | 1.21E-228 | * | Activated | Inhibited | | |
| Transcription of DNA | | | Activated | | | |
| Metastasis of carcinoma cell lines | | | Activated | | | |
| Fusion of muscle cells | | | Activated | | | |
| Aggregation of cells | | 1.14E-83 | Activated | | | |
| Formation of muscle | | | Activated | | | |
| Vascularization of eye | | | Activated | | | |
| Differentiation of muscle cell lines | | | Activated | | | |
| Quantity of cells | 2.72E-102 | 2.87E-233 | Activated | Inhibited | Inhibited | Inhibited |
| Quantity of bone | | | Activated | Inhibited | | |
| Cell movement of breast cell lines | | | Activated | | | |
| Activation of T lymphocytes | | | Activated | | | |
| Activation of lymphocytes | | | Activated | Inhibited | | |
| Activation of blood cells | 1.69E-56 | 3.43E-146 | Activated | Activated | | |
| Quantity of phagocytes | | 4.3E-140 | Activated | Inhibited | | |
| Aggregation of blood platelets | | | Activated | | | |
| Development of vasculature | 1.8E-77 | 1.84E-221 | Activated | | Inhibited | |
| Solid tumor | | | Activated | Inhibited | | |
| Extracranial solid tumor | | | Activated | Activated | Inhibited | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Cancer | | | Activated | | | |
| Activation of leukocytes | 2.75E-57 | 5.84E-135 | Activated | Activated | | |
| G1 phase of tumor cell lines | | | Activated | | | |
| Myelopoiesis of bone marrow | | | Activated | | | |
| Cell-mediated response | | | Activated | | | |
| Interaction of protein | | | Activated | | | |
| Chemotaxis | | 4.9E-120 | Activated | | Inhibited | |
| Cell movement of epithelial cell lines | | | Activated | | | |
| Fusion of cells | | | Activated | | | |
| G1/S phase transition | | | Activated | | | |
| Apoptosis of muscle cells | | 2.49E-119 | Activated | Activated | | |
| Pelvic tumor | 1.81E-59 | | Activated | Inhibited | | |
| Transcription of RNA | | 2.71E-75 | Activated | | Inhibited | |
| Transcription | | 3.3E-92 | Activated | | | |
| G1 phase | | 6.31E-76 | Activated | | | |
| Migration of brain cells | | | Activated | | | |
| Activation of cells | 3.66E-78 | 6.43E-190 | Activated | Activated | | |
| Proliferation of leukemia cell lines | | 5.94E-78 | Activated | | | |
| Migration of neurons | | | Activated | | | |
| Neovascularization of eye | | | Activated | | | |
| Apoptosis of stem cells | | | Activated | | | |
| Leukocyte migration | 1.46E-79 | 3.36E-205 | Activated | Activated | Inhibited | |
| Expression of RNA | | 5.44E-90 | Activated | | | |
| Necrosis of muscle | 3.34E-54 | 1.37E-133 | Activated | | | |
| Cell movement of tumor cell lines | 1.17E-69 | 1.12E-156 | Activated | | Inhibited | |
| Interphase | | 1.99E-94 | Activated | | | |
| Growth of tumor | 2.27E-68 | 2.81E-193 | Activated | | Inhibited | |

(continued)

| Diseases or functions annotation | Diseases and functions affected in dementia and neurodegeneration (p value) | Diseases and functions affected in fibrosis and scarring (p value) | Effect TBI | TBI+1 mg/kg LMW-DS | TBI+5 mg/kg LMW-DS | TBI+15 mg/kg LMW-DS |
|---|---|---|---|---|---|---|
| Genital tumor | 1.07E-52 | | Activated | Activated | | |
| Attachment of tumor cell lines | | | Activated | | | |
| Adipogenesis of connective tissue | | | Activated | | | |
| Quantity of IL-6 in blood | | | Activated | | | |
| Quantity of TNF in blood | | | Activated | | | |
| Inflammation of body cavity | 6.8E-184 | * | Activated | Inhibited | | |
| Inflammation of absolute anatomical region | 1.33E-208 | * | Activated | Inhibited | | |
| Cell movement | 1.08E-108 | 5.26E-246 | Activated | Activated | Inhibited | |
| Metabolism of hormone | | | Activated | | Inhibited | |
| Synthesis of hormone | | | Activated | Activated | Inhibited | |
| Migration of cells | 6.76E-103 | 4.26E-241 | Activated | Activated | Inhibited | |
| Cell movement of vascular smooth muscle cells | | | Activated | Inhibited | | |
| Inflammatory response | 2.02E-74 | 9.77E-181 | Activated | Activated | | |
| Secretion of molecule | 1.66E-75 | | Activated | | Activated | |
| Cell movement of muscle cells | | 6.73E-75 | Activated | Inhibited | | |
| Transport of molecule | 1.58E-117 | | Activated | Activated | Activated | |
| * ambiguous effect | | | | | | |

The effects induced by TBI within 7 days were significant with a large number of genes deregulated. Consequently, the alterations in many neurodegeneration and scaring-related canonical pathways were 5 significant. Most of these pathway alterations were counteracted by ILB given within 30 minutes of the TBI (Table 39 and 40). Similar to the pathways the number of significantly affected molecular processes and diseases within 7 days of TBI was large and the effects were significant. However, the effect of TBI was mostly abolished by LMW-DS given 30 minutes after the injury (Table 41 and 42).

Table 39 - Canonical pathways affected by TBI after 7 days and the effects of LMW-DS relative to control (p values and z scores)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Axonal Guidance Signaling | 11 | 17.3 | * | | | | |
| CREB Signaling in Neurons | 17.8 | 3.94 | -3.703 | | | | |
| Opioid Signaling Pathway | 20.8 | | -3.048 | -0.447 | * | | 0.816 |
| Synaptic Long Term Depression | 13.7 | 4.67 | -4.061 | 1.342 | 1 | | 1.342 |
| Synaptic Long Term Potentiation | 14.3 | 3.49 | -3.479 | | | | |
| GNRH Signaling | 17.9 | 9.75 | -3.592 | | 2 | | |
| Molecular Mechanisms of Cancer | 14.6 | 32.2 | * | | | | |
| CXCR4 Signaling | 4.2 | 10.3 | -1.622 | | | | |
| Neuropathic Pain Signaling In Dorsal Horn Neurons | 16.9 | 3.31 | -3.55 | | | | * |
| Factors Promoting Cardiogenesis in Vertebrates | 4.56 | 12.6 | * | | | | |
| Cholecystokinin/Gastrin-mediated Signaling | 7.43 | 9.52 | -1.219 | | | | |
| Calcium Signaling | 33.2 | 6.28 | -3.781 | | | | |
| Osteoarthritis Pathway | 17.6 | 43.2 | -1.64 | | -1 | | |
| Epithelial Adherens Junction Signaling | 2.74 | 21.8 | * | | | | |
| Endothelin-1 Signaling | 12.2 | 14.1 | -1.155 | 1.342 | 1.633 | 1 | |
| Cardiac Hypertrophy Signaling | 14.6 | 19.9 | -2.828 | | 1 | | |
| Glutamate Receptor Signaling | 12.1 | | -2.53 | | | | |
| GPCR-Mediated Nutrient Sensing in Enteroendocrine Cells | 12.4 | | -2.121 | | | | |
| Actin Cytoskeleton Signaling | 1.66 | 12.5 | -3.286 | | | | |
| UVC-Induced MAPK Signaling | 6.23 | 8.51 | -1.147 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Dopamine-DARPP32 Feedback in cAMP Signaling | 16.2 | 2.58 | -2.611 | | | | |
| Role of NFAT in Cardiac Hypertrophy | 18.1 | 16.1 | -3.244 | | * | | 0.447 |
| Phospholipase C Signaling | 4.22 | 11.6 | -2.534 | | 1 | 2 | |
| Role of Macrophages, Fibroblasts and Endothelial Cells in Rheumatoid Arthritis | 14.2 | 53.2 | * | | | | |
| Role of Osteoblasts, Osteoclasts and Chondrocytes in Rheumatoid Arthritis | 8.77 | 47.7 | * | | | | |
| Agrin Interactions at Neuromuscular Junction | 4.16 | 6.61 | -2.4 | | | | |
| Aldosterone Signaling in Epithelial Cells | 4.23 | 3.44 | -2.335 | | | | |
| Protein Kinase A Signaling | 6.1 | 8.04 | -1.386 | | -1.342 | | |
| PTEN Signaling | 9.31 | 28.9 | 2.828 | | | | |
| Gap Junction Signaling | 13.4 | 21.8 | * | | | | |
| G Beta Gamma Signaling | 14.7 | 5.48 | -3.413 | 1 | | | 2.236 |
| Wnt/β-catenin Signaling | | 8.18 | 0.686 | | -1 | | |
| Thrombin Signaling | 3.11 | 10.2 | -2 | | | | |
| Glioblastoma Multiform Signaling | 3.92 | 16.4 | -1.48 | | | | |
| Corticotropin Releasing Hormone Signaling | 18.1 | 7.67 | -1.414 | | | | |
| Tec Kinase Signaling | 4.92 | 17.4 | -1.257 | | | | |
| nNOS Signaling in Neurons | 13 | 3.94 | -1.89 | | | | |
| Cellular Effects of Sildenafil (Viagra) | 6.22 | 2.54 | * | | | | |
| IL-8 Signaling | 9.79 | 34.7 | -1.982 | | 2.646 | | |
| Ephrin Receptor Signaling | 4.59 | 8.64 | -4.004 | | | | 2.236 |
| Basal Cell Carcinoma Signaling | | 3.44 | 0 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Colorectal Cancer Metastasis Signaling | 10.2 | 38.4 | -1.155 | | -0.378 | | |
| PPARa/RXRa Activation | 8.12 | 16.4 | 2.335 | | * | | |
| Neuregulin Signaling | 6.88 | 10.7 | -2.558 | | | | |
| Hepatic Fibrosis / Hepatic Stellate Cell Activation | 15.1 | 68.7 | * | | | | |
| Ephrin B Signaling | | 4 | -2.668 | | | | |
| GP6 Signaling Pathway | 1.86 | | -2.959 | | | | |
| Regulation of the Epithelial-Mesenchymal Transition Pathway | 3.69 | 30 | * | | | | |
| UVA-Induced MAPK Signaling | 6.66 | 9.44 | -2.683 | | | | |
| Signaling by Rho Family GTPases | 2.29 | 8.92 | -2.412 | | 1 | | 1 |
| Pyridoxal 5'-phosphate Salvage Pathway | 4.9 | | -1.789 | | | | |
| Huntington's Disease Signaling | 20.9 | 6.68 | -2.121 | | | | |
| ErbB Signaling | 6.54 | 14.8 | -2.887 | | | | |
| α-Adrenergic Signaling | 5.91 | 1.99 | -2.357 | | | | |
| Fcy Receptor-mediated Phagocytosis in Macrophages and Monocytes | 7.62 | 6.87 | 0.6 | | 2.236 | | |
| Natural Killer Cell Signaling | 4.39 | 5.95 | * | | | | |
| Renin-Angiotensin Signaling | 13.2 | 18.9 | -2.646 | | | | |
| RhoGDI Signaling | | 2.14 | 1.976 | | | | |
| GPCR-Mediated Integration of Enteroendocrine Signaling Exemplified by an L Cell | 4.53 | | 0.218 | | | | |
| HGF Signaling | 7.48 | 17.4 | -3.138 | | | | |
| Gaq Signaling | 12.2 | 15.2 | -2.401 | | | | |
| 14-3-3-mediated Signaling | 12.2 | 23.7 | -1.134 | | | | |
| P2Y Purigenic Receptor Signaling Pathway | 7.16 | 7.78 | -2.191 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| G-Protein Coupled Receptor Signaling | 22.1 | 18.1 | * | | | | |
| PCP pathway | | 2.56 | -0.243 | | | | |
| Thyroid Cancer Signaling | 9.4 | 7.72 | * | | | | |
| Melatonin Signaling | 8.59 | | -0.471 | | | | |
| Mouse Embryonic Stem Cell Pluripotency | 1.35 | 17.9 | -2.502 | | | | |
| IL-3 Signaling | 4.09 | 16.8 | -2.711 | | | | |
| Integrin Signaling | 1.36 | 12.4 | -2.846 | | | | |
| Androgen Signaling | 12.2 | 2.95 | -2.065 | | | | |
| Nitric Oxide Signaling in the Cardiovascular System | 11.7 | 12.9 | -3 | | | | |
| Paxillin Signaling | 1.56 | 10.6 | -3.578 | | | | |
| Fc Epsilon RI Signaling | 5.05 | 15.7 | -0.756 | | | | -1 |
| NGF Signaling | 9.02 | 14.7 | -3.024 | | | | |
| Adrenomedullin signaling pathway | 10.4 | | -2.03 | -1 | -0.632 | * | -0.378 |
| Semaphorin Signaling in Neurons | | 1.33 | * | | | | |
| FLT3 Signaling in Hematopoietic Progenitor Cells | 1.8 | 14.4 | -3.128 | | | | * |
| fMLP Signaling in Neutrophils | 3.74 | 14.3 | -2.502 | | | | |
| Phagosome Formation | 5.65 | 6.16 | * | | | | |
| Ovarian Cancer Signaling | 6.42 | 21.1 | -3.606 | | | | |
| VDR/RXR Activation | 4.65 | 10.2 | 1.069 | | * | | |
| Leukocyte Extravasation Signaling | 6.36 | 19.7 | -2.92 | | 1.342 | | |
| D-myo-inositol (1,4,5)-Trisphosphate Biosynthesis | | | -0.632 | | | | |
| Salvage Pathways of Pyrimidine Ribonucleotides | 3.02 | | -1.46 | | | | |
| Wnt/Ca+ pathway | 4.79 | 1.59 | -1.698 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Role of NANOG in Mammalian Embryonic Stem Cell Pluripotency | | 17 | -3.051 | | | | |
| Virus Entry via Endocytic Pathways | 3.75 | 11 | * | | | | |
| Type II Diabetes Mellitus Signaling | 19 | 16.1 | -0.894 | | | | |
| Rac Signaling | 2.62 | 13.5 | -4.426 | | | | |
| CCR3 Signaling in Eosinophils | 3.08 | 10.5 | -2.558 | | | | |
| cAMP-mediated signaling | 15.8 | 10 | -2.722 | | -2 | 1 | |
| Notch Signaling | 3.05 | | -0.378 | | | | |
| HER-2 Signaling in Breast Cancer | 3.27 | 13.1 | * | | | | |
| Caveolar-mediated Endocytosis Signaling | 1.96 | 5.58 | * | | | | |
| CCR5 Signaling in Macrophages | 16.3 | 4.77 | 0 | | | | |
| Sperm Motility | 4.03 | 1.76 | -1.961 | | | | |
| Regulation of Actin-based Motility by Rho | | 2.14 | -0.218 | | | | |
| Adipogenesis pathway | 4.87 | 13.9 | * | | | | |
| Growth Hormone Signaling | 6.85 | 9.43 | -2.065 | | | | |
| B Cell Receptor Signaling | 9.59 | 28.2 | -3.212 | | | | -0.447 |
| PI3K Signaling in B Lymphocytes | 7.67 | 20.4 | -2.887 | | 1.89 | | |
| Role of Tissue Factor in Cancer | 5.6 | 27.1 | * | | | | |
| Human Embryonic Stem Cell Pluripotency | 3.32 | 19.9 | * | | | | |
| TGF-β Signaling | 2.26 | 24.2 | -1.886 | | | | |
| Erythropoietin Signaling | 4.67 | 16.7 | * | | | | |
| Antiproliferative Role of Somatostatin Receptor 2 | | 8.4 | -3.207 | | | | |
| ERK/MAPK Signaling | 5.66 | 12.8 | -3.667 | | 1 | | |
| p70S6K Signaling | 6.22 | 11.9 | -3.024 | | | | |
| CNTF Signaling | | 13.2 | -3.638 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| GDNF Family Ligand-Receptor Interactions | 3.68 | 9.29 | -2.183 | | | | |
| BMP signaling pathway | 5.09 | 17.7 | -2.183 | | | | |
| Role of NFAT in Regulation of the Immune Response | 5.53 | 15.1 | -2.921 | 0.816 | 2.53 | | 2.236 |
| Neuroinflammation Signaling Pathway | 54.8 | | -1.809 | | 1.941 | | |
| Germ Cell-Sertoli Cell Junction Signaling | 3.63 | 23.6 | * | | | | |
| Glioma Signaling | 6.44 | 18.2 | -3.13 | | | | |
| Netrin Signaling | 14.4 | 2.95 | * | | | | |
| Role of Wnt/GSK-3β Signaling in the Pathogenesis of Influenza | | | 0.577 | | | | |
| Production of Nitric Oxide and Reactive Oxygen Species in Macrophages | 13.7 | 27.7 | -1 | | 2.236 | | |
| Cardiac β-adrenergic Signaling | 3.77 | | -1.886 | | | | |
| Calcium-induced T Lymphocyte Apoptosis | 3.2 | 4.29 | -1.069 | | | | |
| UVB-Induced MAPK Signaling | 7.17 | 9.71 | -1.5 | | | | |
| ErbB4 Signaling | 3.93 | 8.87 | -2.183 | | | | |
| Gas Signaling | 8.77 | 3.53 | -1.964 | | | | |
| RAR Activation | 6.66 | 8.92 | * | | | | |
| 1D-myo-inositol Hexakisphosphate Biosynthesis II (Mammalian) | | | -1.134 | | | | |
| Acute Myeloid Leukemia Signaling | 2.95 | 14.1 | -1.964 | | | | |
| Relaxin Signaling | 3.61 | 10.1 | -3.3 | | | | |
| NF-κB Activation by Viruses | 3.27 | 15.1 | -3.13 | | | | |
| Telomere Extension by Telomerase | | | * | | | | |
| Superpathway of Inositol Phosphate Compounds | | 2.44 | -2.655 | | | | 2 |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| PAK Signaling | 1.8 | 11.5 | -2.4 | | | | |
| GABA Receptor Signaling | 30.6 | | * | | | | |
| IL-4 Signaling | 3.7 | 11.8 | * | | | | |
| Prolactin Signaling | 4.56 | 12.3 | -2.357 | | | | |
| Phenylalanine Degradation I (Aerobic) | | | * | | | | |
| ILK Signaling | 6.57 | 24.1 | -1.567 | | 1.89 | | |
| Thrombopoietin Signaling | 6.39 | 10.3 | -2.5 | | | | |
| STAT3 Pathway | 9.57 | 25.5 | -2.4 | | * | | |
| Parkinson's Signaling | 7.06 | 1.7 | * | | | | |
| SAPK/JNK Signaling | 2.17 | 7.22 | -1.706 | | | | |
| NRF2-mediated Oxidative Stress Response | 8.95 | 10.5 | -1.4 | | | | |
| Melanocyte Development and Pigmentation Signaling | 2.8 | 7.64 | -3.13 | | | | |
| RhoA Signaling | | 2.58 | -1.043 | | | | |
| FcγRIIB Signaling in B Lymphocytes | 11.9 | 8.78 | -1.265 | | | | |
| eNOS Signaling | 29 | 9.79 | -1.961 | | | | |
| FAK Signaling | 1.82 | 14.4 | * | | | | |
| Serotonin Receptor Signaling | 9.58 | | * | | | | |
| PEDF Signaling | 6.56 | 25.5 | -2.524 | | | | |
| VEGF Family Ligand-Receptor Interactions | 4.77 | 13.3 | -2.357 | | | | |
| Breast Cancer Regulation by Stathmin1 | 5.84 | 11 | * | | | | |
| D-myo-inositol-5-phosphate Metabolism | | | -1.671 | | | | |
| IL-10 Signaling | 6.55 | 23.3 | * | | | | |
| IL-15 Signaling | 3.78 | 25 | * | | | | |
| Sertoli Cell-Sertoli Cell Junction Signaling | 5.76 | 21.6 | * | | | | |
| JAK/Stat Signaling | 2.4 | 20.2 | -2.828 | | | | |
| Apoptosis Signaling | 13 | 13.8 | 2.524 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| PDGF Signaling | 6.67 | 20.4 | -3.441 | | | | |
| Non-Small Cell Lung Cancer Signaling | 3.49 | 13.7 | -2.324 | | | | |
| D-myo-inositol (1,4,5)-trisphosphate Degradation | | | 0 | | | | |
| Gai Signaling | 9.38 | 9.83 | -1.964 | | | | |
| Glutamate Dependent Acid Resistance | 2 | | * | | | | |
| PKCθ Signaling in T Lymphocytes | 10.7 | 17.3 | -2.558 | | 2 | | |
| Role of IL-17F in Allergic Inflammatory Airway Diseases | 4.79 | 11.7 | -2.53 | | | | |
| Amyotrophic Lateral Sclerosis Signaling | 28.1 | 13.5 | -1.886 | | | | |
| TWEAK Signaling | 5 | 4.46 | -0.333 | | | | |
| Sphingosine-1-phosphate Signaling | 5.14 | 7.9 | -0.426 | | | | |
| Superpathway of D-myo-inositol (1,4,5)-trisphosphate Metabolism | 1.37 | | -0.378 | | | | |
| Mechanisms of Viral Exit from Host Cells | 5.27 | | * | | | | |
| CDK5 Signaling | 8.38 | 3.35 | -2.524 | | | | |
| IL-1 Signaling | 3.22 | 7.14 | -1.069 | | 1 | | * |
| D-myo-inositol (1,3,4)-trisphosphate Biosynthesis | | | -0.816 | | | | |
| Leptin Signaling in Obesity | 5.34 | 4.55 | -1.89 | | | | |
| Acute Phase Response Signaling | 18.7 | 37.8 | -1.877 | | 1.89 | | -0.447 |
| Pancreatic Adenocarcinoma Signaling | 9.68 | 35.1 | -1.606 | | | | |
| LPS-stimulated MAPK Signaling | 7.31 | 18.4 | -1.886 | | | | |
| Cancer Drug Resistance By Drug Efflux | 5.87 | 11 | * | | | | |
| Calcium Transport I | | | 0 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Antioxidant Action of Vitamin C | 6.6 | 8.13 | 0.229 | | | | |
| Phospholipases | | 1.76 | -0.277 | | | | |
| 3-phosphoinositide Degradation | | | -2.117 | | | | 2 |
| Urea Cycle | | 1.44 | * | | | | |
| Regulation of Cellular Mechanics by Calpain Protease | 1.3 | 8.67 | -1.667 | | | | |
| Angiopoietin Signaling | 2.01 | 12 | -3.051 | | | | |
| Role of MAPK Signaling in the Pathogenesis of Influenza | 4.53 | 13.7 | * | | | | |
| IL-6 Signaling | 7.42 | 32.4 | -2.711 | | 1 | | * |
| ERK5 Signaling | 3.67 | 6.1 | -2.673 | -2 | -0.447 | | |
| GM-CSF Signaling | 3.32 | 25.7 | -3.606 | | | | |
| Oncostatin M Signaling | 2.22 | 15.3 | -2.333 | | | | |
| Circadian Rhythm Signaling | 4.89 | | * | | | | |
| Inhibition of Angiogenesis by TSP1 | 10.7 | 12.7 | 1.134 | | | | |
| 3-phosphoinositide Biosynthesis | | 3.42 | -2.828 | | | | |
| Tyrosine Biosynthesis IV | | | * | | | | |
| Dendritic Cell Maturation | 10.5 | 33.6 | -0.557 | | 1.897 | | |
| Glycoaminoglycan-protein Linkage Region Biosynthesis | | | * | | | | |
| NF-κB Signaling | 8.97 | 36.4 | -2.921 | -0.447 | * | | 0.447 |
| RAN Signaling | | | * | | | | |
| Macropinocytosis Signaling | 5.53 | 15 | -1.941 | | | | |
| PPAR Signaling | 3.53 | 20.5 | 1.886 | | -1.342 | | |
| nNOS Signaling in Skeletal Muscle Cells | 15.4 | 1.44 | * | | | | |
| HMGB1 Signaling | 8.48 | 38.7 | -1.46 | | 1.134 | | |
| Actin Nucleation by ARP-WASP Complex | | 2.98 | -1.155 | | | | |

(continued)

| Ingenuity canonical pathways | Canonical pathways affected in dementia and neurodegenerative disease (p value) | Canonical pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Insulin Receptor Signaling | 5.78 | 8.97 | -1.877 | | | | |
| mTOR Signaling | 2.43 | 6.06 | -1.89 | | 1 | | |
| * ambiguous effect | | | | | | | |

Table 40 - Canonical pathways affected by TBI after 7 days and the effects of LMW-DS

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Axonal Guidance Signaling | 11 | 17.3 | * | | | | |
| CREB Signaling in Neurons | 17.8 | 3.94 | Inhibited | | | | |
| Opioid Signaling Pathway | 20.8 | | Inhibited | Inhibited | * | | Activated |
| Synaptic Long Term Depression | 13.7 | 4.67 | Inhibited | Activated | Activated | | Activated |
| Synaptic Long Term Potentiation | 14.3 | 3.49 | Inhibited | | | | |
| GNRH Signaling | 17.9 | 9.75 | Inhibited | | Activated | | |
| Molecular Mechanisms of Cancer | 14.6 | 32.2 | * | | | | |
| CXCR4 Signaling | 4.2 | 10.3 | Inhibited | | | | |
| Neuropathic Pain Signaling In Dorsal Horn Neurons | 16.9 | 3.31 | Inhibited | | | | * |
| Factors Promoting Cardiogenesis in Vertebrates | 4.56 | 12.6 | * | | | | |
| Cholecystokinin/ Gastrin-mediated Signaling | 7.43 | 9.52 | Inhibited | | | | |
| Calcium Signaling | 33.2 | 6.28 | Inhibited | | | | |
| Osteoarthritis Pathway | 17.6 | 43.2 | Inhibited | | Inhibited | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Epithelial Adherens Junction Signaling | 2.74 | 21.8 | * | | | | |
| Endothelin-1 Signaling | 12.2 | 14.1 | Inhibited | Activated | Activated | Activated | |
| Cardiac Hypertrophy Signaling | 14.6 | 19.9 | Inhibited | | Activated | | |
| Glutamate Receptor Signaling | 12.1 | | Inhibited | | | | |
| GPCR-Mediated Nutrient Sensing in Enteroendocrine Cells | 12.4 | | Inhibited | | | | |
| Actin Cytoskeleton Signaling | 1.66 | 12.5 | Inhibited | | | | |
| UVC-Induced MAPK Signaling | 6.23 | 8.51 | Inhibited | | | | |
| Dopamine-DARPP32 Feedback in cAMP Signaling | 16.2 | 2.58 | Inhibited | | | | |
| Role of NFAT in Cardiac Hypertrophy | 18.1 | 16.1 | Inhibited | | * | | Activated |
| Phospholipase C Signaling | 4.22 | 11.6 | Inhibited | | Activated | Activated | |
| Role of Macrophages, Fibroblasts and Endothelial Cells in Rheumatoid Arthritis | 14.2 | 53.2 | * | | | | |
| Role of Osteoblasts, Osteoclasts and Chondrocytes in Rheumatoid Arthritis | 8.77 | 47.7 | * | | | | |
| Agrin Interactions at Neuromuscular Junction | 4.16 | 6.61 | Inhibited | | | | |
| Aldosterone Signaling in Epithelial Cells | 4.23 | 3.44 | Inhibited | | | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Protein Kinase A Signaling | 6.1 | 8.04 | Inhibited | | Inhibited | | |
| PTEN Signaling | 9.31 | 28.9 | Activated | | | | |
| Gap Junction Signaling | 13.4 | 21.8 | * | | | | |
| G Beta Gamma Signaling | 14.7 | 5.48 | Inhibited | Activated | | | Activated |
| Wnt/p-catenin Signaling | | 8.18 | Activated | | Inhibited | | |
| Thrombin Signaling | 3.11 | 10.2 | Inhibited | | | | |
| Glioblastoma Multiform Signaling | 3.92 | 16.4 | Inhibited | | | | |
| Corticotropin Releasing Hormone Signaling | 18.1 | 7.67 | Inhibited | | | | |
| Tec Kinase Signaling | 4.92 | 17.4 | Inhibited | | | | |
| nNOS Signaling in Neurons | 13 | 3.94 | Inhibited | | | | |
| Cellular Effects of Sildenafil (Viagra) | 6.22 | 2.54 | * | | | | |
| IL-8 Signaling | 9.79 | 34.7 | Inhibited | | Activated | | |
| Ephrin Receptor Signaling | 4.59 | 8.64 | Inhibited | | | | Activated |
| Basal Cell Carcinoma Signaling | | 3.44 | | | | | |
| Colorectal Cancer Metastasis Signaling | 10.2 | 38.4 | Inhibited | | Inhibited | | |
| PPARa/RXRa Activation | 8.12 | 16.4 | Activated | | * | | |
| Neuregulin Signaling | 6.88 | 10.7 | Inhibited | | | | |
| Hepatic Fibrosis / Hepatic Stellate Cell Activation | 15.1 | 68.7 | * | | | | |
| Ephrin B Signaling | | 4 | Inhibited | | | | |
| GP6 Signaling Pathway | 1.86 | | Inhibited | | | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Regulation of the Epithelial-Mesenchymal Transition Pathway | 3.69 | 30 | * | | | | |
| UVA-Induced MAPK Signaling | 6.66 | 9.44 | Inhibited | | | | |
| Signaling by Rho Family GTPases | 2.29 | 8.92 | Inhibited | | Activated | | Activated |
| Pyridoxal 5'-phosphate Salvage Pathway | 4.9 | | Inhibited | | | | |
| Huntington's Disease Signaling | 20.9 | 6.68 | Inhibited | | | | |
| ErbB Signaling | 6.54 | 14.8 | Inhibited | | | | |
| α-Adrenergic Signaling | 5.91 | 1.99 | Inhibited | | | | |
| Fcy Receptor-mediated Phagocytosis in Macrophages and Monocytes | 7.62 | 6.87 | Activated | | Activated | | |
| Natural Killer Cell Signaling | 4.39 | 5.95 | * | | | | |
| Renin-Angiotensin Signaling | 13.2 | 18.9 | Inhibited | | | | |
| RhoGDI Signaling | | 2.14 | Activated | | | | |
| GPCR-Mediated Integration of Enteroendocrin e Signaling Exemplified by an L Cell | 4.53 | | Activated | | | | |
| HGF Signaling | 7.48 | 17.4 | Inhibited | | | | |
| Gaq Signaling | 12.2 | 15.2 | Inhibited | | | | |
| 14-3-3-mediated Signaling | 12.2 | 23.7 | Inhibited | | | | |
| P2Y Purigenic Receptor Signaling Pathway | 7.16 | 7.78 | Inhibited | | | | |
| G-Protein Coupled Receptor Signaling | 22.1 | 18.1 | * | | | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| PCP pathway | | 2.56 | Inhibited | | | | |
| Thyroid Cancer Signaling | 9.4 | 7.72 | * | | | | |
| Melatonin Signaling | 8.59 | | Inhibited | | | | |
| Mouse Embryonic Stem Cell Pluripotency | 1.35 | 17.9 | Inhibited | | | | |
| IL-3 Signaling | 4.09 | 16.8 | Inhibited | | | | |
| Integrin Signaling | 1.36 | 12.4 | Inhibited | | | | |
| Androgen Signaling | 12.2 | 2.95 | Inhibited | | | | |
| Nitric Oxide Signaling in the Cardiovascular System | 11.7 | 12.9 | Inhibited | | | | |
| Paxillin Signaling | 1.56 | 10.6 | Inhibited | | | | |
| Fc Epsilon RI Signaling | 5.05 | 15.7 | Inhibited | | | | Inhibited |
| NGF Signaling | 9.02 | 14.7 | Inhibited | | | | |
| Adrenomedullin signaling pathway | 10.4 | | Inhibited | Inhibited | Inhibited | * | Inhibited |
| Semaphorin Signaling in Neurons | | 1.33 | * | | | | |
| FLT3 Signaling in Hematopoietic Progenitor Cells | 1.8 | 14.4 | Inhibited | | | | * |
| fMLP Signaling in Neutrophils | 3.74 | 14.3 | Inhibited | | | | |
| Phagosome Formation | 5.65 | 6.16 | * | | | | |
| Ovarian Cancer Signaling | 6.42 | 21.1 | Inhibited | | | | |
| VDR/RXR Activation | 4.65 | 10.2 | Activated | | * | | |
| Leukocyte Extravasation Signaling | 6.36 | 19.7 | Inhibited | | Activated | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| D-myo-inositol (1,4,5)-Trisphosphate Biosynthesis | | | Inhibited | | | | |
| Salvage Pathways of Pyrimidine Ribonucleotides | 3.02 | | Inhibited | | | | |
| Wnt/Ca+ pathway | 4.79 | 1.59 | Inhibited | | | | |
| Role of NANOG in Mammalian Embryonic Stem Cell Pluripotency | | 17 | Inhibited | | | | |
| Virus Entry via Endocytic Pathways | 3.75 | 11 | * | | | | |
| Type II Diabetes Mellitus Signaling | 19 | 16.1 | Inhibited | | | | |
| Rac Signaling | 2.62 | 13.5 | Inhibited | | | | |
| CCR3 Signaling in Eosinophils | 3.08 | 10.5 | Inhibited | | | | |
| cAMP-mediated signaling | 15.8 | 10 | Inhibited | | Inhibited | Activated | |
| Notch Signaling | 3.05 | | Inhibited | | | | |
| HER-2 Signaling in Breast Cancer | 3.27 | 13.1 | * | | | | |
| Caveolar-mediated Endocytosis Signaling | 1.96 | 5.58 | * | | | | |
| CCR5 Signaling in Macrophages | 16.3 | 4.77 | | | | | |
| Sperm Motility | 4.03 | 1.76 | Inhibited | | | | |
| Regulation of Actin-based Motility by Rho | | 2.14 | Inhibited | | | | |
| Adipogenesis pathway | 4.87 | 13.9 | * | | | | |
| Growth Hormone Signaling | 6.85 | 9.43 | Inhibited | | | | |
| B Cell Receptor Signaling | 9.59 | 28.2 | Inhibited | | | | Inhibited |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| PI3K Signaling in B Lymphocytes | 7.67 | 20.4 | Inhibited | | Activated | | |
| Role of Tissue Factor in Cancer | 5.6 | 27.1 | * | | | | |
| Human Embryonic Stem Cell Pluripotency | 3.32 | 19.9 | * | | | | |
| TGF-β Signaling | 2.26 | 24.2 | Inhibited | | | | |
| Erythropoietin Signaling | 4.67 | 16.7 | * | | | | |
| Antiproliferative Role of Somatostatin Receptor 2 | | 8.4 | Inhibited | | | | |
| ERK/MAPK Signaling | 5.66 | 12.8 | Inhibited | | Activated | | |
| p70S6K Signaling | 6.22 | 11.9 | Inhibited | | | | |
| CNTF Signaling | | 13.2 | Inhibited | | | | |
| GDNF Family Ligand-Receptor Interactions | 3.68 | 9.29 | Inhibited | | | | |
| BMP signaling pathway | 5.09 | 17.7 | Inhibited | | | | |
| Role of NFAT in Regulation of the Immune Response | 5.53 | 15.1 | Inhibited | Activated | Activated | | Activated |
| Neuroinflammation Signaling Pathway | 54.8 | | Inhibited | | Activated | | |
| Germ Cell-Sertoli Cell Junction Signaling | 3.63 | 23.6 | * | | | | |
| Glioma Signaling | 6.44 | 18.2 | Inhibited | | | | |
| Netrin Signaling | 14.4 | 2.95 | * | | | | |
| Role of Wnt/GSK-3β Signaling in the Pathogenesis of Influenza | | | Activated | | | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Production of Nitric Oxide and Reactive Oxygen Species in Macrophages | 13.7 | 27.7 | Inhibited | | Activated | | |
| Cardiac β-adrenergic Signaling | 3.77 | | Inhibited | | | | |
| Calcium-induced T Lymphocyte Apoptosis | 3.2 | 4.29 | Inhibited | | | | |
| UVB-Induced MAPK Signaling | 7.17 | 9.71 | Inhibited | | | | |
| ErbB4 Signaling | 3.93 | 8.87 | Inhibited | | | | |
| Gas Signaling | 8.77 | 3.53 | Inhibited | | | | |
| RAR Activation | 6.66 | 8.92 | * | | | | |
| 1D-myo-inositol Hexakisphosphate Biosynthesis II (Mammalian) | | | Inhibited | | | | |
| Acute Myeloid Leukemia Signaling | 2.95 | 14.1 | Inhibited | | | | |
| Relaxin Signaling | 3.61 | 10.1 | Inhibited | | | | |
| N F-$_\kappa$B Activation by Viruses | 3.27 | 15.1 | Inhibited | | | | |
| Telomere Extension by Telomerase | | | * | | | | |
| Superpathway of Inositol Phosphate Compounds | | 2.44 | Inhibited | | | | Activated |
| PAK Signaling | 1.8 | 11.5 | Inhibited | | | | |
| GABA Receptor Signaling | 30.6 | | * | | | | |
| IL-4 Signaling | 3.7 | 11.8 | * | | | | |
| Prolactin Signaling | 4.56 | 12.3 | Inhibited | | | | |
| Phenylalanine Degradation I (Aerobic) | | | * | | | | |
| ILK Signaling | 6.57 | 24.1 | Inhibited | | Activated | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Thrombopoietin Signaling | 6.39 | 10.3 | Inhibited | | | | |
| STAT3 Pathway | 9.57 | 25.5 | Inhibited | | * | | |
| Parkinson's Signaling | 7.06 | 1.7 | * | | | | |
| SAPK/JNK Signaling | 2.17 | 7.22 | Inhibited | | | | |
| NRF2-mediated Oxidative Stress Response | 8.95 | 10.5 | Inhibited | | | | |
| Melanocyte Development and Pigmentation Signaling | 2.8 | 7.64 | Inhibited | | | | |
| RhoA Signaling | | 2.58 | Inhibited | | | | |
| FcyRIIB Signaling in B Lymphocytes | 11.9 | 8.78 | Inhibited | | | | |
| eNOS Signaling | 29 | 9.79 | Inhibited | | | | |
| FAK Signaling | 1.82 | 14.4 | * | | | | |
| Serotonin Receptor Signaling | 9.58 | | * | | | | |
| PEDF Signaling | 6.56 | 25.5 | Inhibited | | | | |
| VEGF Family Ligand-Receptor Interactions | 4.77 | 13.3 | Inhibited | | | | |
| Breast Cancer Regulation by Stathmin1 | 5.84 | 11 | * | | | | |
| D-myo-inositol-5-phosphate Metabolism | | | Inhibited | | | | |
| IL-10 Signaling | 6.55 | 23.3 | * | | | | |
| IL-15 Signaling | 3.78 | 25 | * | | | | |
| Sertoli Cell-Sertoli Cell Junction Signaling | 5.76 | 21.6 | * | | | | |
| JAK/Stat Signaling | 2.4 | 20.2 | Inhibited | | | | |
| Apoptosis Signaling | 13 | 13.8 | Activated | | | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| PDGF Signaling | 6.67 | 20.4 | Inhibited | | | | |
| Non-Small Cell Lung Cancer Signaling | 3.49 | 13.7 | Inhibited | | | | |
| D-myo-inositol (1,4,5)-trisphosphate Degradation | | | | | | | |
| Gai Signaling | 9.38 | 9.83 | Inhibited | | | | |
| Glutamate Dependent Acid Resistance | 2 | | * | | | | |
| PKCθ Signaling in T Lymphocytes | 10.7 | 17.3 | Inhibited | | Activated | | |
| Role of IL-17F in Allergic Inflammatory Airway Diseases | 4.79 | 11.7 | Inhibited | | | | |
| Amyotrophic Lateral Sclerosis Signaling | 28.1 | 13.5 | Inhibited | | | | |
| TWEAK Signaling | 5 | 4.46 | Inhibited | | | | |
| Sphingosine-1-phosphate Signaling | 5.14 | 7.9 | Inhibited | | | | |
| Superpathway of D-myo-inositol (1,4,5)-trisphosphate Metabolism | 1.37 | | Inhibited | | | | |
| Mechanisms of Viral Exit from Host Cells | 5.27 | | * | | | | |
| CDK5 Signaling | 8.38 | 3.35 | Inhibited | | | | |
| IL-1 Signaling | 3.22 | 7.14 | Inhibited | | Activated | | * |
| D-myo-inositol (1,3,4)-trisphosphate Biosynthesis | | | Inhibited | | | | |
| Leptin Signaling in Obesity | 5.34 | 4.55 | Inhibited | | | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Acute Phase Response Signaling | 18.7 | 37.8 | Inhibited | | Activated | | Inhibited |
| Pancreatic Adenocarcinom a Signaling | 9.68 | 35.1 | Inhibited | | | | |
| LPS-stimulated MAPK Signaling | 7.31 | 18.4 | Inhibited | | | | |
| Cancer Drug Resistance By Drug Efflux | 5.87 | 11 | * | | | | |
| Calcium Transport I | | | | | | | |
| Antioxidant Action of Vitamin C | 6.6 | 8.13 | Activated | | | | |
| Phospholipases | | 1.76 | Inhibited | | | | |
| 3-phosphoinositid e Degradation | | | Inhibited | | | | Activated |
| Urea Cycle | | 1.44 | * | | | | |
| Regulation of Cellular Mechanics by Calpain Protease | 1.3 | 8.67 | Inhibited | | | | |
| Angiopoietin Signaling | 2.01 | 12 | Inhibited | | | | |
| Role of MAPK Signaling in the Pathogenesis of Influenza | 4.53 | 13.7 | * | | | | |
| IL-6 Signaling | 7.42 | 32.4 | Inhibited | | Activated | | * |
| ERK5 Signaling | 3.67 | 6.1 | Inhibited | Inhibited | Inhibited | | |
| GM-CSF Signaling | 3.32 | 25.7 | Inhibited | | | | |
| Oncostatin M Signaling | 2.22 | 15.3 | Inhibited | | | | |
| Circadian Rhythm Signaling | 4.89 | | * | | | | |
| Inhibition of Angiogenesis by TSP1 | 10.7 | 12.7 | Activated | | | | |
| 3-phosphoinositid e Biosynthesis | | 3.42 | Inhibited | | | | |

(continued)

| Ingenuity canonical pathways | Canonical pathways affected in dementia and neurodegenerative disease (p value) | Canonical Pathways affected in scar formation and fibrosis (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Tyrosine Bio-synthesis IV | | | * | | | | |
| Dendritic Cell Ma-turation | 10.5 | 33.6 | Inhibited | | Activated | | |
| Glycoaminoglyc an-protein Link-age Region Bio-synthesis | | | * | | | | |
| NF-κB Signaling | 8.97 | 36.4 | Inhibited | Inhibited | * | | Activated |
| RAN Signaling | | | * | | | | |
| Macropinocytosi s Signaling | 5.53 | 15 | Inhibited | | | | |
| PPAR Signaling | 3.53 | 20.5 | Activated | | Inhibited | | |
| nNOS Signaling in Skeletal Muscle Cells | 15.4 | 1.44 | * | | | | |
| HMGB1 Signaling | 8.48 | 38.7 | Inhibited | | Activated | | |
| Actin Nucleation by ARP-WASP Complex | | 2.98 | Inhibited | | | | |
| Insulin Receptor Signaling | 5.78 | 8.97 | Inhibited | | | | |
| mTOR Signaling | 2.43 | 6.06 | Inhibited | | Activated | | |

Table 41 - Diseases and molecular functions affected by TBI after 7 days and the effects of LMW-DS (p values and z scores)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Cell movement | 1.1E-108 | 5.3E-246 | -6.524 | -1.01 | 2.297 | | 0.154 |
| Size of body | | | -6.2 | | 0.748 | | 0.67 |
| Organization of cytoskeleton | 1.61E-68 | 3.76E-76 | -5.922 | | 2.174 | | 1.922 |
| Migration of cells | 6.8E-103 | 4.3E-241 | -5.885 | | 2.659 | | 0.271 |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Organization of cyto-plasm | 4.68E-69 | 7.6E-74 | -5.875 | | | | 1.922 |
| Cell survival | 1.22E-94 | 4E-184 | -5.807 | | 1.966 | | |
| Formation of cellular protrusions | 2.84E-52 | | -5.739 | | | | 1.183 |
| Development of neurons | 7.82E-63 | | -5.726 | | 1.106 | | 0.688 |
| Quantity of cells | 2.7E-102 | 2.9E-233 | -5.577 | | 0.634 | 0.991 | 0.493 |
| Microtubule dynamics | 2.4E-63 | | -5.549 | | 1.82 | | 1.962 |
| Cell viability | 9.14E-94 | 1E-176 | -5.42 | -1.584 | 1.879 | | |
| Cell viability of tumor cell lines | 7.56E-63 | 1.1E-114 | -5.022 | | 0.991 | | |
| Developmental process of synapse | | | -4.97 | -0.152 | | | 0.849 |
| Development of gap junctions | | | -4.826 | | | | 0.849 |
| Formation of plasma membrane | | | -4.725 | -0.152 | | | |
| Cell-cell contact | | | -4.682 | | | | 1.504 |
| Assembly of intercellular junctions | | | -4.584 | | | | |
| Formation of intercellular junctions | | | -4.329 | | | | 0.391 |
| Morphogenesis of neurons | 4.16E-54 | | -4.318 | | | | 0.205 |
| Neuritogenesis | 2.04E-53 | | -4.318 | | | | |
| Invasion of cells | 1.26E-64 | 1.1E-148 | -4.317 | | 1.32 | | |
| Homing of cells | | 2E-126 | -4.314 | | | | |
| Chemotaxis | | 4.9E-120 | -4.232 | | 1.873 | | |
| Angiogenesis | 6.89E-75 | 1E-210 | -4.219 | | 0.294 | | |
| Development of vasculature | 1.8E-77 | 1.8E-221 | -4.218 | | 0.295 | | |
| Collapse of growth cone | | | -4.145 | | | | |
| Cell movement of tumor cell lines | 1.17E-69 | 1.1E-156 | -4.06 | | 1.492 | | |
| Vasculogenesis | 3.63E-68 | 6.7E-185 | -3.982 | | 0.507 | | |
| Neurotransmission | 3.7E-100 | | -3.909 | | | | 1.214 |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Cell movement of endothelial cells | | 2.38E-86 | -3.817 | | 2.084 | | |
| Transactivation of RNA | | | -3.66 | | | | |
| Transactivation | | | -3.651 | | | | |
| Long-term potentiation | 6.19E-76 | | -3.624 | | | | |
| Transcription | | 3.3E-92 | -3.459 | | 1.317 | 0.747 | |
| Transcription of RNA | | 2.71E-75 | -3.445 | | 1.221 | 0.517 | |
| Synaptic transmission of cells | | | -3.371 | | | | |
| Plasticity of synapse | | | -3.364 | | | | |
| Potentiation of synapse | 1.58E-77 | | -3.319 | | | | |
| Migration of endothelial cells | | 1.18E-81 | -3.312 | | 2.16 | | |
| Synaptic transmission | 8.3E-97 | | -3.304 | | | | |
| Long-term potentiation of brain | | | -3.278 | | | | |
| Migration of tumor cell lines | 9.34E-62 | 5.5E-134 | -3.236 | | | | |
| Quantity of neurons | 1.57E-59 | | -3.147 | | | | |
| Quantity of nervous tissue | 4.93E-60 | | -3.126 | | | | |
| Development of genitourinary system | | 1.77E-77 | -3.125 | | -0.336 | | |
| Long-term potentiation of cerebral cortex | | | -3.102 | | | | |
| Cellular homeostasis | 1E-117 | 1.6E-154 | -3.087 | | 1.615 | | |
| Expression of RNA | | 5.44E-90 | -3.057 | | 1.797 | | |
| Growth of connective tissue | | 4.3E-157 | -3.055 | | -0.324 | | |
| Non-hematologic malignant neoplasm | | | -2.986 | | -0.243 | | -0.223 |
| Synaptic transmission of nervous tissue | | | -2.963 | | | | |
| Shape change of neurites | | | -2.953 | | | | |
| Branching of neurites | | | -2.881 | | | | |
| Transcription of DNA | | | -2.793 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Long-term potentiation of hippocampus | | | -2.789 | | | | |
| Behavior | 7.7E-146 | | -2.715 | | | | |
| Development of body trunk | | 7.2E-188 | -2.709 | | 1.09 | | |
| Cognition | 9.8E-112 | | -2.679 | | | | |
| Branching of neurons | | | -2.669 | | | | |
| Learning | 1.2E-108 | | -2.66 | | | | 0.469 |
| Sprouting | 6.17E-59 | | -2.655 | | | | |
| Branching of cells | 8.41E-54 | | -2.65 | | | | 0.397 |
| Coordination | | | -2.648 | | | | |
| Potentiation of hippocampus | | | -2.611 | | | | |
| Long-term memory | | | -2.571 | | | | |
| Differentiation of neurons | | | -2.556 | | | | |
| Cell movement of blood cells | 2.64E-79 | 2.3E-210 | -2.533 | | | | |
| Leukocyte migration | 1.46E-79 | 3.4E-205 | -2.532 | | 3.062 | 2.365 | |
| Shape change of neurons | | | -2.531 | | | | |
| Dendritic growth/-branching | | | -2.491 | | | | -0.169 |
| Memory | 1.31E-83 | | -2.473 | | | | |
| Carcinoma | | | -2.446 | | -0.403 | 1.067 | -0.358 |
| Genitourinary adenocarcinoma | | | -2.425 | | | | |
| Formation of brain | | | -2.415 | | | | |
| Growth of tumor | 2.27E-68 | 2.8E-193 | -2.369 | | 2.295 | | |
| Growth of organism | | 5.6E-102 | -2.364 | | | | |
| Synthesis of lipid | 1.14E-78 | 5.59E-92 | -2.355 | 0.033 | 1.937 | | |
| Respiratory system development | | | -2.335 | | | | |
| Differentiation of osteoblasts | | | -2.329 | | | | |
| Conditioning | | | -2.324 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Proliferation of neuro-nal cells | 4.49E-61 | | -2.298 | | | | |
| Male genital neoplasm | | | -2.296 | | | | |
| Synaptic depression | | | -2.292 | | | | |
| Development of epithelial tissue | 8.97E-54 | 4.4E-109 | -2.287 | | 0.262 | | |
| Density of neurons | | | -2.27 | | | | |
| Proliferation of connective tissue cells | | 4.7E-152 | -2.237 | | -0.747 | | |
| Formation of lung | | | -2.236 | | | | |
| Prostatic carcinoma | | | -2.219 | | | | |
| Formation of rhombencephalon | | | -2.212 | | | | |
| Innervation | | | -2.204 | | | | |
| Guidance of axons | | | -2.194 | | | | |
| Genitourinary carcinoma | | | -2.191 | | 1.131 | | |
| Discomfort | 4.2E-181 | | -2.184 | | | | |
| Metabolism of hormone | | | -2.158 | -1.066 | | | |
| Cell movement of neurons | | | -2.143 | | | | |
| Long term depression | | | -2.107 | | | | |
| Differentiation of osteoblastic-lineage cells | | | -2.093 | | | | |
| Outgrowth of cells | 2.39E-58 | | -2.085 | | | | |
| Malignant solid tumor | | | -2.079 | | 0.423 | | |
| Non-hematological solid tumor | | | -2.073 | | 0.021 | | -0.913 |
| Growth of neurites | 5.41E-59 | | -2.054 | | | | |
| Transport of molecule | 1.6E-117 | | -2.045 | | 1.854 | | 1.143 |
| Formation of hippocampus | | | -2.042 | | | | |
| Prostatic tumor | | | -2.02 | | | | |
| Formation of muscle | | | -2.01 | | | | |
| Genital tumor | 1.07E-52 | | -2.009 | | 0.305 | | |
| Fibrogenesis | | | -1.986 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Prostatic adenocarci-noma | | | -1.982 | | | | |
| Adenocarcinoma | | | -1.939 | | -0.155 | | -0.944 |
| Transport of K$^+$ | | | -1.912 | | | | |
| Abdominal cancer | | | -1.902 | -2.426 | -0.474 | | -2.015 |
| Cardiogenesis | | 2.07E-92 | -1.895 | | | | |
| Malignant neoplasm of retroperitoneum | | | -1.889 | | | | |
| Development of central nervous system cells | | | -1.886 | | | | |
| Development of reproductive system | | | -1.882 | | | | |
| Epithelial neoplasm | | | -1.877 | -1.313 | | 0.775 | -0.999 |
| Malignant neoplasm of male genital organ | | | -1.864 | | | | |
| Development of head | | | -1.851 | | 1.213 | | |
| Development of body axis | | | -1.851 | | 1.213 | | |
| Patterning of rhombencephalon | | | -1.835 | | | | |
| Axonogenesis | | | -1.798 | | | | |
| Tumorigenesis of tissue | | | -1.785 | -0.998 | -0.832 | 0.918 | -1.333 |
| Synthesis of nitric oxide | 2.05E-53 | 1.3E-98 | -1.752 | | | | |
| Melanoma | | | -1.723 | | | | |
| Outgrowth of neurites | 5.63E-52 | | -1.714 | | | | |
| Urinary tract cancer | 6.04E-53 | | -1.698 | | | | |
| Abdominal adenocarcinoma | | | -1.687 | | 0.73 | | |
| Transport of ion | | | -1.687 | | | | 1.109 |
| Hyperalgesia | 1.56E-55 | | -1.679 | | | | |
| Development of cerebral cortex | | | -1.661 | | | | |
| Dyskinesia | 3.5E-136 | | -1.657 | | | | |
| Proliferation of smooth muscle cells | | 5.2E-120 | -1.64 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Differentiation of connective tissue cells | 1.6E-52 | 3.4E-143 | -1.635 | -0.349 | 0.769 | | -0.011 |
| Prostate cancer | | | -1.628 | | | | |
| Muscle contraction | | | -1.623 | | | | |
| Pelvic tumor | 1.81E-59 | | -1.62 | -1.214 | 0.445 | | |
| Transport of metal ion | | | -1.609 | | | | |
| Formation of filaments | | | -1.578 | | | | |
| Genital tract cancer | | | -1.575 | | | | |
| Neoplasia of epithelial cells | | | -1.555 | | | | |
| Transport of cation | | | -1.55 | | | | |
| Quantity of connective tissue | | 4.8E-113 | -1.546 | | 0.609 | | |
| Differentiation of nervous system | | | -1.543 | | | | |
| Migration of neurons | | | -1.538 | | | | |
| Transport of metal | | | -1.527 | | | | 1 |
| Upper gastrointestinal tract cancer | | | -1.501 | | | | |
| Malignant genitourinary solid tumor | 5.22E-63 | | -1.497 | -0.537 | 0.346 | | |
| Development of central nervous system | | | -1.481 | | | | |
| Differentiation of bone | | 3.9E-104 | -1.458 | | 1.012 | | |
| Proliferation of muscle cells | 1.11E-56 | 1.8E-148 | -1.458 | | | | |
| Formation of dendrites | | | -1.436 | | | | |
| Development of cytoplasm | | | -1.435 | | | | |
| Spatial learning | | | -1.431 | | | | |
| Disorder of basal ganglia | 6.6E-167 | | -1.423 | | | | |
| Cued conditioning | | | -1.414 | | | | |
| Formation of cytoskeleton | | | -1.408 | | | | |
| Transport of inorganic cation | | | -1.402 | | | | |
| Neurological signs | 2.6E-167 | | -1.359 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Development of genital tumor | | | -1.353 | | | | |
| Pelvic cancer | 1.1E-54 | | -1.328 | | | | |
| Central nervous system cancer | 2.25E-65 | 1.15E-85 | -1.321 | | | | |
| Cell cycle progression | | 3.6E-129 | -1.3 | | 1.58 | | |
| Heart rate | | 3.1E-76 | -1.29 | | | | |
| Action potential of neurons | | | -1.279 | | | | |
| Action potential of cells | | | -1.279 | | | | |
| Phosphorylation of protein | | | -1.272 | | | | |
| Abdominal carcinoma | | | -1.258 | -1.987 | -0.831 | | |
| Digestive system cancer | | | -1.241 | -1.96 | -1.792 | | -1.513 |
| Squamous-cell carcinoma | | | -1.234 | | | | |
| Formation of forebrain | | | -1.212 | | | | |
| Formation of telencephalon | | | -1.212 | | | | |
| Hyperesthesia | 2.75E-59 | | -1.204 | | | | |
| Differentiation of bone cells | | 1.4E-102 | -1.199 | -1.799 | 0.85 | 0.903 | -0.237 |
| Cancer of secretory structure | 3.5E-54 | | -1.193 | | 0.64 | | |
| Pancreatic ductal carcinoma | | | -1.177 | | | | |
| Pancreatic ductal adenocarcinoma | | | -1.177 | | | | |
| Pancreatic adenocarcinoma | | | -1.177 | | | | |
| Quantity of metal ion | 2.5E-56 | | -1.165 | | | | |
| Organization of actin cytoskeleton | | | -1.164 | | | | |
| Development of carcinoma | | | -1.158 | | | 0.152 | |
| B-cell non-Hodgkin lymphoma | | | -1.154 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Formation of actin stress fibers | | | -1.139 | | | | |
| Mature B-cell neoplasm | 6.27E-65 | | -1.131 | | | | |
| Glioblastoma | 3.36E-56 | | -1.103 | | | | |
| Pancreatic cancer | | | -1.089 | | | | |
| Sensory disorders | 7.43E-58 | | -1.063 | | | | |
| Development of gastrointestinal tract | | | -1.062 | | | | |
| Quantity of metal | 8.53E-63 | 1.99E-81 | -1.061 | 0.415 | | | |
| Cell movement of myeloid cells | 8.57E-58 | 1.3E-173 | -1.047 | | 3.907 | 1.197 | |
| Function of muscle | | 6.94E-87 | -1.043 | | | | |
| Cancer | | | -1.035 | | 0.905 | 1.705 | |
| Formation of actin filaments | | | -1.028 | | | | |
| Head and neck carci-noma | | | -1.026 | | | | |
| Excitatory postsynap-tic potential | | | -1 | | | | |
| Progressive neurologi-cal disorder | 6.6E-215 | | -0.963 | | | | |
| Development of adenocarcinoma | | | -0.952 | | | | |
| Cancer of cells | 7.6E-56 | 1.17E-97 | -0.927 | | 0.742 | | |
| Concentration of hormone | | | -0.917 | -0.32 | | | 0.825 |
| Genitourinary tumor | 6.65E-66 | | -0.908 | | 1.388 | 1.746 | |
| Abdominal neoplasm | | | -0.871 | | 0.061 | -0.272 | -1.116 |
| Spatial memory | | | -0.869 | | | | |
| Urinary tract tumor | 8.53E-58 | 3.28E-74 | -0.863 | | | | |
| Head and neck cancer | | | -0.86 | | -1.154 | | |
| Upper gastrointestinal carcinoma | | | -0.849 | | | | |
| Extraadrenal retroperitoneal tumor | | | -0.821 | | | | |
| Secretion of molecule | 1.66E-75 | | -0.8 | | 1.386 | | |
| Astrocytoma | | | -0.786 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Gonadal tumor | | | -0.732 | | | | |
| Quantity of carbohydrate | 3.84E-52 | 2.39E-87 | -0.732 | 0.49 | | | -0.017 |
| Ductal carcinoma | | | -0.728 | | | | |
| Development of digestive system | | | -0.724 | | | | |
| Tumorigenesis of reproductive tract | | | -0.713 | | | | |
| Development of connective tissue cells | | 1.1E-76 | -0.712 | -0.005 | 1.638 | 0.766 | -0.005 |
| Neoplasia of cells | 1.65E-64 | 4.1E-103 | -0.704 | | 0.474 | | |
| Non-melanoma solid tumor | | | -0.698 | | 0.01 | 1.121 | -1.478 |
| Ovarian tumor | | | -0.668 | | | | |
| Growth of epithelial tissue | 3.1E-59 | 7.7E-164 | -0.65 | | -1.58 | | |
| Pancreatic carcinoma | | | -0.649 | | | | |
| Fear | | | -0.637 | | | | |
| Quantity of $Ca^{2+}$ | 1.96E-55 | | -0.627 | -0.11 | | | 0.224 |
| Lung cancer | 1.74E-74 | 1.33E-95 | -0.602 | | | | |
| Ossification of bone | | | -0.588 | | | | |
| Abnormality of cerebral cortex | | | -0.524 | | | | |
| Function of smooth muscle | | | -0.516 | | | | |
| Female genital neoplasm | | | -0.502 | | | | |
| Emotional behavior | 1.13E-57 | | -0.502 | | | | |
| Solid tumor | | | -0.473 | | 1.29 | 0.992 | |
| Malignant connective or soft tissue neoplasm | | 3.28E-97 | -0.471 | | | | |
| Liver tumor | | | -0.451 | -1.91 | | | |
| Respiratory system tumor | 4.27E-70 | 2.31E-95 | -0.451 | | | | |
| Cognitive impairment | 7.8E-118 | | -0.428 | | | | |
| Thoracic cancer | 5.97E-75 | 6.2E-100 | -0.425 | | | | |
| Glioma | 2.96E-58 | | -0.416 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Central nervous system tumor | 4.16E-69 | 7.45E-77 | -0.411 | | | | |
| Central nervous system solid tumor | 9.68E-69 | 1.55E-76 | -0.411 | | | | |
| Liquid tumor | 3.25E-66 | 1.21E-82 | -0.398 | | | | |
| Skin carcinoma | | | -0.391 | | | | |
| Leukemic tumor | 4.28E-54 | | -0.379 | | | | |
| Gastrointestinal tract cancer | | | -0.377 | | | | |
| Abnormality of cerebrum | | | -0.365 | | | | |
| Concentration of lipid | 2.48E-87 | 6.3E-118 | -0.361 | | -0.575 | | -0.204 |
| Glioma cancer | 1.45E-57 | 5.12E-74 | -0.351 | | | | |
| Tumor in nervous system | 8.7E-72 | 3.4E-77 | -0.337 | | | | |
| Colon cancer | | | -0.314 | | | | |
| Upper gastrointestinal tract tumor | | | -0.295 | | | | |
| Hepatobiliary system cancer | | | -0.293 | | | | |
| Head and neck tumor | | | -0.269 | | -0.355 | | |
| Colorectal cancer | | | -0.251 | | | | |
| Liver cancer | | | -0.25 | | | | |
| Proliferation of epithelial cells | | 4.7E-125 | -0.219 | | -1.196 | | |
| Breast or pancreatic cancer | 1.55E-69 | | -0.211 | | -1.026 | | |
| Tumorigenesis of epithelial neoplasm | | | -0.168 | -1.981 | | 0.152 | |
| Development of colorectal tumor | | | -0.152 | | | | |
| Weight gain | 1.15E-72 | | -0.15 | 0.625 | | | |
| Quantity of steroid hormone | | | -0.127 | | | | |
| Lung carcinoma | 3.9E-61 | | -0.113 | | | | |
| B-cell lymphoproliferative disorder | | | -0.085 | | | | |
| B-cell neoplasm | 1.39E-70 | | -0.085 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| B cell cancer | | | -0.085 | | | | |
| Lung tumor | 1.04E-78 | 8.1E-103 | -0.082 | | | | |
| Gastrointestinal carcinoma | | | -0.068 | | | | |
| Epileptic seizure | | | -0.054 | | | | |
| Endocrine gland tumor | | | -0.049 | | -0.067 | | |
| Oscillation of $Ca^{2+}$ | | | -0.035 | | | | |
| Tauopathy | 0 | 5.43E-89 | * | | | | |
| Extracranial solid tumor | | | 0.01 | | 0.369 | 1.474 | 0.529 |
| Development of sensory organ | | | 0.02 | | 0.669 | | |
| Malignant neoplasm of large intestine | | | 0.048 | | | | |
| Pancreatobiliary tumor | | | 0.052 | | | | |
| Secretion of neurotransmitter | | | 0.083 | | | | |
| Sarcoma | | 1.96E-92 | 0.083 | | | | |
| Connective tissue tumor | | 4.4E-105 | 0.086 | | | | |
| Epilepsy | 1.79E-93 | | 0.091 | | | | |
| Liver carcinoma | | | 0.101 | | | | |
| Cell death of brain | 6.8E-111 | | 0.108 | | | | |
| Thermoregulation | | | 0.122 | | | | |
| Pancreatic tumor | | | 0.125 | | | | |
| Skin tumor | | | 0.148 | | -2.396 | | |
| Thoracic neoplasm | 2.34E-79 | 2.5E-108 | 0.173 | | | | |
| Development of respiratory system tumor | | | 0.174 | | | | |
| Necrosis of epithelial tissue | 4.75E-82 | 6.8E-155 | 0.183 | | 1.674 | | |
| Cell death of central nervous system cells | 3E-107 | | 0.185 | | | | |
| B-cell lymphoma | | | 0.19 | | | | |
| Cell death of tumor cell lines | 3.79E-88 | 5.8E-159 | 0.215 | | -0.811 | 0.178 | |
| Digestive organ tumor | | | 0.227 | -1.396 | -1.348 | | -1.481 |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Connective or soft tissue tumor | | 1.2E-119 | 0.231 | | | | |
| Formation of eye | | | 0.251 | | 1.664 | | |
| Neuronal cell death | 9.9E-137 | 4.87E-88 | 0.254 | | | | |
| Stomach tumor | | | 0.275 | | | | |
| Growth of axons | | | 0.275 | | | | |
| Disorder of pregnancy | | | 0.29 | | | | |
| Breast or colorectal cancer | 6.1E-55 | | 0.33 | | -1.953 | | |
| Sensory system development | | | 0.335 | | -0.307 | | |
| Development of lung tumor | | | 0.347 | | | | |
| Cell death of brain cells | 7.5E-108 | | 0.349 | | | | |
| Neurodegeneration of cerebral cortex | | | 0.385 | | | | |
| Anxiety | | | 0.388 | | | | |
| Breast carcinoma | | | 0.418 | | | | |
| Obesity | 5.6E-152 | | 0.419 | | 0.493 | | 2.18 |
| Development of intestinal tumor | | | 0.44 | | | | |
| Development of malignant tumor | | | 0.455 | -1.326 | | -0.774 | |
| Lung adenocarcinoma | | | 0.468 | | | | |
| Skin cancer | | | 0.488 | | | | |
| Non-small cell lung carcinoma | 1.09E-56 | | 0.493 | | | | |
| Movement Disorders | 2E-227 | | 0.536 | | | | |
| Diffuse lymphoma | | | 0.555 | | | | |
| Gastric lesion | | | 0.565 | | | | |
| Occlusion of artery | 3E-152 | 3.2E-178 | 0.586 | | | | |
| Non-Hodgkin lymphoma | | | 0.621 | | | | |
| Locomotion | 1.34E-66 | | 0.697 | | | | |
| Breast or ovarian carcinoma | | | 0.73 | | | | |
| Breast cancer | 2.25E-70 | 2.2E-134 | 0.73 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Glucose metabolism disorder | 1.4E-184 | 1.4E-170 | 0.75 | | 0.439 | | |
| Incidence of tumor | | | 0.782 | -1.614 | | -0.865 | |
| Atherosclerosis | 9.5E-131 | 2.8E-174 | 0.783 | | | | |
| Amyloidosis | 0 | 1.46E-91 | 0.812 | | | | |
| Liver lesion | | 1.4E-110 | 0.833 | | | | |
| Mood Disorders | 2.4E-173 | | 0.836 | | | | |
| Depressive disorder | 9.7E-162 | | 0.836 | | | | |
| Lymphohematopoietic cancer | 1.28E-94 | 6.2E-121 | 0.845 | | | | |
| Paired-pulse facilita-tion | | | 0.852 | | | | |
| Lymphoreticular neo-plasm | 6.38E-75 | | 0.856 | | -1.224 | | |
| Colon tumor | | | 0.864 | | | | |
| Apoptosis of tumor cell lines | 4.41E-93 | 5.3E-155 | 0.867 | | -0.941 | 0.783 | |
| Cell death of epithelial cells | 4.48E-69 | 3E-123 | 0.886 | | 1.993 | | |
| Vaso-occlusion | 6.2E-151 | 2.9E-179 | 0.909 | 1.264 | | | |
| Subcutaneous tumor | | | 0.911 | | | | |
| Colorectal tumor | | | 0.93 | | | | |
| Occlusion of blood vessel | 1.7E-152 | 3.4E-180 | 0.969 | | | | |
| Lymphatic system tumor | 4.79E-88 | | 0.977 | | -0.956 | | |
| Breast or ovarian cancer | 7.8E-65 | 4.6E-113 | 1.011 | | -1.953 | | |
| Hypertrophy | 1.65E-56 | 2.6E-219 | 1.011 | | | | |
| Hematologic cancer | 1.05E-92 | 2.2E-115 | 1.074 | -1.067 | -1.725 | | -2.216 |
| Large intestine neoplasm | | | 1.126 | -1.192 | | | |
| Lymphoid cancer | 1.85E-77 | 1.8E-114 | 1.127 | | -0.956 | | |
| Hypertension | 4.14E-89 | | 1.128 | | | | |
| Gastrointestinal adenocarcinoma | | | 1.181 | | | | |
| Frequency of tumor | | | 1.228 | -2.128 | | -1.519 | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Lymphohematopoietic neoplasia | 1E-96 | 6.2E-133 | 1.232 | | | | |
| Skin lesion | | | 1.234 | | -0.111 | | 0.532 |
| Neck neoplasm | | | 1.257 | | | | |
| Mammary tumor | 3.35E-72 | 5.2E-153 | 1.261 | | | | |
| Motor dysfunction or movement disorder | 7.7E-228 | | 1.269 | | | | |
| Gastrointestinal tumor | | | 1.279 | -1.029 | -1.215 | | -1.284 |
| Hematologic cancer of cells | 2.64E-71 | 4E-144 | 1.314 | | -1.486 | | |
| Disorder of blood pressure | 3.79E-97 | | 1.325 | | | | |
| Hematopoietic neoplasm | 2.37E-95 | | 1.338 | -0.686 | -1.002 | | -2.027 |
| Seizure disorder | 3E-118 | | 1.343 | | | | 1.376 |
| Seizures | 1.01E-97 | | 1.362 | | | | |
| Necrosis | 3.1E-153 | 1.4E-251 | 1.376 | | 0.228 | 0.213 | |
| Peripheral vascular disease | 5.7E-170 | | 1.389 | | 1 | | |
| Lymphoproliferative disorder | 2.49E-83 | 2E-104 | 1.435 | | -1.727 | | |
| Neoplasia of leukocytes | 5.5E-88 | 1.3E-149 | 1.44 | | -1.486 | | |
| Intestinal tumor | | | 1.486 | | -1.09 | | |
| Lymphocytic cancer | 3.97E-73 | | 1.569 | | -1.486 | | |
| Lymphocytic neoplasm | 2.2E-82 | 4.3E-139 | 1.569 | | -1.486 | | |
| Cell death of muscle cells | 1.7E-54 | 9.9E-127 | 1.829 | | | | |
| Renal impairment | 4.4E-100 | 3.2E-101 | 1.835 | | 0.555 | | |
| Failure of kidney | 4.17E-85 | 4.4E-107 | 1.835 | | 0.555 | | |
| Cerebrovascular dysfunction | 1.3E-186 | | 1.845 | | | | |
| Lymphoma | 4.3E-54 | 1E-143 | 1.896 | | -1.224 | | |
| Development of digestive organ tumor | | | 1.909 | | | | |
| Cell death of muscle | | 1.7E-134 | 1.921 | | | | |
| Necrosis of muscle | 3.34E-54 | 1.4E-133 | 1.921 | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeated dose LMW-DS |
|---|---|---|---|---|---|---|---|
| Neurodegeneration | 3.46E-85 | | 2.046 | | | | |
| Abnormality of heart ventricle | 1.36E-63 | 7.5E-128 | 2.157 | | | | |
| Development of benign tumor | 2.1E-59 | | 2.423 | | | | |
| Benign Tumors | 3.71E-75 | | 2.493 | | | | |
| Benign lesion | 9.74E-87 | | 2.695 | | | | |
| Cell death | 6.5E-155 | 3.7E-254 | 3.326 | | 0.791 | -1.269 | |
| Apoptosis | 7.5E-135 | 1.1E-244 | 3.418 | | -0.676 | -0.256 | |
| Hyperactive behavior | | | 4.022 | | | | |
| Bleeding | 7.55E-94 | 2.5E-102 | 4.287 | | -2.118 | | |
| Neonatal death | | | 6.487 | | | | |
| Perinatal death | | | 8.086 | | | | |
| Morbidity or mortality | 4.8E-108 | 2.3E-216 | 11.646 | | -2.848 | | |
| Organismal death | 2E-109 | 3.5E-213 | 11.962 | | -2.885 | | |

Table 42 - Diseases and molecular functions affected by TBI after 7 days and the effects of LMW-DS

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Cell movement | 1.1E-108 | 5.3E-246 | Inhibited | Inhibited | Activated | | Activated |
| Size of body | | | Inhibited | | Activated | | Activated |
| Organization of cytoskeleton | 1.61E-68 | 3.76E-76 | Inhibited | | Activated | | Activated |
| Migration of cells | 6.8E-103 | 4.3E-241 | Inhibited | | Activated | | Activated |
| Organization of cytoplasm | 4.68E-69 | 7.6E-74 | Inhibited | | | | Activated |
| Cell survival | 1.22E-94 | 4E-184 | Inhibited | | Activated | | |
| Formation of cellular protrusions | 2.84E-52 | | Inhibited | | | | Activated |
| Development of neurons | 7.82E-63 | | Inhibited | | Activated | | Activated |
| Quantity of cells | 2.7E-102 | 2.9E-233 | Inhibited | | Activated | Activated | Activated |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Microtubule dynamics | 2.4E-63 | | Inhibited | | Activated | | Activated |
| Cell viability | 9.14E-94 | 1E-176 | Inhibited | Inhibited | Activated | | |
| Cell viability of tumor cell lines | 7.56E-63 | 1.1E-114 | Inhibited | | Activated | | |
| Developmental process of synapse | | | Inhibited | Inhibited | | | Activated |
| Development of gap junctions | | | Inhibited | | | | Activated |
| Formation of plasma membrane | | | Inhibited | Inhibited | | | |
| Cell-cell contact | | | Inhibited | | | | Activated |
| Assembly of intercellular junctions | | | Inhibited | | | | |
| Formation of intercellular junctions | | | Inhibited | | | | Activated |
| Morphogenesis of neurons | 4.16E-54 | | Inhibited | | | | Activated |
| Neuritogenesis | 2.04E-53 | | Inhibited | | | | |
| Invasion of cells | 1.26E-64 | 1.1E-148 | Inhibited | | Activated | | |
| Homing of cells | | 2E-126 | Inhibited | | | | |
| Chemotaxis | | 4.9E-120 | Inhibited | | Activated | | |
| Angiogenesis | 6.89E-75 | 1E-210 | Inhibited | | Activated | | |
| Development of vasculature | 1.8E-77 | 1.8E-221 | Inhibited | | Activated | | |
| Collapse of growth cone | | | Inhibited | | | | |
| Cell movement of tumor cell lines | 1.17E-69 | 1.1E-156 | Inhibited | | Activated | | |
| Vasculogenesis | 3.63E-68 | 6.7E-185 | Inhibited | | Activated | | |
| Neurotransmission | 3.7E-100 | | Inhibited | | | | Activated |
| Cell movement of endothelial cells | | 2.38E-86 | Inhibited | | Activated | | |
| Transactivation of RNA | | | Inhibited | | | | |
| Transactivation | | | Inhibited | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Long-term potentiation | 6.19E-76 | | Inhibited | | | | |
| Transcription | | 3.3E-92 | Inhibited | | Activated | Activated | |
| Transcription of RNA | | 2.71E-75 | Inhibited | | Activated | Activated | |
| Synaptic transmission of cells | | | Inhibited | | | | |
| Plasticity of synapse | | | Inhibited | | | | |
| Potentiation of synapse | 1.58E-77 | | Inhibited | | | | |
| Migration of endothelial cells | | 1.18E-81 | Inhibited | | Activated | | |
| Synaptic transmission | 8.3E-97 | | Inhibited | | | | |
| Long-term potentiation of brain | | | Inhibited | | | | |
| Migration of tumor cell lines | 9.34E-62 | 5.5E-134 | Inhibited | | | | |
| Quantity of neurons | 1.57E-59 | | Inhibited | | | | |
| Quantity of nervous tissue | 4.93E-60 | | Inhibited | | | | |
| Development of genitourinary system | | 1.77E-77 | Inhibited | | Inhibited | | |
| Long-term potentiation of cerebral cortex | | | Inhibited | | | | |
| Cellular homeostasis | 1E-117 | 1.6E-154 | Inhibited | | Activated | | |
| Expression of RNA | | 5.44E-90 | Inhibited | | Activated | | |
| Growth of connective tissue | | 4.3E-157 | Inhibited | | Inhibited | | |
| Nonhematologic malignant neoplasm | | | Inhibited | | Inhibited | | Inhibited |
| Synaptic transmission of nervous tissue | | | Inhibited | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Shape change of neurites | | | Inhibited | | | | |
| Branching of neurites | | | Inhibited | | | | |
| Transcription of DNA | | | Inhibited | | | | |
| Long-term poten- tiation of hippo- campus | | | Inhibited | | | | |
| Behavior | 7.7E-146 | | Inhibited | | | | |
| Development of body trunk | | 7.2E-188 | Inhibited | | Activated | | |
| Cognition | 9.8E-112 | | Inhibited | | | | |
| Branching of neurons | | | Inhibited | | | | |
| Learning | 1.2E-108 | | Inhibited | | | | Activated |
| Sprouting | 6.17E-59 | | Inhibited | | | | |
| Branching of cells | 8.41E-54 | | Inhibited | | | | Activated |
| Coordination | | | Inhibited | | | | |
| Potentiation of hippocampus | | | Inhibited | | | | |
| Long-term memory | | | Inhibited | | | | |
| Differentiation of neurons | | | Inhibited | | | | |
| Cell movement of blood cells | 2.64E-79 | 2.3E-210 | Inhibited | | | | |
| Leukocyte migration | 1.46E-79 | 3.4E-205 | Inhibited | | Activated | Activated | |
| Shape change of neurons | | | Inhibited | | | | |
| Dendritic growth/- branching | | | Inhibited | | | | Inhibited |
| Memory | 1.31E-83 | | Inhibited | | | | |
| Carcinoma | | | Inhibited | | Inhibited | Activated | Inhibited |
| Genitourinary adenocarcinoma | | | Inhibited | | | | |
| Formation of brain | | | Inhibited | | | | |
| Growth of tumor | 2.27E-68 | 2.8E-193 | Inhibited | | Activated | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Growth of organism | | 5.6E-102 | Inhibited | | | | |
| Synthesis of lipid | 1.14E-78 | 5.59E-92 | Inhibited | Activated | Activated | | |
| Respiratory system development | | | Inhibited | | | | |
| Differentiation of osteoblasts | | | Inhibited | | | | |
| Conditioning | | | Inhibited | | | | |
| Proliferation of neuronal cells | 4.49E-61 | | Inhibited | | | | |
| Male genital neoplasm | | | Inhibited | | | | |
| Synaptic depression | | | Inhibited | | | | |
| Development of epithelial tissue | 8.97E-54 | 4.4E-109 | Inhibited | | Activated | | |
| Density of neurons | | | Inhibited | | | | |
| Proliferation of connective tissue cells | | 4.7E-152 | Inhibited | | Inhibited | | |
| Formation of lung | | | Inhibited | | | | |
| Prostatic carcinoma | | | Inhibited | | | | |
| Formation of rhombencephalon | | | Inhibited | | | | |
| Innervation | | | Inhibited | | | | |
| Guidance of axons | | | Inhibited | | | | |
| Genitourinary carcinoma | | | Inhibited | | Activated | | |
| Discomfort | 4.2E-181 | | Inhibited | | | | |
| Metabolism of hormone | | | Inhibited | Inhibited | | | |
| Cell movement of neurons | | | Inhibited | | | | |
| Long term depression | | | Inhibited | | | | |
| Differentiation of osteoblastic-lineage cells | | | Inhibited | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Outgrowth of cells | 2.39E-58 | | Inhibited | | | | |
| Malignant solid tumor | | | Inhibited | | Activated | | |
| Non-hematological solid tumor | | | Inhibited | | Activated | | Inhibited |
| Growth of neurites | 5.41E-59 | | Inhibited | | | | |
| Transport of molecule | 1.6E-117 | | Inhibited | | Activated | | Activated |
| Formation of hippocampus | | | Inhibited | | | | |
| Prostatic tumor | | | Inhibited | | | | |
| Formation of muscle | | | Inhibited | | | | |
| Genital tumor | 1.07E-52 | | Inhibited | | Activated | | |
| Fibrogenesis | | | Inhibited | | | | |
| Prostatic adenocarcinoma | | | Inhibited | | | | |
| Adenocarcinoma | | | Inhibited | | Inhibited | | Inhibited |
| Transport of K$^+$ | | | Inhibited | | | | |
| Abdominal cancer | | | Inhibited | Inhibited | Inhibited | | Inhibited |
| Cardiogenesis | | 2.07E-92 | Inhibited | | | | |
| Malignant neoplasm of retroperitoneum | | | Inhibited | | | | |
| Development of central nervous system cells | | | Inhibited | | | | |
| Development of reproductive system | | | Inhibited | | | | |
| Epithelial neoplasm | | | Inhibited | Inhibited | | Activated | Inhibited |
| Malignant neoplasm of male genital organ | | | Inhibited | | | | |
| Development of head | | | Inhibited | | Activated | | |
| Development of body axis | | | Inhibited | | Activated | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Patterning of rhombencephalon | | | Inhibited | | | | |
| Axonogenesis | | | Inhibited | | | | |
| Tumorigenesis of tissue | | | Inhibited | Inhibited | Inhibited | Activated | Inhibited |
| Synthesis of nitric oxide | 2.05E-53 | 1.3E-98 | Inhibited | | | | |
| Melanoma | | | Inhibited | | | | |
| Outgrowth of neurites | 5.63E-52 | | Inhibited | | | | |
| Urinary tract cancer | 6.04E-53 | | Inhibited | | | | |
| Abdominal adenocarcinoma | | | Inhibited | | Activated | | |
| Transport of ion | | | Inhibited | | | | Activated |
| Hyperalgesia | 1.56E-55 | | Inhibited | | | | |
| Development of cerebral cortex | | | Inhibited | | | | |
| Dyskinesia | 3.5E-136 | | Inhibited | | | | |
| Proliferation of smooth muscle cells | | 5.2E-120 | Inhibited | | | | |
| Differentiation of connective tissue cells | 1.6E-52 | 3.4E-143 | Inhibited | Inhibited | Activated | | Inhibited |
| Prostate cancer | | | Inhibited | | | | |
| Muscle contraction | | | Inhibited | | | | |
| Pelvic tumor | 1.81E-59 | | Inhibited | Inhibited | Activated | | |
| Transport of metal ion | | | Inhibited | | | | |
| Formation of filaments | | | Inhibited | | | | |
| Genital tract cancer | | | Inhibited | | | | |
| Neoplasia of epithelial cells | | | Inhibited | | | | |
| Transport of cation | | | Inhibited | | | | |
| Quantity of connective tissue | | 4.8E-113 | Inhibited | | Activated | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Differentiation of nervous system | | | Inhibited | | | | |
| Migration of neurons | | | Inhibited | | | | |
| Transport of metal | | | Inhibited | | | | Activated |
| Upper gastrointestinal tract cancer | | | Inhibited | | | | |
| Malignant genitourinary solid tumor | 5.22E-63 | | Inhibited | Inhibited | Activated | | |
| Development of central nervous system | | | Inhibited | | | | |
| Differentiation of bone | | 3.9E-104 | Inhibited | | Activated | | |
| Proliferation of muscle cells | 1.11E-56 | 1.8E-148 | Inhibited | | | | |
| Formation of dendrites | | | Inhibited | | | | |
| Development of cytoplasm | | | Inhibited | | | | |
| Spatial learning | | | Inhibited | | | | |
| Disorder of basal ganglia | 6.6E-167 | | Inhibited | | | | |
| Cued conditioning | | | Inhibited | | | | |
| Formation of cytoskeleton | | | Inhibited | | | | |
| Transport of inorganic cation | | | Inhibited | | | | |
| Neurological signs | 2.6E-167 | | Inhibited | | | | |
| Development of genital tumor | | | Inhibited | | | | |
| Pelvic cancer | 1.1E-54 | | Inhibited | | | | |
| Central nervous system cancer | 2.25E-65 | 1.15E-85 | Inhibited | | | | |
| Cell cycle progression | | 3.6E-129 | Inhibited | | Activated | | |
| Heart rate | | 3.1E-76 | Inhibited | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Action potential of neurons | | | Inhibited | | | | |
| Action potential of cells | | | Inhibited | | | | |
| Phosphorylation of protein | | | Inhibited | | | | |
| Abdominal carcinoma | | | Inhibited | Inhibited | Inhibited | | |
| Digestive system cancer | | | Inhibited | Inhibited | Inhibited | | Inhibited |
| Squamous-cell carcinoma | | | Inhibited | | | | |
| Formation of forebrain | | | Inhibited | | | | |
| Formation of telencephalon | | | Inhibited | | | | |
| Hyperesthesia | 2.75E-59 | | Inhibited | | | | |
| Differentiation of bone cells | | 1.4E-102 | Inhibited | Inhibited | Activated | Activated | Inhibited |
| Cancer of secretory structure | 3.5E-54 | | Inhibited | | Activated | | |
| Pancreatic ductal carcinoma | | | Inhibited | | | | |
| Pancreatic ductal adenocarcinoma | | | Inhibited | | | | |
| Pancreatic adenocarcinoma | | | Inhibited | | | | |
| Quantity of metal ion | 2.5E-56 | | Inhibited | | | | |
| Organization of actin cytoskeleton | | | Inhibited | | | | |
| Development of carcinoma | | | Inhibited | | | Activated | |
| B-cell non-Hodgkin lymphoma | | | Inhibited | | | | |
| Formation of actin stress fibers | | | Inhibited | | | | |
| Mature B-cell neoplasm | 6.27E-65 | | Inhibited | | | | |
| Glioblastoma | 3.36E-56 | | Inhibited | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Pancreatic cancer | | | Inhibited | | | | |
| Sensory disorders | 7.43E-58 | | Inhibited | | | | |
| Development of gastrointestinal tract | | | Inhibited | | | | |
| Quantity of metal | 8.53E-63 | 1.99E-81 | Inhibited | Activated | | | |
| Cell movement of myeloid cells | 8.57E-58 | 1.3E-173 | Inhibited | | Activated | Activated | |
| Function of muscle | | 6.94E-87 | Inhibited | | | | |
| Cancer | | | Inhibited | | Activated | Activated | |
| Formation of actin filaments | | | Inhibited | | | | |
| Head and neck carcinoma | | | Inhibited | | | | |
| Excitatory postsynaptic potential | | | Inhibited | | | | |
| Progressive neurological disorder | 6.6E-215 | | Inhibited | | | | |
| Development of adenocarcinoma | | | Inhibited | | | | |
| Cancer of cells | 7.6E-56 | 1.17E-97 | Inhibited | | Activated | | |
| Concentration of hormone | | | Inhibited | Inhibited | | | Activated |
| Genitourinary tumor | 6.65E-66 | | Inhibited | | Activated | Activated | |
| Abdominal neoplasm | | | Inhibited | | Activated | Inhibited | Inhibited |
| Spatial memory | | | Inhibited | | | | |
| Urinary tract tumor | 8.53E-58 | 3.28E-74 | Inhibited | | | | |
| Head and neck cancer | | | Inhibited | | Inhibited | | |
| Upper gastrointestinal carcinoma | | | Inhibited | | | | |
| Extraadrenal retroperitoneal tumor | | | Inhibited | | | | |
| Secretion of molecule | 1.66E-75 | | Inhibited | | Activated | | |
| Astrocytoma | | | Inhibited | | | | |
| Gonadal tumor | | | Inhibited | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Quantity of carbo-hydrate | 3.84E-52 | 2.39E-87 | Inhibited | Activated | | | Inhibited |
| Ductal carcinoma | | | Inhibited | | | | |
| Development of digestive system | | | Inhibited | | | | |
| Tumorigenesis of reproductive tract | | | Inhibited | | | | |
| Development of connective tissue cells | | 1.1E-76 | Inhibited | Inhibited | Activated | Activated | Inhibited |
| Neoplasia of cells | 1.65E-64 | 4.1E-103 | Inhibited | | Activated | | |
| Non-melanoma solid tumor | | | Inhibited | | Activated | Activated | Inhibited |
| Ovarian tumor | | | Inhibited | | | | |
| Growth of epithelial tissue | 3.1E-59 | 7.7E-164 | Inhibited | | Inhibited | | |
| Pancreatic carcinoma | | | Inhibited | | | | |
| Fear | | | Inhibited | | | | |
| Quantity of Ca$^{2+}$ | 1.96E-55 | | Inhibited | Inhibited | | | Activated |
| Lung cancer | 1.74E-74 | 1.33E-95 | Inhibited | | | | |
| Ossification of bone | | | Inhibited | | | | |
| Abnormality of cerebral cortex | | | Inhibited | | | | |
| Function of smooth muscle | | | Inhibited | | | | |
| Female genital neoplasm | | | Inhibited | | | | |
| Emotional behavior | 1.13E-57 | | Inhibited | | | | |
| Solid tumor | | | Inhibited | | Activated | Activated | |
| Malignant connective or soft tissue neoplasm | | 3.28E-97 | Inhibited | | | | |
| Liver tumor | | | Inhibited | Inhibited | | | |
| Respiratory system tumor | 4.27E-70 | 2.31E-95 | Inhibited | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Cognitive impair-ment | 7.8E-118 | | Inhibited | | | | |
| Thoracic cancer | 5.97E-75 | 6.2E-100 | Inhibited | | | | |
| Glioma | 2.96E-58 | | Inhibited | | | | |
| Central nervous system tumor | 4.16E-69 | 7.45E-77 | Inhibited | | | | |
| Central nervous system solid tumor | 9.68E-69 | 1.55E-76 | Inhibited | | | | |
| Liquid tumor | 3.25E-66 | 1.21E-82 | Inhibited | | | | |
| Skin carcinoma | | | Inhibited | | | | |
| Leukemic tumor | 4.28E-54 | | Inhibited | | | | |
| Gastrointestinal tract cancer | | | Inhibited | | | | |
| Abnormality of cerebrum | | | Inhibited | | | | |
| Concentration of lipid | 2.48E-87 | 6.3E-118 | Inhibited | | Inhibited | | Inhibited |
| Glioma cancer | 1.45E-57 | 5.12E-74 | Inhibited | | | | |
| Tumor in nervous system | 8.7E-72 | 3.4E-77 | Inhibited | | | | |
| Colon cancer | | | Inhibited | | | | |
| Upper gastrointestinal tract tumor | | | Inhibited | | | | |
| Hepatobiliary system cancer | | | Inhibited | | | | |
| Head and neck tumor | | | Inhibited | | Inhibited | | |
| Colorectal cancer | | | Inhibited | | | | |
| Liver cancer | | | Inhibited | | | | |
| Proliferation of epithelial cells | | 4.7E-125 | Inhibited | | Inhibited | | |
| Breast or pancreatic cancer | 1.55E-69 | | Inhibited | | Inhibited | | |
| Tumorigenesis of epithelial neoplasm | | | Inhibited | Inhibited | | Activated | |
| Development of colorectal tumor | | | Inhibited | | | | |
| Weight gain | 1.15E-72 | | Inhibited | Activated | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Quantity of steroid hormone | | | Inhibited | | | | |
| Lung carcinoma | 3.9E-61 | | Inhibited | | | | |
| B-cell lymphoprolifferativ e disorder | | | Inhibited | | | | |
| B-cell neoplasm | 1.39E-70 | | Inhibited | | | | |
| B cell cancer | | | Inhibited | | | | |
| Lung tumor | 1.04E-78 | 8.1E-103 | Inhibited | | | | |
| Gastrointestinal carcinoma | | | Inhibited | | | | |
| Epileptic seizure | | | Inhibited | | | | |
| Endocrine gland tumor | | | Inhibited | | Inhibited | | |
| Oscillation of Ca$^{2+}$ | | | Inhibited | | | | |
| Tauopathy | 0 | 5.43E-89 | * | | | | |
| Extracranial solid tumor | | | Activated | | Activated | Activated | Activated |
| Development of sensory organ | | | Activated | | Activated | | |
| Malignant neoplasm of large intestine | | | Activated | | | | |
| Pancreatobiliary tumor | | | Activated | | | | |
| Secretion of neurotransmitter | | | Activated | | | | |
| Sarcoma | | 1.96E-92 | Activated | | | | |
| Connective tissue tumor | | 4.4E-105 | Activated | | | | |
| Epilepsy | 1.79E-93 | | Activated | | | | |
| Liver carcinoma | | | Activated | | | | |
| Cell death of brain | 6.8E-111 | | Activated | | | | |
| Thermoregulation | | | Activated | | | | |
| Pancreatic tumor | | | Activated | | | | |
| Skin tumor | | | Activated | | Inhibited | | |
| Thoracic neoplasm | 2.34E-79 | 2.5E-108 | Activated | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Development of respiratory system tumor | | | Activated | | | | |
| Necrosis of epithelial tissue | 4.75E-82 | 6.8E-155 | Activated | | Activated | | |
| Cell death of central nervous system cells | 3E-107 | | Activated | | | | |
| B-cell lymphoma | | | Activated | | | | |
| Cell death of tumor cell lines | 3.79E-88 | 5.8E-159 | Activated | | Inhibited | Activated | |
| Digestive organ tumor | | | Activated | Inhibited | Inhibited | | Inhibited |
| Connective or soft tissue tumor | | 1.2E-119 | Activated | | | | |
| Formation of eye | | | Activated | | Activated | | |
| Neuronal cell death | 9.9E-137 | 4.87E-88 | Activated | | | | |
| Stomach tumor | | | Activated | | | | |
| Growth of axons | | | Activated | | | | |
| Disorder of pregnancy | | | Activated | | | | |
| Breast or colorectal cancer | 6.1E-55 | | Activated | | Inhibited | | |
| Sensory system development | | | Activated | | Inhibited | | |
| Development of lung tumor | | | Activated | | | | |
| Cell death of brain cells | 7.5E-108 | | Activated | | | | |
| Neurodegeneration of cerebral cortex | | | Activated | | | | |
| Anxiety | | | Activated | | | | |
| Breast carcinoma | | | Activated | | | | |
| Obesity | 5.6E-152 | | Activated | | Activated | | Activated |
| Development of intestinal tumor | | | Activated | | | | |
| Development of malignant tumor | | | Activated | Inhibited | | Inhibited | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Lung adenocarci-noma | | | Activated | | | | |
| Skin cancer | | | Activated | | | | |
| Non-small cell lung carcinoma | 1.09E-56 | | Activated | | | | |
| Movement Disorders | 2E-227 | | Activated | | | | |
| Diffuse lymphoma | | | Activated | | | | |
| Gastric lesion | | | Activated | | | | |
| Occlusion of artery | 3E-152 | 3.2E-178 | Activated | | | | |
| Non-Hodgkin lymphoma | | | Activated | | | | |
| Locomotion | 1.34E-66 | | Activated | | | | |
| Breast or ovarian carcinoma | | | Activated | | | | |
| Breast cancer | 2.25E-70 | 2.2E-134 | Activated | | | | |
| Glucose metabolism disorder | 1.4E-184 | 1.4E-170 | Activated | | Activated | | |
| Incidence of tumor | | | Activated | Inhibited | | Inhibited | |
| Atherosclerosis | 9.5E-131 | 2.8E-174 | Activated | | | | |
| Amyloidosis | 0 | 1.46E-91 | Activated | | | | |
| Liver lesion | | 1.4E-110 | Activated | | | | |
| Mood Disorders | 2.4E-173 | | Activated | | | | |
| Depressive disorder | 9.7E-162 | | Activated | | | | |
| Lymphohematopo ietic cancer | 1.28E-94 | 6.2E-121 | Activated | | | | |
| Paired-pulse facilitation | | | Activated | | | | |
| Lymphoreticular neoplasm | 6.38E-75 | | Activated | | Inhibited | | |
| Colon tumor | | | Activated | | | | |
| Apoptosis of tumor cell lines | 4.41E-93 | 5.3E-155 | Activated | | Inhibited | Activated | |
| Cell death of epithelial cells | 4.48E-69 | 3E-123 | Activated | | Activated | | |
| Vaso-occlusion | 6.2E-151 | 2.9E-179 | Activated | Activated | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Subcutaneous tumor | | | Activated | | | | |
| Colorectal tumor | | | Activated | | | | |
| Occlusion of blood vessel | 1.7E-152 | 3.4E-180 | Activated | | | | |
| Lymphatic system tumor | 4.79E-88 | | Activated | | Inhibited | | |
| Breast or ovarian cancer | 7.8E-65 | 4.6E-113 | Activated | | Inhibited | | |
| Hypertrophy | 1.65E-56 | 2.6E-219 | Activated | | | | |
| Hematologic cancer | 1.05E-92 | 2.2E-115 | Activated | Inhibited | Inhibited | | Inhibited |
| Large intestine neoplasm | | | Activated | Inhibited | | | |
| Lymphoid cancer | 1.85E-77 | 1.8E-114 | Activated | | Inhibited | | |
| Hypertension | 4.14E-89 | | Activated | | | | |
| Gastrointestinal adenocarcinoma | | | Activated | | | | |
| Frequency of tumor | | | Activated | Inhibited | | Inhibited | |
| Lymphohematopoietic neoplasia | 1E-96 | 6.2E-133 | Activated | | | | |
| Skin lesion | | | Activated | | Inhibited | | Activated |
| Neck neoplasm | | | Activated | | | | |
| Mammary tumor | 3.35E-72 | 5.2E-153 | Activated | | | | |
| Motor dysfunction or movement disorder | 7.7E-228 | | Activated | | | | |
| Gastrointestinal tumor | | | Activated | Inhibited | Inhibited | | Inhibited |
| Hematologic cancer of cells | 2.64E-71 | 4E-144 | Activated | | Inhibited | | |
| Disorder of blood pressure | 3.79E-97 | | Activated | | | | |
| Hematopoietic neoplasm | 2.37E-95 | | Activated | Inhibited | Inhibited | | Inhibited |
| Seizure disorder | 3E-118 | | Activated | | | | Activated |
| Seizures | 1.01E-97 | | Activated | | | | |
| Necrosis | 3.1E-153 | 1.4E-251 | Activated | | Activated | Activated | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Peripheral vascular disease | 5.7E-170 | | Activated | | Activated | | |
| Lymphoproliferative disorder | 2.49E-83 | 2E-104 | Activated | | Inhibited | | |
| Neoplasia of leukocytes | 5.5E-88 | 1.3E-149 | Activated | | Inhibited | | |
| Intestinal tumor | | | Activated | | Inhibited | | |
| Lymphocytic cancer | 3.97E-73 | | Activated | | Inhibited | | |
| Lymphocytic neoplasm | 2.2E-82 | 4.3E-139 | Activated | | Inhibited | | |
| Cell death of muscle cells | 1.7E-54 | 9.9E-127 | Activated | | | | |
| Renal impairment | 4.4E-100 | 3.2E-101 | Activated | | Activated | | |
| Failure of kidney | 4.17E-85 | 4.4E-107 | Activated | | Activated | | |
| Cerebrovascular dysfunction | 1.3E-186 | | Activated | | | | |
| Lymphoma | 4.3E-54 | 1E-143 | Activated | | Inhibited | | |
| Development of digestive organ tumor | | | Activated | | | | |
| Cell death of muscle | | 1.7E-134 | Activated | | | | |
| Necrosis of muscle | 3.34E-54 | 1.4E-133 | Activated | | | | |
| Neurodegeneration | 3.46E-85 | | Activated | | | | |
| Abnormality of heart ventricle | 1.36E-63 | 7.5E-128 | Activated | | | | |
| Development of benign tumor | 2.1E-59 | | Activated | | | | |
| Benign Tumors | 3.71E-75 | | Activated | | | | |
| Benign lesion | 9.74E-87 | | Activated | | | | |
| Cell death | 6.5E-155 | 3.7E-254 | Activated | | Activated | Inhibited | |
| Apoptosis | 7.5E-135 | 1.1E-244 | Activated | | Inhibited | Inhibited | |
| Hyperactive behavior | | | Activated | | | | |
| Bleeding | 7.55E-94 | 2.5E-102 | Activated | | Inhibited | | |
| Neonatal death | | | Activated | | | | |

(continued)

| Diseases or functions annotation | Diseases and functions affected in dementia and neurodegeneration (p value) | Diseases and functions affected in fibrosis and scarring (p value) | TBI | TBI+1 mg/kg LMW-DS | TBI+5 mg/kg LMW-DS | TBI+15 mg/kg LMW-DS | TBI+15 mg/kg repeat dose LMW-DS |
|---|---|---|---|---|---|---|---|
| Perinatal death | | | Activated | | | | |
| Morbidity or mortality | 4.8E-108 | 2.3E-216 | Activated | | Inhibited | | |
| Organismal death | 2E-109 | 3.5E-213 | Activated | | Inhibited | | |
| * ambiguous effect | | | | | | | |

DISCUSSION

LMW-DS was able to counteract and reverse the effects of TBI in most pathways and molecular process. The data indicated that LMW-DS was able to normalize tissue gene expression and function after TBI. The functions and pathways studied were highly relevant to neurodegenerative disease. From the results it was apparent that LMW-DS was able to affect these pathways in a beneficial way even when the disruption was severe.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

**Claims**

1. A method of determining an efficiency of dextran sulfate treatment of a patient suffering from a neurological disease, disorder or condition, said method comprising:

determining (S1) an amount of at least one biomarker selected from each group of group nos. 1 to 6 in a first biological sample taken from said patient prior to administration of dextran sulfate, or a pharmaceutically acceptable salt thereof, to said patient;

determining (S2) an amount of said at least one biomarker selected from each group of said group nos. 1 to 6 in a second biological sample taken from said patient following administration of said dextran sulfate, or said pharmaceutically acceptable salt thereof, to said patient;

determining (S3), for each biomarker, a difference between said amount of said biomarker in said second biological sample and said amount of said biomarker in said first biological sample; and

determining (S4) said dextran sulfate treatment to be efficient if said amounts of said biomarkers selected from group nos. 1, 3 and 5 are reduced in said second biological sample relative to said first biological sample and if said amounts of said biomarkers selected from group nos. 2, 4 and 6 are increased in said second biological sample relative to said first biological sample, wherein

group no. 1 consists of platelet factor 4 (PFA4) and vav guanine nucleotide exchange factor 3 (VAV3);

group no. 2 consists of tumor necrosis factor (TNF) superfamily member 15 (TNFSF15), interleukin 17B (IL-17B), thymic stromal lymphopoietin (TSLP) and corticotropin releasing hormone (CRH);

group no. 3 consists of fibroblast growth factor 1 (FGF1) and KIT-ligand (KITLG);

group no. 4 consists of brain derived neutrophic factor (BDNF), noggin (NOG) and heparin binding epidermal growth factor (EGF) like growth factor (HBEGF);

group no. 5 consists of alpha fetoprotein (AFP), sarcoplasmic/endoplasmic reticulum calcium ATPase 3 (ATP2A3), solute carrier family 29 member 1 (SLC29A1), solute carrier family 40 member 1 (SLC40A1) and transthyretin (TTR); and

group no. 6 consists of solute carrier family 1 member 4 (SLC1A4), solute carrier family 7 member 11 (SLC7A11), solute carrier family 16 member 7 (SLC16A7), low density lipoprotein receptor (LDLR) and ATPase phospholipid transporting 8A1 (ATP8A1).

2. The method according to claim 1, wherein said first biological sample and said second biological sample are a first body fluid sample and a second body fluid sample, and said body fluid is preferably selected from the group consisting of blood, blood serum and blood plasma, preferably said body fluid is blood.

3. The method according to claim 1 or 2, wherein determining (S2) said amount in said second biological sample comprises determining (S2) said amount of said at least one biomarker selected from each group of said group nos. 1 to 6 in said second biological sample taken from said patient within a time period of from one day up to fourteen days following administration of said dextran sulfate, or said pharmaceutically acceptable salt thereof, to said patient, preferably within a time period of from four days up to ten days following administration of said dextran sulfate, or said pharmaceutically acceptable salt thereof, to said patient, and more preferably seven days following administration of said dextran sulfate, or said pharmaceutically acceptable salt thereof, to said patient.

4. The method according to any of the claims 1 to 3, wherein

determining (S1) said amount in said first biological sample comprises determining (S1) said amount of multiple biomarkers selected from each group of said group nos. 1 to 6 in said first biological sample taken from said patient prior to administration of dextran sulfate, or said pharmaceutically acceptable salt thereof, to said patient; and determining (S2) said amount in said second biological sample comprises determining (S2) said amount of said multiple biomarkers selected from each group of said group nos. 1 to 6 in said second biological sample taken from said patient following administration of said dextran sulfate, or said pharmaceutically acceptable salt thereof, to said patient.

5. The method according to claim 4, wherein

determining (S1) said amount in said first biological sample comprises determining (S1) said amount of all biomarkers from each group of said group nos. 1 to 6 in said first biological sample taken from said patient prior to administration of dextran sulfate, or said pharmaceutically acceptable salt thereof, and determining (S2) said amount in said second biological sample comprises determining (S2) said amount of said all biomarkers from each group of said group nos. 1 to 6 in said second biological sample taken from said patient following administration of said dextran sulfate, or said pharmaceutically acceptable salt thereof, to said patient.

6. The method according to any of the claims 1 to 5, wherein determining (S3) said difference comprises determining (S3), for each biomarker $i$, a change $c_i$ in said amount of said biomarker between said first biological sample and said second biological sample relative to said amount of said biomarker in said first biological sample, wherein

$$c_i = 100 \times \frac{A2_i - A1_i}{A1_i}$$ and A1$_i$ represents said amount of said biomarker $i$ in said first biological sample and A2$_i$ represents said amount of said biomarker $i$ in said second biological sample.

7. The method according to claim 6, wherein determining (S4) said efficiency comprises:

determining (S4) said dextran sulfate treatment to be efficient if said change $c_i$ is equal to or larger than X for said biomarkers selected from group nos. 1, 3 and 5 and said change $c_i$ is equal to or smaller than -X for said biomarkers selected from group nos. 2, 4 and 6, wherein X is a threshold value and is preferably 20; and/or determining (S4) said dextran sulfate treatment to be inefficient if said change $c_i$ is below X for at least one of said biomarkers selected from group nos. 1, 3 and 5 and/or said change $c_i$ is above -X for at least one of said biomarkers selected from group nos. 2, 4 and 6.

8. The method according to any of the claims 1 to 7, further comprising:

determining an amount of at least one of interleukin 36 receptor antagonist (IL36RN), golgi soluble N-ethylmaleimide-sensitive factor (NSF) attachment protein (SNAP) receptor complex 1 (GOSR1) and solute carrier family 4 member 1 (SLC4A1) in said first biological sample taken from said patient prior to administration of said dextran sulfate, or said pharmaceutically acceptable salt thereof, to said patient; determining an amount of said at least one of IL36RN, GOSR1 and SLC4A1 in said second biological sample taken from said patient following administration of said dextran sulfate, or said pharmaceutically acceptable salt thereof, to said patient; and

determining a difference between said amount of said at least one of IL36RN, GOSR1 and SLC4A1 in said second biological sample and said amount of said at least one of IL36RN, GOSR1 and SLC4A1 in said first biological sample, wherein

determining (S4) said efficiency comprises determining (S4) said efficiency of said dextran sulfate treatment based on said differences and said difference between said amount of said at least one of IL36RN, GOSR1 and SLC4A1.

9. The method according to any of the claims 1 to 8, further comprising adjusting said dextran sulfate treatment based on said determined efficiency, optionally by:

selecting, based on said determined efficiency, a dose of said dextran sulfate, or said pharmaceutically acceptable salt thereof, to be administered to said patient;

selecting, based on said determined efficiency, a frequency of administration of said dextran sulfate, or said pharmaceutically acceptable salt thereof, to said patient;

selecting, based on said determined efficiency, a duration of administration of said dextran sulfate, or said pharmaceutically acceptable salt thereof, to said patient; and/or

selecting, based on said determined efficiency, a dosage regimen of said dextran sulfate, or said pharmaceutically acceptable salt thereof, for said patient

10. The method according to any of the claims 1 to 9, wherein said neurological disease, disorder or condition is selected from the group consisting of traumatic brain injury (TBI), amyotrophic lateral sclerosis (ALS), Alzheimer's disease (AD), sub-arachnoid hemorrhage (SAH), Parkinson's disease (PD), Huntington's disease (HD), multiple sclerosis (MS), acute disseminated encephalomyelitis (ADEM), central nervous system (CNS) neuropathies, central pontine myelinolysis (CPM), myelopathies, leukoencephalopathies, leukodystrophies, Guillain-Barre syndrome (GBS), peripheral neuropathies, Charcot-Marie-Tooth (CMT) disease, hereditary spastic paraplegia (HSP), primary lateral sclerosis (PLS), progressive muscular atrophy (PMA), progressive bulbar palsy (PBP) pseudobulbar palsy, spinal muscular atrophy (SMA) and post-polio syndrome (PPS), preferably selected from the group consisting of TBI, ALS, AD and SAH and more preferably being TBI.

11. The method according to any of the claims 1 to 10, wherein said dextran sulfate, or said pharmaceutically acceptable salt thereof, has a number average molecular weight ($M_n$) as measured by nuclear magnetic resonance (NMR) spectroscopy within an interval of 1850 and 3500 Da, preferably within an interval of 1850 and 2500 Da, and more preferably within an interval of 1850 and 2300 Da, such as within an interval of 1850 and 2000 Da.

12. The method according to claim 11, wherein said dextran sulfate, or said pharmaceutically acceptable salt thereof, has an average sulfate number per glucose unit within an interval of 2.5 and 3.0, preferably within an interval of 2.5 and 2.8, and more preferably within an interval of 2.6 and 2.7.

13. The method according to any of the claims 1 to 12, wherein said dextran sulfate, or said pharmaceutically acceptable salt thereof, has on average 5.1 glucose units and an average sulfate number per glucose unit of 2.6 to 2.7.

14. The method according to any of the claims 1 to 13, wherein said dextran sulfate, or said pharmaceutically acceptable salt thereof, is administered formulated as an aqueous injection solution.

15. The method according to any of the claims 1 to 14, wherein said pharmaceutically acceptable salt thereof is a sodium salt of dextran sulfate.

## Patentansprüche

1. Verfahren zum Bestimmen einer Effizienz einer Dextransulfattherapie eines Patienten, der von einer neurologischen Erkrankung, Störung oder einem neurologischen Zustand betroffen ist, wobei das Verfahren Folgendes umfasst:

Bestimmen (S1) einer Menge mindestens eines Biomarkers, ausgewählt aus jeder Gruppe der Gruppen Nr. 1 bis 6, in einer ersten biologischen Probe, die dem Patienten vor Verabreichung von Dextransulfat oder einem pharmazeutisch annehmbaren Salz davon entnommen wurde;

Bestimmen (S2) einer Menge des mindestens einen Biomarkers, ausgewählt aus jeder Gruppe der Gruppen Nr. 1 bis 6, in einer zweiten biologischen Probe, die dem Patienten nach Verabreichung des Dextransulfats oder des pharmazeutisch annehmbaren Salzes davon entnommen wurde;

Bestimmen (S3) einer Differenz zwischen der Menge des Biomarkers in der zweiten biologischen Probe und der Menge des Biomarkers in der ersten biologischen Probe für jeden Biomarker; und

Bestimmen (S4), dass die Dextransulfattherapie effizient ist, wenn die Mengen der aus den Gruppen Nr. 1, 3 und 5 ausgewählten Biomarker in der zweiten biologischen Probe relativ zu der ersten biologischen Probe verringert sind und wenn die Mengen der aus den Gruppen Nr. 2, 4 und 6 ausgewählten Biomarker in der zweiten biologischen Probe relativ zu der ersten biologischen Probe erhöht sind, wobei

Gruppe Nr. 1 aus dem Thrombozytenfaktor 4 (PFA4) und dem VAV-Guanin-Nukleotid-Austauschfaktor 3 (VAV3) besteht;

Gruppe Nr. 2 aus dem Element 15 der Tumor-Nekrose-Faktor(TNF)-Superfamilie (TNFSF15), Interleukin 17B (IL-17B), thymischem Stroma-Lymphopoietin (TSLP) und Corticotropin-Freisetzungshormon (CRH) besteht;

Gruppe Nr. 3 aus Fibroblasten-Wachstumsfaktor 1 (FGF1) und KIT-Ligand (KITLG) besteht;

Gruppe Nr. 4 aus dem vom Gehirn stammenden neutrophen Faktor (BDNF), Noggin (NOG) und dem heparinbindenden epidermalen Wachstumsfaktor(EGF)-ähnlichen Wachstumsfaktor (HBEGF) besteht;

Gruppe Nr. 5 aus Alpha-Fetoprotein (AFP), Calcium-ATPase 3 des sarkoplasmatischen/endoplasmatischen Retikulums (ATP2A3), Element 1 der SLC-Transporterfamilie 29 (SLC29A1), Element 1 der SLC-Transporterfamilie 40 (SLC40A1) und Transthyretin (TTR) besteht; und

Gruppe Nr. 6 aus Element 4 der SLC-Transporterfamilie 1 (SLC1A4), Element 11 der SLC-Transporterfamilie 7 (SLC7A11), Element 7 der SLC-Transporterfamilie 16 (SLC16A7), Rezeptor für Lipoprotein niedriger Dichte (LDLR) und ATPase für den Phospholipid-Transport 8A1 (ATP8A1) besteht.

2. Verfahren nach Anspruch 1, wobei die erste biologische Probe und die zweite biologische Probe eine erste Körperflüssigkeitsprobe und eine zweite Körperflüssigkeitsprobe sind und die Körperflüssigkeit vorzugsweise aus der Gruppe ausgewählt ist, die aus Blut, Blutserum und Blutplasma besteht, wobei die Körperflüssigkeit vorzugsweise Blut ist.

3. Verfahren nach Anspruch 1 oder 2, wobei das Bestimmen (S2) der Menge in der zweiten biologischen Probe das Bestimmen (S2) der Menge des mindestens einen Biomarkers, ausgewählt aus jeder Gruppe der Gruppen Nr. 1 bis 6 in der zweiten biologischen Probe, die dem Patienten innerhalb eines Zeitraums von einem Tag bis zu vierzehn Tagen nach Verabreichung des Dextransulfats oder des pharmazeutisch annehmbaren Salzes davon an den Patienten entnommen wurde, vorzugsweise innerhalb eines Zeitraums von vier Tagen bis zu zehn Tagen nach Verabreichung des Dextransulfats oder des pharmazeutisch annehmbaren Salzes davon an den Patienten, und noch bevorzugter sieben Tage nach Verabreichung des Dextransulfats oder des pharmazeutisch annehmbaren Salzes davon an den Patienten.

4. Verfahren nach einem der Ansprüche 1 bis 3, wobei

Bestimmen (S1) der Menge in der ersten biologischen Probe Bestimmen (S1) der Menge mehrerer Biomarker, ausgewählt aus jeder Gruppe der Gruppen Nr. 1 bis 6, in der ersten biologischen Probe umfasst, die dem Patienten vor Verabreichung von Dextransulfat oder dem pharmazeutisch annehmbaren Salz davon an den Patienten entnommen wurde; und

Bestimmen (S2) der Menge in der zweiten biologischen Probe Bestimmen (S2) der Menge der mehreren Biomarker, ausgewählt aus jeder Gruppe der Gruppen Nr. 1 bis 6, in der zweiten biologischen Probe umfasst, die dem Patienten nach Verabreichung von Dextransulfat oder dem pharmazeutisch annehmbaren Salz davon an den Patienten entnommen wurde.

5. Verfahren nach Anspruch 4, wobei

Bestimmen (S1) der Menge in der ersten biologischen Probe Bestimmen (S1) der Menge aller Biomarker aus jeder Gruppe der Gruppen Nr. 1 bis 6 in der ersten biologischen Probe umfasst, die vor Verabreichung von Dextransulfat oder dem pharmazeutisch annehmbaren Salz davon entnommen wurde; und

Bestimmen (S2) der Menge in der zweiten biologischen Probe Bestimmen (S2) der Menge aller Biomarker aus jeder Gruppe der Gruppen Nr. 1 bis 6 in der zweiten biologischen Probe umfasst, die dem Patienten nach Verabreichung des Dextransulfats oder des pharmazeutisch annehmbaren Salzes davon an den Patienten entnommen wurde.

6. Verfahren nach einem der Ansprüche 1 bis 5, wobei Bestimmen (S3) der Differenz Bestimmen (S3) i einer Änderung $c_i$ der Menge des Biomarkers zwischen der ersten biologischen Probe und der zweiten biologischen Probe relativ zu der Menge des Biomarkers in der ersten biologischen Probe für jeden Biomarker umfasst, wobei

$$c_i = 100 \times \frac{A2_i - A1_i}{A1_i}$$

und $A1_i$ die Menge des Biomarkers $i$ in der ersten biologischen Probe und $A2_i$ die Menge des Biomarkers i in der zweiten biologischen Probe darstellt.

7. Verfahren nach Anspruch 6, wobei Bestimmen (S4) der Effizienz Folgendes umfasst:

Bestimmen (S4), dass die Dextransulfattherapie effizient ist, wenn die Änderung $c_i$ gleich oder größer als X für die aus den Gruppen Nr. 1, 3 und 5 ausgewählten Biomarker ist und die Änderung $c_i$ gleich oder kleiner als -X für die aus den Gruppen Nr. 2, 4 und 6 ausgewählten Biomarker ist, wobei X ein Schwellenwert ist und vorzugsweise 20 beträgt; und/oder
Bestimmen (S4), dass die Dextransulfattherapie nicht effizient ist, wenn die Änderung $c_i$ unter X für mindestens einen der Biomarker, ausgewählt aus der Gruppe Nr. 1, 3 und 5, liegt und/oder die Änderung $c_i$ über -X für mindestens einen der Biomarker, ausgewählt aus der Gruppe Nr. 2, 4 und 6, liegt.

8. Verfahren nach einem der Ansprüche 1 bis 7, ferner umfassend:

Bestimmen einer Menge mindestens eines von Interleukin-36-Rezeptor-Antagonist (IL36RN), Golgi löslicher *N*-Ethylmaleimidsensitiver Faktor(NSF)-Bindungsprotein(SNAP)-Rezeptor-Komplex 1 (GOSR1) und Element 1 der SLC-Transporterfamilie 4 (SLC4A1) in der ersten biologischen Probe, die dem Patienten vor Verabreichung des Dextransulfats oder des pharmazeutisch annehmbaren Salzes davon an den Patienten entnommen wurde;
Bestimmen einer Menge des mindestens einen von IL36RN, GOSR1 und/oder SLC4A1 in der zweiten biologischen Probe, die dem Patienten nach Verabreichung des Dextransulfats oder des pharmazeutisch annehmbaren Salzes davon an den Patienten entnommen wurde; und
Bestimmen einer Differenz zwischen der Menge des mindestens einen von IL36RN, GOSR1 und SLC4A1 in der zweiten biologischen Probe und der Menge des mindestens einen von IL36RN, GOSR1 und SLC4A1 in der ersten biologischen Probe, wobei
Bestimmen (S4) der Effizienz Bestimmen (S4) der Effizienz der Dextransulfattherapie basierend auf den Differenzen und der Differenz zwischen der Menge des mindestens einen von IL36RN, GOSR1 und SLC4A1 umfasst.

9. Verfahren nach einem der Ansprüche 1 bis 8, ferner umfassend Anpassen der Dextransulfattherapie basierend auf der bestimmten Effizienz, optional durch:

Auswählen einer Dosis des Dextransulfats oder des pharmazeutisch annehmbaren Salzes davon, die dem Patienten verabreicht werden soll, basierend auf der bestimmten Effizienz;
Auswählen einer Häufigkeit der Verabreichung des Dextransulfats oder des pharmazeutisch annehmbaren Salzes davon an den Patienten basierend auf der bestimmten Effizienz;
Auswählen einer Dauer der Verabreichung des Dextransulfats oder des pharmazeutisch annehmbaren Salzes davon an den Patienten basierend auf der bestimmten Effizienz; und/oder
Auswählen eines Dosierungsschemas des Dextransulfats oder des pharmazeutisch annehmbaren Salzes davon für den Patienten basierend auf der bestimmten Effizienz.

10. Verfahren nach einem der Ansprüche 1 bis 9, wobei die neurologische Erkrankung, Störung oder der neurologische Zustand ausgewählt ist aus der Gruppe bestehend aus traumatischer Hirnverletzung (TBI), amyotropher Lateralsklerose (ALS), Alzheimer-Krankheit (AD), Subarachnoidalblutung (SAH), Parkinson-Krankheit (PD), Huntington-Krankheit (HD), Multiple Sklerose (MS), akuter disseminierter Enzephalomyelitis (ADEM), Neuropathien des zentralen Nervensystems (ZNS), zentraler pontiner Myelinolyse (CPM), Myelopathien, Leukoencephalopathien, Leukodystrophien, Guillain-Barré-Syndrom (GBS), peripheren Neuropathien, Charcot-Marie-Tooth-Krankheit (CMT), hereditärer spastischer Paraplegie (HSP), primärer Lateralsklerose (PLS), progressiver Muskelatrophie (PMA), progressiver bulbärer Lähmung (PBP), pseudobulbärer Lähmung, spinaler Muskelatrophie (SMA) und Post-Polio-Syndrom (PPS), vorzugsweise ausgewählt aus der Gruppe bestehend aus TBI, ALS, AD und SAH und bevorzugt TBI ist.

11. Verfahren nach einem der Ansprüche 1 bis 10, wobei das Dextransulfat oder das pharmazeutisch annehmbare Salz davon ein zahlenmittleres Molekulargewicht (M$_n$), gemessen durch Kernspinresonanzspektroskopie (NMR), innerhalb eines Intervalls von 1850 und 3500 Da, vorzugsweise innerhalb eines Intervalls von 1850 und 2500 Da, und noch bevorzugter innerhalb eines Intervalls von 1850 und 2300 Da, wie beispielsweise innerhalb eines Intervalls von 1850 und 2000 Da, aufweist.

12. Verfahren nach Anspruch 11, wobei das Dextransulfat oder das pharmazeutisch annehmbare Salz davon eine durchschnittliche Sulfatzahl pro Glukoseeinheit innerhalb eines Intervalls von 2,5 und 3,0, vorzugsweise innerhalb eines Intervalls von 2,5 und 2,8 und noch bevorzugter innerhalb eines Intervalls von 2,6 und 2,7 aufweist.

13. Verfahren nach einem der Ansprüche 1 bis 12, wobei das Dextransulfat oder das pharmazeutisch annehmbare Salz davon im Durchschnitt 5,1 Glukoseeinheiten und eine durchschnittliche Sulfatzahl pro Glukoseeinheit von 2,6 bis 2,7 aufweist.

14. Verfahren nach einem der Ansprüche 1 bis 13, wobei das Dextransulfat oder das pharmazeutisch annehmbare Salz davon in einer Formulierung als wässrige Injektionslösung verabreicht wird.

15. Verfahren nach einem der Ansprüche 1 bis 14, wobei das pharmazeutisch annehmbare Salz davon ein Natriumsalz von Dextransulfat ist.

## Revendications

1. Procédé de détermination de l'efficacité d'un traitement au sulfate de dextrane chez un patient souffrant d'une maladie, d'un trouble ou d'une affection neurologique, ledit procédé comprenant :

la détermination (S1) d'une quantité d'au moins un biomarqueur choisi dans chaque groupe des groupes n° 1 à 6 dans un premier échantillon biologique prélevé sur ledit patient avant l'administration de sulfate de dextrane, ou d'un sel pharmaceutiquement acceptable de celui-ci, audit patient ;
la détermination (S2) d'une quantité dudit au moins un biomarqueur choisi dans chaque groupe desdits groupes n° 1 à 6 dans un second échantillon biologique prélevé sur ledit patient après l'administration dudit sulfate de dextrane, ou dudit sel pharmaceutiquement acceptable de celui-ci, audit patient ;
la détermination (S3), pour chaque biomarqueur, d'une différence entre ladite quantité dudit biomarqueur dans ledit second échantillon biologique et ladite quantité dudit biomarqueur dans ledit premier échantillon biologique ; et
la détermination (S4) que ledit traitement au sulfate de dextrane est efficace si lesdites quantités desdits biomarqueurs choisis dans les groupes n° 1, 3 et 5 sont réduites dans ledit second échantillon biologique par rapport audit premier échantillon biologique et si lesdites quantités desdits biomarqueurs choisis dans les groupes n° 2, 4 et 6 sont augmentées dans ledit second échantillon biologique par rapport audit premier échantillon biologique, dans lequel
le groupe n° 1 est constitué du facteur plaquettaire 4 (PFA4) et du facteur d'échange de vav guanine nucléotide 3 (VAV3) ;
le groupe n° 2 est constitué du membre 15 de la superfamille des facteurs de nécrose tumorale (TNF) (TNFSF15), de l'interleukine 17B (IL-17B), de la lymphopoïétine stromale thymique (TSLP) et de l'hormone de libération de la corticotropine (CRH) ;
le groupe n° 3 est constitué du facteur de croissance des fibroblastes 1 (FGF1) et du ligand KIT (KITLG) ;
le groupe n° 4 est constitué du facteur neutrophique dérivé du cerveau (BDNF), du noggin (NOG) et du facteur de croissance de type facteur de croissance épidermique (EGF) liant l'héparine (HBEGF) ;
le groupe n° 5 est constitué de l'alpha-foetoprotéine (AFP), de l'ATPase calcique 3 du réticulum sarcoplasmique/endoplasmique (ATP2A3), du membre 1 de la famille des transporteurs de solutés 29 (SLC29A1), du membre 1 de la famille des transporteurs de solutés 40 (SLC40A1) et de la transthyrétine (TTR) ; et
le groupe n° 6 est constitué du membre 4 de la famille des transporteurs de solutés 1 (SLC1A4), du membre 11 de la famille des transporteurs de solutés 7 (SLC7A11), du membre 7 de la famille des transporteurs de solutés 16 (SLC16A7), du récepteur des lipoprotéines de basse densité (LDLR) et du transporteur de phospholipides ATPase 8A1 (ATP8A1).

2. Procédé selon la revendication 1, dans lequel ledit premier échantillon biologique et ledit second échantillon biologique sont un premier échantillon de fluide corporel et un second échantillon de fluide corporel, et ledit fluide corporel est de préférence choisi dans le groupe constitué par le sang, le sérum sanguin et le plasma sanguin, de préférence ledit fluide corporel est du sang.

**3.** Procédé selon la revendication 1 ou 2, dans lequel la détermination (S2) de ladite quantité dans ledit second échantillon biologique comprend la détermination (S2) de ladite quantité dudit au moins un biomarqueur choisi dans chaque groupe desdits groupes n° 1 à 6 dans ledit second échantillon biologique prélevé sur ledit patient dans un délai d'un jour jusqu'à quatorze jours après l'administration dudit sulfate de dextrane, ou dudit sel pharmaceutiquement acceptable de celui-ci, audit patient, de préférence dans un délai de quatre jours jusqu'à dix jours après l'administration dudit sulfate de dextrane, ou dudit sel pharmaceutiquement acceptable de celui-ci, audit patient, et plus préférablement sept jours après l'administration dudit sulfate de dextrane, ou dudit sel pharmaceutiquement acceptable de celui-ci, audit patient.

**4.** Procédé selon l'une quelconque des revendications 1 à 3, dans lequel

la détermination (S1) de ladite quantité dans ledit premier échantillon biologique comprend la détermination (S1) de ladite quantité de multiples biomarqueurs choisis dans chaque groupe desdits groupes n° 1 à 6 dans ledit premier échantillon biologique prélevé sur ledit patient avant l'administration de sulfate de dextrane, ou dudit sel pharmaceutiquement acceptable de celui-ci, audit patient ; et
la détermination (S2) de ladite quantité dans ledit second échantillon biologique comprend la détermination (S2) de ladite quantité desdits multiples biomarqueurs choisis dans chaque groupe desdits groupes n° 1 à 6 dans ledit second échantillon biologique prélevé sur ledit patient après l'administration de sulfate de dextrane, ou dudit sel pharmaceutiquement acceptable de celui-ci, audit patient.

**5.** Procédé selon la revendication 4, dans lequel

la détermination (S1) de ladite quantité dans ledit premier échantillon biologique comprend la détermination (S1) de ladite quantité de tous les biomarqueurs de chaque groupe desdits groupes n° 1 à 6 dans ledit premier échantillon biologique prélevé sur ledit patient avant l'administration de sulfate de dextrane, ou dudit sel pharmaceutiquement acceptable de celui-ci ; et
la détermination (S2) de ladite quantité dans ledit second échantillon biologique comprend la détermination (S2) de ladite quantité de tous lesdits biomarqueurs de chaque groupe desdits groupes n° 1 à 6 dans ledit second échantillon biologique prélevé sur ledit patient après l'administration de sulfate de dextrane, ou dudit sel pharmaceutiquement acceptable de celui-ci, audit patient.

**6.** Procédé selon l'une quelconque des revendications 1 à 5, dans lequel la détermination (S3) de ladite différence comprend la détermination (S3), pour chaque biomarqueur i, d'un changement dans ladite quantité dudit biomarqueur entre ledit premier échantillon biologique et ledit second échantillon biologique par rapport à ladite quantité dudit biomarqueur dans ledit premier échantillon biologique, dans lequel $c_i = 100 \times \frac{A2_i - A1_i}{A1_i}$ et $A1_i$ représente ladite quantité dudit biomarqueur i dans ledit premier échantillon biologique et $A2_i$ représente ladite quantité dudit biomarqueur i dans ledit second échantillon biologique.

**7.** Procédé selon la revendication 6, dans lequel la détermination (S4) de ladite efficacité comprend :

la détermination (S4) que ledit traitement au sulfate de dextrane est efficace si ledit changement est égal ou supérieur à X pour lesdits biomarqueurs choisis dans les groupes n° 1, 3 et 5 et ledit changement est égal ou inférieur à -X pour lesdits biomarqueurs choisis dans les groupes n° 2, 4 et 6, dans lequel X est une valeur seuil et est de préférence 20 ; et/ou
la détermination (S4) que ledit traitement au sulfate de dextrane est inefficace si ledit changement est inférieur à X pour au moins un desdits biomarqueurs choisis dans les groupes n° 1, 3 et 5 et/ou ledit changement est supérieur à -X pour au moins un desdits biomarqueurs choisis dans les groupes n° 2, 4 et 6.

**8.** Procédé selon l'une quelconque des revendications 1 et 7, comprenant également :

la détermination d'une quantité d'au moins un antagoniste du récepteur de l'interleukine 36 (IL36RN), du complexe récepteur 1 de la protéine d'attachement du facteur sensible *au N*-éthylmaléimide (NSF) soluble (SNAP) de Golgi (GOSR1) et du membre 1 de la famille des transporteurs de solutés 4 (SLC4A1) dans ledit premier échantillon biologique prélevé sur ledit patient avant l'administration dudit sulfate de dextrane, ou dudit sel pharmaceutiquement acceptable de celui-ci, audit patient ;
la détermination d'une quantité dudit au moins un de IL36RN, GOSR1 et SLC4A1 dans ledit second échantillon biologique prélevé sur ledit patient après administration dudit sulfate de dextrane, ou dudit sel pharmaceutiquement acceptable de celui-ci, audit patient ; et
la détermination d'une différence entre ladite quantité dudit au moins un de IL36RN, GOSR1 et SLC4A1 dans ledit second échantillon biologique et ladite quantité dudit au moins un de IL36RN, GOSR1 et SLC4A1 dans ledit premier échantillon biologique, dans lequel
la détermination (S4) de ladite efficacité comprend la détermination (S4) de ladite efficacité dudit traitement au sulfate de dextrane sur la base desdites différences et de ladite différence entre ladite quantité dudit au moins un de IL36RN, GOSR1 et SLC4A1.

9. Procédé selon l'une quelconque des revendications 1 à 8, comprenant également l'ajustement dudit traitement au sulfate de dextrane sur la base de ladite efficacité déterminée, éventuellement par :

sélection, sur la base de ladite efficacité déterminée, d'une dose dudit sulfate de dextrane, ou dudit sel pharmaceutiquement acceptable de celui-ci, à administrer audit patient ;
sélection, sur la base de ladite efficacité déterminée, d'une féquence d'administration dudit sulfate de dextrane, ou dudit sel pharmaceutiquement acceptable de celui-ci, audit patient ;
sélection, sur la base de ladite efficacité déterminée, d'une durée d'administration dudit sulfate de dextrane, ou dudit sel pharmaceutiquement acceptable de celui-ci, audit patient ; et/ou
sélection, sur la base de ladite efficacité déterminée, d'un régime de dose dudit sulfate de dextrane, ou dudit sel pharmaceutiquement acceptable de celui-ci, pour ledit patient.

10. Procédé selon l'une quelconque des revendications 1 à 9, dans lequel ladite maladie, ledit trouble ou ladite affection neurologique est choisi dans le groupe constitué par une lésion cérébrale traumatique (LCT), la sclérose latérale amyotrophique (SLA), la maladie d'Alzheimer (MA), l'hémorragie sous-arachnoïdienne (HSA), la maladie de Parkinson (MP), la maladie de Huntington (MH), la sclérose en plaques (SEP), l'encéphalomyélite aiguë disséminée (EMAD), les neuropathies du système nerveux central (SNC), la myélinolyse centropontine (MPC), les myélopathies, les leucoencéphalopathies, les leucodystrophies, le syndrome de Guillain-Barré (SGB), les neuropathies périphériques, la maladie de Charcot-Marie-Tooth (CMT), la paraplégie spastique héréditaire (PSH), la sclérose latérale primaire (SLP), l'atrophie musculaire progressive (AMP), la paralysie bulbaire progressive (PBP), la paralysie pseudobulbaire, l'amyotrophie spinale (AMS) et le syndrome post-polio (PPS), de préférence choisi dans le groupe constitué de LTC, SLA, MA et HSA et plus préférablement qui est la LTC.

11. Procédé selon l'une quelconque des revendications 1 à 10, dans lequel ledit sulfate de dextrane, ou ledit sel pharmaceutiquement acceptable de celui-ci, a un poids moléculaire moyen en nombre ($M_n$) tel que mesuré par spectroscopie de résonance magnétique nucléaire (RMN) dans un intervalle de 1850 et 3500 Da, de préférence dans un intervalle de 1850 et 2500 Da, et plus préférablement dans un intervalle de 1850 et 2300 Da, tel que dans un intervalle de 1850 et 2000 Da.

12. Procédé selon la revendication 11, dans lequel ledit sulfate de dextrane, ou ledit sel pharmaceutiquement acceptable de celui-ci, a un nombre moyen de sulfate par unité de glucose dans un intervalle de 2,5 et 3,0, de préférence dans un intervalle de 2,5 et 2,8, et plus préférablement dans un intervalle de 2,6 et 2,7.

13. Procédé selon l'une quelconque des revendications 1 à 12, dans lequel ledit sulfate de dextrane, ou ledit sel pharmaceutiquement acceptable de celui-ci, a en moyenne 5,1 unités de glucose et un nombre moyen de sulfate par unité de glucose de 2,6 à 2,7.

14. Procédé selon l'une quelconque des revendications 1 à 13, dans lequel ledit sulfate de dextrane, ou ledit sel pharmaceutiquement acceptable de celui-ci, est administré formulé sous forme d'une solution injectable aqueuse.

15. Procédé selon l'une quelconque des revendications 1 à 14, dans lequel ledit sel pharmaceutiquement acceptable de celui-ci est un sel de sodium de sulfate de dextrane.

Fig. 1

* Significantly different for sTBI @ 5d

Fig. 2A

*SIGNIFICANTLY DIFFERENT FOR sTBI @ 5d

Fig. 2B

*SIGNIFICANTLY DIFFERENT FOR sTBI @ 5d

EP 3 935 388 B1

Fig. 2C

Fig. 2D

Fig. 3A

Fig. 3B

**NADP+** nmol/g ww

*SIGNIFICANTLY DIFFERENT FOR sTBI @ 5d

Fig. 3C

EP 3 935 388 B1

Fig. 3D

*SIGNIFICANTLY DIFFERENT FOR sTBI @ 5d

EP 3 935 388 B1

## Ascorbic acid

*SIGNIFICANTLY DIFFERENT FROM sTBI @ 2d

**SIGNIFICANTLY DIFFERENT FOR sTBI @ 5d

Fig. 4A

Fig. 4B

# MDA

*SIGNIFICANTLY DIFFERENT FROM sTBI @ 2d

**SIGNIFICANTLY DIFFERENT FOR sTBI @ 5d

Fig. 4C

Fig. 5

Fig. 6A

*SIGNIFICANTLY DIFFERENT FROM sTBI @ 2d
**SIGNIFICANTLY DIFFERENT FOR sTBI @ 5d

Acetyl-CoA

*SIGNIFICANTLY DIFFERENT FOR sTBI @ 5d

Fig. 6B

NAA

*SIGNIFICANTLY DIFFERENT FOR sTBI @ 5d

Fig. 6C

Fig. 7

Fig. 8

ASCORBIC
ACID
(μmol/g w.w.)

Fig. 9

GSH
(µmol/g w.w.)

Controls  sTBI 7 days  LMW-DS 1  LMW-DS 5  LMW-DS 15  LMW-DS 15 rip

Fig. 10

Fig. 11

154

START

S1 — DETERMINE AMOUNT OF BIOMARKERS IN 1ST SAMPLE

S2 — DETERMINE AMOUNT OF BIOMARKERS IN 2ND SAMPLE

S3 — DETERMINE DIFFERENCE IN AMOUNTS

S4 — DETERMINE TREATMENT EFFICIENCY

END

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016076780 A

### Non-patent literature cited in the description

- **MARMAROU et al.** *J Neurosurg.*, 1994, vol. 80, 291-300
- **TAVAZZI et al.** *Neurosurgery.*, 2005, vol. 56, 582-589
- **VAGNOZZI et al.** *Neurosurgery.*, 2007, vol. 61, 379-388
- **TAVAZZI et al.** *Neurosurgery.*, 2007, vol. 61, 390-395
- **AMORINI et al.** *J Cell Mol Med.*, 2017, vol. 21, 530-542
- **LAZZARINO et al.** *Anal Biochem.*, 2003, vol. 322, 51-59
- **TAVAZZI et al.** *Clin Biochem.*, 2005, vol. 38, 997-1008
- **AMORINI et al.** *Mol Cell Biochem.*, 2012, vol. 359, 205-216
- **VAGNOZZI et al.** *J Neurotrauma.*, 1999, vol. 16, 903-913
- **SIGNORETTI et al.** *J Neurotrauma.*, 2001, vol. 18, 977-993
- **DI PIETRO et al.** *Mol Cell Biochem.*, 2013, vol. 375, 185-198
- **DI PIETRO et al.** *Mol Med.*, 2014, vol. 20, 147-157
- **DI PIETRO et al.** *Free Radic Biol Med.*, 2014, vol. 69, 258-264
- **AMORINI et al.** *Biochim Biophys Acta Mol Basis of Dis.*, 2016, vol. 1862, 679-687
- **MARMAROU et al.** *J. Neurosurg.*, 1994, vol. 80, 291-300
- **AMORINI et al.** *J Cell Mol Med.*, 2017, vol. 21 (3), 530-542
- **AMORINO et al.** *Mol Cell Biochem.*, 2012, vol. 359, 205-216
- **DI PIETRO et al.** *Sci Rep.*, 2017, vol. 7 (1), 9189